(19) Europäisches Patentamt
European Patent Office
Office européen des brevets

(11) EP 2 111 847 A1

(12) # EUROPÄISCHE PATENTANMELDUNG

(43) Veröffentlichungstag:
**28.10.2009 Patentblatt 2009/44**

(21) Anmeldenummer: **09004222.7**

(22) Anmeldetag: **25.03.2009**

(51) Int Cl.:
***A61K 8/31*** *(2006.01)* ***A61K 8/92*** *(2006.01)*
***A61K 8/02*** *(2006.01)* ***A61Q 1/02*** *(2006.01)*
***A61Q 1/04*** *(2006.01)* ***A61Q 1/06*** *(2006.01)*
***A61Q 1/10*** *(2006.01)* ***A61Q 5/00*** *(2006.01)*
***A61Q 5/06*** *(2006.01)* ***A61Q 5/12*** *(2006.01)*
***A61Q 7/00*** *(2006.01)* ***A61Q 15/00*** *(2006.01)*
***A61Q 17/04*** *(2006.01)* ***A61Q 19/00*** *(2006.01)*
***A61Q 19/08*** *(2006.01)* ***A61Q 19/10*** *(2006.01)*
***C07C 1/22*** *(2006.01)* ***C07C 9/15*** *(2006.01)*

(84) Benannte Vertragsstaaten:
**AT BE BG CH CY CZ DE DK EE ES FI FR GB GR HR HU IE IS IT LI LT LU LV MC MK MT NL NO PL PT RO SE SI SK TR**
Benannte Erstreckungsstaaten:
**AL BA RS**

(30) Priorität: **03.04.2008 DE 102008017031**

(71) Anmelder: **Cognis IP Management GmbH**
**40589 Düsseldorf (DE)**

(72) Erfinder:
- **Jackwerth, Bettina**
  **40764 Langenfeld (DE)**
- **Dierker, Markus**
  **40593 Düsseldorf (DE)**
- **Kawa, Rolf**
  **40789 Monheim (DE)**
- **Maurer, Stefanie**
  **68163 Mannheim (DE)**

(54) **Kohlenwasserstoff Gemische und ihre Verwendung**

(57) Die vorliegende Erfindung betrifft Kohlenwasserstoff Gemische, die $^{14}C$ Isotope enthalten, ihre Verwendung sowie kosmetische und/oder pharmazeutischen Zubereitungen, welche Kohlenwasserstoff Gemische, die $^{14}C$ Isotope enthalten, enthalten.

EP 2 111 847 A1

## Beschreibung

Die vorliegende Erfindung betrifft Kohlenwasserstoff Gemische, die $^{14}C$ Isotope enthalten, ihre Verwendung in kosmetischen und/oder pharmazeutischen Zubereitungen, sowie kosmetische und/oder pharmazeutische Zubereitungen, enthaltend Kohlenwasserstoff Gemische, die $^{14}C$ Isotope enthalten.

### Stand der Technik

Sensorisch leichte Ölkörper, so genannte "light emollients", werden von der kosmetischen Industrie in einer Vielzahl von Formulierungen verwendet. Insbesondere für die dekorative Kosmetik bzw. in pflegenden Formulierungen werden so genannte "leichte" Komponenten eingesetzt. Bei diesen Komponenten kann es sich beispielsweise um flüchtige, cyclische Silikone (z.B. Cyclopentasiloxan oder Cyclomethicone) oder Kohlenwasserstoffe aus petrochemischen Prozessen handeln. Bei den zuletzt genannten Stoffen handelt es sich aufgrund ihrer Herstellung überwiegend um Gemische aus linearen, cyclischen und verzweigten Kohlenwasserstoffen, deren Flammpunkt durchaus unter 50 ˚C (wie z.B. beim Isododecan) liegen kann. Beispiele und anwendungstechnische Beschreibungen derartiger Formulierungen können in Standardwerken, wie zum Beispiel: ‚Handbook of Cosmetic Science and Technology', A. Barel, M. Paye, H. Maibach, Marcel Dekker Inc. 2001 nachgelesen werden. Aus toxikologischen, ökologischen bzw. sicherheitstechnischen Gründen besteht jedoch in Zukunft Bedarf nach alternativen Rohstoffen für derartige Formulierungsaufgaben.

In kosmetischen und pharmazeutischen Zubereitungen werden unter der Bezeichnung "Mineral Öl" die aus mineralischen Rohstoffen (Erdöl, Braun- u. Steinkohlen) gewonnenen flüssigen Destillations-Produkte, die im Wesentlichen aus Gemischen von gesättigten Kohlenwasserstoffen mit linearer, cyclischer und/oder verzweigter Struktur bestehen, eingesetzt. Diese Kohlenwasserstoff Gemische müssen jedoch aufwendig gereinigt und chemisch modifiziert werden, bevor sie den Anforderungen an kosmetische Rohstoffe entsprechen.

Die Aufgabe der Erfindung bestand darin, alternative Rohstoffe zu finden, die ökologisch bzw. toxikologisch unbedenklich sind. Dabei war es insbesondere von Interesse Rohstoffe bereit zu stellen, welche ohne aufwendige Reinigungsschritte direkt in kosmetischen bzw. pharmazeutischen Zubereitungen eingesetzt werden können. Vorzugsweise sollten diese Rohstoffe auf Basis nachwachsender Rohstoffe erhältlich sein. Diese Rohstoffe sollten in typischen kosmetischen und/oder pharmazeutischen Formulierungen ohne anwendungstechnische Einschränkungen direkt eingesetzt werden können. Darüber hinaus sollten die Rohstoffe gegenüber den Kohlenwasserstoff Gemischen des Standes der Technik eine verbesserte Sensorik aufweisen, wünschenswert war auch, dass diese Rohstoffe eine bessere Hautverträglichkeit aufweisen. Von besonderem Interesse war es, Rohstoffe bereit zu stellen, welche hinsichtlich ihrer sensorischen Einsatzmöglichkeiten mit Silikonölen, insbesondere mit niedrigviskosen Silikonölen, wie z.B. Dimethiconen vergleichbar sind. Wünschenswert war es insbesondere Rohstoffe zu Verfügung zu stellen, welche sich als Ersatzstoffe für Silikonöle eignen. Darüber hinaus war es von Interesse Rohstoffe bereit zu stellen, die gegenüber den Rohstoffen des Standes der Technik eine verbesserte $CO_2$-Bilanz aufweisen.

Eine weitere Aufgabe bestand darin Rohstoffe zur Verfügung zu stellen, welche eine stabile Formulierung mit AP/Deo (=Antiperspirant/Desodorant) Wirkstoffen ermöglicht. Kosmetische Zubereitungen der Kategorie Antiperspirantien/Desodorantien, insbesondere in so genannten "Stick-Formulierungen" haben immer noch das Problem der unzureichenden Stabilität der kosmetischen Grundlage, insoweit daß sich geruchliche Veränderungen während der Lagerung ergeben. Eine weitere Aufgabe der Erfindung bestand daher darin, Rohstoffe zu Verfügung zu stellen, welche es ermöglichen antiperspirierende bzw. desodorierende Zubereitungen, insbesondere solche in "Stick-Formulierung" stabil bereit zu stellen. Diese Zubereitungen sollten insbesondere bei längerer Lagerung keine unerwünschten Geruchsentwicklungen zeigen. Eine weitere Aufgabe bestand darin Rohstoffe zu Verfügung zu stellen, welche einen sensorisch "leichten" Eindruck vermitteln, möglichst bei gleichzeigter verbesserter Hautverträglichkeit, insbesondere in Kombination mit UV-Lichtschutzfiltern sowie in Verbindung mit Selbstbräunern. Von besonderem Interesse ist die Bereitstellung von neuen Rohstoffen, die in Formulierungen der dekorativen Kosmetik einen sensorisch vorteilhaften Eindruck ermöglichen. An Formulierungen der dekorativen Kosmetik, wie beispielsweise Lippenstifte, Lidschatten, Mascara, Nagellack etc. werden aufgrund des Applikationsorts (hauptsächlich Gesicht und Hände) erhöhte Anforderungen an die Sensorik, insbesondere an die Flüchtigkeit gestellt, damit diese Produkte nicht den Eindruck von "Schwere" vermitteln. Desweiteren ist bei diesen Produkten eine gute Dispergierbarkeit von Pigmenten wünschenswert.

### Beschreibung der Erfindung

Ein Gegenstand der Erfindung betrifft ein Kohlenwasserstoff Gemisch, das $^{14}C$ Isotope enthält und wobei das Kohlenwasserstoff Gemisch mindestens 2 voneinander verschiedene Kohlenwasserstoffe enthält, deren Kohlenstoff Zahl sich um mehr als 1 unterscheidet.

Der Begriff "2 voneinander verschiedene Kohlenwasserstoffe" bezeichnet Kohlenwasserstoffe mit einer unterschiedlichen C-Zahl.

Der Begriff "Kohlenstoff Zahl" oder "C-Zahl" umfasst alle im Kohlenwasserstoff vorhandenen C-Atome. Er beträgt somit z.B. für Undecan = 11 oder für Tridecan = 13.

Das bedeutet, wenn das Kohlenwasserstoff Gemisch einen Kohlenwasserstoff mit einer C Zahl von n (n = ganzzahlige Zahl) enthält, so enthält das Gemisch mindestens noch einen weiteren Kohlenwasserstoff mit einer C-Zahl von größer gleich n+2 bzw. kleiner gleich n-2.

Vorzugsweise ist n eine ungrade Zahl, insbesondere 7,9,11,13,15,17,19, 21 und/oder 23.

Eine bevorzugte Ausführungsform der Erfindung betrifft ein Kohlenwasserstoff Gemisch, das $^{14}C$ Isotope enthält, wobei das Kohlenwasserstoff Gemisch mindestens 2 voneinander verschiedene Kohlenwasserstoffe enthält, deren Kohlenstoff Zahl sich um 2 unterscheidet. In dieser Ausführungsform der Erfindung enthält das Kohlenwasserstoff Gemisch neben dem Kohlenwasserstoff mit einer C Zahl von n noch mindestens einen Kohlenwasserstoff mit einer C Zahl von n+2.

In einer bevozugten Ausführungsform unterscheiden sich die im Kohlenwasserstoff Gemisch enthaltenden Kohlenwasserstoffe um maximal 10 Kohlenstoffatome, vorzugsweise um maximal 8 Kohlenstoffatome, insbesondere um maximal 6 Kohlenstoffatome, vorzugsweise um maximal 4 Kohlenstoffatome.

Die Erfindung umfasst weiterhin ein Kohlenwasserstoff Gemisch, das $^{14}C$ Isotope enthält, das einen Kohlenwasserstoff mit einer C Zahl von n enthält, sowie mindestens einen weiteren Kohlenwasserstoff mit einer C-Zahl von n+2 und/oder n+4 und/oder n+6 und/oder n+8 und/oder n+10.

Vorzugsweise sind die Kohlenwasserstoffe ausgewählt sind aus der Gruppe der Kohlenwasserstoffen mit 7 bis 23 Kohlenstoffatomen, vorzugsweise mit 11 bis 21 Kohlenstoffatomen.

Als Kohlenwasserstoffe werden organische Verbindungen bezeichnet, die nur aus Kohlenstoff und Wasserstoff bestehen. Sie umfassen sowohl cyclische als auch acyclische (=aliphatische) Verbindungen. Sie umfassen sowohl gesättigte wie einfach oder mehrfach ungesättigte Verbindungen. Die Kohlenwasserstoffe können linear oder verzweigt sein. Je nach Anzahl der Kohlenstoffatome im Kohlenwasserstoff kann man die Kohlenwasserstoffe einteilen in ungradzahlige Kohlenwasserstoffe (wie beispielsweise Nonan, Undecan, Tridecan) oder geradzahlige Kohlenwasserstoffe (wie beispielsweise Octan, Dodecan, Tetradecan). Je nach Verweigung kann man die Kohlenwasserstoffe einteilen in lineare (= unverzweigte) oder verzweigte Kohlenwasserstoffe. Gesättigte, aliphatische Kohlenwasserstoffe werden auch als Paraffine bezeichnet.

Als "Kohlenwasserstoff Gemisch" im Sinne der Erfindung werden Mischungen von Kohlenwasserstoffen verstanden, die bis zu 10 Gew.-% Substanzen enthalten, die nicht zu den Kohlenwasserstoffen zählen. Die Gew.-% Angaben der Kohlenwasserstoffe beziehen sich jeweils auf die Summe der im Gemisch vorhandenen Kohlenwasserstoffe. Die bis zu 10 Gew.-% vorhandenen Nicht-Kohlenwasserstoffe werden für diese Berechnung nicht berücksichtigt.

Bei den Substanzen, die nicht zu den Kohlenwasserstoffen zählen und die bis zu 10 Gew.-%, insbesondere bis zu 8 Gew.-%, vorzugsweise bis zu 5 Gew.-% im erfindungsgemäßen Kohlenwasserstoff Gemisch enthalten sein können, handelt sich beispielsweise um Fettalkohole, die als nicht umgesetzte Edukte im Kohlenwasserstoff Gemisch verbleiben.

Der Begriff "CX-Kohlenwasserstoff" umfasst Kohlenwasserstoffe mit einer C-Zahl von X, so umfasst beispielsweise der Begriff C11-Kohlenwasserstoff alle Kohlenwasserstoffe mit einer C-Zahl von 11.

Die Bezugsgröße "Summe der Kohlenwasserstoffe" umfasst alle im Gemisch enthaltenen Kohlenwasserstoffe, unabhängig von ihrer Kohlenstoffzahl.

Der Begriff Nuklide bezeichnet Atomarten (Atome einschließlich Elektronenhülle), die charakterisiert sind durch die Anzahl der Protonen und Neutronen im Kern, d.h. durch Ordnungszahl und Massenzahl (Zahl der Neutronen = Massenzahl - Ordnungszahl).

Isotope sind Nuklide gleicher Ordnungszahl (= Kernladungszahl, Atomnummer, Protonenzahl), aber unterschiedlicher Anzahl der im Kern enthaltenen Neutronen und damit unterschiedlicher Massenzahl [=Nukleonenzahl, Zahl der in einem Atomkern enthaltenen Nukleonen (Protonen und Neutronen)]. Isotope unterscheiden sich außer durch die Masse auch durch Drehimpuls (Kernspin), magnetisches Moment und elektrisches Quadrupolmoment.

Zur eindeutigen Kennzeichnung der Isotope benutzt man die für Nuklide allgemein gebräuchliche Schreibweise $^{A}{}_{Z}X$ (X = chemisches Symbol, A= Massenzahl, Z = Kernladungszahl), für die stabilen Isotope des Kohlenstoff also $^{12}{}_{6}C$, alternativ wird als Schreibweise $^{12}C$ oder C-12 verwendet.

Das Element Kohlenstoff hat insgesamt 2 stabile Isotope $^{12}C$ und $^{13}C$. $^{12}C$ kommt zu 98,9 % in der Natur vor, $^{13}C$ zu 1,1 %. Neben diesen beiden stabilen Isotopen gibt es noch mehrere instabile Isotope. Das bekannteste instabile Isotop ist dabei $^{14}C$ mit einer Halbwertszeit von 5730 Jahren. $^{14}C$ entsteht durch natürliche Kernreaktion in der Atmosphäre aus $^{14}N$: die Erde ist ständig kosmischer Strahlung ausgesetzt, trifft diese Strahlung auf die obersten Schichten der Erdatmosphäre erzeugt dies freie Neutronen. Diese wiederum reagieren mit dem in der unteren Atmosphäre zu etwa 80% in der Luft enthaltenen Stickstoff. Dabei läuft folgende Reaktion ab:

$$^{14}N + {}^{1}n \rightarrow {}^{14}C + {}^{1}p$$

Der Kern eines Stickstoffatoms mit der Massezahl 14 (7 Neutronen, 7 Protonen) nimmt ein Neutron auf. Unter Abgabe eines Protons entsteht aus dem Stickstoffatom das radioaktive Kohlenstoffisotop $^{14}C$ (8 Neutronen, 6 Protonen), die Massezahl bleibt also gleich. Kohlenstoff $^{12}C$ hat dagegen 6 Neutronen und 6 Protonen - ist also leichter als $^{14}C$.

Der in der Atmosphäre erzeugte $^{14}C$ verbindet sich mit vorhandenem Sauerstoff zu Kohlendioxid. Durch die Photosynthese der Pflanzen gelangt $^{14}C$ so anschließend in die Biosphäre. Da Lebewesen bei ihrem Stoffwechsel ständig Kohlenstoff mit der Atmosphäre austauschen, stellt sich in lebenden Organismen dasselbe Verteilungsverhältnis der 3 Kohlenstoff-Isotope $^{12}C$-12, $^{13}C$-12 und $^{14}C$ ein, wie es in der Atmosphäre vorliegt: Lebende Organismen enthalten pro $10^{12}$ stabilen $^{12}C$-u. $^{13}C$-Isotopen ca. 1,2 radioaktive $^{14}C$-Isotope.

Wird Kohlenstoff aus diesem Kreislauf herausgenommen (d.h. wird er fossil), dann ändert sich das Verhältnis zwischen $^{14}C$ und $^{12}C$, da die zerfallenden $1^{14}C$ Isotope nicht durch neue ersetzt werden.

Fossile Brennstoffe, wie Erdöl, Erdgas oder Kohle, sind vor über 100 Millionen Jahren entstanden, d.h. diese Brennstoffe enthalten keine $^{14}C$ Isotope mehr, da die ursprünglich vorhandenen $^{14}C$ Isotope zerfallen sind und keine neuen $^{14}C$ Isotope aufgenommen wurden. Demnach enthalten Kohlenwasserstoffe, die aus fossilen Quellen stammen, keine $^{14}C$ Isotope.

Gegenstand der Erfindung sind Kohlenwasserstoff Gemische, die $^{14}C$ Isotope enthalten.

Das erfindungsgemäße Kohlenwasserstoff Gemisch enthält mindestens zwei voneinander verschiedene Kohlenwasserstoffe. Die Erfindung betrifft Gemische, in denen mindestens ein Kohlenwasserstoff $^{14}C$ Isotope enthält, vorzugsweise enthalten 2 voneinander verschiedene Kohlenwasserstoffe $^{14}C$ Isotope. In einer bevorzugten Ausführungsform der Erfindung enthalten alle im Kohlenwasserstoff Gemisch vorhandene Kohlenwasserstoffe $^{14}C$ Isotope.

Zur Illustration: enthält das erfindungsgemäße Kohlenwasserstoff Gemisch n-Undecan und n-Tridecan so muss mindestens einer der Kohlenwasserstoff $^{14}C$ Isotope enthalten, d.h. entweder n-Undecan oder n-Tridecan, vorzugsweise enthalten beide Kohlenwasserstoffe $^{14}C$ Isotope.

In einer bevorzugten Ausführungsform der Erfindung liegt der Anteil der $^{14}C$ Isotope zu den $^{12}C$ Isotopen bei größer gleich 1 x $10^{-16}$, insbesondere bei größer gleich 1 x $10^{-15}$, vorzugsweise größer gleich 7,5 x $10^{-14}$, vorzugsweise größer gleich 1,5 x $10^{-13}$, insbesondere größer gleich 3 x $10^{-13}$, vorzugsweise im Bereich von 6 x $10^{-13}$ bis 1,2 x $10^{-12}$. Bezugsgröße sind alle im Kohlenwasserstoff Gemisch vorhandenen Kohlenwasserstoffe.

Der $^{14}C$-Gehalt einer Probe kann entweder durch Zählung der zerfallenden $^{14}C$-Isotope im Zählrohr (Zählrohr Methode nach Libby), im Flüssigkeits-Szintillations-Spektrometer oder durch Zählung der noch vorhandenen $^{14}C$-Isotope mit der Beschleuniger-Massenspektrometrie bestimmt werden. Mit der Beschleuniger-Massenspektrometrie (Abkürzung: AMS von Accelerator *M*ass Spectrometry) können $^{14}C$ Isotope mit Hilfe kernphysikalischer Meßmethoden im ppt- bis ppq-Bereich (von $10^{-12}$ bis $10^{-16}$) in kleinsten Probenmengen (Milligrammbereich) nachgewiesen werden.

Eine bevorzugte Ausführungsform der Erfindung betrifft ein Kohlenwasserstoff Gemisch, das $^{14}C$ Isotope enthält, wobei das Kohlenwasserstoff Gemisch mindestens 2 voneinander verschiedene Kohlenwasserstoffe enthält, deren Kohlenstoff Zahl sich um mehr als 1 unterscheidet und welches kleiner gleich 50 Gew.-%, insbesondere kleiner gleich 20 Gew.-%, insbesondere kleiner gleich 10 Gew.%, vorzugsweise kleiner gleich 8 Gew.-%, vorzugsweise kleiner gleich 5 Gew.-%, vorzugsweise kleiner gleich 3 Gew.-%, vorzugsweise kleiner gleich 2 Gew.-%, insbesondere kleiner gleich 1 Gew.-% verzweigte Kohlenwasserstoffe bezogen auf die Summe der Kohlenwasserstoffe enthält.

Eine bevorzugte Ausführungsform der Erfindung betrifft ein Kohlenwasserstoff Gemisch, das $^{14}C$ Isotope enthält, wobei das Kohlenwasserstoff Gemisch mindestens 2 voneinander verschiedene Kohlenwasserstoffe enthält, deren Kohlenstoff Zahl sich um mehr als 1 unterscheidet und welches kleiner gleich 20 Gew.-%, insbesondere kleiner gleich 10 Gew.-%, vorzugsweise kleiner gleich 8 Gew.-%, vorzugsweise kleiner gleich 5 Gew.-%, vorzugsweise kleiner gleich 3 Gew.-%, vorzugsweise kleiner gleich 2 Gew.-%, insbesondere kleiner gleich 1 Gew.-% aromatische Kohlenwasserstoffe bezogen auf die Summe der Kohlenwasserstoffe enthält. In einer bevorzugten Ausführungsform der Erfindung enthält das Kohlenwasserstoff Gemisch kleiner gleich 0,1, insbesondere kleiner gleich 0,01, insbesondere kleiner gleich 0,001 Gew.-% aromatische Kohlenwasserstoffe bezogen auf die Summe der Kohlenwasserstoffe.

Eine bevorzugte Ausführungsform der Erfindung betrifft ein Kohlenwasserstoff Gemisch, das $^{14}C$ Isotope enthält, wobei das Kohlenwasserstoff Gemisch mindestens 2 voneinander verschiedene Kohlenwasserstoffe enthält, deren Kohlenstoff Zahl sich um mehr als 1 unterscheidet und welches kleiner gleich 50 Gew.-%, insbesondere kleiner gleich 20 Gew.-%, insbesondere kleiner gleich 10 Gew.-%, vorzugsweise kleiner gleich 8 Gew.-%, vorzugsweise kleiner gleich 5 Gew.-%, vorzugsweise kleiner gleich 3 Gew.-%, vorzugsweise kleiner gleich 2 Gew.-%, insbesondere kleiner gleich 1 Gew.-% ungesättigte Kohlenwasserstoffe bezogen auf die Summe der Kohlenwasserstoffe enthält. In einer bevorzugten Ausführungsform der Erfindung enthält das Kohlenwasserstoff Gemisch kleiner gleich 0,1, insbesondere kleiner gleich 0,01, insbesondere kleiner gleich 0,001 Gew.-% ungesättigte Kohlenwasserstoffe bezogen auf die Summe der Kohlenwasserstoffe.

Eine bevorzugte Ausführungsform der Erfindung betrifft ein Kohlenwasserstoff Gemisch, das $^{14}C$ Isotope enthält, wobei das Kohlenwasserstoff Gemisch mindestens 2 voneinander verschiedene Kohlenwasserstoffe enthält, deren Kohlenstoff Zahl sich um mehr als 1 unterscheidet und welches kleiner gleich 20 Gew.-% insbesondere kleiner gleich 15 Gew.-%, insbesondere kleiner gleich 10 Gew.-%, vorzugsweise kleiner gleich 9 Gew.-%, vorzugsweise kleiner gleich 8 Gew.-%, vorzugsweise kleiner gleich 5 Gew.-%, geradzahlige Kohlenwasserstoffe bezogen auf die Summe der Kohlenwasserstoffe enthält.

Eine bevorzugte Ausführungsform der Erfindung betrifft ein Kohlenwasserstoff Gemisch, das $^{14}C$ Isotope enthält, wobei das Kohlenwasserstoff Gemisch mindestens 2 voneinander verschiedene Kohlenwasserstoffe enthält, deren Kohlenstoff Zahl sich um mehr als 1 unterscheidet und worin die 2 voneinander verschiedenen Kohlenwasserstoffe mindestens 50 Gew.-%, vorzugsweise mindestens 60 Gew.-%, vorzugsweise mindestens 70 Gew.-%, vorzugsweise mindestens 80 Gew.-%, vorzugsweise mindestens 90 Gew.-% - bezogen auf die Summe der Kohlenwasserstoffe ausmachen.

Besonders bevorzugt ist es, wenn es sich bei diesen 2 voneinander verschiedenen Kohlenwasserstoffen um Kohlenwasserstoffe handelt, die sich in der C-Zahl um 2 unterscheiden. D.h. in einer bevorzugten Ausführungsform des erfindungsgemäßen Kohlenwasserstoff Gemischs bestehen mindestens 50 Gew.-%, vorzugsweise mindestens 60 Gew.-%, vorzugsweise mindestens 70 Gew.-%, vorzugsweise mindestens 80 Gew.-%, vorzugsweise mindestens 90 Gew.-% - bezogen auf die Summe der Kohlenwasserstoffe, aus einem Kohlenwasserstoff mit der C-Zahl n und einem Kohlenwasserstoff mit der C-Zahl n+2.

<u>Kohlenwasserstoff Gemisch mit linearen C 11 und linearen C13 Kohlenwasserstoffen</u>

Eine bevorzugte Ausführungsform der Erfindung betrifft ein Kohlenwasserstoff Gemisch, das $^{14}C$ Isotope enthält, wobei das Kohlenwasserstoff Gemisch mindestens 2 voneinander verschiedene Kohlenwasserstoffe enthält, deren Kohlenstoff Zahl sich um mehr als 1 unterscheidet und welches lineare C11 und lineare C13 Kohlenwasserstoffe enthält. Vorzugsweise ist der lineare C11 Kohlenwasserstoff n-Undecan. Vorzugsweise ist der lineare C 13 Kohlenwasserstoff n-Tridecan. In einer bevorzugten Ausführungsform der Erfindung enthält das erfindungsgemäße Kohlenwasserstoff Gemisch lineare C11 und lineare C13 Kohlenwasserstoffe sowie mindestens einen weiteren linearen Kohlenwasserstoff ausgewählt aus der Gruppe bestehend aus C12, C15, C16, C17, C18, C19, C20, C21 und C23 Kohlenwasserstoffen, vorzugsweise ausgewählt aus der Gruppe bestehend aus C15, C17, C 19, C21 und C23 Kohlenwasserstoffen. Bevorzugt sind lineare, gesättigte Kohlenwasserstoffe. Als linearer C15 Kohlenstoff ist n-Pentadecan bevorzugt, als linearer C17 Kohlenwasserstoff ist n-Heptadecan bevorzugt, als linearer C19 Kohlenwasserstoff ist n-Nonadecan bevorzugt.

In einer bevorzugten Ausführungsform der Erfindung enthält das erfindungsgemäße Kohlenwasserstoff Gemisch lineare C11 und lineare C13 Kohlenwasserstoffe sowie mindestens einen linearen C15 Kohlenwasserstoff und/ oder einen linearen C17 Kohlenwasserstoff. Als linearer C 15 Kohlenstoff ist n-Pentadecan bevorzugt, als linearer C 17 Kohlenwasserstoff ist n-Heptadecan bevorzugt.

Eine Ausführungsform der Erfindung betrifft ein Kohlenwasserstoff Gemisch, das $^{14}C$ Isotope enthält, wobei das Gemisch enthält

(a) 50 bis 90 Gew.-% lineare C-11 Kohlenwasserstoffe, vorzugsweise n-Undecan
(b) 10 bis 50 Gew.-% lineare C-13 Kohlenwasserstoffe, vorzugsweise n-Tridecan bezogen auf die Summe der Kohlenwasserstoffe.

Besonders bevorzugt sind Kohlenwasserstoff Gemische, die $^{14}C$ Isotope enthalten, welche

(a) 55 bis 80 Gew.-%, insbesondere 60 bis 75 Gew.-%, insbesondere 65 bis 70 Gew.% lineare C-11 Kohlenwasserstoffe, vorzugsweise n-Undecan
(b) 20 bis 45 Gew.-% insbesondere 24 bis 40 Gew.-%, insbesondere 24 bis 30 Gew.% lineare C-13 Kohlenwasserstoffe, vorzugsweise, vorzugsweise n-Tridecan
bezogen auf die Summe der Kohlenwasserstoffe enthalten.

Eine bevorzugte Ausführungsform der Erfindung betrifft ein Kohlenwasserstoff Gemisch, das $^{14}C$ Isotope enthält, dadurch gekennzeichnet, dass die Summe der linearen C11- und linearen C13-Kohlenwasserstoffe größer gleich 70 Gew.-%, insbesondere größer gleich 80 Gew.-%, bevorzugt größer gleich 90 Gew.%, besonders bevorzugt größer gleich 95 Gew.-%, insbesondere größer gleich 99 Gew.-%, bezogen auf die Summe der Kohlenwasserstoffe, beträgt. In einer bevorzugten Ausführungsform der Erfindung beträgt das Gewichtsverhältnis von linearen C11-Kohlenwasserstoffen zu linearen C13-Kohlenwasserstoffen 1,5 bis 3,5.

Besonders bevorzugt sind erfindungsgemäße Kohlenwasserstoff Gemische, bei denen die linearen C11 und/ oder linearen C13 Kohlenwasserstoffe gesättigte Kohlenwasserstoffe sind, vorzugsweise ist sowohl der lineare C11 als

auch der lineare C13 Kohlenwasserstoff ein gesättigter Kohlenwasserstoff (n-Undecan und n-Tridecan).

In einer bevorzugten Ausführungsform enthalten die erfindungsgemäßen Kohlenwasserstoff Gemische kleiner gleich 10 Gew.-%, insbesondere kleiner gleich 5 Gew.-%, bevorzugt kleiner gleich 3 Gew.-%, an C-12 Kohlenwasserstoffen bezogen auf die Summe der Kohlenwasserstoffe.

Eine bevorzugte Ausführungsform der Erfindung betrifft ein Kohlenwasserstoff Gemisch, das $^{14}C$ Isotope enthält, enthaltend lineare C11 und lineare C13 Kohlenwasserstoffe, wobei die Summe der linearen C11- und linearen C13-Kohlenwasserstoffe größer gleich 60 Gew. % bezogen auf die Summe der Kohlenwasserstoffe beträgt und wobei die Summe der Kohlenwasserstoffe mit einer C-Kettenlänge größer gleich 14, kleiner gleich 15 Gew.-%, bezogen auf die Summe der Kohlenwasserstoffe, beträgt. Besonders bevorzugt sind Kohlenwasserstoff Gemische, bei denen die Summe der Kohlenwasserstoffe mit einer C-Kettenlänge kleiner gleich 14, kleiner gleich 10 Gew.-%, insbesondere kleiner gleich 8 Gew.-%, bevorzugt kleiner gleich 4 Gew.-%, insbesondere kleiner gleich 2 Gew.-% beträgt, jeweils bezogen auf die Summe der Kohlenwasserstoffe.

Eine bevorzugte Ausführungsform der Erfindung betrifft ein Kohlenwasserstoff Gemisch, das $^{14}C$ Isotope enthält, enthaltend lineare C11 und lineare C13 Kohlenwasserstoffe, wobei die Summe der linearen C11- und linearen C 13-Kohlenwasserstoffe größer gleich 60 Gew. % bezogen auf die Summe der Kohlenwasserstoffe beträgt und die Summe der Kohlenwasserstoffe mit einer C-Kettenlänge von kleiner gleich 10, kleiner gleich 3 Gew.-%, insbesondere kleiner gleich 2 Gew.-%, bevorzugt kleiner gleich 1,5 Gew.-%, insbesondere kleiner gleich 1 Gew.-%, bezogen auf die Summe der Kohlenwasserstoffe, beträgt.

In einer Ausführungsform der Erfindung enthält das erfindungsgemäße Kohlenwasserstoff Gemisch C12 und C14 Kohlenwasserstoffe, vorzugsweise im selben Gewichtsverhältnis zueinander wie die linearen C11 Kohlenwasserstoffe zu den linearen C13 Kohlenwasserstoffe. In einer bevorzugten Ausführungsform der Erfindung beträgt sowohl das Gewichtsverhältnis von linearen C11-Kohlenwasserstoffen zu linearen C13-Kohlenwasserstoffen als auch das Gewichtsverhältnis von C12 zu C 14 Kohlenwasserstoffen 1,5 bis 3,5.

<u>Kohlenwasserstoff Gemisch mit linearen C 11 und linearen C15 Kohlenwasserstoffen</u>

Eine bevorzugte Ausführungsform der Erfindung betrifft ein Kohlenwasserstoff Gemisch, das $^{14}C$ Isotope enthält, wobei das Kohlenwasserstoff Gemisch mindestens 2 voneinander verschiedene Kohlenwasserstoffe enthält, deren Kohlenstoff Zahl sich um mehr als 1 unterscheidet und welches lineare C11 und lineare C15 Kohlenwasserstoffe enthält. Vorzugsweise ist der lineare C11 Kohlenwasserstoff n-Undecan. Vorzugsweise ist der lineare C15 Kohlenwasserstoff n-Pentadecan. In einer bevorzugten Ausführungsform der Erfindung enthält das erfindungsgemäße Kohlenwasserstoff Gemisch lineare C11 und lineare C15 Kohlenwasserstoffe sowie mindestens einen weiteren linearen Kohlenwasserstoff ausgewählt aus der Gruppe bestehend aus C12, C13, C14, C16, C17, C18, C19, C20, C 21 und C23 Kohlenwasserstoffen, vorzugsweise ausgewählt aus der Gruppe bestehend aus C13, C17, C19, C21 und C23 Kohlenwasserstoffen. Bevorzugt sind lineare, gesättigte Kohlenwasserstoffe. Als linearer C13 Kohlenstoff ist n-Tridecan bevorzugt, als linearer C17 Kohlenwasserstoff ist n-Heptadecan bevorzugt, als linearer C19 Kohlenwasserstoff ist n-Nonadecan bevorzugt.

In einer bevorzugten Ausführungsform der Erfindung enthält das erfindungsgemäße Kohlenwasserstoff Gemisch lineare C11 und lineare C15 Kohlenwasserstoffe sowie mindestens einen linearen C13 Kohlenwasserstoff und/ oder einen linearen C17 Kohlenwasserstoff. Als linearer C13 Kohlenstoff ist n-Tridecan bevorzugt, als linearer C 17 Kohlenwasserstoff ist n-Heptadecan bevorzugt.

Eine Ausführungsform der Erfindung betrifft ein Kohlenwasserstoff Gemisch, das $^{14}C$ Isotope enthält, wobei das Gemisch enthält

(c) 50 bis 90 Gew.-% lineare C-11 Kohlenwasserstoffe, vorzugsweise n-Undecan
(d) 10 bis 50 Gew.-% lineare C-15 Kohlenwasserstoffe, vorzugsweise n-Pentadecan
bezogen auf die Summe der Kohlenwasserstoffe.

Besonders bevorzugt sind Kohlenwasserstoff Gemische, die $^{14}C$ Isotope enthalten, welche

(a) 55 bis 80 Gew.-%, insbesondere 60 bis 75 Gew.-%, insbesondere 65 bis 70 Gew.% lineare C-11 Kohlenwasserstoffe, vorzugsweise n-Undecan
(b) 20 bis 45 Gew.-% insbesondere 24 bis 40 Gew.-%, insbesondere 24 bis 30 Gew.% lineare C-15 Kohlenwasserstoffe, vorzugsweise, vorzugsweise n-Pentadecan
bezogen auf die Summe der Kohlenwasserstoffe enthalten.

Eine bevorzugte Ausführungsform der Erfindung betrifft ein Kohlenwasserstoff Gemisch, das $^{14}C$ Isotope enthält, dadurch gekennzeichnet, dass die Summe der linearen C11- und linearen C15-Kohlenwasserstoffe größer gleich 70 Gew.-%, insbesondere größer gleich 80 Gew.-%, bevorzugt größer gleich 90 Gew.%, besonders bevorzugt größer

gleich 95 Gew.-%, insbesondere größer gleich 99 Gew.-%, bezogen auf die Summe der Kohlenwasserstoffe, beträgt. In einer bevorzugten Ausführungsform der Erfindung beträgt das Gewichtsverhältnis von linearen C11-Kohlenwasserstoffen zu linearen C15-Kohlenwasserstoffen 1,5 bis 3,5.

Besonders bevorzugt sind erfindungsgemäße Kohlenwasserstoff Gemische, bei denen die linearen C11 und/oder linearen C 15 Kohlenwasserstoffe gesättigte Kohlenwasserstoffe sind, vorzugsweise ist sowohl der lineare C11 als auch der lineare C 13 Kohlenwasserstoff ein gesättigter Kohlenwasserstoff (n-Undecan und n-Pentadecan).

In einer bevorzugten Ausführungsform enthalten die erfindungsgemäßen Kohlenwasserstoff Gemische kleiner gleich 10 Gew.-%, insbesondere kleiner gleich 5 Gew.-%, bevorzugt kleiner gleich 3 Gew.-%, an C-12 Kohlenwasserstoffen bezogen auf die Summe der Kohlenwasserstoffe.

Eine bevorzugte Ausführungsform der Erfindung betrifft ein Kohlenwasserstoff Gemisch, das $^{14}C$ Isotope enthält, enthaltend lineare C11 und lineare C15 Kohlenwasserstoffe, wobei die Summe der linearen C11- und linearen C15-Kohlenwasserstoffe größer gleich 60 Gew. % bezogen auf die Summe der Kohlenwasserstoffe beträgt und wobei die Summe der Kohlenwasserstoffe mit einer C-Kettenlänge größer gleich 16, kleiner gleich 15 Gew.-%, bezogen auf die Summe der Kohlenwasserstoffe, beträgt. Besonders bevorzugt sind Kohlenwasserstoff Gemische, bei denen die Summe der Kohlenwasserstoffe mit einer C-Kettenlänge kleiner gleich 14, kleiner gleich 10 Gew.-%, insbesondere kleiner gleich 8 Gew.-%, bevorzugt kleiner gleich 4 Gew.-%, insbesondere kleiner gleich 2 Gew.-% beträgt, jeweils bezogen auf die Summe der Kohlenwasserstoffe.

Eine bevorzugte Ausführungsform der Erfindung betrifft ein Kohlenwasserstoff Gemisch, das $^{14}C$ Isotope enthält, enthaltend lineare C11 und lineare C15 Kohlenwasserstoffe, wobei die Summe der linearen C11- und linearen C15-Kohlenwasserstoffe größer gleich 60 Gew. % bezogen auf die Summe der Kohlenwasserstoffe beträgt und die Summe der Kohlenwasserstoffe mit einer C-Kettenlänge von kleiner gleich 10, kleiner gleich 3 Gew.-%, insbesondere kleiner gleich 2 Gew.-%, bevorzugt kleiner gleich 1,5 Gew.-%, insbesondere kleiner gleich 1 Gew.-%, bezogen auf die Summe der Kohlenwasserstoffe, beträgt.

In einer Ausführungsform der Erfindung enthält das erfindungsgemäße Kohlenwasserstoff Gemisch C12 und C16 Kohlenwasserstoffe, vorzugsweise im selben Gewichtsverhältnis zueinander wie die linearen C11 Kohlenwasserstoffe zu den linearen C15 Kohlenwasserstoffe. In einer bevorzugten Ausführungsform der Erfindung beträgt sowohl das Gewichtsverhältnis von linearen C 11-Kohlenwasserstoffen zu linearen C 15-Kohlenwasserstoffen als auch das Gewichtsverhältnis von C12 zu C16 Kohlenwasserstoffen 1,5 bis 3,5.

<u>Kohlenwasserstoff Gemisch mit linearen C13 und linearen C15 Kohlenwasserstoffen</u>

Eine bevorzugte Ausführungsform der Erfindung betrifft ein Kohlenwasserstoff Gemisch, das $^{14}C$ Isotope enthält, wobei das Kohlenwasserstoff Gemisch mindestens 2 voneinander verschiedene Kohlenwasserstoffe enthält, deren Kohlenstoff Zahl sich um mehr als 1 unterscheidet und welches lineare C13 und lineare C15 Kohlenwasserstoffe enthält. Vorzugsweise ist der lineare C13 Kohlenwasserstoff n-Tridecan. Vorzugsweise ist der lineare C 15 Kohlenwasserstoff n-Pentadecan. In einer bevorzugten Ausführungsform der Erfindung enthält das erfindungsgemäße Kohlenwasserstoff Gemisch lineare C13 und lineare C15 Kohlenwasserstoffe sowie mindestens einen weiteren linearen Kohlenwasserstoff ausgewählt aus der Gruppe bestehend aus C11, C12, C14, C16, C17, C18, C19, C20, C21 und C23 Kohlenwasserstoffen, vorzugsweise ausgewählt aus der Gruppe bestehend aus C11, C17, C19, C21 und C23 Kohlenwasserstoffen. Bevorzugt sind lineare, gesättigte Kohlenwasserstoffe. Als linearer C11 Kohlenstoff ist n-Undecan bevorzugt, als linearer C 17 Kohlenwasserstoff ist n-Heptadecan bevorzugt, als linearer C19 Kohlenwasserstoff ist n-Nonadecan bevorzugt.

Eine bevorzugte Ausführungsform der Erfindung betrifft ein Kohlenwasserstoff Gemisch, das $^{14}C$ Isotope enthält, wobei das Gemisch enthält

(e) 50 bis 90 Gew.-% lineare C-13 Kohlenwasserstoffe, vorzugsweise n-Tridecan
(f) 10 bis 50 Gew.-% lineare C-15 Kohlenwasserstoffe, vorzugsweise n-Pentadecan
bezogen auf die Summe der Kohlenwasserstoffe.

Besonders bevorzugt sind Kohlenwasserstoff Gemische, die $^{14}C$ Isotope enthalten, welche

(a) 55 bis 80 Gew.-%, insbesondere 60 bis 75 Gew.-%, insbesondere 65 bis 70 Gew.% lineare C-13 Kohlenwasserstoffe, vorzugsweise n-Tridecan
(b) 20 bis 45 Gew.-% insbesondere 24 bis 40 Gew.-%, insbesondere 24 bis 30 Gew.% lineare C-15 Kohlenwasserstoffe, vorzugsweise, vorzugsweise n-Pentadecan
bezogen auf die Summe der Kohlenwasserstoffe, enthalten

Eine bevorzugte Ausführungsform der Erfindung betrifft ein Kohlenwasserstoff Gemisch, das $^{14}C$ Isotope enthält, dadurch gekennzeichnet, dass die Summe der linearen C13- und linearen C15-Kohlenwasserstoffe größer gleich

70 Gew.-%, insbesondere größer gleich 80 Gew.-%, bevorzugt größer gleich 90 Gew.%, besonders bevorzugt größer gleich 95 Gew.-%, insbesondere größer gleich 99 Gew.-%, bezogen auf die Summe der Kohlenwasserstoffe, beträgt.

In einer bevorzugten Ausführungsform der Erfindung beträgt das Gewichtsverhältnis von linearen C13-Kohlenwasserstoffen zu linearen C15-Kohlenwasserstoffen 1,5 bis 3,5.

Besonders bevorzugt sind erfindungsgemäße Kohlenwasserstoff Gemische, bei denen die linearen C13 und/ oder linearen C15 Kohlenwasserstoffe gesättigte Kohlenwasserstoffe sind, vorzugsweise ist sowohl der lineare C13 als auch der lineare C15 Kohlenwasserstoff ein gesättigter Kohlenwasserstoff (n-Tridecan und n-Pentadecan).

In einer bevorzugten Ausführungsform enthalten die erfindungsgemäßen Kohlenwasserstoff Gemische kleiner gleich 10 Gew.-%, insbesondere kleiner gleich 5 Gew.-%, bevorzugt kleiner gleich 3 Gew.-%, an C-14 Kohlenwasserstoffen bezogen auf die Summe der Kohlenwasserstoffe.

Eine bevorzugte Ausführungsform der Erfindung betrifft ein Kohlenwasserstoff Gemisch, das $^{14}C$ Isotope enthält, enthaltend lineare C13 und lineare C15 Kohlenwasserstoffe, wobei die Summe der linearen C13- und linearen C15-Kohlenwasserstoffe größer gleich 60 Gew. % bezogen auf die Summe der Kohlenwasserstoffe beträgt und wobei die Summe der Kohlenwasserstoffe mit einer C-Kettenlänge größer gleich 16, kleiner gleich 15 Gew.-%, bezogen auf die Summe der Kohlenwasserstoffe, beträgt. Besonders bevorzugt sind Kohlenwasserstoff Gemische, bei denen die Summe der Kohlenwasserstoffe mit einer C-Kettenlänge größer gleich 16, kleiner gleich 10 Gew.-%, insbesondere kleiner gleich 8 Gew.-%, bevorzugt kleiner gleich 4 Gew.-%, insbesondere kleiner gleich 2 Gew.-% beträgt, jeweils bezogen auf die Summe der Kohlenwasserstoffe.

Eine bevorzugte Ausführungsform der Erfindung betrifft ein Kohlenwasserstoff Gemische, das $^{14}C$ Isotope enthält, enthaltend lineare C13 und lineare C15 Kohlenwasserstoffe, wobei die Summe der linearen C13- und linearen C15-Kohlenwasserstoffe größer gleich 60 Gew. % bezogen auf die Summe der Kohlenwasserstoffe beträgt und die Summe der Kohlenwasserstoffe mit einer C-Kettenlänge von kleiner gleich 12, kleiner gleich 3 Gew.-%, insbesondere kleiner gleich 2 Gew.-%, bevorzugt kleiner gleich 1,5 Gew.-%, insbesondere kleiner gleich 1 Gew.-%, bezogen auf die Summe der Kohlenwasserstoffe, beträgt.

In einer Ausführungsform der Erfindung enthalten die erfindungsgemäßen Kohlenwasserstoff Gemische C14 und C16 Kohlenwasserstoffe, vorzugsweise im selben Gewichtsverhältnis zueinander wie die linearen C13 Kohlenwasserstoffe zu den linearen C15 Kohlenwasserstoffe. In einer bevorzugten Ausführungsform der Erfindung beträgt sowohl das Gewichtsverhältnis von linearen C13-Kohlenwasserstoffen zu linearen C15-Kohlenwasserstoffen als auch das Gewichtsverhältnis von C14 zu C16 Kohlenwasserstoffen 1,5 bis 3,5.

Kohlenwasserstoff Gemisch mit linearen C15 und linearen C17 Kohlenwasserstoffen

Eine bevorzugte Ausführungsform der Erfindung betrifft ein Kohlenwasserstoff Gemisch, das $^{14}C$ Isotope enthält, wobei das Kohlenwasserstoff Gemisch mindestens 2 voneinander verschiedene Kohlenwasserstoffe enthält, deren Kohlenstoff Zahl sich um mehr als 1 unterscheidet und welches lineare C15 und lineare C17 Kohlenwasserstoffe enthält. Vorzugsweise ist der lineare C15 Kohlenwasserstoff n-Pentadecan. Vorzugsweise ist der lineare C17 Kohlenwasserstoff n-Heptadecan. In einer bevorzugten Ausführungsform der Erfindung enthält das erfindungsgemäße Kohlenwasserstoff Gemisch lineare C15 und lineare C17 Kohlenwasserstoffe sowie mindestens einen weiteren linearen Kohlenwasserstoff ausgewählt aus der Gruppe bestehend aus C11, C12, C13, C14, C16, C18, C19, C20, C21 und C23 Kohlenwasserstoffen, vorzugsweise ausgewählt aus der Gruppe bestehend aus C11, C13, C19, C21 und C23 Kohlenwasserstoffen. Bevorzugt sind lineare, gesättigte Kohlenwasserstoffe. Als linearer C11 Kohlenstoff ist n-Undecan bevorzugt, als linearer C13 Kohlenwasserstoff ist n-Tridecan bevorzugt, als linearer C19 Kohlenwasserstoff ist n-Nonadecan bevorzugt.

Eine bevorzugte Ausführungsform der Erfindung betrifft ein Kohlenwasserstoff Gemisch, das $^{14}C$ Isotope enthält, wobei das Gemisch enthält

(g) 50 bis 90 Gew.-% lineare C-15 Kohlenwasserstoffe, vorzugsweise n-Pentadecan
(h) 10 bis 50 Gew.-% lineare C-17 Kohlenwasserstoffe, vorzugsweise n-Heptadecan
bezogen auf die Summe der Kohlenwasserstoffe.

Besonders bevorzugt sind Kohlenwasserstoff Gemische, die $^{14}C$ Isotope enthalten, welche

(a) 55 bis 80 Gew.-%, insbesondere 60 bis 75 Gew.-%, insbesondere 65 bis 70 Gew.% lineare C-15 Kohlenwasserstoffe, vorzugsweise n-Pentadecan.
(b) 20 bis 45 Gew.-% insbesondere 24 bis 40 Gew.-%, insbesondere 24 bis 30 Gew.% lineare C-17 Kohlenwasserstoffe, vorzugsweise, vorzugsweise n-Heptadecan.
bezogen auf die Summe der Kohlenwasserstoffe, enthalten.

Eine bevorzugte Ausführungsform der Erfindung betrifft ein Kohlenwasserstoff Gemisch, das $^{14}C$ Isotope ent-

hält, dadurch gekennzeichnet, dass die Summe der linearen C15- und linearen C17-Kohlenwasserstoffe größer gleich 70 Gew.-%, insbesondere größer gleich 80 Gew.-%, bevorzugt größer gleich 90 Gew.%, besonders bevorzugt größer gleich 95 Gew.-%, insbesondere größer gleich 99 Gew.-%, bezogen auf die Summe der Kohlenwasserstoffe, beträgt.

In einer bevorzugten Ausführungsform der Erfindung beträgt das Gewichtsverhältnis von linearen C15-Kohlenwasserstoffen zu linearen C17-Kohlenwasserstoffen 1,5 bis 3,5.

Besonders bevorzugt sind erfindungsgemäße Kohlenwasserstoff Gemische, bei denen die linearen C15 und/oder linearen C17 Kohlenwasserstoffe gesättigte Kohlenwasserstoffe sind, vorzugsweise ist sowohl der lineare C15 als auch der lineare C17 Kohlenwasserstoff ein gesättigter Kohlenwasserstoff (n-Pentadecan und n-Heptadecan).

In einer bevorzugten Ausführungsform enthalten die erfindungsgemäßen Kohlenwasserstoff Gemische kleiner gleich 10 Gew.-%, insbesondere kleiner gleich 5 Gew.-%, bevorzugt kleiner gleich 3 Gew.-%, an C-16 Kohlenwasserstoffen bezogen auf die Summe der Kohlenwasserstoffe.

Eine bevorzugte Ausführungsform der Erfindung betrifft ein Kohlenwasserstoff Gemische das $^{14}C$ Isotope enthält, enthaltend lineare C15 und lineare C 17 Kohlenwasserstoffe, wobei die Summe der linearen C15- und linearen C17-Kohlenwasserstoffe größer gleich 60 Gew. % bezogen auf die Summe der Kohlenwasserstoffe beträgt und wobei die Summe der Kohlenwasserstoffe mit einer C-Kettenlänge größer gleich 18, kleiner gleich 15 Gew.-%, bezogen auf die Summe der Kohlenwasserstoffe, beträgt. Besonders bevorzugt sind Kohlenwasserstoff Gemische, bei denen die Summe der Kohlenwasserstoffe mit einer C-Kettenlänge größer gleich 18, kleiner gleich 10 Gew.-%, insbesondere kleiner gleich 8 Gew.-%, bevorzugt kleiner gleich 4 Gew.-%, insbesondere kleiner gleich 2 Gew.-% beträgt, jeweils bezogen auf die Summe der Kohlenwasserstoffe.

Eine bevorzugte Ausführungsform der Erfindung betrifft ein Kohlenwasserstoff Gemisch, das $^{14}C$ Isotope enthält, enthaltend lineare C15 und lineare C17 Kohlenwasserstoffe, wobei die Summe der linearen C15- und linearen C17-Kohlenwasserstoffe größer gleich 60 Gew. % bezogen auf die Summe der Kohlenwasserstoffe beträgt und die Summe der Kohlenwasserstoffe mit einer C-Kettenlänge von kleiner gleich 14, kleiner gleich 3 Gew.-%, insbesondere kleiner gleich 2 Gew.-%, bevorzugt kleiner gleich 1,5 Gew.-%, insbesondere kleiner gleich 1 Gew.-%, bezogen auf die Summe der Kohlenwasserstoffe, beträgt.

In einer Ausführungsform der Erfindung enthalten die erfindungsgemäßen Kohlenwasserstoff Gemische C16 und C18 Kohlenwasserstoffe, vorzugsweise im selben Gewichtsverhältnis zueinander wie die linearen C15 Kohlenwasserstoffe zu den linearen C17 Kohlenwasserstoffe. In einer bevorzugten Ausführungsform der Erfindung beträgt sowohl das Gewichtsverhältnis von linearen C 15-Kohlenwasserstoffen zu linearen C17-Kohlenwasserstoffen als auch das Gewichtsverhältnis von C16 zu C 18 Kohlenwasserstoffen 1,5 bis 3,5.

Kohlenwasserstoff Gemisch mit linearen C17 und linearen C19 Kohlenwasserstoffen

Eine bevorzugte Ausführungsform der Erfindung betrifft ein Kohlenwasserstoff Gemisch, das $^{14}C$ Isotope enthält, wobei das Kohlenwasserstoff Gemisch mindestens 2 voneinander verschiedene Kohlenwasserstoffe enthält, deren Kohlenstoff Zahl sich um mehr als 1 unterscheidet und welches lineare C17 und lineare C19 Kohlenwasserstoffe enthält. Vorzugsweise ist der lineare C17 Kohlenwasserstoff n-Heptadecan. Vorzugsweise ist der lineare C 19 Kohlenwasserstoff n-Nonadecan. In einer bevorzugten Ausführungsform der Erfindung enthält das erfindungsgemäße Kohlenwasserstoff Gemisch lineare C17 und lineare C19 Kohlenwasserstoffe sowie mindestens einen weiteren linearen Kohlenwasserstoff ausgewählt aus der Gruppe bestehend aus C11, C12, C13, C14, C15, C16, C18, C20, C21 und C23 Kohlenwasserstoffen, vorzugsweise ausgewählt aus der Gruppe bestehend aus C11, C13, C15, C21 und C23 Kohlenwasserstoffen. Bevorzugt sind lineare, gesättigte Kohlenwasserstoffe. Als linearer C11 Kohlenstoff ist n-Undecan bevorzugt, als linearer C13 Kohlenwasserstoff ist n-Tridecan bevorzugt, als linearer C19 Kohlenwasserstoff ist n-Nonadecan bevorzugt. Besonders bevorzugt ist als weiterer linearer Kohlenwasserstoff ist n-Henicosan (C21 linear, gesättigt).

Eine bevorzugte Ausführungsform der Erfindung betrifft ein Kohlenwasserstoff Gemisch, das $^{14}C$ Isotope enthält, wobei das Gemisch enthält

(i) 50 bis 90 Gew.-% lineare C-17 Kohlenwasserstoffe, vorzugsweise n-Heptadecan
(j) 10 bis 50 Gew.-% lineare C-19 Kohlenwasserstoffe, vorzugsweise n-Nonadecan
bezogen auf die Summe der Kohlenwasserstoffe.

Besonders bevorzugt sind Kohlenwasserstoff Gemische, die $^{14}C$ Isotope enthalten, welche

(a) 55 bis 80 Gew.-%, insbesondere 60 bis 75 Gew.-%, insbesondere 65 bis 70 Gew.% lineare C-17 Kohlenwasserstoffe, vorzugsweise n-Heptadecan.
(b) 20 bis 45 Gew.-% insbesondere 24 bis 40 Gew.-%, insbesondere 24 bis 30 Gew.% lineare C-19 Kohlenwasserstoffe, vorzugsweise, vorzugsweise n-Nonadecan
bezogen auf die Summe der Kohlenwasserstoffe, enthalten

Eine bevorzugte Ausführungsform der Erfindung betrifft ein Kohlenwasserstoff Gemisch, das $^{14}C$ Isotope enthält, dadurch gekennzeichnet, dass die Summe der linearen C17- und linearen C 19-Kohlenwasserstoffe größer gleich 70 Gew.-%, insbesondere größer gleich 80 Gew.-%, bevorzugt größer gleich 90 Gew.%, besonders bevorzugt größer gleich 95 Gew.-%, insbesondere größer gleich 99 Gew.-%, bezogen auf die Summe der Kohlenwasserstoffe, beträgt.

In einer bevorzugten Ausführungsform der Erfindung beträgt das Gewichtsverhältnis von linearen C 17-Kohlenwasserstoffen zu linearen C 19-Kohlenwasserstoffen 1,5 bis 3,5.

Besonders bevorzugt sind erfindungsgemäße Kohlenwasserstoff Gemische, bei denen die linearen C 17 und/ oder linearen C 19 Kohlenwasserstoffe gesättigte Kohlenwasserstoffe sind, vorzugsweise ist sowohl der lineare C17 als auch der lineare C19 Kohlenwasserstoff ein gesättigter Kohlenwasserstoff (n-Heptadecan und n-Nonadecan).

In einer bevorzugten Ausführungsform enthalten die erfindungsgemäßen Kohlenwasserstoff Gemische kleiner gleich 10 Gew.-%, insbesondere kleiner gleich 5 Gew.-%, bevorzugt kleiner gleich 3 Gew.-%, an C-18 Kohlenwasserstoffen bezogen auf die Summe der Kohlenwasserstoffe.

Eine bevorzugte Ausführungsform der Erfindung betrifft ein Kohlenwasserstoff Gemisch, das $^{14}C$ Isotope enthält, enthaltend lineare C 17 und lineare C 19 Kohlenwasserstoffe, wobei die Summe der linearen C17- und linearen C19-Kohlenwasserstoffe größer gleich 60 Gew. % bezogen auf die Summe der Kohlenwasserstoffe beträgt und wobei die Summe der Kohlenwasserstoffe mit einer C-Kettenlänge größer gleich 20, kleiner gleich 15 Gew.-%, bezogen auf die Summe der Kohlenwasserstoffe, beträgt. Besonders bevorzugt sind Kohlenwasserstoff Gemische, bei denen die Summe der Kohlenwasserstoffe mit einer C-Kettenlänge größer gleich 18, kleiner gleich 10 Gew.%, insbesondere kleiner gleich 8 Gew.-%, bevorzugt kleiner gleich 4 Gew.-%, insbesondere kleiner gleich 2 Gew.-% beträgt, jeweils bezogen auf die Summe der Kohlenwasserstoffe.

Eine bevorzugte Ausführungsform der Erfindung betrifft ein Kohlenwasserstoff Gemisch, das $^{14}C$ Isotope enthält, enthaltend lineare C17 und lineare C19 Kohlenwasserstoffe, wobei die Summe der linearen C17- und linearen C19-Kohlenwasserstoffe größer gleich 60 Gew. % bezogen auf die Summe der Kohlenwasserstoffe beträgt und die Summe der Kohlenwasserstoffe mit einer C-Kettenlänge von kleiner gleich 16, kleiner gleich 3 Gew.-%, insbesondere kleiner gleich 2 Gew.-%, bevorzugt kleiner gleich 1,5 Gew.%, insbesondere kleiner gleich 1 Gew.%, bezogen auf die Summe der Kohlenwasserstoffe, beträgt.

In einer Ausführungsform der Erfindung enthalten die erfindungsgemäßen Kohlenwasserstoff Gemische C18 und C20 Kohlenwasserstoffe, vorzugsweise im selben Gewichtsverhältnis zueinander wie die linearen C17 Kohlenwasserstoffe zu den linearen C19 Kohlenwasserstoffe. In einer bevorzugten Ausführungsform der Erfindung beträgt sowohl das Gewichtsverhältnis von linearen C17-Kohlenwasserstoffen zu linearen C19-Kohlenwasserstoffen als auch das Gewichtsverhältnis von C18 zu C20 Kohlenwasserstoffen 1,5 bis 3,5.

Kohlenwasserstoff Gemisch mit linearen C19 und linearen C21 Kohlenwasserstoffen

Eine bevorzugte Ausführungsform der Erfindung betrifft ein Kohlenwasserstoff Gemisch, das $^{14}C$ Isotope enthält, wobei das Kohlenwasserstoff Gemisch mindestens 2 voneinander verschiedene Kohlenwasserstoffe enthält, deren Kohlenstoff Zahl sich um mehr als 1 unterscheidet und welches lineare C19 und lineare C21 Kohlenwasserstoffe enthält. Vorzugsweise ist der lineare C19 Kohlenwasserstoff n-Nonadecan. Vorzugsweise ist der lineare C 21 Kohlenwasserstoff n-Henicosan. In einer bevorzugten Ausführungsform der Erfindung enthält das erfindungsgemäße Kohlenwasserstoff Gemisch lineare C19 und lineare C21 Kohlenwasserstoffe sowie mindestens einen weiteren linearen Kohlenwasserstoff ausgewählt aus der Gruppe bestehend aus C11, C12, C13, C14, C15, C16, C17, C18, C20 und C23 Kohlenwasserstoffen, vorzugsweise ausgewählt aus der Gruppe bestehend aus C11, C13, C15, C17 und C23 Kohlenwasserstoffen. Bevorzugt sind lineare, gesättigte Kohlenwasserstoffe. Als linearer C11 Kohlenstoff ist n-Undecan bevorzugt, als linearer C13 Kohlenwasserstoff ist n-Tridecan bevorzugt, als linearer C17 Kohlenwasserstoff ist n-Heptadecan bevorzugt.

Eine bevorzugte Ausführungsform der Erfindung betrifft ein Kohlenwasserstoff Gemisch, das $^{14}C$ Isotope enthält, wobei das Gemisch enthält

(a) 50 bis 90 Gew.-% lineare C-19 Kohlenwasserstoffe, vorzugsweise n-Nonadecan
(b) 10 bis 50 Gew.-% lineare C-21 Kohlenwasserstoffe, vorzugsweise n-Henicosan bezogen auf die Summe der Kohlenwasserstoffe.

Besonders bevorzugt sind Kohlenwasserstoff Gemische, die $^{14}C$ Isotope enthalten, welche

(a) 55 bis 80 Gew.-%, insbesondere 60 bis 75 Gew.-%, insbesondere 65 bis 70 Gew.% lineare C-19 Kohlenwasserstoffe, vorzugsweise n-Nonadecan.
(b) 20 bis 45 Gew.-% insbesondere 24 bis 40 Gew.-%, insbesondere 24 bis 30 Gew.% lineare C-21 Kohlenwasserstoffe, vorzugsweise, vorzugsweise n-Henicosan
bezogen auf die Summe der Kohlenwasserstoffe, enthalten

Eine bevorzugte Ausführungsform der Erfindung betrifft ein Kohlenwasserstoff Gemisch, das $^{14}C$ Isotope enthält, dadurch gekennzeichnet, dass die Summe der linearen C19- und linearen C21-Kohlenwasserstoffe größer gleich 70 Gew.-%, insbesondere größer gleich 80 Gew.-%, bevorzugt größer gleich 90 Gew.%, besonders bevorzugt größer gleich 95 Gew.-%, insbesondere größer gleich 99 Gew.-%, bezogen auf die Summe der Kohlenwasserstoffe, beträgt.

In einer bevorzugten Ausführungsform der Erfindung beträgt das Gewichtsverhältnis von linearen C19-Kohlenwasserstoffen zu linearen C21-Kohlenwasserstoffen 1,5 bis 3,5.

Besonders bevorzugt sind erfindungsgemäße Kohlenwasserstoff Gemische, bei denen die linearen C19 und/oder linearen C21 Kohlenwasserstoffe gesättigte Kohlenwasserstoffe sind, vorzugsweise ist sowohl der lineare C19 als auch der lineare C21 Kohlenwasserstoff ein gesättigter Kohlenwasserstoff (n-Nonadecan und n-Henicosan).

In einer bevorzugten Ausführungsform enthalten die erfindungsgemäßen Kohlenwasserstoff Gemische kleiner gleich 10 Gew.-%, insbesondere kleiner gleich 5 Gew.-%, bevorzugt kleiner gleich 3 Gew.-%, an C-20 Kohlenwasserstoffen bezogen auf die Summe der Kohlenwasserstoffe.

Eine bevorzugte Ausführungsform der Erfindung betrifft ein Kohlenwasserstoff Gemisch, das $^{14}C$ Isotope enthält, enthaltend lineare C19 und lineare C21 Kohlenwasserstoffe, wobei die Summe der linearen C19- und linearen C21-Kohlenwasserstoffe größer gleich 60 Gew. % bezogen auf die Summe der Kohlenwasserstoffe beträgt und wobei die Summe der Kohlenwasserstoffe mit einer C-Kettenlänge größer gleich 22, kleiner gleich 15 Gew.-%, bezogen auf die Summe der Kohlenwasserstoffe, beträgt. Besonders bevorzugt sind Kohlenwasserstoff Gemische, bei denen die Summe der Kohlenwasserstoffe mit einer C-Kettenlänge größer gleich 22, kleiner gleich 10 Gew.-%, insbesondere kleiner gleich 8 Gew.-%, bevorzugt kleiner gleich 4 Gew.-%, insbesondere kleiner gleich 2 Gew.-% beträgt, jeweils bezogen auf die Summe der Kohlenwasserstoffe.

Eine bevorzugte Ausführungsform der Erfindung betrifft ein Kohlenwasserstoff Gemisch, das $^{14}C$ Isotope enthält, enthaltend lineare C19 und lineare C21 Kohlenwasserstoffe, wobei die Summe der linearen C19- und linearen C21-Kohlenwasserstoffe größer gleich 60 Gew. % bezogen auf die Summe der Kohlenwasserstoffe beträgt und die Summe der Kohlenwasserstoffe mit einer C-Kettenlänge von kleiner gleich 18, kleiner gleich 3 Gew.-%, insbesondere kleiner gleich 2 Gew.-%, bevorzugt kleiner gleich 1,5 Gew.-%, insbesondere kleiner gleich 1 Gew.-%, bezogen auf die Summe der Kohlenwasserstoffe, beträgt.

In einer Ausführungsform der Erfindung enthalten die erfindungsgemäßen Kohlenwasserstoff Gemische C20 und C22 Kohlenwasserstoffe, vorzugsweise im selben Gewichtsverhältnis zueinander wie die linearen C19 Kohlenwasserstoffe zu den linearen C21 Kohlenwasserstoffe. In einer bevorzugten Ausführungsform der Erfindung beträgt sowohl das Gewichtsverhältnis von linearen C19-Kohlenwasserstoffen zu linearen C21-Kohlenwasserstoffen als auch das Gewichtsverhältnis von C20 zu C22 Kohlenwasserstoffen 1,5 bis 3,5.
Die erfindungsgemäßen Kohlenwasserstoff Gemische eignen sich insbesondere zur Verwendung in kosmetischen und/oder pharmazeutischen Zubereitungen, insbesondere als Ölkörper und/oder als Dispergatoren.

Ein weiterer Gegenstand der Erfindung betrifft ein Verfahren zur Herstellung einer kosmetischen und/oder pharmazeutischen Zubereitung, wobei ein Kohlenwasserstoff Gemisch gemäß einem der Ansprüche 1 bis 13 zu einen kosmetisch und/oder pharmazeutisch geeigneten Träger gegeben wird.

Die erfindungsgemäßen Kohlenwasserstoff Gemische eignen sich insbesondere zur Verwendung in kosmetischen Zubereitungen zur Pflege von Haut und/oder Haaren.

Die erfindungsgemäßen Kohlenwasserstoff Gemische eignen sich insbesondere zur Verwendung in kosmetischen Zubereitungen zum Sonnenschutz.

Die erfindungsgemäßen Kohlenwasserstoff Gemische eignen sich insbesondere zur Verwendung in Zubereitungen der dekorativen Kosmetik, wie beispielsweise Lippenstifte, Lip Gloss, Lidschatten, Wimperntuschen (Mascara), Lidstifte (Kajal), Nagellack sowie in Make-up Formulierungen jeder Art (Puder, Creme, Foundation, Abdeckstifte etc.).

Die erfindungsgemäßen Kohlenwasserstoff Gemische eignen sich insbesondere zur Verwendung in Zubereitungen zur Reinigung von Haut und/oder Haaren, wie beispielsweise Shampoos, Duschgels, Badezusätze, Conditioner etc.

Die erfindungsgemäßen Kohlenwasserstoffgemische eignen sich weiterhin zur Herstellung von feinteiligen Emulsionen, so z.B. Nanoemulsionen, Microemulsionen oder PIT Emulsionen. In solchen feinteiligen Emulsionen liegen die Öltröpfchen in der Regel im Bereich von 10 bis 1000 nm, vorzugsweise 100 bis 500 nm Durchmesser vor. Diese werden nach dem Fachmann bekannten Verfahren hergestellt, für PIT Emulsionen beispielsweise in Parfümerie und Kosmetik, 77. Jahrgang, Nr. 4/96, S. 250 - 254 von Wadle et al. beschrieben.

Herstellung der Kohlenwasserstoff Gemische

Die erfindungsgemäßen Kohlenwasserstoff Gemische können beispielsweise durch reduktive Demethylierung von pflanzlichen Fettalkoholen erhalten werden. Besonders geeignet zur Herstellung der erfindungsgemäßen Kohlenwasserstoff Gemische ist das in der internationalen Anmeldung PCT/EP2006/011647 (Cognis) beschrieben Verfahren der reduktiven Dehydroxymethylierung ausgehend von Fettalkoholen pflanzlichen Ursprungs. Dabei können beispielsweise Fettalkohole gewünschter C Kettenlänge einzelnen dem beschriebenen Verfahren unterzogen werden und die so erhaltenen Kohlenwasserstoffe zu den erfindungsgemäßen Kohlenwasserstoff Gemischen gemischt werden. Bevorzugt ist es jedoch ein Gemisch welche die entsprechenden Fettalkohole enthält direkt der reduktiven Dehydroxymethylierung zu unterwerfen, und so dass als Reaktionsprodukt direkt das erfindungsgemäße Kohlenwasserstoff Gemisch zu erhalten. Dieses kann dann direkt, ohne weitere Aufreinigung, in kosmetischen und/oder pharmazeutischen Zubereitung eingesetzt werden.

Kosmetische und/oder pharmazeutische Zubereitungen

Ein weiterer Gegenstand der Erfindung betrifft kosmetische und/oder pharmazeutische Zubereitungen, enthaltend 0,1 bis 80 Gew.%, vorzugsweise 5 bis 50 Gew.-%, insbesondere 10 bis 30 Gew.-% eines Kohlenwasserstoff Gemischs, das $^{14}C$ Isotope enthält. Die Gew.-% beziehen sich auf das Gesamtgewicht der kosmetischen und/oder pharmazeutischen Zubereitung.

Bei den in den erfindungsgemäßen Zubereitungen vorhandenen Kohlenwasserstoffen Gemischen handelt es sich immer um Mischungen von mindestens 2 voneinander verschiedenen Kohlenwasserstoffen. Diese können sich in ihrer C-Zahl um 1, 2, 3, 4 etc. Kohlenstoffatome unterscheiden. In einer bevorzugten Ausführungsform enthaltend die kosmetischen und/oder pharmazeutischen Zubereitungen Kohlenwasserstoffe, deren Kohlenstoff Zahl sich um mehr als 1, vorzugsweise um 2 unterscheidet.

Vorzugsweise beträgt der Anteil der $^{14}C$ Isotope zu den $^{12}C$ Isotopen in dem in den erfindungsgemäßen Zubereitungen enthaltende Kohlenwasserstoff Gemisch größer gleich 1 x $10^{-16}$, insbesondere größer gleich 1 x $10^{-15}$, vorzugsweise größer gleich 7,5 x $10^{-14}$, vorzugsweise größer gleich 1,5 x $10^{-13}$, insbesondere größer gleich 3 x $10^{-13}$, vorzugsweise liegt der Anteil der $^{14}C$ Isotope zu den $^{12}C$ Isotopen im Bereich von 6 x $10^{-13}$ bis 1,2 x $10^{-12}$. Bezugsgröße sind hier alle in der erfindungsgemäßen Zubereitung enthaltenen Kohlenwasserstoffe.

Vorzugsweise enthält das in der erfindungsgemäßen Zubereitung enthaltende Kohlenwasserstoff Gemisch mindestens einen Kohlenwasserstoff, der $^{14}C$ Isotope enthält, vorzugsweise enthalten 2 voneinander verschiedene Kohlenwasserstoffe $^{14}C$ Isotope. Besonders bevorzugt enthalten die 2 voneinander verschiedenen Kohlenwasserstoffe, deren Kohlenstoffzahl sich um mehr als 1, vorzugsweise um 2 unterscheidet, $^{14}C$ Isotope.

In einer bevorzugten Ausführungsform der Erfindung enthalten die kosmetischen und/oder pharmazeutischen Zubereitungen die Kohlenwasserstoff Gemische gemäß einem der Ansprüche 1 bis 13.

In einer bevorzugten Ausführungsform enthalten die erfindungsgemäßen kosmetischen und/oder pharmazeutischen Zubereitungen Kohlenwasserstoffe, die ausgewählt sind aus der Gruppe der Kohlenwasserstoffe mit 7 bis 23 Kohlenstoffatomen, vorzugsweise 11 bis 21 Kohlenstoffatome.

In einer bevorzugten Ausführungsform enthalten die erfindungsgemäßen kosmetischen und/oder pharmazeutischen Zubereitungen Kohlenwasserstoff Gemische, die kleiner gleich 50 Gew.-%, insbesondere kleiner gleich 20 Gew.-%, kleiner gleich 10 Gew.%, vorzugsweise kleiner gleich 5 Gew.-%, insbesondere kleiner gleich 1 Gew.-% verzweigte Kohlenwasserstoffe bezogen auf die Summe der Kohlenwasserstoffe enthalten

Die Bezugsgröße "Summe der Kohlenwasserstoffe" in kosmetischen und/oder pharmazeutischen Zubereitungen umfasst alle in der kosmetischen und/oder pharmazeutischen Zubereitung enthaltenen Kohlenwasserstoffe, unabhängig von ihrer Kohlenstoffzahl.

In einer bevorzugten Ausführungsform enthalten die erfindungsgemäßen kosmetischen und/oder pharmazeutischen Zubereitungen Kohlenwasserstoff Gemische, die kleiner gleich 20 Gew.-%, insbesondere kleiner gleich 10 Gew.-%, insbesondere kleiner gleich 5 Gew.-%, bevorzugt kleiner gleich 1 Gew.-%, aromatische Kohlenwasserstoffe bezogen auf die Summe der Kohlenwasserstoffe enthalten. In einer bevorzugten Ausführungsform der Erfindung enthalten die Zubereitungen kleiner gleich 0,1, insbesondere kleiner gleich 0,01 Gew.-%, insbesondere kleiner gleich 0,001 Gew.-% aromatische Kohlenwasserstoffe bezogen auf die Summe der Kohlenwasserstoffe.

In einer bevorzugten Ausführungsform enthalten die erfindungsgemäßen kosmetischen und/oder pharmazeutischen Zubereitungen Kohlenwasserstoff Gemische, die kleiner gleich 50 Gew.-%, insbesondere kleiner gleich 20 Gew.-%, kleiner gleich 10 Gew.-%, insbesondere kleiner gleich 5 Gew.-%, bevorzugt kleiner gleich 1 Gew.-%, ungesättigte Kohlenwasserstoffe bezogen auf die Summe der Kohlenwasserstoffe enthalten. In einer bevorzugten Ausführungsform der Erfindung enthalten die Zubereitungen kleiner gleich 0,1, insbesondere kleiner gleich 0,01 Gew.-%, insbesondere kleiner gleich 0,001 Gew.-% ungesättigte Kohlenwasserstoffe bezogen auf die Summe der Kohlenwasserstoffe

In einer bevorzugten Ausführungsform enthalten die erfindungsgemäßen kosmetischen und/oder pharmazeutischen Zubereitungen Kohlenwasserstoff Gemische, die kleiner gleich 20 Gew.-% insbesondere kleiner gleich 15 Gew.-%, insbesondere kleiner gleich 10 Gew.-%, vorzugsweise kleiner gleich 9 Gew.-%, insbesondere kleiner gleich 8 Gew.-% insbesondere kleiner gleich 5 Gew.-% geradzahlige Kohlenwasserstoffe, bezogen auf die Summe der Kohlenwasserstoffe enthalten.

Ein weiterer Gegenstand der Erfindung betrifft kosmetische und/oder pharmazeutische Zubereitungen, enthaltend 0,1 bis 80 Gew.-%, vorzugsweise 5 bis 50 Gew.-%, insbesondere 10 bis 30 Gew.-% eines Kohlenwasserstoff

Gemischs, das $^{14}C$ Isotope enthält, und worin 2 voneinander verschiedenen Kohlenwasserstoffe mindestens 50 Gew.-%, vorzugsweise mindestens 60 Gew.-% - bezogen auf die Summe der Kohlenwasserstoffe ausmachen.

Besonders bevorzugt sind erfindungsgemäße kosmetische und/oder pharmazeutische Zubereitungen, in denen die Summe der 2 voneinander verschiedenen Kohlenwasserstoffe größer gleich 70 Gew.-%, insbesondere größer gleich 80 Gew.-%, bevorzugt größer gleich 90 Gew.%, besonders bevorzugt größer gleich 95 Gew.-%, insbesondere größer gleich 99 Gew.-%, bezogen auf die Summe der Kohlenwasserstoffe, beträgt. In einer Ausführungsform der Erfindung besteht das Kohlenwasserstoff Gemisch der kosmetischen und/oder pharmazeutischen Zubereitung ausschließlich aus 2 voneinander verschiedenen Kohlenwasserstoffen.

In einer bevorzugten Ausführungsform enthalten die erfindungsgemäßen kosmetischen und/oder pharmazeutischen Zubereitungen 0,1 bis 80 Gew.-%, vorzugsweise 5 bis 50 Gew.-%, insbesondere 10 bis 30 Gew.-% eines Kohlenwasserstoff Gemischs, das $^{14}C$ Isotope enthält, worin 2 voneinander verschiedenen Kohlenwasserstoff ausgewählt sind aus der Gruppe bestehend aus

- linearen C 11 und linearen C 13 Kohlenwasserstoffen,
- linearen C11 und linearen C15 Kohlenwasserstoffen
- linearen C13 und linearen C15 Kohlenwasserstoffen,
- linearen C15 und linearen C17 Kohlenwasserstoffen,
- linearen C17 und linearen C19 Kohlenwasserstoffen und/oder
- linearen C19 und linearen C21 Kohlenwasserstoffen.

Diese Zubereitungen können beispielsweise erhalten werden, indem ein erfindungsgemäßes Kohlenwasserstoff Gemisch eingesetzt wird oder indem definierte Mengen einzelner Kohlenwasserstoffe eingesetzt werden.

Besonders bevorzugt sind kosmetische und/oder pharmazeutische Zubereitungen, enthaltend 0,1 bis 80 Gew. % eines Kohlenwasserstoff Gemischs, das $^{14}C$ Isotope enthält und wobei das Kohlenwasserstoff Gemisch

- 50 bis 90 Gew.-% eines Kohlenwasserstoffes mit der C-Zahl n enthält
- 10 bis 50 Gew.-% eines Kohlenwasserstoffs mit der C-Zahl n+2 enthält bezogen auf die Summe der Kohlenwasserstoffe enthalten.

wobei n eine ganze Zahl von 7 bis 23, vorzugsweise 11 bis 21 darstellt.

Besonders bevorzugt sind kosmetische und/oder pharmazeutische Zubereitungen, enthaltend 0,1 bis 80 Gew. % eines Kohlenwasserstoff Gemischs, das $^{14}C$ Isotope enthält, wobei das Kohlenwasserstoff Gemisch

- 55 bis 80 Gew.-%, insbesondere 60 bis 75 Gew.-%, insbesondere 65 bis 70 Gew.% eines Kohlenwasserstoffes mit der C-Zahl n enthält
- 20 bis 45 Gew.-% insbesondere 24 bis 40 Gew.-%, insbesondere 24 bis 30 Gew.% eines Kohlenwasserstoffes mit der C-Zahl n enthält
  bezogen auf die Summe der Kohlenwasserstoffe.

wobei n eine ganze Zahl von 7 bis 23, vorzugsweise 11 bis 21 darstellt.

In einer bevorzugten Ausführungsform der Erfindung beträgt das Gewichtsverhältnis des Kohlenwasserstoffe mit der C-Zahl n zum Kohlenwasserstoff mit der C-Zahl n+2 in den kosmetischen und/oder pharmazeutischen Zubereitungen 1,5 bis 3,5.

Die erfindungsgemäßen kosmetischen und/oder pharmazeutischen Zubereitungen stellen leichte und stabile kosmetische und/oder pharmazeutische Zubereitungen dar, dies ist insbesondere dann der Fall, wenn sie weiterhin Antiperspirant-/Desodorant-Wirkstoffen enthalten.

Ein Gegenstand der Erfindung betrifft daher kosmetische und/oder pharmazeutische Zubereitungen, enthaltend 0,1 bis 80 Gew.-% eines Kohlenwasserstoff Gemischs, das $^{14}C$ Isotope enthält, und mindestens einen Antiperspirant-/Desodorant-Wirkstoff. Erfindungsgemäß sind als Antiperspirant/Desodorant Wirkstoff alle Wirkstoffe geeignet, die Körpergerüchen entgegen wirken, diese überdecken oder beseitigen. Körpergerüche entstehen durch die Einwirkung von Hautbakterien auf apokrinen Schweiß, wobei unangenehm riechende Abbauprodukte gebildet werden. Als Antiperspirant /Desodorant Wirkstoffe eignen sich insbesondere Verbindungen ausgewählt aus der Gruppe bestehend Antiperspirantien, Esteraseinhibitoren, bakterizide bzw. bakteriostatische Wirkstoffe und/oder schweißabsorbierende Substanzen.

<u>Antiperspirantien</u>

Bei Antiperspirantien handelt es sich um Salze des Aluminiums, Zirkoniums oder des Zinks. Solche geeigneten antihydrotisch wirksamen Wirkstoffe sind z.B. Aluminiumchlorid, Aluminiumchlorhydrat, Aluminiumdichlorhydrat, Aluminiumsesquichlorhydrat und deren Komplexverbindungen z. B. mit Propylenglycol-1,2. Aluminiumhydroxyallantoinat, Aluminiumchloridtartrat, Aluminium-Zirkonium-Trichlorohydrat, Aluminium-Zirkonium-tetrachlorohydrat, Aluminium-Zirkonium-pentachlorohydrat und deren Komplexverbindungen z. B. mit Aminosäuren wie Glycin. Vorzugsweise werden Aluminiumchlorhydrat, Aluminium-Zirkonium-tetrachlorohydrat, Aluminium-Zirkonium-pentachlorohydrat und deren Komplexverbindungen eingesetzt.

Die erfindungsgemäßen Zubereitungen können die Antiperspirantien in Mengen von 1 bis 50, vorzugsweise 5 bis 30 und insbesondere 8 bis 25 Gew.-% - bezogen auf das Gesamtgewicht der kosmetischen und/oder pharmazeutischen Zubereitung - enthalten.

Esteraseinhibitoren

Beim Vorhandensein von Schweiß im Achselbereich werden durch Bakterien extrazelluläre Enzyme - Esterasen, vorzugsweise Proteasen und/oder Lipasen - gebildet, die im Schweiß enthaltene Ester spalten und dadurch Geruchsstoffe freisetzen. Als Esteraseinhibitoren geeignet sind vorzugsweise Trialkylcitrate wie Trimethylcitrat, Tripropylcitrat, Triisopropylcitrat, Tributylcitrat und insbesondere Triethylcitrat (Hydagen® CAT, Cognis GmbH, Düsseldorf/FRG). Die Stoffe inhibieren die Enzymaktivität und reduzieren dadurch die Geruchsbildung. Weitere Stoffe, die als Esteraseinhibitoren in Betracht kommen, sind Sterolsulfate oder -phosphate, wie beispielsweise Lanosterin-, Cholesterin-, Campesterin-, Stigmasterin- und Sitosterinsulfat bzw -phosphat, Dicarbonsäuren und deren Ester, wie beispielsweise Glutarsäure, Glutarsäuremonoethylester, Glutarsäurediethylester, Adipinsäure, Adipinsäuremonoethylester, Adipinsäurediethylester, Malonsäure und Malonsäurediethylester, Hydroxycarbonsäuren und deren Ester wie beispielsweise Citronensäure, Äpfelsäure, Weinsäure oder Weinsäurediethylester sowie Zinkglycinat.

Die erfindungsgemäßen Zubereitungen können die Esteraseinhibitoren in Mengen von 0,01 bis 20, vorzugsweise 0,1 bis 10 und insbesondere 0,3 bis 5 Gew.-% - bezogen auf das Gesamtgewicht der kosmetischen und/oder pharmazeutischen Zubereitung - enthalten.

Bakterizide bzw. bakteriostatische Wirkstoffe

Typische Beispiele für geeignete bakterizide bzw. bakteriostatische Wirkstoffe sind insbesondere Chitosan und Phenoxyethanol. Besonders wirkungsvoll hat sich auch 5-Chlor-2-(2,4-dichlorphenoxy)-phenol erwiesen, das unter der Marke Irgasan® von der Ciba-Geigy, Basel/CH vertrieben wird. Als keimhemmende Mittel sind grundsätzlich alle gegen grampositive Bakterien wirksamen Stoffe geeignet, wie z. B. 4-Hydroxybenzoesäure und ihre Salze und Ester, N-(4-Chlorphenyl)-N'-(3,4 dichlorphenyl)harnstoff, 2,4,4'-Trichlor-2'-hydroxydiphenylether (Triclosan), 4-Chlor-3,5-dimethylphenol, 2,2'-Methylen-bis(6-brom-4-chlorphenol), 3-Methyl-4-(1-methylethyl)phenol, 2-Benzyl-4-chlorphenol, 3-(4-Chlorphenoxy)-1,2-propandiol, 3-Iod-2-propinylbutylcarbamat, Chlorhexidin, 3,4,4'-Trichlorcarbanilid (TTC), antibakterielle Riechstoffe, Thymol, Thymianöl, Eugenol, Nelkenöl, Menthol, Minzöl, Farnesol, Phenoxyethanol, Glycerinmonocaprinat, Glycerinmonocaprylat, Glycerinmonolaurat (GML), Diglycerinmonocaprinat (DMC), Salicylsäure-N-alkylamide wie z. B. Salicylsäure-n-octylamid oder Salicylsäure-n-decylamid.

Die erfindungsgemäßen Zubereitungen können die bakteriziden bzw. bakteriostatischen Wirkstoffe in Mengen von 0,01 bis 5 und vorzugsweise 0,1 bis 2 Gew.% - bezogen auf das Gesamtgewicht der kosmetischen und/oder pharmazeutischen Zubereitung - enthalten.

Schweißabsorbierende Substanzen

Als schweißabsorbierende Substanzen kommen modifizierte Stärke, wie z.B. Dry Flo Plus (Fa. National Starch), Silikate, Talkum und andere Substanzen ähnlicher Modifikation, die zur Schweißabsorption geeignet erscheinen. Die erfindungsgemäßen Zubereitungen können die schweißabsorbierende Substanzen in Mengen von 0,1 bis 30, vorzugsweise 1 bis 20 und insbesondere 2 bis 8 Gew.-% - bezogen auf das Gesamtgewicht der kosmetischen und/oder pharmazeutischen Zubereitung - enthalten.

Die erfindungsgemäßen kosmetischen und/oder pharmazeutischen Zubereitungen stellen leichte und stabile kosmetische und/oder pharmazeutische Zubereitungen dar, dies ist insbesondere dann der Fall, wenn sie weiterhin mindestens einen UV-Lichtschutzfilter enthalten.

Ein Gegenstand der Erfindung betrifft daher kosmetische und/oder pharmazeutische Zubereitungen, enthaltend 0,1 bis 80 Gew.% eines Kohlenwasserstoff Gemischs, das $^{14}C$ Isotope enthält, und mindestens mindestens einen UV-Lichtschutzfilter.

Ein Gegenstand der Erfindung betrifft vorzugsweise kosmetische und/oder pharmazeutische Zubereitungen, enthaltend 0,1 bis 80 Gew.% eines Kohlenwasserstoff Gemischs, das $^{14}C$ Isotope enthält und wobei die Kohlenwasserstoffe mindestens 2 voneinander verschiedene Kohlenwasserstoffe enthalten, deren Kohlenstoff Zahl sich um mehr als 1, vorzugsweise um 2 unterscheidet, und mindestens einen UV-Lichtschutzfilter.

Erfindungsgemäß sind als UV-Lichtschutzfilter bei Raumtemperatur flüssige oder kristalline organische Substanzen (Lichtschutzfilter) geeignet, die in der Lage sind, ultraviolette Strahlen zu absorbieren und die aufgenommene Energie in Form längerwelliger Strahlung, z.B. Wärme wieder abzugeben. UV-Filter können öllöslich oder wasserlöslich sein. Als typische öllösliche UV-B-Filter bzw. Breitspektrum-UV A/B-Filter sind z.B. zu nennen:

- ➢ 3-Benzylidencampher bzw. 3-Benzylidennorcampher (Mexoryl SDS 20) und dessen Derivate, z.B. 3-(4-Methylbenzyliden)campher wie in der EP 0693471 B1 beschrieben
- ➢ 3-(4'-Trimethylammonium) benzyliden- bornan-2-on-methylsulfat (Mexoryl SO)
- ➢ 3,3'-(1,4-Phenylendimethin)-bis (7,7- dimethyl-2-oxobicyclo-[2.2.1] heptan-1-methansulfonsäure) and salts (Mexoryl SX)
- ➢ 3-(4'-Sulfo)-benzyliden-bornan-2-on and salts (Mexoryl SL)
- ➢ Polymer von N-{(2und 4)- [2-oxoborn-3-yliden)methyl}benzyl]acrylamid (Mexoryl SW)
- ➢ 2-(2H-Benzotriazol-2-yl)-4-methyl-6-(2-methyl-3-(1,3,3,3-tetramethyl-1-(trimethylsilyloxy) disiloxanyl)propyl) phenol (Mexoryl SL)
- ➢ 4-Aminobenzoesäurederivate, vorzugsweise 4-(Dimethylamino)benzoesäure-2-ethylhexylester, 4-(Dimethylamino)benzoesäure-2-octylester und 4-(Dimethylamino)benzoe-säureamylester;
- ➢ Ester der Zimtsäure, vorzugsweise 4-Methoxyzimtsäure-2-ethylhexylester, 4-Methoxyzimtsäurepropylester, 4-Methoxyzimtsäureisoamylester, 2-Cyano-3,3-phenylzimtsäure-2-ethylhexylester (Octocrylene);
- ➢ Ester der Salicylsäure, vorzugsweise Salicylsäure-2-ethylhexylester, Salicylsäure-4-isopropylbenzylester, Salicylsäurehomomenthylester;
- ➢ Derivate des Benzophenons, vorzugsweise 2-Hydroxy-4-methoxybenzophenon, 2-Hydroxy-4-methoxy-4'-methylbenzophenon, 2,2'-Dihydroxy-4-methoxybenzophenon;
- ➢ Ester der Benzalmalonsäure, vorzugsweise 4-Methoxybenzmalonsäuredi-2-ethylhexylester;
- ➢ Triazinderivate, wie z.B. 2,4,6-Trianilino-(p-carbo-2'-ethyl-1'-hexyloxy)-1,3,5-triazin und 2,4,6-Tris[p-(2-ethylhexyl-oxycar-bonyl) anilino]-1,3,5-triazin (Uvinul T 150) wie in der EP 0818450 A1 beschrieben oder 4,4'-[(6-[4-((1,1-Dimethylethyl)amino-carbonyl) phenyl-amino]-1,3,5-triazin-2,4- diyl)diimino] bis(benzoesäure-2- ethylhexylester) (Uvasorb® HEB);
- ➢ 2,2(-Methylen-bis(6-(2H-benzotriazol-2-yl)-4- (1,1,3,3-tetramethyl-butyl)phenol) (Tinosorb M);
- ➢ 2,4-Bis[4-(2-ethylhexyloxy)-2- hydroxyphenyl]-6-(4- methoxyphenyl)-1,3,5- triazin (Tinosorb S);
- ➢ Propan-1,3-dione, wie z.B. 1-(4-tert.Butylphenyl)-3-(4'methoxyphenyl)propan-1,3-dion;
- ➢ Ketotricyclo(5.2.1.0)decan-Derivate, wie in der EP 0694521 B1 beschrieben;
- ➢ Dimethicodiethylbenzalmalonate (Parsol SLX).

Als wasserlösliche UV - Filter kommen in Frage:

- ➢ 2-Phenylbenzimidazol-5-sulfonsäure und deren Alkali-, Erdalkali-, Ammonium-, Alkylammonium-, Alkanolammonium- und Glucammoniumsalze;
- ➢ 2,2(-(1,4-Phenylen)bis(1H-benzimidazol- 4,6-disulfon-säure, Mononatriumsalz) (Neo Heliopan AP)
- ➢ Sulfonsäurederivate von Benzophenonen, vorzugsweise 2-Hydroxy-4-methoxybenzophenon-5-sulfonsäure und ihre Salze;
- ➢ Sulfonsäurederivate des 3-Benzylidencamphers, wie z.B. 4-(2-Oxo-3-bornylidenmethyl)-benzolsulfonsäure und 2-Methyl-5-(2-oxo-3-bornyliden)sulfonsäure und deren Salze.

Als typische UV-A-Filter kommen insbesondere Derivate des Benzoylmethans in Frage, wie beispielsweise 1-(4'-tert.Butylphenyl)-3-(4'-methoxyphenyl)propan-1,3-dion, 4-tert.-Butyl-4'-methoxydibenzoylmethan (Parsol® 1789), 1-Phenyl-3-(4'-isopropylphenyl)-propan-1,3-dion sowie Enaminverbindungen, wie beschrieben in der DE 19712033 A1 (BASF) sowie Benzoic Acid, 2-[4-(Diethylamino)-2-Hydroxybenzoyl]-, Hexyl Ester (Uvinul® A plus).

Die UV-A und UV-B-Filter können selbstverständlich auch in Mischungen eingesetzt werden. Besonders günstige Kombinationen bestehen aus den Derivaten des Benzoylmethans, z.B. 4-tert.-Butyl-4'-methoxydibenzoylmethan (Parsol® 1789) und 2-Cyano-3,3-phenylzimtsäure-2-ethyl-hexylester (Octocrylene) in Kombination mit Estern der Zimtsäure, vorzugsweise 4-Methoxyzimtsäure-2-ethylhexylester und/oder 4-Methoxyzimtsäurepropylester und/oder 4-Methoxyzimtsäureisoamylester. Vorteilhaft werden derartige Kombinationen mit wasserlöslichen Filtern wie z.B. 2-Phenylbenzimidazol-5-sulfonsäure und deren Alkali-, Erdalkali-, Ammonium-, Alkylammonium-, Alkanolammonium- und Glucammoniumsalze kombiniert.

Als UV-Lichtschutzfilter eignen sich insbesondere die gemäß Annex VII der Kommissions Direktive (in der **Fassung Commission Directive 2005/9/EC of 28 January 2005 amending Council Directive 76/768/EEC,** concerning cosmetic products, for the purposes of adapting Annexes VII thereof to technical progress) zugelassen Stoffe, auf die hier explizit Bezug genommen wird.

Neben den genannten löslichen Stoffen kommen für diesen Zweck auch unlösliche Lichtschutzpigmente, nämlich feindisperse Metalloxide bzw. Salze in Frage. Beispiele für geeignete Metalloxide sind insbesondere Zinkoxid und Titandioxid und daneben Oxide des Eisens, Zirkoniums, Siliciums, Mangans, Aluminiums und Cers sowie deren Gemische. Als Salze können Silicate (Talk), Bariumsulfat oder Zinkstearat eingesetzt werden. Die Oxide und Salze werden in Form der Pigmente für hautpflegende und hautschützende Emulsionen und auch für die dekorative Kosmetik verwendet. Die Partikel sollten einen mittleren Durchmesser von weniger als 100 nm, vorzugsweise zwischen 5 und 50 nm und insbesondere zwischen 15 und 30 nm aufweisen. Sie können eine sphärische Form aufweisen, es können jedoch auch solche Partikel zum Einsatz kommen, die eine ellipsoide oder in sonstiger Weise von der sphärischen Gestalt abweichende Form besitzen. Die Pig-mente können auch oberflächenbehandelt, d.h. hydrophilisiert oder hydrophobiert vorliegen. Typische Beispiele sind gecoatete Titandioxide, wie z.B. Titandioxid T 805 (Degussa) oder Eusolex® T, Eusolex® T-2000, Eusolex® T-Aqua, Eusolex® AVO, Eusolex® T-ECO, Eusolex® T-OLEO und Eusolex® T-S (Merck). Typische Beispiele für sind Zinkoxide, wie z.B. Zinc Oxide neutral, Zinc Oxide NDM (Symrise) oder Z-Cote® (BASF) oder SUNZnO-AS und SUNZnO-NAS (Sunjun Chemical Co. Ltd.). Als hydrophobe Coatingmittel kommen dabei vor allem Silicone und dabei speziell Trialkoxyoctylsilane oder Simethicone in Frage. In Sonnenschutzmitteln werden bevorzugt sogenannte Mikro- oder Nanopigmente eingesetzt. Vorzugsweise wird mikronisiertes Zinkoxid verwendet. Weitere geeignete UV-Lichtschutzfilter sind der Übersicht von P.Finkel in SÖFW-Journal 122, 8/1996, S. 543-548 sowie Parf.Kosm. 80. Jahrgang, Nr. 3/1999, S. 10 bis 16 zu entnehmen.

Neben den beiden vorgenannten Gruppen primärer Lichtschutzstoffe können auch sekundäre Lichtschutzmittel vom Typ der Antioxidantien eingesetzt werden, die die photochemische Reaktionskette unterbrechen, welche ausgelöst wird, wenn UV-Strahlung in die Haut eindringt. Typische Beispiele hierfür sind Aminosäuren (z.B. Glycin, Histidin, Tyrosin, Tryptophan) und deren Derivate, Imidazole (z.B. Urocaninsäure) und deren Derivate, Peptide wie D,L-Carnosin, D-Carnosin, L-Carnosin und deren Derivate (z.B. Anserin), Carotinoide, Carotine (z.B. -Carotin, -Carotin, Lycopin) und deren Derivate, Chlorogensäure und deren Derivate, Liponsäure und deren Derivate (z.B. Dihydroliponsäure), Aurothioglucose, Propylthiouracil und andere Thiole (z.B. Thioredoxin, Glutathion, Cystein, Cystin, Cysta-min und deren Glycosyl-, N-Acetyl-, Methyl-, Ethyl-, Propyl-, Amyl-, Butyl- und Lauryl-, Palmitoyl-, Oleyl-, -Linoleyl-, Cholesteryl- und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleo-side und Salze) sowie Sulfoximinverbindungen (z.B. Buthioninsulfoximine, Homocysteinsulfoximin, Butioninsulfone, Penta-, Hexa-, Heptathioninsulfoximin) in sehr geringen verträglichen Dosierungen (z.B. pmol bis mol/kg), ferner (Metall)-Chelatoren (z.B. $\alpha$-Hydroxyfettsäure$_n$, Palmitinsäure, Phytinsäure, Lactoferrin), $\alpha$-Hydroxysäuren (z.B. Citronensäure, Milchsäure, Äpfelsäure), Huminsäure, Gallensäure, Gallenextrakte, Bilirubin, Biliverdin, EDTA, EGTA und deren Derivate, ungesättigte Fettsäuren und deren Derivate (z.B. gamma-Linolensäure, Linolsäure, Ölsäure), Folsäure und deren Derivate, Ubichinon und Ubichinol und deren Derivate, Vitamin C und Derivate (z.B. Ascorbylpalmitat, Mg-Ascor-bylphosphat, Ascorbylacetat), Tocopherole und Derivate (z.B. Vitamin-E-acetat), Vitamin A und Derivate (Vitamin-A-palmitat) sowie Koniferylbenzoat des Benzoeharzes, Rutinsäure und deren Derivate, $\alpha$-Glycosylrutin, Ferulasäure, Furfurylidenglucitol, Carnosin, Butylhydroxytoluol, Butylhydroxyanisol, Nordihydroguajakharzsäure, Nordihydro-guajaretsäure, Trihydroxybutyrophenon, Harnsäure und deren Derivate, Mannose und deren Derivate, Superoxid-Dismutase, Zink und dessen Derivate (z.B. ZnO, ZnSO4) Selen und dessen Derivate (z.B. Selen-Methionin), Stilbene und deren Derivate (z.B. Stilbenoxid, trans-Stilbenoxid) und die erfindungsgemäß geeigneten Derivate (Salze, Ester, Ether, Zucker, Nukleotide, Nukleoside, Peptide und Lipide) dieser genannten Wirkstoffe.

Ein Gegenstand der Erfindung betrifft daher kosmetische und/oder pharmazeutische Zubereitungen, enthaltend 0,1 bis 80 Gew.% eines Kohlenwasserstoff Gemischs, das $^{14}c$ Isotope enthält, und mindestens mindestens einen UV-Lichtschutzfilter ausgewählt aus der Gruppe bestehend aus 4-Methybenzylidene Camphor, Benzophenone-3, Butyl Methoxydibenzoylmethane, Bis-Ethylhexyloxyphenol Methoxyphenyl Triazine, Methylene Bis-Benzotriazolyl Tetramethylbutylphenol, Diethylhexyl Butamido Triazone, Ethylhexyl Triazone und Diethylamino Hydroxybenzoyl Hexyl Benzoate, 3-(4'-Trimethylammonium) benzyliden- bornan-2-on-methylsulfat, 3,3'-(1,4-Phenylendimethin)-bis(7,7-dimethyl-2-oxobicyclo-[2.2.1]heptan-1-methansulfonsäure) und ihre Salze, 3-(4'-Sulfo)-benzyliden-bornan-2-on und ihre Salze, Polymer von N-{(2und 4)- [2-oxoborn-3-yliden)methyl}benzyl]acrylamid, 2-(2H-Benzotriazol-2-yl)-4-methyl-6-(2-methyl-3-(1,3,3,3-tetramethyl-1-(trimethylsilyloxy) disiloxanyl)propyl) phenol, Dimethicodiethylbenzalmalonate und ihren Mischungen.

Ein Gegenstand der Erfindung betrifft daher kosmetische und/oder pharmazeutische Zubereitungen, enthaltend 0,1 bis 80 Gew.% eines Kohlenwasserstoff Gemischs, das $^{14}C$ Isotope enthält und wobei die Kohlenwasserstoffe mindestens 2 voneinander verschiedene Kohlenwasserstoffe enthalten, deren Kohlenstoff Zahl sich um mehr als 1, vorzugsweise um 2 unterscheidet, und mindestens einen UV-Lichtschutzfilter ausgewählt aus der Gruppe bestehend aus 4-Methybenzylidene Camphor, Benzophenone-3, Butyl Methoxydibenzoylmethane, Bis-Ethylhexyloxyphenol Methoxyphenyl Triazine, Methylene Bis-Benzotriazolyl Tetramethylbutylphenol, Diethylhexyl Butamido Triazone, Ethylhexyl Triazone und Diethylamino Hydroxybenzoyl Hexyl Benzoate, 3-(4'-Trimethylammonium) benzyliden- bornan-2-on-methylsulfat, 3,3'-(1,4-Phenylendimethin)-bis(7,7-dimethyl-2-oxobicyclo-[2.2.1]heptan-1-methansulfonsäure) und ihre Salze, 3-(4'-Sulfo)-benzyliden-bornan-2-on und ihre Salze, Polymer von N-{(2und 4)- [2-oxoborn-3-yliden)methyl}benzyl]acrylamid, 2-(2H-Benzotriazol-2-yl)-4-methyl-6-(2-methyl-3-(1,3,3,3-tetramethyl-1-(trimethylsilyloxy) disiloxanyl)propyl) phenol, Dimethicodiethylbenzalmalonate und ihren Mischungen.

Diese UV-Lichtschutzfilter sind beispielsweise unter den folgenden Handelsnamen kommerziell erhältlich:

NeoHeliopan®MBC (INCI: 4-Methylbenzylidene Camphor; Hersteller: Symrise); NeoHeliopan® BB (INCI: Benzophenone-3, Hersteller: Symrise); Parsol®1789 (INCI: Butyl Methoxydibenzoylmethane, Hersteller: Hoffmann-La Roche (Givaudan); Tinosorb®S (INCI: Bis-Ethylhexyloxyphenol Methoxyphenyl Triazine); Tinosorb®M (INCI: Methylene Bis-Benzotriazolyl Tetramethylbutylphenol): Herstelller: Ciba Specialty Chemicals Corporation; Uvasorb®HEB (INCI: Diethylhexyl Butamido Triazone, Hersteller: 3V Inc.), Uvinul®T 150 (INCI: Ethylhexyl Triazone, Hersteller: BASF AG); Uvinul® A plus (INCI: Diethylamino Hydroxybenzoyl Hexyl Benzoate: Hersteller: BASF AG; Mexoryl® SO: 3-(4'-Trimethylammonium) benzyliden- bornan-2-on-methylsulfat, INCI: Camphor Benzalkonium Methosulfate; Mexoryl®SX: 3,3'-(1,4-Phenylendimethin)-bis (7,7- dimethyl-2-oxobicyclo-[2.2.1] heptan-1-methansulfonsäure), CTFA: INCI Terephthalylidene Dicamphor Sulfonic Acid; Mexory® SL: 3-(4'-Sulfo)-benzyliden-bornan-2-on, INCI Benzylidene Camphor Sulfonic Acid; Mexoryl®SW: Polymer von N-{(2und 4)-[2-oxoborn-3-yliden)methyl}benzyl]acrylamid, INCI Polyacrylamidomethyl Benzylidene Camphor; Mexoryl®SL: 2-(2H-Benzotriazol-2-yl)-4-methyl-6-(2-methyl-3-(1,3,3,3-tetramethyl-1-(trimethylsilyloxy) disiloxanyl)propyl) phenol; INCI: DROMETRIZOLE TRISILOXANE; Parsol® SLX: Dimethicodiethylbenzalmalonate, INCI Polysilicone-15.

Die erfindungsgemäßen Zubereitungen können die UV-Lichtschutzfilter in Mengen von 0,5 bis 30 Gew.-%, vorzugsweise 2,5 bis 20 Gew.-%, besonders bevorzugt 5 - 15 Gew.-% - bezogen auf das Gesamtgewicht der kosmetischen und/oder pharmazeutischen Zubereitung - enthalten.

Ein Gegenstand der Erfindung betrifft kosmetische und/oder pharmazeutische Zubereitungen, enthaltend 0,1 bis 80 Gew.% eines Kohlenwasserstoff Gemischs, das $^{14}C$ Isotope enthält, und mindestens mindestens einen Selbstbräuner.

Ein bevorzugter Gegenstand der Erfindung betrifft kosmetische und/oder pharmazeutische Zubereitungen, enthaltend 0,1 bis 80 Gew.% eines Kohlenwasserstoff Gemischs, das $^{14}C$ Isotope enthält und wobei die Kohlenwasserstoffe mindestens 2 voneinander verschiedene Kohlenwasserstoffe enthalten, deren Kohlenstoff Zahl sich um mehr als 1, vorzugsweise um 2 unterscheidet, und mindestens einen Selbstbräuner.

Als Selbstbräuner sind Substanzen zu verstehen, welche eine Bräunung der Haut verursachen. Beispielsweise seien sowie alpha, beta-ungestättige Aldehyde genannt, welche mit den Aminosäuren der Haut im Sinne einer Maillard Reaktion zu gefärbten Verbindungen abreagieren. Als Wirkstoffe für Selbstbräuner kommen weiterhin in Frage natürliche oder synthetische Ketole oder Aldole. Als geeignete Wirkstoffe seien exemplarisch genannt Dihydroxyaceton, Erythrulose Glycerolaldehyd, Alloxan, Hydroxymethylglyoxal, gamma-Dialdehyd, 6-Aldo-D-Fructose, Ninhydrin und meso-Weinsäuredialdehyd. Als Selbstbräuner eignen sich insbesondere Dihydroxyaceton und/oder Erythrulose.

Als besonders vorteilhaft haben sich Mischungen der o.g. Wirkstoffe untereinander oder mit Mucondialdehyd oder/und Naphthochinone wie z. B. 5-Hydroxy-1,4-naphthochinon (Juglon) und 2-Hydroxy-1,4-Naphthochinon erwiesen. Die erfindungsgemäßen Zusammensetzungen enthalten die Selbstbräuner üblicherweise in Konzentrationen von 1 bis 10, insbesondere von 2 bis 5 Gew.-% -bezogen auf das Gesamtgewicht der kosmetischen und/oder pharmazeutischen Zubereitung.

Ein Gegenstand der Erfindung betrifft kosmetische und/oder pharmazeutische Zubereitungen, enthaltend 0,1 bis 80 Gew.% eines Kohlenwasserstoff Gemischs, das $^{14}C$ Isotope enthält, und mindestens mindestens einen Selbstbräuner und mindestens einen UV-Lichtschutzfilter.

Ein bevorzugter Gegenstand der Erfindung betrifft kosmetische und/oder pharmazeutische Zubereitungen, enthaltend 0,1 bis 80 Gew.% eines Kohlenwasserstoff Gemischs, das $^{14}C$ Isotope enthält und wobei die Kohlenwasserstoffe mindestens 2 voneinander verschiedene Kohlenwasserstoffe enthalten, deren Kohlenstoff Zahl sich um mehr als 1, vorzugsweise um 2 unterscheidet, und mindestens einen UV-Lichtschutzfilter und mindestens einen Selbstbräuner.

Die erfindungsgemäßen kosmetischen und/oder pharmazeutischen Zubereitungen können beispielsweise als O/W oder W/O Pflegeemulsionen, Sonnenschutzformulierung, AP/Deo Konzepte, Formulierungen für die dekorative Kosmetik, ölige Pflegezubereitungen, Tränkflüssigkeiten für Substrate, wie beispielsweise Papier- und Vliessprodukte vorliegen. Exemplarisch seien genannt Wet Wipes, Taschentücher, Windeln oder Hygieneprodukte.

Die erfindungsgemäßen Kohlenwasserstoff Gemische sowie die erfindungsgemäßen kosmetischen und/oder pharmazeutischen Zubereitungen, eignen sich insbesondere auch für leichte, sprühbare Anwendungen und/oder als Bestandteile von Pflegeemulsionen für Tissues, Papiere, Wipes, Schwämme (z.B. Polyurethanschwämme), Pflaster im Bereich Baby-Hygiene, Baby-Pflege, Hautpflege, Sonnenschutz, After-SunTreatment, Insektrepellent, Reinigung, Gesichtsreinigung und AP/Deo - Anwendung. Sie lassen sich auf Tissues, Papiere, Wipes, Vlies-Produkte, Schwämme, Puffs, Pflaster und Bandagen applizieren, die ihren Einsatz im Bereich der Reinigung, Hygiene und/oder Pflege finden (Feuchttücher zur Baby-Hygiene und Baby-Pflege, Reinigungstücher, Gesichtreinigungstücher, Hautpflegetücher, Pflegetücher mit Wirkstoffen gegen die Hautalterung, Wipes mit Sonnenschutzformulierungen und Insektenrepellentien

sowie Wipes zur dekorativen Kosmetik oder zum After-Sun-Treatment, Toiletten-Feuchttücher, Antitranspirant-Wipes, Windeln, Taschentücher, Wet Wipes, Hygieneprodukte, Selbstbräunungs Wipes, Toilettenpapier, Erfrischungstücher, After-shave Tücher). Sie lassen sich u.a. auch in Zubereitungen zur Haarpflege, Haarreinigung oder Haarfärbung einsetzen. Durch den Einsatz der erfindungsgemäßen Kohlenwasserstoff Gemische wird das sensorische Verhalten bei Applikation positiv beeinflusst

Die erfindungsgemäßen Kohlenwasserstoff Gemische eignen sich insbesondere als Bestandteile von Zubereitungen der dekorativen Kosmetik, wie beispielsweise Lippenstifte, Augen-Make up, wie beispielsweise Lidschatten, Mascara, Lidstifte, Kajal, Nagellack, etc. sowie Make-up Formulierungen.

Ein Gegenstand der Erfindung betrifft kosmetische und/oder pharmazeutische Zubereitungen, enthaltend 0,1 bis 80 Gew.% eines Kohlenwasserstoff Gemischs, das $^{14}C$ Isotope enthält, und mindestens mindestens ein Pigment und/oder einen Farbstoff.

Ein bevorzugter Gegenstand der Erfindung betrifft kosmetische und/oder pharmazeutische Zubereitungen, enthaltend 0,1 bis 80 Gew.% eines Kohlenwasserstoff Gemischs, das $^{14}C$ Isotope enthält und wobei die Kohlenwasserstoffe mindestens 2 voneinander verschiedene Kohlenwasserstoffe enthalten, deren Kohlenstoff Zahl sich um mehr als 1, vorzugsweise um 2 unterscheidet, und mindestens ein Pigment und/oder einen Farbstoff.

Der Begriff Pigment umfasst Partikel jeder Form, die weiß oder gefärbt, organisch oder anorganisch sind, in den Zubereitungen nicht löslich sind, und dem Zweck dienen die Zubereitung zu färben.
In einer bevorzugten Ausführungsform werden anorganische Pigmente verwendet, besonders bevorzugt sind Metalloxide.
Als anorganische Pigmente seien exemplarisch genannt: Titandioxid, optional oberflächenbeschichtet, Zirkonium oder Ceriumoxide und Zink-, Eisen- (Schwarz, Gelb oder Rot) und Chrom-oxide, Manganviolett, Ultramarine Blau, Chromhydroate und Eisen(III)blau, Metallpulver wie Aluminiumpulver oder Kupferpulver.
In einer bevorzugten Ausführungsform der Erfindung ist das Pigment ausgewählt aus den anorganischen Pigmenten, vorzugweise aus den Metalloxiden. In einer bevorzugten Ausführungsform ist das Pigment ausgewählt aus der Gruppe bestehend aus Titandioxid, Zinkoxid, Eisenoxid und Mischungen daraus.
Die Pigmente können sowohl einzeln als auch in Mischungen vorliegen.

Bevorzugt im Sinne der vorliegenden Erfindung sind Pigmentmischungen aus Weiss-Pigmenten (z. B. Kaolin, Titandioxid oder Zinkoxid) und anorganischen Farbpigmenten (z. B. Eisenoxid Pigmente, Chromoxide), wobei die Pigmente beschichtet ("gecoatet") oder unbeschichtet vorliegen koennen. Unter den Farbpigmenten sind Eisenoxide besonders bevorzugt.
Vorteilhaft im Sinne der vorliegenden Erfindung können das oder die Pigmente auch aus der Gruppe der Effektpigmente gewählt werden, welche der kosmetischen Zubereitung neben der reinen Farbe eine zusätzliche Eigenschaft - wie z. B. eine Winkelabhängigkeit der Farbe (changieren, Flop), Glanz (nicht Oberflächenglanz) oder Textur - verleihen. Solche Effektpigmente werden erfindungsgemäss vorteilhaft zusätzlich zu einem oder mehreren Weiss- und/oder Farbpigmenten eingesetzt.

Die bedeutendste Gruppe der Effektpigmente stellen die Glanzpigmente dar, zu denen nach **DIN 55944: 2003-11** die Metalleffektpigmente und die Perlglanzpigmente gehören. Einige spezielle Effektpigmente lassen sich diesen beiden Gruppen nicht zuordnen, z. B. plättchenfoermiges Graphit, plättchenförmiges Eisenoxid und mikronisiertes Titandioxid, wobei mikronisiertes Titandioxid keinen Glanzeffekt, sondern einen winkelabhängigen Lichtstreueffekt erzeugt. Bei den Glanzpigmenten nach DIN 55943: 2001-10 handelt es sich vorwiegend um plättchenfoermige Effektpigmente. Parallel orientiert zeigen Glanzpigmente einen charakteristischen Glanz. Die optische Wirkung von Glanzpigmenten beruht auf der gerichteten Reflexion an metallischen Teilchen (Metalleffektpigmente), an transparenten Teilchen mit hoher Brechzahl (Perlglanzpigmente) oder auf dem Phaenomen der Interferenz (Interferenzpigmente) (DIN 55944: 2003-11).

Beispiele für erfindungsgemäss bevorzugte handelsübliche Effektpigmente sind: Timiron and #174; von Merck, Iriodin and #174; von Merck (Perl- und Farbglanzpigmente für dekorative technische Anwendungen), Xirallic and #174; von Merck (farbintensive Kristalleffektpigmente).
Ferner können die erfindungsgemässen Zubereitungen vorteilhaft auch organische Farbpigmente enthalten, d. h. organische Farbstoffe, welche in der Zubereitung praktisch unlöslich sind. Nach DIN 55944: 1990-04 können organische Pigmente nach chemischen Gesichtspunkten in Azopigmente und polycyclische Pigmente sowie nach farblichen Gesichtspunkten in Bunt- oder Schwarzpigmente eingeteilt werden. Organische Weisspigmente sind ohne praktische Bedeutung.
Die Pigmente können vorteilhaft im Sinne der vorliegenden Erfindung auch in Form kommerziell erhältlicher öliger oder wässriger Vordispersionen zur Anwendung kommen. Die erfindungsgemäßen Zubereitungen enthalten üblicherweise 0,1 bis 40 Gew.-% Pigmente - bezogen auf das Gesamtgewicht der kosmetischen und/oder pharmazeutischen Zubereitung. Es ist ferner vorteilhaft im Sinne der vorliegenden Erfindung, wenn die erfindungsgemässe Zubereitung einen oder mehrere Farbstoffe enthält.

Die Farbstoffe können sowohl synthetischen als auch natürlichen Ursprungs sein.

Eine Liste von geeigneten Farbstoffen findet sich in EP 1 371 359 A2**,** S.8, Z. 25-57, S.9 und S.10 sowie S. 11, Z. 1 bis 54, auf welche hiermit explizit Bezug genommen wird.

Die erfindungsgemäßen Zubereitungen enthalten üblicherweise 0,01 bis 5, vorzugsweise 0,1 bis 1,0 Gew.-% Farbstoffebezogen auf das Gesamtgewicht der kosmetischen und/oder pharmazeutischen Zubereitung. Die erfindungsgemäßen Zubereitungen enthalten üblicherweise eine Gesamtmenge an Farbstoffen und Pigmenten im Bereich von 0,01 bis 30 Gew.-%, insbesonderen 0,1 bis 15 Gew.-%, vorzugsweise 1 bis 10 Gew.-%, bezogen auf das Gesamtgewicht der kosmetischen und/oder pharmazeutischen Zubereitung.

Als Farbstoffe und Pigmente eignen sich insbesondere die gemäß Annex IV der Kommissions Direktive zugelassenen Farbstoffe und Pigmente (in der Fassung: **Commission Directive 2007/22/EC of 17 April 2007 amending Council Directive 76/768/EEC,** concerning cosmetic products, for the purposes of adapting Annexes IV and VI thereto to technical progress) zugelassen Stoffe, auf die hiermit explizit Bezug genommen wird.

Die kosmetischen und/oder pharmazeutischen Zubereitungen können Formulierungen zur Körperpflege sein, z. B. eine Körpermilch, Cremes, Lotionen, sprühbare Emulsionen, Produkte zur Eliminierung des Körpergeruchs etc. Die Kohlenwasserstoff Gemische lassen sich auch in tensidhaltigen Formulierungen wie z. B. Schaum- und Duschbädern, Haarshampoos und Pflegespülungen einsetzen. Je nach Applikationszweck enthalten die kosmetischen und/oder pharmazeutischen Zubereitungen eine Reihe weiterer Hilfs- und Zusatzstoffe, wie beispielsweise Tenside, weitere Ölkörper, Emulgatoren, Perlglanzwachse, Konsistenzgeber, Verdickungsmittel, Überfettungsmittel, Stabilisatoren, Polymere, Fette, Wachse, Lecithine, Phospholipide, biogene Wirkstoffe, Antischuppenmittel, Filmbildner, Quellmittel, Insektenrepellentien, Selbstbräuner, Tyrosinaseinhibitoren (Depigmentierungsmittel), Hydrotrope, Solubilisatoren, Konservierungsmittel, Parfümöle, Farbstoffe etc., die nachstehend exemplarisch aufgelistet sind.

Ein Gegenstand der Erfindung betrifft kosmetische und/oder pharmazeutische Zubereitungen, enthaltend 0,1 bis 80 Gew.% eines Kohlenwasserstoff Gemischs, das $^{14}C$ Isotope enthält, und mindestens einen Emulgator und/oder ein Tensid und/oder eine Wachskomponente und/oder ein Polymer und/oder einen weiteren Ölkörper.

Ein bevorzugter Gegenstand der Erfindung betrifft kosmetische und/oder pharmazeutische Zubereitungen, enthaltend 0,1 bis 80 Gew.% eines Kohlenwasserstoff Gemischs, das $^{14}C$ Isotope enthält und wobei die Kohlenwasserstoffe mindestens 2 voneinander verschiedene Kohlenwasserstoffe enthalten, deren Kohlenstoff Zahl sich um mehr als 1, vorzugsweise um 2 unterscheidet, und mindestens einen Emulgator und/oder ein Tensid und/oder eine Wachskomponente und/oder ein Polymer und/oder einen weiteren Ölkörper.

<u>Emulgator</u>

In einer Ausführungsform der Erfindung enthalten die erfindungsgemäßen Zubereitungen mindestens einen Emulgator.

Ein Gegenstand der Erfindung betrifft kosmetische und/oder pharmazeutische Zubereitungen, enthaltend 0,1 bis 80 Gew.% eines Kohlenwasserstoff Gemischs, das $^{14}C$ Isotope enthält, und mindestens einen Emulgator.

Ein bevorzugter Gegenstand der Erfindung betrifft kosmetische und/oder pharmazeutische Zubereitungen, enthaltend 0,1 bis 80 Gew.% Kohlenwasserstoffe, eines Kohlenwasserstoff Gemischs, das $^{14}C$ Isotope enthält und wobei die Kohlenwasserstoffe mindestens 2 voneinander verschiedene Kohlenwasserstoffe enthalten, deren Kohlenstoff Zahl sich um mehr als 1, vorzugsweise um 2 unterscheidet, und mindestens einen Emulgator.

Die erfindungsgemäßen Zusammensetzungen enthalten den/die Emulgator(en) üblicherweise in einer Menge von 0 bis 40 Gew.-%, vorzugsweise 0,1 bis 20 Gew.-% vorzugsweise 0,1 bis 15 Gew.-% und insbesondere 0,1 bis 10 Gew.-% bezogen auf das Gesamtgewicht der Zusammensetzung.

Jedem Emulgator wird ein sogenannter HLB-Wert (eine dimensionslose Zahl zwischen 0 und 20) zugeschrieben, der angibt, ob eine bevorzugte Wasser-oder Öllöslichkeit vorliegt. Zahlen unter 9 kennzeichnen bevorzugt öllösliche, hydrophobe Emulgatoren, Zahlen über 11 wasserlösliche, hydrophile Emulgatoren.

Der HLB-Wert sagt etwas über das Gleichgewicht der Grösse und Stärke der hydrophilen und der lipophilen Gruppen eines Emulgators aus.

Der HLB-Wert eines Emulgators lässt sich auch aus Inkrementen errechnen, wobei die HLB-Inkremente für die verschiedenen hydrophilen und hydrophoben Gruppen, aus denen sich ein

Molekül zusammensetzt. In der Regel kann er Tabellenwerken (z. B. H. P. Fiedler, Lexikon der Hilfsstoffe für Pharmazie, Kosmetik und angrenzende Gebiete, Editio Cantor Verlag, Aulendorf, 4. Aufl. 1996) oder den Herstellerangaben entnommen werden. Die Löslichkeit des Emulgators in den beiden Phasen bestimmt praktisch den Emulsionstyp. Ist der Emulgator besser in Wasser löslich erhält man eine O/W-Emulsion. Hat der Emulgator hingegen eine bessere Löslichkeit in der Öiphase entsteht unter sonst gleichen Herstellungsbedingungen eine W/O-Emulsion.

In einer Ausführungsform der Erfindung enthält die erfindungsgemäße Zubereitung mehr als einen Emulgator. Der Fachmann setzt in Abhängigkeit der übrigen Komponenten übliche Emulgator Systeme (wie z.B. Emulgator und Co-Emulgator) ein.

Nicht-ionische Emulgatoren

Zur Gruppe der nicht-ionischen Emulgatoren gehören beispielsweise:

(1) Anlagerungsprodukte von 2 bis 50 Mol Ethylenoxid und/oder 1 bis 20 Mol Propylenoxid an lineare Fettalkohole mit 8 bis 40 C-Atomen, an Fettsäuren mit 12 bis 40 C-Atomen und an Alkylphenole mit 8 bis 15 C-Atomen in der Alkylgruppe.
(2) $C_{12}$-$C_{18}$-Fettsäuremono- und -diester von Anlagerungsprodukten von 1 bis 50 Mol Ethylenoxid an Glycerin.
(3) Sorbitanmono- und -diester von gesättigten und ungesättigten Fettsäuren mit 6 bis 22 Kohlenstoffatomen und deren Ethylenoxidanlagerungsprodukte.
(4) Alkylmono- und -oligoglycoside mit 8 bis 22 Kohlenstoffatomen im Alkylrest und deren ethoxylierte Analoga.
(5) Anlagerungsprodukte von 7 bis 60 Mol Ethylenoxid an Ricinusöl und/oder gehärtetes Ricinusöl.
(6) Polyol- und insbesondere Polyglycerinester, wie z. B. Polyolpoly-12-hydroxystearate, Polyglycerinpolyricinoleat, Polyglycerindiisostearat oder Polyglycerindimerat. Ebenfalls geeignet sind Gemische von Verbindungen aus mehreren dieser Substanzklassen.
(7) Anlagerungsprodukte von 2 bis 15 Mol Ethylenoxid an Ricinusöl und/oder gehärtetes Ricinusöl.
(8) Partialester auf Basis linearer, verzweigter, ungesättigter bzw. gesättigter $C_6$-$C_{22}$-Fettsäuren, Ricinolsäure sowie 12-Hydroxystearinsäure und Polyglycerin, Pentaerythrit, Dipentaerythrit, Zuckeralkohole (z. B. Sorbit), Alkylglucoside (z. B. Methylglucosid, Butylglucosid, Laurylglucosid) sowie Polyglucoside (z. B. Cellulose), oder Mischester wie z. B. Glycerylstearatcitrat und Glycerylstearatlactat.
(9) Polysiloxan-Polyalkyl-Polyether-Copolymere bzw. entsprechende Derivate.
(10) Mischester aus Pentaerythrit, Fettsäuren, Citronensäure und Fettalkohol und/oder Mischester von Fettsäuren mit 6 bis 22 Kohlenstoffatomen, Methylglucose und Polyolen, vorzugsweise Glycerin oder Polyglycerin.

Die Anlagerungsprodukte von Ethylenoxid und/oder von Propylenoxid an Fettalkohole, Fettsäuren, Alkylphenole, Glycerinmono- und -diester sowie Sorbitanmono- und -diester von Fettsäuren oder an Ricinusöl stellen bekannte, im Handel erhältliche Produkte dar. Es handelt sich dabei um Homologengemische, deren mittlerer Alkoxylierungsgrad dem Verhältnis der Stoffmengen von Ethylenoxid und/oder Propylenoxid und Substrat, mit denen die Anlagerungsreaktion durchgeführt wird, entspricht. Je nach Ethoxylierungsgrad handelt es sich um W/O- oder O/W-Emulgatoren. $C_{12/18}$-Fettsäuremono- und -diester von Anlagerungsprodukten von Ethylenoxid an Glycerin sind als Rückfettungsmittel für kosmetische Zubereitungen bekannt.

Erfindungsgemäß besonders gut geeignete und milde Emulgatoren sind Polyolpoly-12-hydroxystearate und Abmischungen davon, welche beispielsweise unter den Marken "Dehymuls® PGPH" (W/O-Emulgator) oder "Eumulgin® VL 75" (Abmischung mit Lauryl Glucosides im Gewichtsverhältnis 1:1, O/W-Emulgator) oder Dehymuls® SBL (W/O-Emulgator) von der Cognis Deutschland GmbH vertrieben werden. In diesem Zusammenhang sei insbesondere auf das Europäische Patent EP 766 661 B1 verwiesen. Die Polyolkomponente dieser Emulgatoren kann sich von Stoffen ableiten, die über mindestens zwei, vorzugsweise 3 bis 12 und insbesondere 3 bis 8 Hydroxylgruppen und 2 bis 12 Kohlenstoffatome verfügen.

Besonders bevorzugte Emulgatoren sind z. B. Cetyl Dimethicone Copolyol (z.B. Abil EM-90), Polyglyceryl-2 Dipolyhydroxystearate (z.B. Dehymuls PGPH), Polyglycerin-3-Diisostearate (z.B. Lameform TGI), Polyglyceryl-4 Isostearate (z.B. Isolan GI 34), Polyglyceryl-3 Oleate (z.B. Isolan GO 33), Diisostearoyl Polyglyceryl-3 Diisostearate (z.B. Isolan PDI), Polyglyceryl-3 Methylglucose Distearate (z.B. Tego Care 450), Polyglyceryl-3 Beeswax (z.B. Cera Bellina), Polyglyceryl-4 Caprate (z.B. Polyglycerol Caprate T2010/90), Polyglyceryl-3 Cetyl Ether (z.B. Chimexane NL), Polyglyceryl-3 Distearate (z. B. Cremophor GS 32) und Polyglyceryl Polyricinoleate (z.B. Admul WOL 1403), Glyceryl Oleate (z.B. Monomuls 90-O 18), Alkyl Glucoside (z.B. Plantacare 1200, Emulgade PL 68/50, Montanov 68, Tego Care CG 90, Tego Glucosid L 55), Methyl Glucose Isostearate (z.B. Tego Care IS), Methyl Glucose Sesquistearate (Tego Care PS), Sodium Cocoyl Hydrolyzed Wheat Protein (z.B. Gluadin WK), Potassium Cetyl Phosphate (z.B. Amphisol K, Crodafos CKP), Sodium Alkylsulfate (z.B. Lanette E), Sucrose Ester (z.B. Crodesta F-10, F-20, F-50, F-70, F-110, F-160, SL-40, Emulgade® Sucro), ethoxylierte und/oder propoxylierte Fettalkohole Fettsäuren, Rizinusöle bzw. hydrierte Rizinusöle (z.B. Eumulgin B2, B2, B3, L, HRE 40, HRE 60, RO 40, Cremophor HRE 40, HRE 60, L, WO 7, Dehymuls HRE 7, Arlacel 989), PEG-30 Dipolyhydroxystearate (z.B. Arlacel P 135, Dehymuls LE), Sorbitan Ester, Sorbitan Ester ethoxyliert und/oder propoxyliert sowie deren Gemische. Ein besonders effektives Gemisch besteht aus Polyglyceryl-2 Dipolyhydroxystearate und Lauryl Glucoside und Glycerin (z.B. Eumulgin VL 75). Geeignet sind weiterhin Polyglyceryl-4 Diisostearate/ Polyhydroxystearate/Sebacate (Isolan® GPS), Diisostearoyl Polyglyceryl-3 Diisostearate (z. B. Isolan PDI), Alkalisalze Acylglutamate (z.B. Eumulgin SG).

Als lipophile W/O-Emulgatoren eignen sich prinzipiell Emulgatoren mit einem HLB-Wert von 1 bis 8, die in zahlreichen Tabellenwerken zusammengefaßt und dem Fachmann bekannt sind. Einige dieser Emulgatoren sind beispielsweise in Kirk-Othmer,"Encyclopedia of Chemical Technology", 3. Aufl., 1979, Band 8, Seite 913, aufgelistet.

Für ethoxylierte Produkte lässt sich der HLB-Wert auch nach folgender Formel berechnen: HLB = (100 - L) : 5, wobei L der Gewichtsanteil der lipophilen Gruppen, d. h. der Fettalkyl- oder Fettacylgruppen in Gewichtsprozent, in den Ethylenoxidaddukten ist.

Besonders vorteilhaft aus der Gruppe der W/O-Emulgatoren sind Partialester von Polyolen, insbesondere von $C_4$-$C_6$-Polyolen, wie beispielsweise Partialester des Pentaerythrits oder Zuckerestern, z. B. Saccharosedistearat, Sorbitanmonoisostearat, Sorbitansesquiisostearat, Sorbitandiisostearat, Sorbitantriisostearat, Sorbitanmonooleat, Sorbitansesquioleat, Sorbitandioleat, Sorbitantrioleat, Sorbitanmonoerucat, Sorbitansesquierucat, Sorbitandierucat, Sorbitantrierucat, Sorbitanmonoricinoleat, Sorbitansesquiricinoleat, Sorbitandiricinoleat, Sorbitantriricinoleat, Sorbitanmonohydroxystearat, Sorbitansesquihydroxystearat, Sorbitandihydroxystearat, Sorbitantrihydroxystearat, Sorbitanmonotartrat, Sorbitansesquitartrat, Sorbitanditartrat, Sorbitantritartrat, Sorbitanmonocitrat, Sorbitansesquicitrat, Sorbitandicitrat, Sorbitantricitrat, Sorbitanmonomaleat, Sorbitansesquimaleat, Sorbitandimaleat, Sorbitantrimaleat sowie deren technische Gemische. Als Emulgatoren geeignet sind auch Anlagerungsprodukte von 1 bis 30, vorzugsweise 5 bis 10 Mol Ethylenoxid an die genannten Sorbitanester.

Je nach Formulierung kann es vorteilhaft sein, zusätzlich wenigstens einen Emulgator aus der Gruppe nichtionischer O/W-Emulgatoren (HLB-Wert: 8 - 18) und/oder Solubilisatoren einzusetzen. Hierbei handelt es sich beispielsweise um die bereits einleitend erwähnten Ethylenoxid-Addukte mit einem entsprechend hohen Ethoxylierungsgrad, z. B. 10 - 20 Ethylenoxid-Einheiten für O/W-Emulgatoren und 20 - 40 Ethylenoxid-Einheiten für sogenannte Solubilisatoren. Erfindungsgemäß besonders vorteilhaft als O/W-Emulgatoren sind Ceteareth-12 und PEG-20 Stearat. Als Solubilisatoren bevorzugt geeignet sind Eumulgin® HRE 40 (INCI: PEG-40 Hydrogenated Castor Oil), Eumulgin® HRE 60 (INCI: PEG-60 Hydrogenated Castor Oil), Eumulgin® L (INCI: PPG-1-PEG-9 Laurylglycolether), sowie Eumulgin® SML 20 (INCI: Polysorbat-20).

Nicht-ionische Emulgatoren aus der Gruppe der Alkyloligoglycoside sind besonders hautfreundlich und daher bevorzugt als O/W-Emulgatoren geeignet. $C_8$-$C_{22}$-Alkylmono- und - oligoglycoside, ihre Herstellung und ihre Verwendung sind aus dem Stand der Technik bekannt. Ihre Herstellung erfolgt insbesondere durch Umsetzung von Glucose oder Oligosacchariden mit primären Alkoholen mit 8 bis 22 C-Atomen. Bezüglich des Glycosidrestes gilt, dass sowohl Monoglycoside, bei denen ein zyklischer Zuckerrest glycosidisch an den Fettalkohol gebunden ist, als auch oligomere Glycoside mit einem Oligomerisationsgrad bis vorzugsweise etwa 8 geeignet sind. Der Oligomerisierungsgrad ist dabei ein statistischer Mittelwert, dem eine für solche technischen Produkte übliche Homologenverteilung zugrunde liegt. Produkte, die unter der Bezeichnung Plantacare® zur Verfügung stehen, enthalten eine glucosidisch gebundene $C_8$-$C_{16}$-Alkylgruppe an einem Oligoglucosidrest, dessen mittlerer Oligomerisationsgrad bei 1 bis 2 liegt. Auch die vom Glucamin abgeleiteten Acylglucamide sind als nicht-ionische Emulgatoren geeignet. Erfindungsgemäß bevorzugt ist ein Produkt, das unter der Bezeichnung Emulgade® PL 68/50 von der Cognis Deutschland GmbH vertrieben und ein 1:1-Gemisch aus Alkylpolyglucosiden und Fettalkoholen darstellt. Erfindungsgemäß vorteilhaft einsetzbar ist auch ein Gemisch aus Lauryl Glucoside, Polyglyceryl-2-Dipolyhydroxystearate, Glycerin und Wasser, das unter der Bezeichnung Eumulgin® VL 75 im Handel ist.

Als Emulgatoren kommen weiterhin Substanzen, wie Lecithine und Phospholipide in Frage. Als Beispiele für natürliche Lecithine seien die Kephaline genannt, die auch als Phosphatidsäuren bezeichnet werden und Derivate der 1,2-Diacyl-sn-glycerin-3-phosphorsäuren darstellen. Dem gegenüber versteht man unter Phospholipiden gewöhnlich Mono- und vorzugsweise Diester der Phosphorsäure mit Glycerin (Glycerinphosphate), die allgemein zu den Fetten gerechnet werden. Daneben kommen auch Sphingosine bzw. Sphingolipide in Frage.

Als Emulgatoren können beispielsweise Silikonemulgatoren enthalten sein. Diese können beispielsweise aus der Gruppe der Alkylmethicon-copolyole und/oder Alkyl-Dimethiconcopolyole gewählt werden, insbesondere aus der Gruppe der Verbindungen, welche gekennzeichnet sind durch die folgende chemische Struktur:

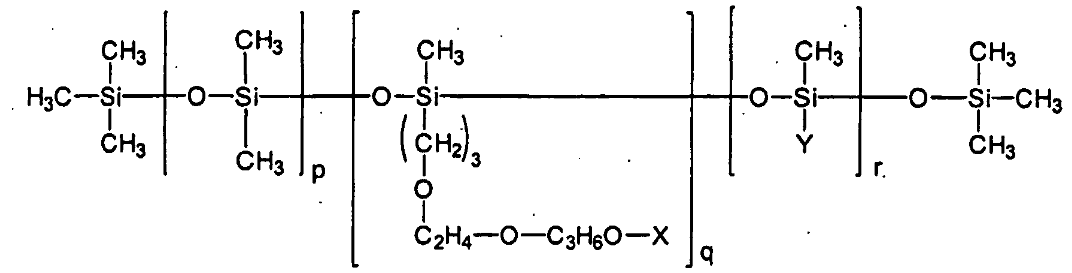

bei welcher X und Y unabhängig voneinander gewählt werden aus der Gruppe H (Wasserstoff) sowie der verzweigten und unverzweigten Alkylgruppen, Acylgruppen und Alkoxygruppen mit 1-24 Kohlenstoffatomen, p eine Zahl von 0-200 darstellt, q eine Zahl von 1-40 darstellt, und r eine Zahl von 1-100 darstellt.

Ein Beispiel für besonders vorteilhaft im Sinne der vorliegenden Erfindung zu verwendende Silikonemulgatoren sind Dimethiconcopolyole, welche von Evonik Goldschmidt unter den Warenbezeichnungen AXIL® B 8842, ABIL® B 8843, ABIL® B 8847, ABIL® B 8851, ABIL® B 8852, ABIL® B 8863, ABIL® B 8873 und ABIL®B 88183 verkauft werden.

Ein weiteres Beispiel für besonders vorteilhaft im Sinne der vorliegenden Erfindung zu verwendende grenzflächenaktiven Substanzen ist das Cetyl PEG/PPG-10/1 Dimethicone (Cetyl Dimethiconcopolyol), welches von EvonikGoldschmidt unter der Warenbezeichnung ABIL® EM 90 verkauft wird.

Ein weiteres Beispiel für besonders vorteilhaft im Sinne der vorliegenden Erfindung zu verwendende grenzflächenaktiven Substanzen ist das Cyclomethicon Dimethiconcopo- lyol, welches von Evonik Goldschmidt unter der Warenbezeichnung ABIL®EM 97 und ABIL®WE 09 verkauft wird.

Weiterhin hat sich als ganz besonders vorteilhaft der Emulgator Lauryl PEG/PPG-18/18 Methicone (Laurylmethiconcopolyol) herausgestellt, welcher unter der Warenbezeichnung Dow Corning® 5200 Formulation Aid von der Gesellschaft Dow Corning Ltd. erhältlich ist.

Ein weiterer vorteilhafter Silikonemulgator ist, Octyl Dimethicon Ethoxy Glucosid der Firma Wacker.
Für eine erfindungsgemäße Wasser-in-Silikonöl-Emulsion können alle bekannten für diesen Emulsionstyp verwendeten Emulgatoren eingesetzt werden. Erfindungsgemäß besonders bevorzugte Wasser-in-Silikon-Emulgatoren sind dabei Cetyl PEG/PPG- 10/1 Dimethicone und Lauryl PEG/PPG-18/18 Methicone [z. B. ABIL® EM 90 Evonik Goldschmidt), DC5200 Formulation Aid (Dow Corning) ] sowie beliebige Mischungen aus beiden Emulgatoren.

Tenside

In einer Ausführungsform der Erfindung enthalten die erfindungsgemäßen Zubereitungen mindestens ein Tensid.

Tenside sind amphiphile Stoffe, die organische, unpolare Substanzen in Wasser lösen können. Sie sorgen, bedingt durch ihren spezifischen Molekülaufbau mit mindestens einem hydrophilen und einem hydrophoben Molekuelteil, für eine Herabsetzung der Oberflächenspannung des Wassers, die Benetzung der Haut, die Erleichterung der Schmutzentfernung und -lösung, ein leichtes Abspülen und - je nach Wunsch- für Schaumregulierung.

Als Tenside werden üblicherweise oberflächen aktive Substanzen verstanden, die einen HLB-Wert von größer 20 aufweisen.

Ein Gegenstand der Erfindung betrifft kosmetische und/oder pharmazeutische Zubereitungen, enthaltend 0,1 bis 80 Gew.-% eines Kohlenwasserstoff Gemischs, das $^{14}C$ Isotope enthält, und mindestens ein Tensid.

Ein bevorzugter Gegenstand der Erfindung betrifft kosmetische und/oder pharmazeutische Zubereitungen, enthaltend 0,1 bis 80 Gew.-% eines Kohlenwasserstoff Gemischs, das $^{14}C$ Isotope enthält und wobei die Kohlenwasserstoffe mindestens 2 voneinander verschiedene Kohlenwasserstoffe enthalten, deren Kohlenstoff Zahl sich um mehr als 1, vorzugsweise um 2 unterscheidet, und mindestens ein Tensid.

Als oberflächenaktive Stoffe können anionische, nichtionische, kationische und/oder amphotere bzw. zwitterionische Tenside enthalten sein. In tensidhaltigen kosmetischen Zubereitungen, wie beispielsweise Duschgelen, Schaumbädern, Shampoos etc. ist vorzugsweise wenigstens ein anionisches Tensid enthalten.

Die erfindungsgemäßen Zusammensetzungen enthalten den/die Tenside üblicherweise in einer Menge von 0 bis 40 Gew.-%., vorzugsweise 0,05 bis 30 Gew.-% insbesondere 0,05 bis 20 Gew.-%, vorzugsweise 0,1 bis 15 Gew.-% und insbesondere 0,1 bis 10 Gew.-% bezogen auf das Gesamtgewicht der Zusammensetzung.

Typische Beispiele für nichtionische Tenside sind Fettalkoholpolyglycolether, Alkylphenolpolyglycolether, Fettsäurepolyglycolester, Fettsäureamidpolyglycolether, Fettamin-polyglycolether, alkoxylierte Triglyceride, Mischether bzw. Mischformale, gegebenenfalls partiell oxidierte Alk(en)yloligoglykoside bzw. Glucoronsäurederivate, Fettsäure-N-alkylglucamide, Proteinhydrolysate (insbesondere pflanzliche Produkte auf Weizenbasis), Polyolfettsäureester, Zuckerester, Sorbitanester, Polysorbate und Aminoxide. Sofern die nichtionischen Tenside Polyglycoletherketten enthalten, können diese eine konventionelle, vorzugsweise jedoch eine eingeengte Homologenverteilung aufweisen.

Als zwitterionische Tenside werden solche oberflächenaktiven Verbindungen bezeichnet, die im Molekül mindestens eine quartäre Ammoniumgruppe und mindestens eine $-COO^{(-)}$- oder - $SO_3^{(-)}$-Gruppe tragen. Besonders geeignete zwitterionische Tenside sind die so genannten Betaine wie die N-Alkyl-N,N-dimethylammoniumglycinate, beispielsweise das Kokosalkyldimethylammoniumglycinat, N-Acyl-aminopropyl-N,N-dimethylammoniumglycinate, beispielsweise das Kokosacylaminopropyldimethylammoniumglycinat, und 2-Alkyl-3-car-boxylmethyl-3-hydroxyethylimidazolin mit jeweils 8 bis 18 C-Atomen in der Alkyl- oder Acylgruppe sowie das Kokosacylaminoethylhydroxyethylcarboxymethylglycinat. Ein bevorzugtes zwitterionisches Tensid ist das unter der INCI-Bezeichnung Cocamidopropyl Betaine bekannte Fettsäureamid-Derivat.

Ebenfalls, insbesondere als Co-Tenside geeignet, sind ampholytische Tenside. Unter ampholytischen Tensiden werden solche oberflächenaktiven Verbindungen verstanden, die außer einer $C_8$-$C_{18}$-Alkyl- oder Acylgruppe im Molekül mindestens eine freie Aminogruppe und mindestens eine -COOH- oder $-SO_3H$-Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N- Alkylsarcosine, 2-Alkylaminopropionsäuren und Alkylaminoessigsäuren mit jeweils etwa 8 bis 18 C-Atomen in der Alkylgruppe. Besonders bevorzugte ampholytische Tenside sind das N-Kokosalkylaminopropionat, das Kokosacylaminoethylaminopropionat und das $C_{12-18}$-Acylsarcosin.

Typische Beispiele für amphotere bzw. zwitterionische Tenside sind Alkylbetaine, Alkylamidobetaine, Aminopropionate, Aminoglycinate, Imidazoliniumbetaine und Sulfobetaine. Bei den genannten Tensiden handelt es sich ausschließlich um bekannte Verbindungen. Hinsichtlich Struktur und Herstellung dieser Stoffe sei auf einschlägige Übersichtsarbeiten auf diesem Gebiet verwiesen. Typische Beispiele für besonders geeignete milde, d.h. besonders hautverträgliche Tenside sind Fettalkoholpolyglycolethersulfate, Monoglyceridsulfate, Mono- und/oder Dialkylsulfosuccinate, Fettsäureisethionate, Fettsäuresarcosinate, Fettsäuretauride, Fettsäureglutamate, $\alpha$-Olefinsulfonate, Ethercarbonsäuren, Alkyloligoglucoside und/ oder deren Gemische mit Alkyloligoglucosidcarboxylaten, Fettsäureglucamide, Alkylamidobetaine, Amphoacetale und/oder Proteinfettsäurekondensate, letztere vorzugsweise auf Basis von Weizenproteinen bzw. deren Salzen.

Anionische Tenside sind gekennzeichnet durch eine wasserlöslich machende, anionische Gruppe wie z. B. eine Carboxylat-, Sulfat-, Sulfonat- oder Phosphat-Gruppe und einen lipophilen Rest. Hautverträgliche anionische Tenside sind dem Fachmann in großer Zahl aus einschlägigen Handbüchern bekannt und im Handel erhältlich. Es handelt sich dabei insbesondere um Alkylsulfate in Form ihrer Alkali-, Ammonium- oder Alkanolammoniumsalze, Alkylethersulfate, Alkylethercarboxylate, Acylisethionate, Acylsarkosinate, Acyltaurine mit linearen Alkyl- oder Acylgruppen mit 12 bis 18 C-Atomen sowie Sulfosuccinate und Acylglutamate in Form ihrer Alkali- oder Ammoniumsalze.

Typische Beispiele für anionische Tenside sind Seifen, Alkylbenzolsulfonate, Alkansulfonate, Olefinsulfonate, Alkylethersulfonate, Glycerinethersulfonate, $\alpha$-Methylestersulfonate, Sulfofettsäuren, Alkylsulfate, Fettalkoholethersulfate, Glycerinethersulfate, Fettsäureethersulfate, Hydroxymisch-ethersulfate, Monoglycerid(ether)sulfate, Fettsäureamid(ether)sulfate, Mono- und Dialkylsulfosuccinate, Mono- und Dialkylsulfosuccinamate, Sulfotriglyceride, Amidseifen, Ethercarbonsäuren und deren Salze, Fettsäureisethionate, Fettsäuresarcosinate, Fettsäuretauride, N-Acylaminosäuren, wie beispielsweise Acyllactylate, Acyltartrate, Acylglutamate und Acylaspartate, Alkyloligoglucosidsulfate, Proteinfettsäurekondensate (insbesondere pflanzliche Produkte auf Weizenbasis) und Alkyl(ether)phosphate. Sofern die anionischen Tenside Polyglycoletherketten enthalten, können diese eine konventionelle, vorzugsweise jedoch eine eingeengte Homologenverteilung aufweisen.

Als kationische Tenside sind insbesondere quartäre Ammoniumverbindungen verwendbar. Bevorzugt sind Ammoniumhalogenide, insbesondere Chloride und Bromide, wie Alkyltrimethylammoniumchloride, Dialkyldimethylammoniumchloride und Trialkylmethylammoniumchloride, z. B. Cetyltrimethylammoniumchlorid, Stearyltrimethylammoniumchlorid, Distearyldimethylammoniumchlorid, Lauryldimethylammoniumchlorid, Lauryldimethylbenzylammoniumchlorid und Tricetylmethylammoniumchlorid. Weiterhin können die sehr gut biologisch abbaubaren quaternären Esterverbindungen, wie beispielsweise die unter dem Warenzeichen Stepantex® vertriebenen Dialkylammoniummethosulfate und Methylhydroxyalkyldialkoyloxyalkylammoniummethosulfate und die entsprechenden Produkte der Dehyquart®-Reihe, als kationische Tenside eingesetzt werden. Unter der Bezeichnung "Esterquats" werden im allgemeinen quaternierte Fettsäuretriethanolaminestersalze verstanden. Sie können den erfindungsgemäßen Zusammensetzungen einen besonderen Weichgriff verleihen. Es handelt sich dabei um bekannte Stoffe, die man nach den einschlägigen Methoden der organischen Chemie herstellt. Weitere erfindungsgemäß verwendbare kationische Tenside stellen die quaternisierten Proteinhydrolysate dar.

Wachskomponente

In einer Ausführungsform der Erfindung enthalten die erfindungsgemäßen Zubereitungen mindestens eine Wachskomponente.

Ein Gegenstand der Erfindung betrifft kosmetische und/oder pharmazeutische Zubereitungen, enthaltend 0,1 bis 80 Gew.% eines Kohlenwasserstoff Gemischs, das $^{14}C$ Isotope enthält, und mindestens eine Wachskomponente.

Ein bevorzugter Gegenstand der Erfindung betrifft kosmetische und/oder pharmazeutische Zubereitungen, enthaltend 0,1 bis 80 Gew.% eines Kohlenwasserstoff Gemischs, das $^{14}C$ Isotope enthält und wobei die Kohlenwasserstoffe mindestens 2 voneinander verschiedene Kohlenwasserstoffe enthalten, deren Kohlenstoff Zahl sich um mehr als 1, vorzugsweise um 2 unterscheidet, und mindestens eine Wachskomponente.

Die erfindungsgemäßen Zusammensetzungen enthalten den/die Wachskomponente(n) üblicherweise in einer Menge von 0 bis 40 Gew.-%, insbesondere von 0 bis 20 Gew.-%, vorzugsweise 0,1 bis 15 Gew.-% und insbesondere 0,1 bis 10 Gew.-% bezogen auf das Gesamtgewicht der Zusammensetzung.

Unter dem Begriff Wachs werden üblicherweise alle natürlichen oder künstlich gewonnenen Stoffe und Stoffgemische mit folgenden Eigenschaften verstanden: sie sind von fester bis brüchig harter Konsistenz, grob bis feinkristallin, durchscheinend bis trüb und schmelzen oberhalb von 30˚C ohne Zersetzung. Sie sind schon wenig oberhalb des Schmelzpunktes niedrigviskos und nicht fadenziehend und zeigen eine stark temperaturabhängige Konsistenz und

Löslichkeit. Erfindungsgemäß einsetzbar ist eine Wachskomponente oder ein Gemisch von Wachskomponenten, die bei 30 °C oder darüber schmelzen.

Als Wachse können erfindungsgemäß auch Fette und fettähnliche Substanzen mit wachsartiger Konsistenz eingesetzt werden, solange sie den geforderten Schmelzpunkt haben. Hierzu gehören u.a. Fette (Triglyceride), Mono- und Diglyceride, natürliche und synthetische Wachse, Fett- und Wachsalkohole, Fettsäuren, Ester von Fettalkoholen und Fettsäuren sowie Fettsäureamide oder beliebige Gemische dieser Substanzen.

Unter Fetten versteht man Triacylglycerine, also die Dreifachester von Fettsäuren mit Glycerin. Bevorzugt enthalten sie gesättigte, unverzweigte und unsubstituierte Fettsäurereste. Hierbei kann es sich auch um Mischester, also um Dreifachester aus Glycerin mit verschiedenen Fettsäuren handeln. Erfindungsgemäß einsetzbar und als Konsistenzgeber besonders gut geeignet sind so genannte gehärtete Fette und Öle, die durch Partialhydrierung gewonnen werden. Pflanzliche gehärtete Fette und Öle sind bevorzugt, z. B. gehärtetes Rizinusöl, Erdnußöl, Sojaöl, Rapsöl, Rübsamenöl, Baumwollsaatöl, Sojaöl, Sonnenblumenöl, Palmöl, Palmkernöl, Leinöl, Mandelöl, Maisöl, Olivenöl, Sesamöl, Kakaobutter und Kokosfett.

Geeignet sind u.a. die Dreifachester von Glycerin mit C12-C60-Fettsäuren und insbesondere C12-C36-Fettsäuren. Hierzu zählt gehärtetes Rizinusöl, ein Dreifachester aus Glycerin und einer Hydroxystearinsäure, der beispielsweise unter der Bezeichnung Cutina HR im Handel ist. Ebenso geeignet sind Glycerintristearat, Glycerintribehenat (z. B. Syncrowax HRC), Glycerintripalmitat oder die unter der Bezeichnung Syncrowax HGLC bekannten TriglyceridGemische, mit der Vorgabe, dass der Schmelzpunkt der Wachskomponente bzw. des Gemisches bei 30 °C oder darüber liegt.

Als Wachskomponenten sind erfindungsgemäß insbesondere Mono- und Diglyceride bzw. Mischungen dieser Partialglyceride einsetzbar. Zu den erfindungsgemäß einsetzbaren Glyceridgemischen zählen die von der Cognis Deutschland GmbH & Co. KG vermarkteten Produkte Novata AB und Novata B (Gemisch aus C12-C18-Mono-, Di- und Triglyceriden) sowie Cutina MD oder Cutina GMS (Glycerylstearat).

Zu den erfindungsgemäß als Wachskomponente einsetzbaren Fettalkoholen zählen die C12-C50-Fettalkohole. Die Fettalkohole können aus natürlichen Fetten, Ölen und Wachsen gewonnen werden, wie beispielsweise Myristylalkohol, 1-Pentadecanol, Cetylalkohol, 1-Heptadecanol, Stearylalkohol, 1-Nonadecanol, Arachidylalkohol, 1-Heneicosanol, Behenylalkohol, Brassidylalkohol, Lignocerylalkohol, Cerylalkohol oder Myricylalkohol. Erfindungsgemäß bevorzugt sind gesättigte unverzweigte Fettalkohole. Aber auch ungesättigte, verzweigte oder unverzweigte Fettalkohole können erfindungsgemäß als Wachskomponente verwendet werden, solange sie den geforderten Schmelzpunkt aufweisen. Erfindungsgemäß einsetzbar sind auch Fettalkoholschnitte, wie sie bei der Reduktion natürlich vorkommender Fette und Öle wie z. B. Rindertalg, Erdnußöl, Rüböl, Baumwollsaatöl, Sojaöl, Sonnenblumenöl, Palmkernöl, Leinöl, Rizinusöl, Maisöl, Rapsöl, Sesamöl, Kakaobutter und Kokosfett anfallen. Es können aber auch synthetische Alkohole, z. B. die linearen, geradzahligen Fettalkohole der Ziegler-Synthese (Alfole) oder die teilweise verzweigten Alkohole aus der Oxosynthese (Dobanole) verwendet werden. Erfindungsgemäß besonders bevorzugt geeignet sind C14-C22-Fettalkohole, die beispielsweise von der Cognis Deutschland GmbH unter der Bezeichnung Lanette 18 (C18-Alkohol), Lanette 16 (C16-Alkohol), Lanette 14 (C14-Alkohol), Lanette O (C16/C18-Alkohol) und Lanette 22 (C18/C22-Alkohol) vermarktet werden. Fettalkohole verleihen den Zusammensetzungen ein trockeneres Hautgefüh1 als Triglyceride und sind daher gegenüber letzteren bevorzugt.

Als Wachskomponenten können auch C14-C40-Fettsäuren oder deren Gemische eingesetzt werden. Hierzu gehören beispielsweise Myristin-, Pentadecan-, Palmitin-, Margarin-, Stearin-, Nonadecan-, Arachin-, Behen-, Lignocerin-, Cerotin-, Melissin-, Eruca- und Elaeostearinsäure sowie substituierte Fettsäuren, wie z. B. 12-Hydroxystearinsäure, und die Amide oder Monoethanolamide der Fettsäuren, wobei diese Aufzählung beispielhaften und keinen beschränkenden Charakter hat.

Erfindungsgemäß verwendbar sind beispielsweise natürliche pflanzliche Wachse, wie Candelillawachs, Carnaubawachs, Japanwachs, Espartograswachs, Korkwachs, Guarumawachs, Reiskeimölwachs, Zuckerrohrwachs, Ouricurywachs, Montanwachs, Sonnenblumenwachs, Fruchtwachse wie Orangenwachse, Zitronenwachse, Grapefruitwachs, Lorbeerwachs (=Bayberrywax) und tierische Wachse, wie z. B. Bienenwachs, Schellackwachs, Walrat, Wollwachs und Bürzelfett. Im Sinne der Erfindung kann es vorteilhaft sein, hydrierte oder gehärtete Wachse einzusetzen. Zu den erfindungsgemäß verwendbaren natürlichen Wachsen zählen auch die Mineralwachse, wie z. B. Ceresin und Ozokerit oder die petrochemischen Wachse, wie z. B. Petrolatum, Paraffinwachse und Mikrowachse. Als Wachskomponente sind auch chemisch modifizierte Wachse, insbesondere die Hartwachse, wie z. B. Montanesterwachse, Sasolwachse und hydrierte Jojobawachse einsetzbar. Zu den synthetischen Wachsen, die erfindungsgemäß einsetzbar sind, zählen beispielsweise wachsartige Polyalkylenwachse und Polyethylenglycolwachse. Pflanzliche Wachse sind erfindungsgemäß bevorzugt.

Die Wachskomponente kann ebenso gewählt werden aus der Gruppe der Wachsester aus gesättigten und/oder ungesättigten, verzweigten und/oder unverzweigten Alkancarbonsäuren und gesättigten und/oder ungesättigten, verzweigten und/oder unverzweigten Alkoholen, aus der Gruppe der Ester aus aromatischen Carbonsäuren, Dicarbonsäuren, Tricarbonsäuren bzw. Hydroxycarbonsäuren (z. B. 12-Hydroxystearinsäure) und gesättigten und/oder ungesättigten, verzweigten und/oder unverzweigten Alkoholen, sowie ferner aus der Gruppe der Lactide langkettiger Hydroxycarbonsäuren. Beispiel solcher Ester sind die C16-C40-Alkylstearate, C20-C40-Alkylstearate (z. B. Kesterwachs K82H), C20-C40-Dialkylester von Dimersäuren, C18-C38-Alkylhydroxystearoylstearate oder C20-C40-Alkylerucate. Ferner sind C30-C50-Alkylbienenwachs, Tristearylcitrat, Triisostearylcitrat, Stearylheptanoat, Stearyloctanoat, Trilaurylcitrat, Ethylenglycoldipalmitat, Ethylenglycoldistearat, Ethylenglykoldi(12-hydroxystearat), Stearylstearat, Palmitylstearat, Stearylbehenat, Cetylester, Cetearylbehenat und Behenylbehenat einsetzbar. Auch Fettsäurepartialglyceride, d. h. technische Mono-und/oder Diester des Glycerins mit Fettsäuren mit 12 bis 18 Kohlenstoffatomen wie etwa Glycerinmono/dilaurat, -palmitat, - myristat oder -stearat kommen hierfür in Frage.

Als Wachse eignen sich weiterhin Perlglanzwachse. Als Perlglanzwachse, insbesondere für den Einsatz in tensidischen Formulierungen, kommen beispielsweise in Frage: Alkylenglycolester, speziell Ethylenglycoldistearat; Fettsäurealkanolamide, speziell Kokosfettsäurediethanolamid; Partialglyceride, speziell Stearinsäuremonoglycerid; Ester von mehrwertigen, gegebenenfalls hydroxy-substituierte Carbonsäuren mit Fettalkoholen mit 6 bis 22 Kohlenstoffatomen, speziell langkettige Ester der Weinsäure; Fettstoffe, wie beispielsweise Fettalkohole, Fettketone, Fettaldehyde, Fettether und Fettcarbonate, die in Summe mindestens 24 Kohlenstoffatome aufweisen, speziell Lauron und Distearylether; Fettsäuren wie Stearinsäure, Hydroxystearinsäure oder Behensäure, Ringöffnungsprodukte von Olefinepoxiden mit 12 bis 22 Kohlenstoffatomen mit Fettalkoholen mit 12 bis 22 Kohlenstoffatomen und/oder Polyolen mit 2 bis 15 Kohlenstoffatomen und 2 bis 10 Hydroxylgruppen sowie deren Mischungen.

Polymere

In einer Ausführungsform der Erfindung enthalten die erfindungsgemäßen Zubereitungen mindestens ein Polymer.

Ein Gegenstand der Erfindung betrifft kosmetische und/oder pharmazeutische Zubereitungen, enthaltend 0,1 bis 80 Gew.% eines Kohlenwasserstoff Gemischs, das $^{14}C$ Isotope enthält, und mindestens ein Polymer.

Ein bevorzugter Gegenstand der Erfindung betrifft kosmetische und/oder pharmazeutische Zubereitungen, enthaltend 0,1 bis 80 Gew.% eines Kohlenwasserstoff Gemischs, das $^{14}C$ Isotope enthält und wobei die Kohlenwasserstoffe mindestens 2 voneinander verschiedene Kohlenwasserstoffe enthalten, deren Kohlenstoff Zahl sich um mehr als 1, vorzugsweise um 2 unterscheidet, und mindestens ein Polymer.

Die erfindungsgemäßen Zusammensetzungen enthalten das/die Polymere(n) üblicherweise in einer Menge von 0 bis 20 Gew.-%, vorzugsweise 0,1 bis 15 Gew.-% und insbesondere 0,1 bis 10 Gew.-% bezogen auf das Gesamtgewicht der Zusammensetzung.

Geeignete kationische Polymere sind beispielsweise kationische Cellulosederivate, wie z. B. eine quaternierte Hydroxyethylcellulose, die unter der Bezeichnung Polymer JR 400® von Amerchol erhältlich ist, kationische Stärke, Copolymere von Diallylammoniumsalzen und Acrylamiden, quaternierte Vinylpyrrolidon/Vinylimidazol-Polymere, wie z.B. Luviquat® (BASF), Kondensationsprodukte von Polyglycolen und Aminen, quaternierte Kollagenpolypeptide, wie beispielsweise Lauryldimonium hydroxy-propyl hydrolyzed collagen (Lamequat®L/Grünau), quaternierte Weizenpolypeptide, Polyethylenimin, kationische Siliconpolymere, wie z. B. Amidomethicone, Copolymere der Adipinsäure und Dimethylaminohydroxypropyldiethylentriamin (Cartaretine®/Sandoz), Copolymere der Acrylsäure mit Dimethyldiallylammoniumchlorid (Merquat® 550/Chemviron), Polyaminopolyamide, kationische Chitinderivate wie beispielsweise quaterniertes Chitosan, gegebenenfalls mikrokristallin verteilt, Kondensationsprodukte aus Dihalogenalkylen, wie z.B. Dibrombutan mit Bisdialkylaminen, wie z. B. Bis-Dimethylamino-1,3-propan, kationischer Guar-Gum, wie z.B. Jaguar® CBS, Jaguar® C-17, Jaguar® C-16 der Firma Celanese, quaternierte Ammoniumsalz-Polymere, wie z. B. Mirapol® A-15, Mirapol® AD-1, Mirapol® AZ-1 der Firma Miranol.

Als anionische, zwitterionische, amphotere und nichtionische Polymere kommen beispielsweise Vinylacetat/Crotonsäure-Copolymere, Vinylpyrrolidon/Vinylacrylat-Copolymere, Vinylacetat/Butylmaleat/ Isobornylacrylat-Copolymere, Methylvinylether/Maleinsäureanhydrid-Copolymere und deren Ester, unvernetzte und mit Polyolen vernetzte Polyacrylsäuren, Acrylamidopropyltrimethylammoniumchlorid/ Acrylat-Copolymere, Octylacrylamid/Methylmethacrylat/tert.Butylaminoethylmethacrylat/2-Hydroxypropylmethacrylat-Copolymere, Polyvinylpyrrolidon, Vinylpyrrolidon/Vinylacetat-Copolymere, Vinylpyrrolidon/ Dimethylaminoethylmethacrylat/Vinylcaprolactam-Terpolymere sowie gegebenenfalls derivatisierte Celluloseether und Silicone in Frage.

Als Polymere eigen sich ebenso Polysaccharide, insbesondere Xanthan-Gum, Guar-Guar, Agar-Agar, Alginate und Tylosen sowie beispielsweise Aerosil-Typen (hydrophile Kieselsäuren), Carboxymethylcellulose und Hydroxyethyl- und Hydroxypropylcellulose, Polyvinylalkohol, Polyvinylpyrrolidon und Bentonite wie z. B. Bentone® Gel VS-5PC (Rheox).

Ebenso geeignet sind sogenannte quaternäre Polymere, z.B. mit der INCI - Bezeichnung Polyquaternium-37, die der folgenden allgemeinen Formel entsprechen: Alternativ können auch andere Dialkylaminoalkyl (meth)acrylate sowie deren durch Alkylierung oder Protonierung erhältliche Ammoniumsalze oder Dialkylaminoalkyl (meth)acrylamide sowie deren durch Alkylierung oder Protonierung erhältliche Ammoniumsalze eingesetzt werden. Besonders bevorzugt sind Polymere enthaltend MAPTAC, APTAC, MADAME, ADAME, DMAEMA und TMAEMAC. Darüberhinaus können auch

Co-polymere mit anionischen, weiteren kationischen oder ungeladenen Monomeren erfindungsgemäß eingesetzt

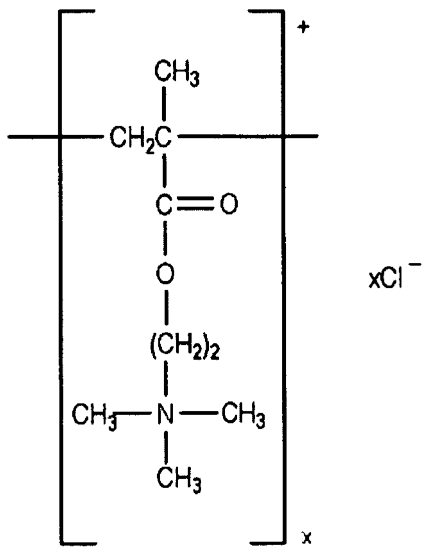

werden, insbesondere solche, die neben den genannten Alkylaminoalkyl (meth)acrylat oder - (meth)acrylamid Monomeren zusätzlich (Meth)acrylsäure und/oder 2-Acrylamido-2-methyl-propansulfonsäure und/oder Acrylamid und/oder Vinylpyrrolidon und/oder Alkyl(meth)acrylate enthalten.

Beispielhaft seien solche Polymere mit der INCI Bezeichnung Polyquaternium-11, Polyquaternium-13, Polyquaternium-14, Polyquaternium-15, Polyquaternium-28, Polyquaternium-32, Polyquaternium-43, Polyquaternium-47 genannt.

Ölkörper

In einer Ausführungsform der Erfindung enthalten die erfindungsgemäßen Zubereitungen mindestens einen Ölkörper. Üblicherweise enthalten die erfindungsgemäßen Zubereitungen das Kohlenwasserstoff Gemisch als Ölkörper. In der hier als bevorzugte genannten Ausführungsform enthalten die Zubereitungen somit einen von dem erfindungsgemäßen Kohlenstoff Gemisch verschiedenen Ölkörper, auch als "weiterer Ölkörper" bezeichnet.

Ein Gegenstand der Erfindung betrifft kosmetische und/oder pharmazeutische Zubereitungen, enthaltend 0,1 bis 80 Gew.% eines Kohlenwasserstoff Gemischs, das $^{14}C$ Isotope enthält, und mindestens eine einen (weiteren) Ölkörper.

Ein bevorzugter Gegenstand der Erfindung betrifft kosmetische und/oder pharmazeutische Zubereitungen, enthaltend 0,1 bis 80 Gew.% eines Kohlenwasserstoff Gemischs, das $^{14}C$ Isotope enthält und wobei die Kohlenwasserstoffe mindestens 2 voneinander verschiedene Kohlenwasserstoffe enthalten, deren Kohlenstoff Zahl sich um mehr als 1, vorzugsweise um 2 unterscheidet, und mindestens einen (weiteren) Ölkörper.

Die Ölkörper (erfindungsgemäßes Kohlenwasserstoff Gemisch plus weitere Ölkörper) sind üblicherweise in einer Gesamtmenge von 0,1 - 90, insbesondere 0,1 - 80, insbesondere 0,5 bis 70, bevorzugt 1 bis 60, insbesondere 1 bis 50 Gew.-%, insbesondere 1 bis 40 Gew.-%, vorzugsweise 5 - 25 Gew.-% und insbesondere 5 - 15 Gew.-% enthalten. Die weiteren Ölkörper sind üblicherweise in einer Menge von 0,1 bis 40 Gew.-% -bezogen auf das Gesamtgewicht der Zubereitung- enthalten.

Als weitere Ölkörper kommen beispielsweise Guerbetalkohole auf Basis von Fettalkoholen mit 6 bis 18, vorzugsweise 8 bis 10 Kohlenstoffatomen, in Frage sowie weitere, zusätzliche Ester wie Myristylmyristat, Myristylpalmitat, Myristylstearat, Myristylisostearat, Myristyloleat, Myristylbehenat, Myristylerucat, Cetylmyristat, Cetylpalmitat, Cetylstearat, Cetylisostearat, Cetyloleat, Cetylbehenat, Cetylerucat, Stearylmyristat, Stearylpalmitat, Stearylstearat, Stearylisostearat, Stearyloleat, Stearylbehenat, Stearylerucat, Isostearylmyristat, Isostearylpalmitat, Isostearylstearat, Isostearylisostearat, Isostearyloleat, Isostearylbehenat, Isostearyloleat, Oleylmyristat, Oleylpalmitat, Oleylstearat, Oleylisostearat, Oleyloleat, Oleylbehenat, Oleylerucat, Behenylmyristat, Behenylpalmitat, Behenylstearat, Behenylisostearat, Behenyloleat, Behenylbehenat, Behenylerucat, Erucylmyristat, Erucylpalmitat, Erucylstearat, Erucylisostearat, Erucyloleat, Erucylbehenat und Erucylerucat. Daneben eignen sich Ester von $C_{18}$-$C_{38}$-Alkylhydroxycarbonsäuren mit linearen oder verzweigten $C_6$-$C_{22}$-Fettalkoholen, insbesondere Dioctyl Malate, Ester von linearen und/oder verzweigten Fettsäuren mit mehrwertigen Alkoholen (wie z. B. Propylenglycol, Dimerdiol oder Trimertriol), Triglyceride auf Basis

$C_6$-$C_{10}$-Fettsäuren, flüssige Mono-/Di-/Triglyceridmischungen auf Basis von $C_6$-$C_{18}$-Fettsäuren, Ester von $C_6$-$C_{22}$-Fettalkoholen und/oder Guerbetalkoholen mit aromatischen Carbonsäuren, insbesondere Benzoesäure, Ester von $C_2$-$C_{12}$-Dicarbonsäuren mit Polyolen mit 2 bis 10 Kohlenstoffatomen und 2 bis 6 Hydroxylgruppen, pflanzliche Öle, verzweigte primäre Alkohole, substituierte Cyclohexane, lineare und verzweigte $C_6$-$C_{22}$-Fettalkoholcarbonate, wie z. B. Dicaprylyl Carbonate (Cetiol® CC), Guerbetcarbonate auf Basis von Fettalkoholen mit 6 bis 18, vorzugsweise 8 bis 10 C Atomen, Ester der Benzoesäure mit linearen und/oder verzweigten $C_6$-$C_{22}$-Alkoholen (z. B. Finsolv® TN), lineare oder verzweigte, symmetrische oder unsymmetrische Dialkylether mit 6 bis 22 Kohlenstoffatomen pro Alkylgruppe, wie z. B. Dicaprylylether (Cetiol® OE), Ringöffnungsprodukte von epoxidierten Fettsäureestern mit Polyolen und Kohlenwasserstoffen oder deren Gemische.

Als weitere Ölkörper kommen beispielsweise Silikonöle in Frage. Sie können als cyclische und/oder lineare Silikonöle vorliegen. Silikonöle sind hochmolekulare synthetische polymere Verbindungen, in denen Silicium- Atome über Sauerstoff-Atome ketten-und/oder netzartig verknüpft und die restlichen Valenzen des Siliciums durch Kohlenwasserstoff-Reste (meist Methyl-, seltener Ethyl-, Propyl-, Phenyl-Gruppen u. a.) abgesättigt sind. Systematisch werden die Silikonöle als Polyorganosiloxane bezeichnet. Die methylsubstituierten Polyorganosiloxane, welche die mengenmäßig bedeutendsten Verbindungen dieser Gruppe darstellen und sich durch die folgende Strukturformel

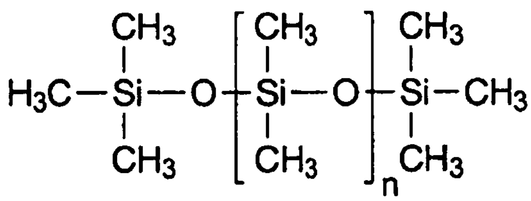

auszeichnen werden auch als Polydimethylsiloxan bzw. Dimethicon (INCI) bezeichnet. Dimethicone gibt es in verschiedenen Kettenlängen bzw. mit verschiedenen Molekulargewichten.

Vorteilhafte Polyorganosiloxane im Sinne der vorliegenden Erfindung sind beispielsweise Dimethylpolysiloxan [Poly (dimethylsiloxan)], welche beispielsweise unter den Handelsbezeichnungen Abil 10 bis 10 000 bei Evonik Goldschmidt erhältlich sind. Ferner vorteilhaft sind Phenylmethylpolysiloxan (INCI: Phenyl Dimethicon, Phenyl Trimethicon), cyclische Silikone (Octamethylcyclotetrasiloxan bzw. Decamethylcyclopentasiloxan), welche nach INCI auch als Cyclomethicon bezeichnet werden, aminomodifizierte Silikone (INCI: Amodimethicone) und Silikonwachse, z. B. Polysiloxan-Polyalkylen-Copolymere (INCI: Stearyl Dimethicon und Cetyl Dimethicon) und Dialkoxydimethylpolysiloxane (Stearoxy Dimethicon und Behenoxy Stearyl Dimethicon), welche als verschiedene Abil-Wax-Typen bei Evonik Goldschmidt erhältlich sind. Aber auch andere Silikonöle sind vorteilhaft im Sinne der vorliegenden Erfindung zu verwenden, beispielsweise Cetyidimethicon, Hexamethylcyclo- trisiloxan, Polydimethylsiloxan, Poly (methylphenylsiloxan). Erfindungsgemäß besonders bevorzugte Silikone sind Dimethicon und Cyclomethicon.

Die erfindungsgemäßen Zubereitungen können weiterhin biogenen Wirkstoffe, Insekten-Repellentien, Tyrosinase Inhibitoren, Konservierungsmittel, Parfümöle, Überfettungsmittel, Stabilitsatoren und/oder Hydrotrope enthalten.

Ein Gegenstand der Erfindung betrifft kosmetische und/oder pharmazeutische Zubereitungen, enthaltend 0,1 bis 80 Gew.% eines Kohlenwasserstoff Gemischs, das $^{14}C$ Isotope enthält, und mindestens einen biogenen Wirkstoff, Insekten-Repellent, Tyrosinase Inhibitor, Konservierungsmittel, Parfümöl, Stabilisator und/oder Hydrotrop.

Ein bevorzugter Gegenstand der Erfindung betrifft kosmetische und/oder pharmazeutische Zubereitungen, enthaltend 0,1 bis 80 Gew.% eines Kohlenwasserstoff Gemischs, das $^{14}C$ Isotope enthält und wobei die Kohlenwasserstoffe mindestens 2 voneinander verschiedene Kohlenwasserstoffe enthalten, deren Kohlenstoff Zahl sich um mehr als 1, vorzugsweise um 2 unterscheidet, und mindestens einen biogenen Wirkstoff, Insekten-Repellent, Tyrosinase Inhibitor, Konservierungsmittel, Parfümöl, Stabilisator und/oder Hydrotrop.

Unter biogenes Wirkstoffen sind beispielsweise Tocopherol, Tocopherolacetat, Tocopherolpalmitat, Ascorbinsäure, (Desoxy)Ribonucleinsäure und deren Fragmentierungsprodukte, β-Glucane, Retinol, Bisabolol, Allantoin, Phytantriol, Panthenol, AHA-Säuren, Aminosäuren, Ceramide, Pseudoceramide, essentielle Öle, Pflanzenextrakte, wie z. B. Aloe vera, Prunusextrakt, Bambaranussextrakt und Vitaminkomplexe zu verstehen.

Als Insekten-Repellentien kommen beispielsweise N,N-Diethyl-m-toluamid, 1,2-Pentandiol oder 3-(N-n-Butyl-N-acetyl-amino)-propionsäureethylester), welches unter der Bezeichnung Insect Repellent® 3535 von der Merck KGaA vertrieben wird, sowie Butylacetylaminopropionate in Frage.

Als Tyrosinhinbitoren, die die Bildung von Melanin verhindern und Anwendung in Depigmentierungsmitteln finden, kommen beispielsweise Arbutin, Ferulasäure, Kojisäure, Cumarinsäure und Ascorbinsäure (Vitamin C) in Frage.

Als Konservierungsmittel eignen sich beispielsweise Phenoxyethanol, Formaldehydlösung, Parabene, Pentandiol oder Sorbinsäure sowie die unter der Bezeichnung Surfacine® bekannten Silberkomplexe. Weiterhin eignen

sich als Konservierungsmittel die in WO 07/048757 beschriebenen 1,2 Alkandiole mit 5 bis 8 C-Atomen.

Als Konservierungsmittel eignen sich insbesondere die gemäß Annex VI der Kommissions Direktive (in der **Fassung Commission Directive 2007/22/EC of 17 April 2007 amending Council Directive 76/768/EEC,** concerning cosmetic products, for the purposes of adapting Annexes IV and VI thereto to technical progress) zugelassen Stoffe, auf die hier explizit Bezug genommen wird.

Als Parfümöle seien genannt Gemische aus natürlichen und synthetischen Riechstoffen. Natürliche Riechstoffe sind Extrakte von Blüten, Stengeln und Blättern, Früchten, Fruchtschalen, Wurzeln, Hölzern, Kräutern und Gräsern, Nadeln und Zweigen, Harzen und Balsamen. Weiterhin kommen tierische Rohstoffe, wie beispielsweise Zibet und Castoreum sowie synthetische Riechstoffverbindungen vom Typ der Ester, Ether, Aldehyde, Ketone, Alkohole und Kohlenwasserstoffe in Frage.

Als Stabilisatoren können Metallsalze von Fettsäuren, wie z. B. Magnesium-, Aluminium-und/oder Zinkstearat bzw. -ricinoleat eingesetzt werden.

Zur Verbesserung des Fließverhaltens können ferner Hydrotrope, wie beispielsweise Ethanol, Isopropylalkohol, oder Polyole eingesetzt werden. Polyole, die hier in Betracht kommen, besitzen vorzugsweise 2 bis 15 Kohlenstoffatome und mindestens zwei Hydroxylgruppen. Die Polyole können noch weitere funktionelle Gruppen, insbesondere Aminogruppen, enthalten bzw. mit Stickstoff modifiziert sein.

## Beispiele

### Herstellbeispiel 1: Herstellung eines erfindungsgemässen Kohlenwasserstoff Gemischs

Zur Herstellung eines erfindungsgemäßen Kohlenwasserstoff Gemischs wurden zunächst Tridecan und Undecan getrennt voneinander aus den jeweiligen Fettalkoholen hergestellt und dann im gewünschten Verhältnis zueinander gemischt:

#### 1a) Herstellung von Tridecan aus 1-Tetradecanol

1000 g 1-Tetradecanol (4,7 mol; Lorol®C 14 der Fa. Cognis) wurden in einem rührbaren Druckbehälter mit 10 g eines Nickelkatalysators (Ni-5249 P der Fa. Engelhard; Ni-Gehalt = 63 Gew.-%) vorgelegt und auf 240 ˚C aufgeheizt. Anschließend wurde über einen Zeitraum von 12 h Wasserstoff über ein Begasungsrohr bei einem Druck von 20 bar zugegeben und gleichzeitig über ein Ventil am Reaktordeckel die Reaktionsgase ausgeschleust. Danach wurde das Produkt gekühlt, abgelassen und filtriert. Es ergab sich eine Auswaage von 845 g Reaktionsprodukt.

Eine GC-Analyse ergibt folgende Zusammensetzung: 89,0 % Tridecan, 2,1 % Tetradecan, 4,1 % 1-Tetradecanol, 4,2 % Dimere Reaktionsprodukte. Dieses Reaktionsprodukt wurde in einer Destillation zum reinen Tridecan fraktioniert und anschließend mit Stickstoff desodoriert. Man erhält ein farbloses, dünnflüssiges und geruchsarmes Produkt.

#### 1b) Herstellung von Undecan aus 1-Dodecanol

1000 g 1-Dodecanol (5,4 mol; Lorol® C 12 der Fa. Cognis) wurden in einem rührbaren Druckbehälter mit 10 g eines Nickelkatalysators (Ni-5249 P der Fa. Engelhard; Ni-Gehalt = 63 Gew%) vorgelegt und auf 240˚C aufgeheizt. Anschließend wurde über einen Zeitraum von 8 h Wasserstoff über ein Begasungsrohr bei einem Druck von 20 bar zugegeben und gleichzeitig über ein Ventil am Reaktordeckel die Reaktionsgase ausgeschleust. Danach wurde das Produkt gekühlt und abgelassen und filtriert. Es ergab sich eine Auswaage von 835g Reaktionsprodukt.

Eine GC-Analyse ergibt folgende Zusammensetzung: 68,4 % Undecan, 0,6 % Dodecan, 21,7 % 1-Dodecanol, 7,2 % Dimere Reaktionsprodukte. Dieses Reaktionsprodukt wurde destilliert, um das Undecan rein zu erhalten. Dieses wurde anschließend mit Stickstoff desodoriert. Man erhält ein farbloses, dünnflüssiges und geruchsarmes Produkt.

Aus den gemäß Beispiel 1a) sowie gemäß Beispiel 1b) erhaltenen Verbindungen wurde folgendes erfindungsgemäßes Kohlenwasserstoff Gemisch hergestellt:

Zusammensetzung des Kohlenwasserstoff Gemischs nach Beispiel 1:
76 Gew.-% n-Undecan, 24 Gew.-% n-Tridecan.

### Herstellbeispiel 2

Zur Herstellung eines erfindungsgemäßen Kohlenwasserstoff Gemischs wurde ein Fettalkohol Gemisch, welches C12 und C14 Fettalkohole entsprechend dem herzustellenden Kohlenwasserstoff Gemisch enthält, einer reduktiven Dehydroxymethylierung unterzogen:

1000 g Lorol® Spezial (Fa. Cognis; Fettalkoholverteilung C 12 Fettalkohol 70-75 %, C 14 Fettalkohol 24-30%, C16 unter 4%) und 10 g eines Nickelkatalysators (Ni-5249 P der Fa. Engelhard; Ni-Gehalt = 63 Gew.-%) wurden im Autoklaven vorgelegt. Der Reaktor wurde verschlossen und evakuiert. Das Reaktionsgemisch wurde anschließend unter Vakuum auf ca. 80°C erhitzt und 30 min. gerührt. Danach wurde der Reaktor mit Wasserstoff auf ca. 80 bar gebracht und kontinuierlich auf 250°C erhitzt. Die Reaktion ist abgeschlossen, wenn der Druck konstant bleibt und die Hauptmenge des Fettalkohols zum gewünschten Kohlenwasserstoff umgesetzt ist. Nach Entspannen und Belüften mit Stickstoff wurde das Produkt destillativ gereinigt. Das gereinigte Produkt fällt als farblose Flüssigkeit an.

Das gemäß Herstellbeispiel 2 erhältliche Kohlenwasserstoff Gemisch ist wie folgt zusammensetzt: (GC Analyse)

| Kohlenwasserstoffe mit einer C Zahl von: | Anteil an Gesamtsumme der Kohlenwasserstoffe [Gew.-%] |
|---|---|
| C11 (linear) | 67 |
| C12 | 4 |
| 13 (linear) | 26 |
| C14 | 1,5 |
| C9, C10, C15 | 1,5 |

Das Gewichtsverhältnis von linearem C11 Kohlenwasserstoff zu linearem C13 Kohlenwasserstoff beträgt 2,57. Ebenso beträgt das Gewichtsverhältnis der C12 Kohlenwasserstoffe zu den C14 Kohlenwasserstoffen 2,57.

<u>Herstellbeispiel 3</u>

Zur Herstellung eines erfindungsgemäßen Kohlenwasserstoff Gemischs wird ein Fettalkohol Gemisch, welches C12, C14, C16 und C18 Fettalkohole entsprechend dem herzustellenden Kohlenwasserstoff Gemisch enthält, einer reduktiven Dehydroxymethylierung unterzogen: 1000 g Lorol® Technisch (Fa. Cognis; Fettalkohol Gemisch aus C 12, C 14, C16 und C 18 Fettalkoholen) und 10 g eines Nickelkatalysators (Ni-5249 P der Fa. Engelhard; Ni-Gehalt = 63 Gew.-%) werden im Autoklaven vorgelegt. Der Reaktor wird verschlossen und evakuiert. Das Reaktionsgemisch wird anschließend unter Vakuum auf ca. 80°C erhitzt und 30 min. gerührt. Danach wird der Reaktor mit Wasserstoff auf ca. 80 bar gebracht und kontinuierlich auf 250°C erhitzt. Die Reaktion ist abgeschlossen, wenn der Druck konstant bleibt und die Hauptmenge des Fettalkohols zum gewünschten Kohlenwasserstoff umgesetzt ist. Nach Entspannen und Belüften mit Stickstoff wird das Produkt destillativ gereinigt. Das gereinigte Produkt fällt als farblose Flüssigkeit an.

Das gemäß Herstellbeispiel 3 erhältliche Kohlenwasserstoff Gemisch ist wie folgt zusammensetzt: (GC Analyse) C 11 Kohlenwasserstoff 55 %, C 13 Kohlenwasserstoff 20%, C15 Kohlenwasserstoff 10%, C 17 Kohlenwasserstoff 15%.

<u>Herstellbeispiele 4 A bis C</u>

Zur Herstellung eines erfindungsgemäßen Kohlenwasserstoff Gemischs wird ein Fettalkohol Gemisch, welches C16 und C18 Fettalkohole entsprechend dem herzustellenden Kohlenwasserstoff Gemisch enthält, einer reduktiven Dehydroxymethylierung unterzogen: 1000 g Stenol ®16 65 (Fa. Cognis; Fettalkohol Gemisch aus C 16 und C 18 Fettalkoholen) und 10 g eines Nickelkatalysators (Ni-5249 P der Fa. Engelhard; Ni-Gehalt = 63 Gew.-%) werden im Autoklaven vorgelegt. Der Reaktor wird verschlossen und evakuiert. Das Reaktionsgemisch wird anschließend unter Vakuum auf ca. 80°C erhitzt und 30 min. gerührt. Danach wird der Reaktor mit Wasserstoff auf ca. 80 bar gebracht und kontinuierlich auf 250°C erhitzt. Die Reaktion ist abgeschlossen, wenn der Druck konstant bleibt und die Hauptmenge des Fettalkohols zum gewünschten Kohlenwasserstoff umgesetzt ist. Nach Entspannen und Belüften mit Stickstoff wird das Produkt destillativ gereinigt. Das gereinigte Produkt fällt als farblose Flüssigkeit an.

Das gemäß Herstellbeispiel 4 A erhältliche Kohlenwasserstoff Gemisch ist wie folgt zusammensetzt: (GC Analyse) C 15 Kohlenwasserstoff 65 %, C 17 Kohlenwasserstoff 35%. In identischer Weise werden 1000 g Stenol ®16-18 (Fa. Cognis; Fettalkohol Gemisch aus C 16 und C 18 Fettalkoholen) umgesetzt (= Herstellbeispiel 4 B) sowie 1000 g Hydrenol® D (Fa. Cognis; Fettalkohol Gemisch aus C 16 und C18 Fettalkoholen) umgesetzt (= Herstellbeispiel 4 C)

Das gemäß Herstellbeispiel 4 B erhältliche Kohlenwasserstoff Gemisch ist wie folgt zusammensetzt: (GC Analyse) C 15 Kohlenwasserstoff 50 %, C 17 Kohlenwasserstoff 50%. Das gemäß Herstellbeispiel 4 C erhältliche Kohlenwasserstoff Gemisch ist wie folgt zusammensetzt: (GC Analyse) C 15 Kohlenwasserstoff 30 %, C 17 Kohlenwasserstoff 70%.

Herstellbeispiele 5 A bis C

Zur Herstellung eines erfindungsgemäßen Kohlenwasserstoff Gemischs wird ein Fettalkohol Gemisch, welches C18, C20 und C22 Fettalkohole entsprechend dem herzustellenden Kohlenwasserstoff Gemisch enthält, einer reduktiven Dehydroxymethylierung unterzogen: 1000 g Stenol ®1822 - 45 (Fa. Cognis; Fettalkohol Gemisch aus C 18, C 20 und C 22 Fettalkoholen) und 10 g eines Nickelkatalysators (Ni-5249 P der Fa. Engelhard; Ni-Gehalt = 63 Gew.-%) werden im Autoklaven vorgelegt. Der Reaktor wird verschlossen und evakuiert. Das Reaktionsgemisch wird anschließend unter Vakuum auf ca. 80˚C erhitzt und 30 min. gerührt. Danach wird der Reaktor mit Wasserstoff auf ca. 80 bar gebracht und kontinuierlich auf 250˚C erhitzt. Die Reaktion ist abgeschlossen, wenn der Druck konstant bleibt und die Hauptmenge des Fettalkohols zum gewünschten Kohlenwasserstoff umgesetzt ist. Nach Entspannen und Belüften mit Stickstoff wird das Produkt destillativ gereinigt. Das gereinigte Produkt fällt als farblose Flüssigkeit an.

Das gemäß Herstellbeispiel 5 A erhältliche Kohlenwasserstoff Gemisch ist wie folgt zusammensetzt: (GC Analyse) C 17 Kohlenwasserstoff 45 %, C 19 Kohlenwasserstoff 10%, C21 Kohlenwasserstoff 45%.

In identischer Weise werden 1000 g Stenol ®AT (Fa. Cognis; Fettalkohol Gemisch aus C 18, C 20 und C 22 Fettalkoholen) umgesetzt (= Herstellbeispiel 5 B) sowie 1000 g Stenol®1822 - 70 (Fa. Cognis; Fettalkohol Gemisch aus C 18, C 20 und C 22 Fettalkoholen) umgesetzt (= Herstellbeispiel 5 C)

Das gemäß Herstellbeispiel 5 B erhältliche Kohlenwasserstoff Gemisch ist wie folgt zusammensetzt: (GC Analyse) C 17 Kohlenwasserstoff 40 %, C 19 Kohlenwasserstoff 12%, C 21 Kohlenwasserstoff 48%. Das gemäß Herstellbeispiel 5 C erhältliche Kohlenwasserstoff Gemisch ist wie folgt zusammensetzt: (GC Analyse) C 17 Kohlenwasserstoff 10 %, C 19 Kohlenwasserstoff 15%, C 21 Kohlenwasserstoff 75%.

Rezepturbeispiele

Die nachfolgenden Rezepturbeispiele wurden jeweils erhalten, in dem die Kohlenwasserstoff Gemische gemäß Herstellbeispielen 1 bis 5 in die angegebenen kosmetischen Zubereitungen eingearbeitet wurden. Der Ausdruck "Kohlenwasserstoff Gemisch gemäß Herstellbeispiel 1 bis 5" beschreibt demnach eine kosmetische Zubereitung einmal mit dem Kohlenwasserstoff Gemisch gemäß Herstellbeispiel 1, einmal mit einem Kohlenwasserstoff Gemisch gemäß Herstellbeispiel 2, einmal mit einem Kohlenwasserstoff Gemisch gemäß Herstellbeispiel 3, einmal mit einem Kohlenwasserstoff Gemisch gemäß Herstellbeispiel 4A, einmal mit dem Kohlenwasserstoff Gemisch gemäß Herstellbeispiel 4 B usw.

Allzweck Creme (W/O)

| Phase | Komponente/ Handelsname | INCI | Gew.% |
|---|---|---|---|
| I. | DEHYMULS® E | Dicocoyl Pentaerhytithyl Distearyl Citrate (and) Sorbitan Sesquioleate (and) Cera Alba (Beeswax) (and) Aluminum Stearate | 3,00 |
| | DEHYMULS® PGPH | Polyglyceryl 2 Dipolyhydroxystearate | 2,00 |
| | CETIOL® OE | Dicaprylyl Ether | 5,00 |
| | CETIOL® 868 | Ethylhexyl Stearate | 5,00 |
| | MYRITOL® 331 | Cocoglycerides | 1,00 |
| | Kohlenwasserstoff Gemisch gemäß Herstellbeispiel 1 bis 5 | | 6,00 |
| II. | Glycerin 86% | | 5,00 |
| | $MgSO_4$ x $7H_2O$ | | 1,00 |
| | Wasser, deionisiert | | 72,00 |
| III. | Konservierungsmittel | | q.s. |

Herstellung:

Die Komponenten der Phase I wurden bei 80 bis 85 ˚C geschmolzen und zur Homogenität verrührt. Die

Komponenten der Phase II wurden auf 80 bis 85 ˚C erhitzt und langsam, unter Rühren zur Phase I zugegeben. Es wurde für weitere 5 Minuten bei dieser Temperatur gerührt. Danach wurde die Emulsion unter Rühren abgekühlt und bei 65 bis 55 ˚C homogenisiert. Sobald die Emulsion homogen erscheint, wurde unter Rühren weiter auf 30˚C abgekühlt. Danach wurden Komponenten der Phase III zugegeben und erneut gerührt.

Balsam zur Befeuchtung und zum Schutz der Lippen

| Phase | Komponente Handelsname | INCI | Gew.-% |
|---|---|---|---|
| I. | Cerilla Raffinée G* | Candelilla (Euphorbia Cerifera) Wax | 7,53 |
| | CUTINA® LM conc. | Polyglyceryl-2 Dipolyhydroxystearate and Octyldodecanol and Copernicia Cerifera (Carnauba) Wax and Euphorbia Cerifera (Candelilla) Wax and Beeswax and Cetearyl Glucoside and Cetearyl Alcohol | 6,57 |
| | Colophane claire type Y | Rosin | 1,89 |
| | Cerauba T1* | Carnauba(Copernica Cerifera)Wax | 1,86 |
| | Cerabeil blanche 1* | Beeswax | 5.31 |
| | Kohlenwasserstoff-G Gemisch gemäß Herstellbeispiel 1 bis 5 | | 15.57 |
| | EUTANOL® G | Octyldodecanol | 21,71 |
| | Crodamol ML(Croda) | Myristyl Lactate | 1,13 |
| | ELESTAB®366 | | 0.43 |
| II. | Castor oil | Castor Oil | 35.00 |
| III. | IRWINOL® LS 9319 | African wild mango butter | 3.00 |
| * erhältlich von Lambert- Riviere (France) | | | |

Herstellung: Phase I wurde bei 85˚C geschmolzen, Phase II wurde zugefügt und die Temperatur auf 80˚C gehalten. Phase III wurde kurz vor dem Einfüllen in die Gießform (welche mit Dimethicon 50 cts befeuchtet und auf 40 ˚C vorgeheizt war) zugegeben. Die Masse wurde in die Gießform gegeben und auf 40 ˚C abgekühlt. Die Gießformen wurde im Gefrierschrank auf nahe 0 ˚C abgekühlt.

<u>Styling Wachs</u>

| Phase | Komponente Handelsname | INCI | Gew.-% |
|---|---|---|---|
| I. | CUTINA® MD | Glyceryl Stearate | 47,0 |
| | COMPERLAN® 100 | Cocamide MEA | 2,50 |
| | CUTINA® HR Powder | Hydrogenated Castor Oil | 2,50 |
| | PLANTACARE® 1200 UP | Lauryl Glucoside | 5,00 |
| | LANETTE® O | Cetearyl Alcohol | 7,00 |
| | CUTINA® CP | Cetyl Palmitate | 7,00 |
| | EUMULGIN® O 20 | O1eth-20 | 5,00 |
| | Kohlenwasserstoff Gemisch gemäß Herstellbeispiel 1 bis 5 | | 23,5 |
| | Wacker Siliconoil AK 350 | Dimethicone | 0,50 |

Die Herstellung erfolgt durch Erhitzen aller Komponenten auf 80 ˚C und Homogenisierung.

Feuchtigkeitsspendende Körpermilch

| Phase | Komponente Handelsname | INCI | Gew.-% |
|---|---|---|---|
| | EMULGADE® CM | Cetearyl Isononanoate (and) Ceteareth-20 (and) Cetearyl Alcohol (and)Glyceryl Stearate (and) Glycerin (and) Ceteareth | 5.0 |
| | EUMULGIN® VL 75 | Lauryl Glucoside (and) Polyglyceryl-2 Dipolyhydroxystearate (and) Glycerin | 2.0 |
| | CETIOL® OE | Dicaprylyl Ether | 4.0 |
| | CETIOL® J 600 | Oleyl Erucate | 1.0 |
| | ISOPROPYLMYRISTATE | Isopropyl Myristate | 7.0 |
| | Kohlenwasserstoff Gemisch gemäß Herstellbeispiel 1 bis 5 | | 7.0 |
| II. | Wasser, deionisiert | | ad 100 |
| III. | Cosmedia SP | Sodium Polyacrylate | 0.4 |
| IV. | HISPAGEL® 200 | Glycerin (and) Glyceryl Polyacrylate | 20.0 |
| V. | Konservierungsmittel, Parfum | | q.s. |
| | pH 5.5 | | |

Die Herstellung erfolgte durch Mischen von Phase I und Wasser bei Raumtemperatur unter Rühren. Dann wurde Phase III zugefügt und solange gerührt bis eine homogene, gequollene Mischung vorlag. Dann wurde Phase IV zugefügt, gefolgt von Phase V, danach wurde der pH Wert eingestellt.

O/W Soft Creme

| Phase | Komponente Handelsname | INCI | Gew.-% |
|---|---|---|---|
| I. | EMULGADE® SE-PF | Glyceryl Stearate (and) Ceteareth-20 (and) Ceteareth-12 (and) Stearyl Alcohol (and) Ceteareth-20 (and) Distearyl Ether | 6.0 |
| | LANETTE® O | Cetearyl Alcohol | 1.0 |
| | CUTINA® MD | Glyceryl Stearate | 2.0 |
| | CETIOL® MM | Myristyl Myristate | 2.0 |
| | Kohlenwasserstoff Gemisch gemäß Herstellbeispiel 1 bis 5 | | 8.0 |
| | Jojoba Oil | Simmondsia Chinensis (jojoba) Seed Oil | 2.0 |
| | COPHEROL® 1250 | Tocopheryl Acetate | 0.5 |
| | | Dimethicone | 0.5 |
| | | Cyclomethicone | 3.0 |
| II. | Wasser | Aqua | ad 100 |
| | | Propylene Glycol | 3.0 |
| III. | HISPAGEL® 200 | Glycerin (and) Glyceryl Polyacrylate | 15.0 |
| IV. | Konservierungsmittel | | q.s. |
| | pH 5.5-6.5 | | |

Diese Creme wurde hergestellt, indem Phase I auf 80˚C erhitzt wurde, Phase II wurde ebenfalls auf 80˚C erhitzt und zu Phase I unter Rühren hinzugefügt. Diese Mischung wurde unter Rühren abgekühlt und bei ca. 55˚C mit einem geeigneten Dispersionsgerät (z.B. Ultra Turrax) homogenisiert. Danach wurde Phase III unter kontinuierlichem Rühren hinzugefügt, Phase IV wurde zugegeben und der pH-Wert eingestellt.

W/O Creme

| Phase | Komponente / Handelsname | INCI | Gew.-% |
|---|---|---|---|
| I. | MONOMULS® 90 O 18 | Glyceryl Oleate | 2,00 |
| | LAMEFORM® TGI | Polyglyceryl 3 Diisostearate | 4,00 |
| | CETIOL® A | Hexyl Laurate | 12,00 |
| | Kohlenwasserstoff Gemisch gemäß Herstellbeispiel 1 bis 5 | | 12,00 |
| | SIPOL® C 16/18 OR | Cetearyl Alcohol | 1,00 |
| | Zinc stearate | Zinc Stearate | 2,00 |
| | Zinc Oxide | CI 77947 (or) Zinc Oxide | 15,00 |
| | Magnesium Sulphate | Magnesium Sulphate | 1,00 |
| | Glycerin | Glycerin | 3,00 |
| | Konservierungsmittel | | q.s. |
| | Benzyl Alcohol | Benzyl Alcohol | 0,40 |
| | HYDAGEN® B | Bisabolol | 0,50 |
| | Wasser | aqua | 100,00 |

Die ersten 7 Komponenten wurden bei 85˚C geschmolzen. Magnesium Sulfate und Glycerin wurden im Wasser gelöst und diese Mischung wurde auf 85 ˚C erhitzt. Diese wässrige Phase wurde zur Ölphase gegeben und dispergiert. Unter kontinuierlichem Rühren wurde bis auf 40˚C abgekühlt und dann wurden Benzyl Alkohol und Hydagen B gemischt und zu der Emulsion gegeben. Unter weiterem Rühren wurde bis auf 30˚C abgekühlt und homogenisiert.

"Body Wash" Reinigungsemulsion

| Phase | Komponente Handelsname | INCI | Gew.-% |
|---|---|---|---|
| I | Texapon ALS-IS | Ammonium Lauryl Sulfate | 30,00 |
| | TEXAPON® NSO | Sodium Laureth Sulfate | 18,00 |
| | Kohlenwasserstoff Gemisch gemäß Herstellbeispiel 1 bis 5 | | 18,00 |
| | Plantacare® 1200 | Lauryl Glucoside | 8,00 |
| II | Jaguar HP 105 | Hydroxypopyl Guar | 2,00 |
| | Euxyl K400 | MethyldibromoGlutaronitrile and Phenoxyethanol | 0,10 |
| | Wasser | Aqua | 23,90 |
| | pH-value | 5,6 | |

**Tabelle 1:** O/W-Sonnenschutzemulsionen Die im Folgenden genannten Beispiele enthalten alle das gemäß Herstellbeispiel 1 oder gemäß Herstellbeispiel 2 erhaltene Kohlenwasserstoff Gemisch. Alle Angaben sind in Gew.-%.

| **Komponente** | **1** | **2** | **3** | **4** | **5** | **6** | **7** | **8** | **9** | **10** | **11** |
|---|---|---|---|---|---|---|---|---|---|---|---|
| **L = Lotion, C = Creme,** | **L** | **C** | **S** | **L** | **C** | **L** | **L** | **C** | **L** | **C** | **L** |
| Eumulgin® VL 75 | | | | | | 4 | 4 | 2 | | | |
| Eumulgin® B2 | 2 | | | | | | | | | | |
| Tween® 60 | | | | 1 | | | | | | | |
| Myrj® 51 | | 3 | | 2 | | | | | | | |
| Cutina® E 24 | 1 | | | 1 | | | | | | | |
| Hostaphat® KL 340 N | | | | | | | | | 2 | | |
| Lanette® E | | | 0.5 | | | | | 0.5 | | | |
| Amphisol® K | | | 1 | | | 1 | | 0.5 | | 1 | |
| Natriumstearat | | | | | | | 1 | | | | 2 |
| Emulgade® PL 68/50 | | | 1 | | 5 | | | | | 4 | |
| Tego® Care 450 | | | | | | | | | | 3 | |
| Cutina® MD | 2 | | | 6 | | | 4 | | | 6 | |
| Lanette® 14 | 1 | | | 1 | | | | 2 | | | 4 |
| Lanette® O | 1 | 6 | | | 5 | 2 | | 2 | | | |
| Antaron V 216 | | | 1 | | 2 | 2 | | | | 1 | |
| Lanolin, wasserfrei USP | | | | | | | 5 | | | | |
| Kohlenwasserstoff Gemisch gemäß Herstellbsp. 1 bis 5 | 2 | 2 | 4 | 1 | 2 | 2 | 2 | 1 | 2 | 2 | 1 |
| Myritol® PC | | | | | 5 | | | | | | |
| Myritol® 331 | 5 | | 8 | | | 6 | | 10 | | 2 | |
| Finsolv® TN | | | 1 | | | | | 1 | 8 | | |
| Cetiol® CC | | 2 | 5 | | | 4 | 4 | 2 | | 2 | |
| Cetiol® OE | | | 3 | | | | | | 2 | 3 | |
| Dow Corning DC® 244 | 4 | | 1 | | 5 | | | 2 | | | 2 |
| Dow Corning DC® 2502 | | 1 | | | 2 | | | | | | |
| Silikonöl Wacker AK® 350 | | 2 | | | | | | | | | |
| Cetiol® 868 | | | | | 2 | | 8 | | | | 7 |
| Cetiol® J 600 | | | | | 3 | 2 | | | | 5 | |
| Mineralöl | | | | 9 | | | | | | | |
| Cetiol® B | | | 1 | | | | | | | 2 | |
| Eutanol® G | | | | | | | | | | | |
| Eutanol® G 16 | | | | | | | | | | | |
| Cetiol® PGL | | 5 | | | | | | | | 5 | |

(fortgesetzt)

| Komponente | 1 | 2 | 3 | 4 | 5 | 6 | 7 | 8 | 9 | 10 | 11 |
|---|---|---|---|---|---|---|---|---|---|---|---|
| Mandelöl | | | 2 | | | | 1 | | | | |
| Photonyl® LS | | | | 2 | | | | | | 2 | |
| Panthenol | 1 | | | | | | | | | | |
| Bisabolol | 0.2 | | | | | | | | | | |
| Tocopherol / Tocopherylacetat | 1 | | | | | | | | | | |
| Photonyl® LS | 1 | | | | | | | | | | |
| Neo Heliopan® Hydro (Na-Salz) | 2 | | 2.2 | | 3 | 3 | | | | | 2 |
| Neo Heliopan AP (Na-Salz) | 2 | | | | 1,5 | 2 | 2 | | 1 | | 1 |
| Neo Heliopan® 303 | 3 | 5 | 9 | 4 | | | | | | | |
| Neo Heliopan® BB | | | | | 1 | | | | | | 2 |
| Neo Heliopan® MBC | 2 | | | 3 | | 2 | 2 | 2 | | | 1 |
| Neo Heliopan® OS | | | | | | | | | 10 | 7 | |
| Neo Heliopan® E 1000 | | 7.5 | | 6 | | | | | | | 6 |
| Neo Heliopan® AV | | | 7.5 | | | 7.5 | 4 | 5 | | | |
| Uvinul® T 150 | 2 | | | | 2.5 | | | 1 | | | |
| Parsol® 1789 | | 1 | 1 | | | | 2 | | 2 | 2 | |
| Zinkoxid NDM | 10 | | 5 | | | 10 | | 3 | | 5 | 4 |
| Eusolex® T 2000 | | | | | 5 | | 3 | 3 | | | 4 |
| Veegum® Ultra | | | 0.75 | | | | | 1 | 1 | | |
| Keltrol® T | | | 0.25 | | | | | 0.5 | 0.5 | | |
| Carbopol® 980 | | 0.5 | | 0.2 | 0.2 | 0.2 | | 0.5 | 0.1 | 0.3 | 0.2 |
| Ethanol | | | | | | | | | | 10 | |
| Butylenglykol | | 2 | | 4 | 3 | | 2 | 5 | 2 | | 2 |
| Glycerin | 5 | 5 | 5 | | 3 | 3 | 2 | | 4 | | 3 |
| Konservierungsmittel, NaOH | q.s. | | | | | | | | | | |
| Wasser | Ad 100 | | | | | | | | | | |

**Tabelle 2: O/W-Sonnenschutzemulsionen**

| Komponente | 12 | 13 | 14 | 15 | 16 | 17 | 18 | 19 | 20 | 21 | 22 |
|---|---|---|---|---|---|---|---|---|---|---|---|
| **L = Lotion, C = Creme,** | **L** | **L** | **L** | **C** | **L** | **C** | **S** | **C** | **C** | **L** | **L** |
| Eumulgin® VL 75 | 4 | 3 | 4.5 | | 3 | | | | 4 | | |
| Eumulgin® B2 | | | | | | | | | | 1 | |
| Tween® 60 | | | | | | | | | | 1 | |
| Cutina® E 24 | | | | 2 | | | | | | | |

(fortgesetzt)

| **Komponente** | **12** | **13** | **14** | **15** | **16** | **17** | **18** | **19** | **20** | **21** | **22** |
|---|---|---|---|---|---|---|---|---|---|---|---|
| Hostaphat® KL 340 N | | | | | | | | | | 0.5 | |
| Lanette® E | 0.5 | | 0.5 | 0.5 | | | 0.1 | | 0.5 | | |
| Amphisol® K | 0.5 | | | | | 1 | 1 | 1 | | | |
| Natriumstearat | | | | | 1 | | | | | | |
| Emulgade® PL 68/50 | | 6 | | | | 4.5 | 1 | 5 | | | |
| Tego® Care 450 | 1 | | | | | | | | 4 | | |
| Cutina® MD | 1 | | | 8 | 6 | 1 | | | | 4 | 1 |
| Lanette® 14 | | 2 | | | | | | 2 | | 1 | |
| Lanette® O | | | | 2 | | | | | 1 | 1 | |
| Antaron V 220 | 1 | | | 2 | | | 0.5 | | | 2 | 0.5 |
| Kohlenwasserstoff-Gemisch nach | 4 | 2 | 4 | 6 | 10 | 4 | 2 | 8 | 2 | 1 | 3 |
| Herstellbsp. 1 bis 5 | | | | | | | | | | | |
| Myritol® PC | | | | | | | | | 5 | | |
| Myritol® 331 | 12 | | 12 | | | 8 | 8 | | | 10 | 8 |
| Finsolv® TN | | | | | 5 | | | 3 | 3 | | |
| Cetiol® CC | 6 | | 6 | | | 5 | 5 | | | | |
| Cetiol® OE | | | | | 2 | | | | | | 2 |
| Dow Corning DC® 244 | | 2 | | | 1 | | | | | | |
| Dow Corning DC® 2502 | | 1 | | | 1 | | | | | | |
| Ceraphyl® 45 | | | | | | | | | | 2 | 2 |
| Silikonöl Wacker AK® 50 | | | | | 1 | | | | | | |
| Cetiol® 868 | | 2 | | | | | | | | | |
| Cetiol® J 600 | | 2 | | | | | | | | | |
| Cetiol® B | 4 | | 4 | | | | | 4 | | | |
| Eutanol® G | | 3 | | 5 | | 3 | | | | | |
| Eutanol® G 16 S | 10 | | | | | | | | | | |
| Cetiol® PGL | | | | 5 | | | | | 2 | | |
| Photonyl® LS | | | | | | | | | | 2 | |
| Panthenol | | | | | | 1 | | | | | |
| Bisabolol | | | | | | 0,2 | | | | | |
| Tocopherol / Tocopherylacetat | | | | | | 1 | | | | | |
| Neo Heliopan® Hydro (Na-Salz) | | | | | | | | | | 3 | |
| Neo Heliopan AP (Na-Salz) | | 2 | | 2 | | | 2 | | | | 1 |
| Eusolex® OCR | 6 | | 9 | | 5 | 7 | 9 | | 4 | | 7 |
| Neo Heliopan® BB | | | | | | | | 1 | 1 | | 1 |
| Neo Heliopan® MBC | | 2 | | 1 | | | | 3 | 1 | | 3 |
| Neo Heliopan® OS | 2 | | | | | | | | 7 | | |
| Neo Heliopan® E1000 | | 4 | | | | | | 5 | | | |

(fortgesetzt)

| Komponente | 12 | 13 | 14 | 15 | 16 | 17 | 18 | 19 | 20 | 21 | 22 |
|---|---|---|---|---|---|---|---|---|---|---|---|
| Neo Heliopan® AV | | 4 | 7.5 | 5 | | | | 5 | 4 | 7.5 | |
| Uvinul® T 150 | 1 | | | | | | | | 1.3 | 1 | 1 |
| Parsol® 1789 | 1 | | | | | | | | 2 | | 1 |
| Z-Cote® HP 1 | 7 | 2 | 5 | | | 7 | 5 | | 6 | 2 | |
| Eusolex® T 2000 | 5 | 2 | | | 10 | | | 10 | | 2 | |
| Veegum® Ultra | 1.5 | | 1.5 | | | 1.5 | 1.2 | | 1 | | |
| Keltrol® T | 0.5 | | 0.5 | | | 0.5 | 0.4 | | 0.5 | | |
| Pemulen® TR 2 | | 0.3 | | 0.3 | | | 0.1 | 0.2 | | | 0.3 |
| Ethanol | | 5 | | 8 | | | | | | | |
| Butylenglykol | 1 | | | 3 | 3 | | | | | 8 | 1 |
| Glycerin | 2 | 4 | 3 | 3 | | 3 | 3 | 3 | 5 | | 3 |
| Wasser/ Konservierungsmittel/ NaOH | ad 100/ q.s./ q.s | | | | | | | | | | |

**Tabelle 3: W/O-Sonnenschutzemulsionen**

| Komponente | 23 | 24 | 25 | 26 | 27 | 28 | 29 | 30 | 31 | 32 | 33 |
|---|---|---|---|---|---|---|---|---|---|---|---|
| **L = Lotion; C = Creme** | **C** | **L** | **C** | **L** | **C** | **L** | **L** | **L** | **L** | **C** | **C** |
| Dehymuls® PGPH | 4 | 2 | 1 | 3 | 3 | 1 | 1 | 2 | 2 | 4 | 1 |
| Monomuls® 90-018 | | | 2 | | | | | | | | |
| Lameform® TGI | 2 | | 4 | | 3 | | | | | 1 | 3 |
| Abil® EM 90 | | | | | | | 4 | | | | |
| Glucate® DO | | | | | | | | | | | 3 |
| Isolan® PDI | | | | | | 4 | | 2 | | | |
| Arlacel® 83 | | | | 2 | | | | | | | |
| Elfacos® ST9 | | | | | | | | | 2 | | |
| Elfacos® ST37 | | | | | | | | | | | |
| Arlacel® P 135 | | 2 | | | | | | | | | |
| Dehymuls® HRE 7 | | | | | | | | | | | |
| Zinkstearat | 1 | | | 1 | 1 | | | 1 | | 1 | |
| Bienenwachs | 1 | | 2 | 1 | | 1 | | 3 | | 2 | 3 |
| Tego® Care CG | | | | | 1 | | | | | | .5 |
| Prisorine® 3505 | 1 | | 1 | 1 | | 1 | 1 | | | | 1 |
| Wollwachsalkohol, wasserfrei, USP | | | | | | | | | | | 1 |
| Antaron V 216 | 2 | | | | | | | | | | |
| Kohlenwasserstoff-Gemisch | 3 | 4 | 2 | 1 | 10 | 2 | 2 | 6 | 3 | 12 | 1 |
| nach Herstellbsp. 1 bis 5 | | | | | | | | | | | |
| Myritol® PC | | | | | 3 | | | 4 | | | |
| Myritol® 331 | 10 | | | | 3 | 6 | | | | | 8 |

(fortgesetzt)

| **Komponente** | **23** | **24** | **25** | **26** | **27** | **28** | **29** | **30** | **31** | **32** | **33** |
|---|---|---|---|---|---|---|---|---|---|---|---|
| Finsolv® TN | | | | 5 | | | 5 | | | | |
| Cetiol® CC | 12 | 22 | | | | 2 | | | 2 | | 5 |
| Cetiol® OE | | | | | 4 | | 5 | | 4 | 2 | |
| Dow Corning DC® 244 | | | | | | | 2 | | | | |
| Dow Corning DC® 2502 | | | 1 | | 2 | | | | | | |
| Prisorine® 3758 | | | | | | | | | | 2 | |
| Silikonöl Wacker AK® 350 | | | | 4 | | | | 3 | | | |
| Cetiol® 868 | | | | | | | | | | 2 | |
| Eutanol® G 16 | | 3 | | | | | | | | | |
| Eutanol® G 16S | | | | | | | | | | | |
| Cetiol® J 600 | | | 4 | | | 2 | | | | | |
| Ceraphyl® 45 | | | | 2 | | | | 2 | | 6 | |
| Cetiol® B | | | 2 | 4 | | | | | | 3 | |
| Eutanol® G | | | 3 | | 2 | | | 8 | | | |
| Cetiol® GL | | 11 | | | 2 | 4 | | | 9 | | |
| Mandelöl | | | | | 1 | | 5 | | | | |
| Photonyl® LS | | 2 | | 1 | | | | | 4 | | |
| Panthenol | 1.0 | | | | | | | | | | |
| Bisabolol | 0.2 | | | | | | | | | | |
| Tocopherol / Tocopherylacetat | 1,0 | | | | | | | | | | |
| Magnesiumsulfat x 7 Wasser | 1 | | | | | | | | | | |
| Neo Heliopan® Hydro (Na-Salz) | | 2 | | 3 | | | | 2 | | | |
| Neo Heliopan AP (Na-Salz) | 2 | 1 | | | 2 | | | 1 | 2 | | 1 |
| Neo Heliopan® 303 | | | | | 4 | | | | | 6 | |
| Neo Heliopan® BB | | 4 | 2 | | | | 2 | | | | |
| Neo Heliopan® MBC | | | | | | | | 4 | | 3 | |
| Neo Heliopan® OS | | | | | | | | | | | |
| Neo Heliopan® E 1000 | | | | | | | | | 5 | | |
| Neo Heliopan® AV | | 3 | 6 | 6 | | 7.5 | 7.5 | | 5 | | 7.5 |
| Uvinul® T 150 | | | | | 2.5 | | | 1 | | 2 | |
| Parsol® 1789 | | 2 | | | | | | 1 | | 2 | |
| Zinkoxid NDM | | | | | | 6 | | | | | |
| Eusolex® T 2000 | 15 | | 10 | | 5 | | 4 | | | | 4 |
| Ethanol | | | | | | | | | | 8 | |
| Butylenglykol | | | 2 | 6 | | | 2 | 5 | | | 2 |
| Glycerin | 5 | 3 | 3 | | 5 | 3 | 2 | | 10 | 4 | |
| Wasser, Konservierungsmittel | ad 1 00, q.s. | | | | | | | | | | |

**Tabelle 4: W/O-Sonnenschutzemulsionen**

| **Komponente** | **34** | **35** | **36** | **37** | **38** | **39** | **40** | **41** | **42** | **43** | **44** |
|---|---|---|---|---|---|---|---|---|---|---|---|
| **L = Lotion; C = Creme** | **L** | **C** | **L** | **L** | **C** | **L** | **L** | **L** | **L** | **C** | **C** |
| Dehymuls® PGPH | 3 | 1 | 5 | 1 | 1 | 3 | 2 | 4 | 0.5 | 1 | 4 |
| Monomuls® 90-O18 | | 1 | | | | | | | | | |
| Lameform® TGI | | | | | 4 | | | 1 | | 3 | 1 |
| Abil® EM 90 | | | | 1 | | | | | | 2 | |
| Glucate® DO | | | | 3 | | | | | 2 | | |
| Isolan® PDI | | 3 | | | | | 4 | | | | |
| Arlacel® 83 | | | | | | 3 | | | | | |
| Elfacos® ST9 | | | | | | | | | | | 2 |
| Elfacos® ST37 | 2 | | | | | | | | | | |
| Arlacel® P 135 | | | | | | 3 | | | | | |
| Dehymuls® HRE 7 | | | | | | | | | 4 | | |
| Zinkstearat | | 2 | 2 | 1 | 1 | | | 1 | 1 | | |
| Bienenwachs | | 1 | | 2 | 2 | | 3 | | 2 | 1 | 1 |
| Tego® Care CG | | | | | | | | | | | |
| Isostearinsäure | 1 | 1 | | | | | 1 | 1 | | 1 | 1 |
| Wollwachsalkohol, wasserfrei | | | | | | | | | | | |
| Antaron V 220 | | 0.5 | 2 | 1 | 1 | 1 | | | | | |
| Kohlenwasserstoff-Gemisch | 2 | 4 | 3 | 3 | 2 | 2 | 1 | 3 | 3 | 1 | 4 |
| nach Herstellbsp. 1 bis 5 | | | | | | | | | | | |
| Myritol® PC | | | | | | | | | | | |
| Myritol® 331 | 4 | 2 | 3 | | 5 | | | 8 | 5 | 4 | |
| Finsolv® TN | | 5 | 5 | | | 7 | | | | | |
| Cetiol® CC | 3 | 1 | | | | | 3 | 16 | | | 12 |
| Cetiol® OE | | 3 | | 2 | | | 3 | | | | |
| Dow Corning DC® 244 | | 4 | | 2 | | | | | | | |
| Dow Corning DC® 2502 | | | | 1 | | | | | | | |
| Prisorine® 3578 | | 1 | | | | | | | | | |
| Silikonöl Wacker AK® 350 | | | | 1 | | | | | | | |
| Cetiol® 868 | | | | | | | | | | | |
| Eutanol® G 16 | | | | | | | | | | | 3 |
| Eutanol® G 16S | | | | | | | | | | | 7 |
| Cetiol® J 600 | | | | 3 | | | | | | | |
| Ceraphyl® 45 | | | | 1 | | | | | 5 | 4 | |
| Cetiol® B | | | | | 3 | 3 | | 2 | 2 | | |
| Eutanol® G | | | | 2 | | | 4 | | 5 | | |
| Cetiol® PGL | | | | | | | 5 | 2 | | | |

(fortgesetzt)

| Komponente | 34 | 35 | 36 | 37 | 38 | 39 | 40 | 41 | 42 | 43 | 44 |
|---|---|---|---|---|---|---|---|---|---|---|---|
| Mandelöl | | | 2 | | | | | | | | |
| Photonyl® LS | | | | | | | 3 | | | | 2 |
| Panthenol | 1.0 | | | | | | | | | | |
| Bisabolol | 0.2 | | | | | | | | | | |
| Tocopherol / Tocopherylacetat | 1.0 | | | | | | | | | | |
| Magnesiumsulfat x 7 Wasser | 1 | | | | | | | | | | |
| Neo Heliopan® Hydro (Na-Salz) | | 4 | | | | | | 4 | | | |
| Neo Heliopan AP (Na-Salz) | 2 | | | 1 | 2 | | 1 | | | | |
| Neo Heliopan® 303 | 6 | 2 | | | | | | | 6 | | |
| Neo Heliopan® BB | | 2 | | 2 | | 2 | | | | | |
| Neo Heliopan® MBC | 2 | | | | 3 | | 4 | | 2 | | |
| Neo Heliopan® OS | | | | | 10 | | 8 | | | | |
| Neo Heliopan® E 1000 | | | 5 | 6 | | | | | | 5 | |
| Neo Heliopan® AV | | 5 | 5 | | | 7.5 | | | | 5 | |
| Uvinul® T 150 | 1 | | | 2 | 2 | | | | 3 | 2 | |
| Parsol® 1789 | | 1 | 1 | | | | 1 | | 0.5 | | |
| Z-Cote® HP 1 | 4 | 10 | | | | | | 5 | | | 5 |
| Titandioxid T 805 | | | | 2 | | 3 | | 7 | | 4 | 7 |
| Ethanol | | | | 8 | | 10 | | | | | |
| Butylenglykol | 5 | 1 | | 3 | 3 | | | | 8 | 2 | |
| Glycerin | | | 6 | 2 | | | 5 | 5 | | 3 | 5 |
| Wasser, Konservierungsmittel | ad 100, q.s. | | | | | | | | | | |

**Tabelle 5: W/O-Pflegeemulsionen**

| Komponente | 45 | 46 | 47 | 48 | 49 | 50 | 51 | 52 | 53 | 54 | 55 |
|---|---|---|---|---|---|---|---|---|---|---|---|
| **L = Lotion. C = Creme** | **C** | **L** | **C** | **L** | **C** | **L** | **L** | **L** | **C** | **C** | **C** |
| Dehymuls® PGPH | 1 | 3 | 1 | 2 | 3 | 1 | 1 | 2 | 1 | 1 | 1 |
| Monomuls® 90-018 | 2 | | | | | | | | 2 | | 2 |
| Lameform® TGI | 4 | 1 | | | 3 | | | 1 | 4 | 3 | 3 |
| Abil® EM 90 | | | | | | | 4 | | | | |
| Isolan® PDI | | | | | | 4 | | | | | |
| Glucate® DO | | | | 5 | | | | | | | |
| Arlacel® 83 | | | 5 | | | | | | | | |
| Dehymuls® FCE | | | | | | | | | | | |
| Dehymuls® HRE 7 | | | | | | | | 4 | | 1 | |
| Zinkstearat | 2 | 1 | | 1 | 1 | | | 1 | 1 | 1 | |
| Bienenwachs | 2 | | 3 | 1 | | 1 | | 1 | 3 | 2 | 1 |
| Tego Care® CG | | | | | 1 | | | | | | 0.5 |

(fortgesetzt)

| Komponente | 45 | 46 | 47 | 48 | 49 | 50 | 51 | 52 | 53 | 54 | 55 |
|---|---|---|---|---|---|---|---|---|---|---|---|
| Prisorine® 3505 | | | 1 | 1 | | 1 | 1 | | | | 1 |
| Dry Flo® Plus | | | | | | | | | | | |
| SFE 839 | | | | | | | 3 | | | | |
| Lanolin; anhydrous USP | | | 5 | | | | | | | 4 | |
| Kohlenwasserstoff-Gemisch | 3 | 4 | 2 | 12 | 10 | 2 | 2 | 6 | 3 | 12 | 1 |
| nach Herstellbsp. 1 bis 5 | | | | | | | | | | | |
| Cegesoft® C 17 | | | 3 | | | | | | | 1 | |
| Myritol® PC | | | | | | 2 | | 4 | | | |
| Myritol® 331 | 6 | | | | 2 | 6 | 2 | | | | |
| Finsolv® TN | | | | 5 | | 2 | 5 | | | | |
| Cetiol® A | | 6 | | | | 4 | | | | | |
| Cetiol® CC | | 8 | | | 2 | 2 | 2 | | | | 5 |
| Cetiol® SN | | 5 | | | | | | 3 | | | |
| Cetiol® OE | 3 | | | | 4 | | 2 | | 4 | 2 | 8 |
| Dow Corning DC® 244 | | | | | 1 | | 2 | | | | |
| Dow Corning DC® 2502 | | | 1 | | 2 | | | | | | |
| Prisorine® 3758 | | | | | 3 | | | | | | |
| Silikonöl Wacker AK® 350 | | | | 4 | | | | 3 | | | |
| Cetiol® 868 | | | | | | | | | | 2 | |
| Cetiol® J 600 | | | 4 | | | 2 | | | | | |
| Ceraphyl® 45 | | | | 2 | | | | 2 | | 6 | |
| Cetiol® B | | | 2 | 4 | | | | | | 3 | |
| Eutanol® G 16 | | 1 | | | 1 | | | | | 3 | 7 |
| Eutanol® G | | | 3 | | 1 | | | 8 | | | |
| Cetiol® PGL | | | | | | 4 | | | 9 | | |
| Mandelöl | | | | | 3 | | 5 | | | | |
| Insect Repellent® 3535 | 2 | | | | | | | | | | |
| N,N-Diethyl-m-toluamid | | | | 3 | | | | 5 | | | |
| Photonyl® LS | 2 | 2 | | | | | | | | | |
| Panthenol | 1.0 | | | | | | | | | | |
| Bisabolol | 0.2 | | | | | | | | | | |
| Tocopherol / Tocopheryl Acetate | 1.0 | | | | | | | | | | |
| Magnesiumsulfat x 7 Wasser | 1 | | | | | | | | | | |
| Bentone® 38 | | | | | 1 | | | | | | |
| Aluminum chlorohydrate | | 8 | | | | | | | | | |
| Propylencarbonat | | | | | 0.5 | | | | | | |
| Ethanol | | | | | | | | | | 8 | |
| Butylene Glycol | | | 2 | 6 | | | 2 | 5 | | | 2 |

(fortgesetzt)

| Komponente | 45 | 46 | 47 | 48 | 49 | 50 | 51 | 52 | 53 | 54 | 55 |
|---|---|---|---|---|---|---|---|---|---|---|---|
| Glycerin | 5 | 3 | 3 | | 5 | 3 | 2 | | 10 | 4 | |
| Wasser, Konservierungsmittel | ad 100, q.s. | | | | | | | | | | |

**Tabelle 6: W/O-Pflegeemulsionen**

| Komponente | 56 | 57 | 58 | 59 | 60 | 61 | 62 | 63 | 64 | 65 | 66 |
|---|---|---|---|---|---|---|---|---|---|---|---|
| **L = Lotion. C = Creme** | **L** | **C** | **L** | **L** | **L** | **L** | **L** | **L** | **L** | **C** | **C** |
| Dehymuls® PGPH | 3 | 1 | 5 | 1 | | 3 | 3 | 4 | 1 | 1 | 1 |
| Monomuls® 90-018 | | 1 | | 1 | | | | | | | |
| Lameform® TGI | | | | | 4 | | | 1 | | 3 | |
| Abil® EM 90 | | | | 3 | | | | | | 2 | |
| Isolan® PDI | | 3 | | | | | | | | | 4 |
| Glucate® DO | 1 | | | | | | | | | | |
| Arlacel® 83 | | | | | | 3 | | | | | |
| Dehymuls® FCE | | | | | 4 | | 1 | | | | |
| Dehymuls® HRE 7 | | | | | | | | | 7 | | |
| Zinkstearat | | 2 | 2 | 1 | 1 | 1 | | 1 | 1 | | 1 |
| Bienenwachs | | 4 | | 2 | 1 | 2 | 2 | 1 | 2 | 3 | 5 |
| Tego® Care CG | | | | | | | | | | | |
| Prisorine® 3505 | 1 | 1 | | | | | 1 | 1 | | 1 | 1 |
| Dry Flo® Plus | 1 | | | | | | | | | | |
| SFE® 839 | | 5 | | | | 4 | | | | | |
| Emery® 1780 | | | | | | | | | | | |
| Lanolin anhydrous USP | | 7 | 3 | | | | | | | | |
| Kohlenwasserstoff-Gemisch | 3 | 4 | 4 | 8 | 10 | 2 | 8 | 6 | 3 | 12 | 7 |
| nach Herstellbsp. 1 bis 5 | | | | | | | | | | | |
| Cegesoft® C 17 | | 2 | | | | | | | | | |
| Myritol® PC | | | | 8 | | | | | | | |
| Myritol® 331 | 4 | | 3 | | 5 | 3 | | | 5 | 4 | |
| Finsolv® TN | | | 5 | | | 7 | | | | | |
| Cetiol® A | | | | | | | | 6 | | | |
| Cetiol® CC | 3 | | | 6 | | 3 | 3 | | | 8 | |
| Cetiol® SN | | | | | 5 | | | | | | |
| Cetiol® OE | | 3 | | 2 | | | 3 | | | | 8 |
| Dow Corning® DC 244 | | 4 | | 2 | | 2 | | | | | |
| Dow Corning® DC 2502 | | | | 1 | | | | | | | |
| Prisorine® 3758 | | | | | | 1 | | | | | |
| Silikonöl Wacker AK® 350 | | | | 1 | | 1 | | 4 | | | |
| Cetiol® 868 | | | | | | | | | | | 10 |

(fortgesetzt)

| Komponente | 56 | 57 | 58 | 59 | 60 | 61 | 62 | 63 | 64 | 65 | 66 |
|---|---|---|---|---|---|---|---|---|---|---|---|
| Cetiol®J 600 | 4 | | | 3 | | | | | | | |
| Ceraphyl® 45 | | | | 1 | | | | | 5 | 4 | |
| Cetiol® B | | | | | 3 | 3 | | 2 | 2 | | |
| Eutanol® G 16 | 1 | | | | | | 3 | | | | |
| Eutanol® G | | | | 2 | | | 2 | | 5 | | |
| Cetiol® PGL | | | 10 | | | | 4 | 6 | | | 3 |
| Mandelöl | | | 2 | | 5 | | 2 | | | | |
| Photonyl® LS | | | | 2 | | | | | | | 2 |
| Panthenol | 1.0 | | | | | | | | | | |
| Bisabolol | 0.2 | | | | | | | | | | |
| Tocopherol / Tocopherylacetat | 1.0 | | | | | | | | | | |
| Magnesiumsulfat x 7 Wasser | 1 | | | | | | | | | | |
| Bentone® 38 | | | | | | 1 | | | | | |
| Propylencarbonat | | | | | | 0.5 | | | | | |
| Ethanol | | | | 8 | | 10 | | | | | |
| Butylenglykol | 5 | 1 | | 3 | 3 | | | | 8 | 2 | 1 |
| Glycerin | | | 6 | 2 | | | 5 | 5 | | 3 | 5 |
| Wasser, Konservierungsmittel | ad 100, q.s. | | | | | | | | | | |

**Tabelle 7: O/W-Pflegeemulsionen**

| Komponente | 67 | 68 | 69 | 70 | 71 | 72 | 73 | 74 | 75 | 76 | 77 |
|---|---|---|---|---|---|---|---|---|---|---|---|
| **L = Lotion, C = Creme** | **C** | **C** | **C** | **L** | **C** | **L** | **L** | **C** | **L** | **C** | **C** |
| Eumulgin® VL 75 | | | | | | 4 | | | | | |
| Dehymuls® PGPH | | 2 | | | | | | | | | |
| Generol® R | | | 1 | | | | | | | | |
| Eumulgin® B2 | | | 0.8 | | | | | | | | |
| Tween® 60 | | | | 1 | | | | | | | |
| Cutina® E 24 | | | 0.6 | 2 | | | | | | | |
| Hostaphat® KL 340 N | | | | | | | | | 2 | | |
| Lanette® E | | | | | | | | 1 | | | |
| Amphisol® K | | 0.5 | | | | 1 | | | | 1 | 0.5 |
| Natriumstearat | | | | | 0.5 | | | | | | |
| Emulgade® PL 68/50 | | 2.5 | | | | | | | | 4 | |
| Tego® are CG | | | | | | | | | | | 2 |
| Tego® Care 450 | | | | | | | | 5 | | | |
| Cutina® MD | | 1 | | 6 | 5 | | 4 | | | 6 | |
| Lanette® 14 | | | | 1 | | | | 2 | | | 4 |
| Lanette® O | 4.5 | | 4 | | 1 | 2 | | | | | 2 |

(fortgesetzt)

| Komponente | 67 | 68 | 69 | 70 | 71 | 72 | 73 | 74 | 75 | 76 | 77 |
|---|---|---|---|---|---|---|---|---|---|---|---|
| Novata® AB | | 1 | | | | | | | | | 1 |
| Lanolin, wasserfrei, USP | | | | | | | 5 | | | | |
| Cetiol® SB 45 | | | 1.5 | | | | 2 | | | | |
| Kohlenwasserstoff-Gemisch | 3 | 4 | 2 | 1 | 10 | 2 | 2 | 6 | 3 | 12 | 1 |
| nach Herstellbsp. 1 bis 5 | | | | | | | | | | | |
| Cegesoft® C 17 | | | | | | | | | | | |
| Myritol® PC | | | | | 5 | | | | | | |
| Myritol® 331 | 2 | 5 | | | | 6 | | 12 | | | |
| Finsolv® TN | | | | | | 2 | | | 8 | | |
| Cetiol® CC | 4 | 6 | | | | 4 | 4 | | | | 5 |
| Cetiol® OE | | | 7 | | | | | | 4 | 3 | |
| Dow Corning DC® 245 | | | 2 | | 5 | 1 | | | | | |
| Dow Corning DC® 2502 | | | | | 2 | 1 | | | | | |
| Prisorine® 3758 | | | | | | 1 | | | | | |
| Silikonöl Wacker AK® 350 | 0.5 | 0.5 | 0.5 | | | 1 | 4 | | | | |
| Cetiol® 868 | | | | | 2 | | 4 | | | | |
| Cetiol® J 600 | 2 | | 3 | | 3 | 2 | | | | 5 | |
| Ceraphyl® 45 | | | | | | | 3 | | | | |
| Cetiol® SN | | | 5 | | | | | | | | |
| Cetiol® B | | | | | | | | | | 2 | |
| Eutanol® G | | 2 | | 5 | | | | | | | |
| Cetiol® PGL | | | | 7 | | | | | 5 | 5 | |
| Dry Flo® Plus | 5 | | | | | | 1 | | | | |
| SFE 839 | 5 | | | | | | | | | | 2 |
| Mandelöl | | | | | | | 1 | | | | |
| Insect Repellent® 3535 | | 2 | 4 | | | 2 | | | | 3 | |
| N,N-Diethyl-m-toluamid | | 2 | | | | | | | | 3 | |
| Photonyl® LS | 2 | 2 | | | | 2 | | | | | |
| Panthenol | 1 | | | | | | | | | | |
| Bisabolol | 0.2 | | | | | | | | | | |
| Tocopherol / Tocopherylacetat | 1 | | | | | | | | | | |
| Veegum® ultra | | | | | | | | | 1 | | |
| Keltrol® T | | | 0.4 | | | | | | 0.5 | | |
| Pemulen® TR 2 | 0.3 | | | | | | | 0.3 | | | |
| Carbopol® Ultrez 10 | | | 0.3 | 0.2 | | 0.2 | | | 0.1 | 0.3 | |
| Cosmedia SP | | 0,3 | | | 0.2 | | | | | | 0.2 |
| Aluminum Chlorohydrate | | | 7 | | | | | | | | |
| Ethanol | | | | | | | | | | 10 | |

(fortgesetzt)

| Komponente | 67 | 68 | 69 | 70 | 71 | 72 | 73 | 74 | 75 | 76 | 77 |
|---|---|---|---|---|---|---|---|---|---|---|---|
| Butylenglykol | | | | 4 | 3 | | 2 | 5 | 2 | | |
| Glycerin | 2 | 5 | 5 | | 3 | 3 | 2 | | 4 | | 3 |
| Wasser, Konservierungsmittel NaOH 1, | ad 100, q.s.,pH 6,5 - 7,5 | | | | | | | | | | |

**Tabelle 8: O/W-Pflegeemulsionen**

| Komponente | 78 | 79 | 80 | 81 | 82 | 83 | 84 | 85 | 86 | 87 | 88 |
|---|---|---|---|---|---|---|---|---|---|---|---|
| **L = Lotion, C = Creme** | **C** | **C** | **L** | **C** | **L** | **C** | **L** | **L** | **L** | **L** | **C** |
| Eumulgin® L 75 | 4 | 3 | | | | | 1 | | | | 2 |
| Generol® R | | | | | | 2 | | | | | |
| Eumulgin® B2 | | | | | | 2 | | | | 1 | |
| Tween® 60 | | | | | | | | | | 1 | |
| Cutina® E 24 | | | | 2 | | | | | | | |
| Hostaphat® KL 340 N | | | | | | | | | | | |
| Lanette® E | 0.5 | | | | | | | | | | 1 |
| Amphisol® K | 0.5 | 1 | | | | | | 1 | 1 | | |
| Natriumstearat | | | | | 1 | | | | | | |
| Emulgade® PL 68/50 | | 6 | | | | | | 5 | | | 4 |
| Tego® Care CG | | | | | | | | | | | |
| Tego® Care 450 | | | | | | | | | 4 | | |
| Cutina® MD | 3 | | 3 | 8 | 6 | 8 | | | | 4 | |
| Lanette® 14 | | 2 | | | | | | 2 | | 1 | |
| Lanette® O | 2 | | | 2 | | 3 | 1 | | 1 | 1 | 6 |
| Novata® AB | | | | | | | | | | | |
| Lanolin, wasserfrei, USP | | | | | | 4 | | | | | |
| Cetiol® SB 45 | | | | | | | 2 | | | | |
| Kohlenwasserstoff-Gemisch nacht | 3 | 4 | 2 | 1 | 10 | 2 | 2 | 6 | 3 | 12 | 1 |
| Herstellbsp. 1 bis 5 | | | | | | | | | | | |
| Cegesoft® C 17 | 4 | | | | | | | | | | |
| Myritol® PC | 6 | | | | | 5 | | | 5 | | |
| Myritol® 331 | 5 | | 5 | | | | 7 | | | 10 | 3 |
| Finsolv® TN | | 5 | | | 5 | | | 3 | 3 | | 1 |
| Cetiol® CC | | | | | | | | | | | 2 |
| Cetiol® OE | | | | | 2 | | 2 | | 5 | | |
| Dow Corning DC® 245 | | 2 | | | 1 | | | | | 8 | 2 |
| Dow Corning DC® 2502 | | 1 | | | 1 | | | | | | 3 |
| Prisorine® 3758 | 3 | | | | | | | | | | 2 |
| Silikonöl Wacker AK® 350 | | | | | 1 | | | | | | 1 |

(fortgesetzt)

| Komponente | 78 | 79 | 80 | 81 | 82 | 83 | 84 | 85 | 86 | 87 | 88 |
|---|---|---|---|---|---|---|---|---|---|---|---|
| Cetiol® 868 | | 2 | | | | | | | | | |
| Cetiol® J 600 | | 2 | | | | | | | | | |
| Ceraphyl® 45 | | | | | | | 3 | | | | |
| Cetiol® SN | | | | | | | | | | | |
| Cetiol® B | | | 5 | | | 5 | | 4 | | | 3 |
| Eutanol® G | | 3 | 5 | | 5 | | | | | | |
| Cetiol® PGL | | | | | | | | 5 | 2 | | |
| Dry Flo® Plus | | 1 | | | | | | | | | 1 |
| SFE 839 | 1 | 1 | | | | | | | | | |
| Mandelöl | | | | | | 2 | | | | | |
| Photonyl® LS | | | | | | 2 | | | | | |
| Panthenol | 1 | | | | | | | | | | |
| Bisabolol | 0.2 | | | | | | | | | | |
| Tocopherol / Tocopherylacetat | 1 | | | | | | | | | | |
| Veegum® Ultra | | | | | | | | | 1 | | |
| Keltrol® T | | | | | | | | | 0.5 | | |
| Carbopol® ETD 2001 | | 0.3 | | 0.3 | | 0.5 | 0.2 | 0.2 | | | |
| Pemulen® TR 2 | | | 0.3 | | | 0.3 | | | | | 0.5 |
| Ethanol | | 5 | | 8 | | | | | | | 10 |
| Butylenglykol | 5 | | 2 | 3 | 3 | | | | | 8 | |
| Glycerin | 2 | 4 | 3 | 3 | | 7 | 5 | 3 | 5 | | |
| Wasser, Konservierungsmittel, NaOH | ad 100, q.s. (pH 6,5 - 7,5) | | | | | | | | | | |

**Tabelle 9: Sprayformulierungen**

| Komponente | 89 | 90 | 91 | 92 | 93 | 94 | 95 | 96 | 97 | 98 | 99 |
|---|---|---|---|---|---|---|---|---|---|---|---|
| **S = Körperspray, S* = Sonnenschutzspray** | **S** | **S** | **S** | **S** | **S** | **S*** | **S*** | **S*** | **S*** | **S*** | **S*** |
| Emulgade® SE-PF | 8,9 | | 7,5 | 7.5 | 4,3 | 9.8 | 8.2 | 9.9 | | | |
| Eumulgin® B2 | 3,1 | | 3 | | | | | 4.2 | | | |
| Eumulgin® B3 | | | | | | 4.2 | 3.3 | | | | |
| Eumulgin® HRE 40 | | | | | 4,7 | | | | | | |
| Cutina® E 24 | | 5,9 | | 4 | | | | | | | |
| Amphisol® K | | | | | | | | | 1 | 1 | 1 |
| Eumulgin® VL 75 | | | | | | | | | | | 2 |
| Emulgade® PL 68/50 | | 0.5 | | | | | | | 2.5 | 1 | |
| Cutina® MD | | 3,1 | | | | | | | | | |
| Antaron V 220 | | | | | | 1 | 1 | 1 | | 1 | 1 |

(fortgesetzt)

| **Komponente** | **89** | **90** | **91** | **92** | **93** | **94** | **95** | **96** | **97** | **98** | **99** |
|---|---|---|---|---|---|---|---|---|---|---|---|
| **S = Körperspray, S* = Sonnenschutzspray** | **S** | **S** | **S** | **S** | **S** | **S*** | **S*** | **S*** | **S*** | **S*** | **S*** |
| Kohlenwasserstoff-Gemisch gemäß Herstellbsp. 1 bis 5 | 11 | 5 | 7 | 7 | 7 | 5 | 4 | 5 | 5 | 4 | 6 |
| Myritoi® PC | | | | | | | | | | | |
| Myritol® 331 | | | 3 | 4 | 3 | 3 | 3 | 3 | | | |
| Finsolv® TN | | 4 | | | | | | 8 | | | |
| Cetiol® CC | 6 | | | 5 | 5 | 2 | 2 | 4 | | | |
| Cetiol® OE | | 5 | 7 | | | 2 | | | | | |
| Dow Corning DC® 244 | | 4 | 4 | 5 | | | | | | | |
| Cetiol® 868 | 3 | | | | | | | | | | |
| Cetiol® J 600 | | | | 2 | 2 | | | | | | |
| Cetiol® B | | | | | | | 2 | | | | |
| Eutanol® G | 2 | | | | 1 | | | | | | |
| Photonyl® LS | 2 | | | | | | 2 | | | 2 | 2 |
| Panthenol | 1 | | | | | | | | | | |
| Bisabolol | 0,2 | | | | | | | | | | |
| Tocopherol / Tocopherylacetat | 1 | | | | | | | | | | |
| Neo Heliopan® Hydro (Na-Salz) | | | | | | 2 | | | | 3 | |
| Neo Heliopan AP (Na-Salz) | | | | | | 2 | 2 | 2 | | | 1 |
| Eusolex® OCR | | | | | | | 2 | | | | 3 |
| Neo Heliopan® BB | | | | | | | | | | 1 | |
| Neo Heliopan® MBC | | | | | | 2 | 2 | 2 | | 1 | 1 |
| Neo Heliopan® OS | | | | | | 5 | | | | | |
| Neo Heliopan® AV | | | | | | 6 | 6 | 2 | | 7.5 | 2 |
| Uvinul® T 150 | | | | | | 1 | 1 | 1 | | 1 | |
| Parsol® 1789 | | | | | | 1 | | 1 | | 1 | |
| Z-Cote® HP 1 | | | | | | | | | | 2 | 2 |
| Eusolex® T 2000 | | | | | | | | | | 2 | 2 |
| Veegum® Ultra | | | | | | | | | | | 1.5 |
| Laponite® XLG | | | | | | | | | | 1.5 | |
| Keltrol® T | | | | | | | | | | | 0.5 |
| Pemulen® TR 2 | | | | | | | | | 0.2 | | |
| Insect Repellent® 3535 | 1 | | | | | | | | | | |
| N,N-Diethyl-m-toluamid | 1 | | | | | | | | | | |
| Ethanol | | | | | | | | | | | |
| Butylenglykol | | | | | | | 1 | | | 2 | 1 |
| Glycerin | | | | | | 3 | 2 | 3 | 2 | | 3 |

(fortgesetzt)

| Komponente | 89 | 90 | 91 | 92 | 93 | 94 | 95 | 96 | 97 | 98 | 99 |
|---|---|---|---|---|---|---|---|---|---|---|---|
| **S = Körperspray, S* = Sonnenschutzspray** | **S** | **S** | **S** | **S** | **S** | **S*** | **S*** | **S*** | **S*** | **S*** | **S*** |
| Wasser/ Konservierungsmittel/ NaOH | ad 100/ q.s./ q.s | | | | | | | | | | |

**Tabelle 10A: Antiperspirant Suspensionsstifte sowie Soft Solids - Mengenangaben in Gew.-% -**

| Zusammensetzung | 1 | 2 | 3 | 4 | 5 | 6 |
|---|---|---|---|---|---|---|
| Distearylether | - | 15 | - | - | - | - |
| Stearylalkohol | - | - | 15 | 10 | 14,7 | - |
| Guerbetalkohol C36 | 15 | - | - | - | - | - |
| Tribehenin | - | - | - | - | - | 20 |
| Hydrogenated Castor Oil | - | - | 4 | - | 3,7 | - |
| Erfindungsgemäßes Kohlenwasserstoff Gemisch nach Herstellbeispiel 1 bis 5 | 30 | 60 | 51 | 60 | 31,6 | 55 |
| Octyldodecanol | 5 | - | - | - | - | - |
| Dicaprylylether | 5 | - | - | - | - | - |
| Hexyldecanol + Hexyldecyl Laurate | - | 10 | - | - | - | - |
| Cyclomethicone | 20 | - | - | - | 30 | - |
| Dry Flo Plus* | - | - | 5 | - | - | - |
| Silica | - | - | - | 2,5 | - | - |
| Talc | - | - | - | 2,5 | - | - |
| Aluminium Zirconium Tetrachlorohydrex GLY | - | - | 25 | 25 | - | 25 |
| Aluminium Chlorohydrate | 25 | 15 | - | - | 20 | - |
| * National Starch | | | | | | |

**Tabelle 10B: Antiperspirant Suspensionsstifte sowie Soft Solids - Mengenangaben in Gew.-% -**

| Zusammensetzung | 7 | 8 |
|---|---|---|
| 12-Hydroxystearinsäure | 10 | 5 |
| Erfindungsgemäßes Kohlenwasserstoff Gemisch nach Herstellbsp. 1 bis 5 | 65 | 65 |
| Dry Flo Plus* | - | 5 |
| Aluminium Zirconium Tetrachlorohydrex GLY | 25 | 25 |
| * National Starch | | |

**Tabelle 11: Rezepturen 1 bis 13**

| **Komponenten INCI (Handelsname)** | **1** | **2** | **3** | **4** | **5** | **6** | **7** | **8** | **9** | **10** | **11** | **12** | **13** |
|---|---|---|---|---|---|---|---|---|---|---|---|---|---|
| Glyceryl Stearate, Ceteareth-20, Ceteareth-12, Cetearyl Alcohol, Cetyl Palmitate (Emulgade® E) | | | | | | | | | | | | 10.7 | 5.1 |
| Ceteareth-20 (Eumulgin® B2) | | | | | | | | | | | | 5.8 | 3.4 |
| Cetearyl Glucoside, Cetearyl Alcohol (Emulgade® PL 68/50) | 1 | | 1 | 1 | | 2 | 2 | | 2 | | 2 | | |
| Polyglyceryl-2 Dipolyhydroxystearate, Lauryl Glucoside, Glycerin (Eumulgin® VL 75) | | 1 | | | 1 | | | 3 | | 2.5 | | | |
| Sodium Cetearyl Sulfate (Lanette® E) | 1 | 1 | 1 | | 1 | | | | 1 | 1 | | | |
| Kohlenwasserstoff Gemisch nach Herstellbsp. 1 bis 5 | 5 | 4 | 8 | 3 | 5 | 8 | 4 | 2 | 4 | 3 | 5 | 10 | 2 |
| Dicaprylyl Carbonate (Cetiol® CC) | 5 | 5 | 5 | | | | | 4 | | 5 | 3 | 4 | |
| Cocoglycerides (Myritol® 331) | 3 | 4 | | 4 | 4 | | | | 5 | | | 3 | 3 |
| Dicaprylyl Ether (Cetiol® OE) | | | | | 5 | | 3 | | 2 | | | | |
| Dibutyl Adipate (Cetiol® B) | | | | 4 | | | | 4 | | 4 | | | |
| Hydrogenated Dimer Dilinoleyl/Dimethylcarbonate Copolymer (Cosmedia® DC) | 1 | 1 | 1 | 1 | 1 | 1.5 | 1.5 | 2 | 3 | 2 | 1.5 | 2 | 2 |
| Ethyl Butylacetylaminopropionate | | | | | | | | | | | 5 | 5 | |
| Tocopherol | 1 | 1 | 1 | 1 | 1 | 1 | 1 | 1 | 1 | 1 | 1 | 1 | 1 |
| Zinc oxide, nanonisiert, | 5 | 5 | 5 | 5 | 5 | | 2 | | 5 | 3 | | | |
| gecoated | | | | | | | | | | | | | |
| Titanium dioxide, nanonisiert, Gecoated | | | | 5 | 5 | | 2 | 3 | 5 | 2 | | | |
| Ethyl hexyl Methoxycinnamate | 7.5 | 7.5 | 7.5 | | | | 3 | 1 | 3 | 5 | | 5 | 5 |
| Octocrylene | 9 | 9 | 9 | | | 2 | 1 | | | | 2 | | 1.5 |
| Butyl Methoxydibenzoylmethane | | | | | | 2 | 2 | | | 1 | 2 | 2 | |
| 4-Methylbenzylidene Camphor | | | | | | | 2 | | | | | | 2 |
| Ethylhexyl Triazone | | | | | | 1 | 1 | 2 | | | 1 | | |
| Diethylhexyl Butamido Triazone | | | | | | 1 | 1 | 2 | | | 1 | 2 | |
| Phenylbenzimidazole Sulfonic Acid als Na-Salz, 15% wäßrige Lsg. | | | | | | | | | | | | | 13.3 |

(fortgesetzt)

| Komponenten INCI (Handelsname) | 1 | 2 | 3 | 4 | 5 | 6 | 7 | 8 | 9 | 10 | 11 | 12 | 13 |
|---|---|---|---|---|---|---|---|---|---|---|---|---|---|
| Glycerin | 3 | 3 | 3 | 3 | 3 | 3 | 3 | 3 | 3 | 3 | 3 | 5 | 5 |
| Magnesium aluminium silicate (and) cellulose Gum | 0.75 | 0.75 | | 0.5 | 0.5 | | 0.5 | | | 0.35 | | | |
| Xanthan Gum | 0.25 | 0.25 | | 0.5 | 0.5 | | 0.5 | | | 0.35 | | | |
| Sodium Polyacrylate (Cosmedia® SP) | | | 0.1 | | | 0.1 | 0.2 | | | | 0.1 | | |
| Acrylates/C10-30 Alkyl Acrylate Crosspolymer | | | | | | | | 0.2 | 0.1 | | | | |
| Wasser, Perfume, Preservatives | q.s. | q.s. | q.s. | q.s. | q.s. | q.s. | q.s. | q.s. | q.s. | q.s. | q.s. | q.s. | q.s. |

**Tabelle 12: Rezepturen 14 bis 26**

| **Komponenten INCI (Handelsname)** | **14** | **15** | **16** | **17** | **18** | **19** | **20** | **21** | **22** | **23** | **24** | **25** | **26** |
|---|---|---|---|---|---|---|---|---|---|---|---|---|---|
| Glyceryl Stearate, Ceteareth-20, Ceteareth-12, Cetearyl Alcohol, Cetyl Palmitate (Emulgade® SE) | 3.7 | 3.7 | | | | | | | | | | 4.9 | 4.1 |
| Ceteareth-12 (Eumulgin®B1) | 1.3 | 1.3 | | | | | | | | | | | |
| Ceteareth-20 (Eumulgin®B2) | | | | | | | | | | | | 1.1 | 0.9 |
| Cetearyl Glucoside, Cetearyl Alcohol (Emulgade® PL 68/50) | | | 5 | 1 | 1 | 1 | 1 | 3 | | | | | |
| Polyglyceryl-2 Dipolyhydroxystearate, Lauryl Glucoside, | | | | | | | | | 3 | 5 | 5 | | |
| Glycerin (Eumulgin® VL 75) | | | | | | | | | | | | | |
| Sodium Cetearyl Sulfate (Lanette® E) | | | | 0.25 | 0.25 | 0.25 | 0.25 | 0.25 | | | | | |
| Potassium Cetyl Phosphate | | | 0.5 | | | | | | | | | | |
| Kohlenwasserstoff Gemisch nach Herstellbsp. 1 bis 5 | 4 | 5 | 6 | 8 | 5 | 8 | 8 | 10 | 7 | 4 | 10 | 5 | 5 |
| Dicaprylyl Carbonate (Cetiol® CC) | 5 | | 5 | | | | | | 2.5 | 4 | 4 | 5 | 5 |
| Coco-Caprylate/Caprate (Cetiol® LC) | | | | 1 | 1 | 1 | 1 | 1 | | | | | |
| Caprylic/Capric Triglyceride (Myritol® 312) | | | | 1 | 1 | 1 | 1 | 1 | | | | | |
| Cocoglycerides (Myritol® 331) | | | | | | | | | | 4 | 4 | | |
| Cetearyl Isononanoate (Cetiol® SN) | 3 | 3 | 3.5 | | | | | | | | | | |
| Octyldodecanol (Eutanol® G) | | | | | | | | | 3.5 | 2 | 2 | | |
| Hexyldecanol (Eutanol® G16) | | | | 1 | 1 | 1 | 1 | 1 | | | | | |
| Olus (Cegesoft® PS6) | | 1.5 | 1.5 | | | | | | | | | | |
| Passiflora Incarnata (Cegesoft® PFO) | 1.5 | | | | | | | | | | | | |
| Dimethicone | | | | 0.5 | 0.5 | 0.5 | 0.5 | 0.5 | | | | | |
| Dimer Distearyltricarbonate (Cosmedia® DC) | 1 | | 1.5 | | | | | 1.5 | | 2,5 | 2,5 | | 0.5 |
| Triethyl Citrate (Hydagen® C.A.T) | | | | | | | | | | | | 1.5 | |
| Tocopherol | | | | | | | | | 0.5 | 0.5 | 0.5 | | |
| Tocopheryl Acetate | 0.5 | 0.5 | | | | | | | | | | | |
| Ethanol | | | | | | | | | | | 5 | | |

(fortgesetzt)

| Komponenten INCI (Handelsname) | 14 | 15 | 16 | 17 | 18 | 19 | 20 | 21 | 22 | 23 | 24 | 25 | 26 |
|---|---|---|---|---|---|---|---|---|---|---|---|---|---|
| Aluminum Chlorhydrate (Locron L) | | | | | | | | | | | | | 40 |
| Chitosan (Hydagen® DCMF) | | | | | | | | | | | | 0.1 | |
| Glycolic Acid | | | | | | | | | | | | 0.04 | |
| Glycerin | 2 | 2 | 2 | 3 | 3 | 3 | 3 | 3 | 2 | 2 | 2 | 3 | 2 |
| Potassium Hydroxyde, 20% wäßrige Lsg. | | | | | | 0.3 | 0.2 | 0.1 | 0.4 | 0.3 | 0.5 | | |
| Glycerin, Glyceryl Polyacrylate (Hispagel® 50) | | | | | | | | | | 10 | | | |
| Carbomer | | | | | | | 0.1 | | 0.2 | | 0.2 | | |
| Sodium Polyacrylate (Cosmedia® SP) | | | | | 0.15 | | | | | | | | |
| Acrylates/C10-30 Alkyl Acrylate Crosspolymer | | | | | | 0.15 | | 0.05 | | | | | |
| Wasser, Perfume, Preservatives | q.s | q.s. | q.s. | q.s. | q.s. | q.s. | q.s. | q.s. | q.s. | q.s. | q.s. | q.s. | |

**Tabelle 13: Rezepturen 27 bis 33 (Formulierungen für AP/Deo)**

| **Komponenten INCI (Handelsname)** | **27** | **28** | **29** | **30** | **31** | **32** | **33** |
|---|---|---|---|---|---|---|---|
| Glyceryl Stearate, Ceteareth-20, Ceteareth-12, Cetearyl Alcohol, Cetyl Palmitate (Emulgade® SE) | 6 | | 4,5 | | | 6 | |
| Ceteareth-20 (Eumulgin® B2) | | | 1 | | | | |
| Cetearyl Isononanoate, Glyceryl Stearate, Ceteareth-20, Ceteareth-12, Cetearyl Alcohol, Cetyl Palmitate (Emulgade® CM) | | | | | 20 | | |
| Polyglyceryl-3 Diisostearate Lameform TGI | | 3 | | | | | |
| Cocoglycerides (Novata® AB) | | | | | | | 4 |
| Stearyl alcohol (Lanette 18) | | | | 14,7 | | | |
| Hydrogenated Castor Oil (Cutina® HR) | | | | 3,7 | | | 6,5 |
| Polyglyceryl-2 Dipolyhydroxystearate, (Dehymuls® PGPH) | | 1 | | | | | |
| Sodium Cetearyl Sulfate (Lanette® E) | 0,3 | | | | | 0,3 | |
| Behenyl Alcohol (Lanette® 22) | 2 | | | | | 4 | |
| Kohlenwasserstoff Gemisch nach Herstellbsp. 1 bis 5 | 4 | 4 | 5 | 5 | 4 | 4 | 15 |
| Dicaprylyl Carbonate (Cetiol® CC) | | 3 | | | | | |
| Dicaprylylether (Cetiol® OE) | 2 | | | 4 | | 3 | 9 |
| Cocoglycerides (Myritol® 331) | | | | | | | |
| Cyclopentasiloxane | 3 | 5 | | 34 | | 2 | 14 |
| Cyclopentasiloxane and Dimethicone/Vinyldimethicone Crosspolymer SFE 839 (GE Bayer) | | 3 | | | | | |
| Dimethicone | 1 | | | | | | |
| Dimer Distearyltricarbonate (Cosmedia® DC) | 1 | 1 | 1 | 1 | 1 | 1 | 1 |
| Triethyl Citrate (Hydagen® C.A.T) | | | 2 | | | | |
| PEG-40 Hydrogenated Castor Oil | | | | | 1 | | |
| Tocopheryl Acetate | | | | 1 | | | |
| Aluminium Zirconium Tetrachlorohydrex GLY (Rezal 36) | 30 | 40 | | 22,9 | | 30 | 25 |
| Aluminum Chlorhydrate (Locron L) | | | 10 | | | | |
| Chitosan (Hydagen® DCMF) | 0,05 | | | | | | |
| Glycolic Acid | 0,02 | | | | | | |
| Glycerin | | 5 | 5 | | | | |
| Propylene Carbonate ( Fluka) | | | | | | | 0,5 |
| Quaternium-18 Hectorite (Bentone 18) | | | | | | | 1 |
| Talc (Merck) | | | | | | 5 | 5 |
| MgSO4x 7H2O | | 1 | | | | | |
| Wasser Phase II | 46,7 | | 35 | | | | |

(fortgesetzt)

| Komponenten INCI (Handelsname) | 27 | 28 | 29 | 30 | 31 | 32 | 33 |
|---|---|---|---|---|---|---|---|
| Wasser, Perfume, Preservatives | q.s. | q.s. | q.s. | q.s. | q.s. | q.s. | q.s. |
| 27 - Antiperspirant / Deo Creme / 28 - Antiperspirant Creme *(W/O)* / 29 - Antiperspirant /Deo Spray<br>30 - Antiperspirant Stift mit Vitamin E / 31 - Deodorant Wipe - Formulierung / 32 - Antiperspirant Creme<br>33 - Antiperspirant Creme «Soft Solid » | | | | | | | |

In der Tabelle 14 werden Sonnenschutzformulierungen vom Typ O/W beschrieben, in der Tabelle 15 werden Pflegeemulsionen beschrieben. Durch den Einsatz der erfindungsgemäßen Kohlenwasserstoff Gemische wird das sensorische Verhalten bei Applikation positiv beeinflusst. Die Mengenangaben beziehen sich jeweils auf Gew.-% der handelsüblichen Substanzen in der Gesamtzusammensetzung.

**Tabelle 14: O/W-Sonnenschutzemulsionen**

| Komponente | 12 | 13 | 14 | 15 | 16 | 17 | 18 | 19 | 20 | 21 | 22 |
|---|---|---|---|---|---|---|---|---|---|---|---|
| **L = Lotion, C = Creme, S = Spray** | L | C | L | C | L | C | S | C | C | L | L |
| Eumulgin® VL 75 | 2 | | | | 3 | | | | 1 | | |
| Eumulgin® B2 | | | | 2 | | | | | | 1 | |
| Tween® 60 | | | | | | | | | | 1 | |
| Cutina® E 24 | | | | 0.5 | | | | | | | |
| Hostaphat® KL 340 N | | | | | | | | | | | 0.5 |
| Eumulgin® SG | | | 0,5 | | | 0,5 | | 0,3 | 0,1 | | |
| Lanette® E | | | | | | | 0.1 | | 0.5 | | |
| Amphisol® K | 0.5 | | | | | | 1 | | | | |
| Natriumstearat | | | | | 1 | | | | | | |
| Emulgade® PL 68/50 | | 2 | 1 | | | 3 | | | | | |
| Tego® Care 450 | | 2 | | | | | | | 2 | | |
| Cutina® MD | | | | 2 | 1 | 3 | | | | | 1 |
| Lanette® 14 | | 1 | | | | | | | | | |
| Lanette® O | | | | 2 | | | | 2 | 1 | 1 | |
| Cutina® ES | 1 | 1 | | 2 | | | | | | 1 | |
| Allianz® OPT | 1 | | | 1 | 1 | | | 2 | | | 2 |
| Cosmedia® DC | | 1.5 | 2 | | | 1.5 | 2 | | 1.5 | 1.5 | |
| Lanolin, wasserfrei, USP | | | | | | 1 | 1 | | | | |
| Kohlenwasserstoff Gemisch nach | 6 | 2 | 4 | 7 | 3 | 7 | 6 | 6 | 4 | 4 | 5 |
| Herstellbsp. 1 bis 5 | | | | | | | | | | | |
| Myritol® PC | | | | | | | | | 5 | | |
| Myritol® 331 | 6 | | 4 | | | 5 | 8 | | | 10 | 8 |
| Finsol® TN | | | | | 5 | | | 3 | 3 | | |
| Cetiol® CC | 6 | | 6 | | | 5 | 5 | | | | |
| Cetiol® OE | | | | | 2 | | | | | | 2 |
| Dow Corning DC® 244 | | 2 | | | 1 | | | | | | |
| Dow Corning DC® 2502 | | 1 | | | 1 | | | 3 | | | |

(fortgesetzt)

| Komponente | 12 | 13 | 14 | 15 | 16 | 17 | 18 | 19 | 20 | 21 | 22 |
|---|---|---|---|---|---|---|---|---|---|---|---|
| Ceraphyl® 45 | | | | | | | | | | 2 | 2 |
| Silikonöl Wacker AK® 350 | | | | | 1 | | | | | | |
| Cetiol® 868 | | 2 | | | | | | | | | |
| Cetiol® J 600 | | 2 | | | | | | | | | |
| Cetiol® B | 4 | | 4 | | | | | 4 | | | |
| Eutanol® G | | 3 | | | | 3 | | | | | |
| Eutanol® G 16 S | 3 | | | | | | | | | | |
| Cetiol® PGL | | | | | | | | | 2 | | |
| Photonyl® LS | | | | | | | | | | 2 | |
| Panthenol | 1 | | | | | | | | | | |
| Bisabolol | 0,2 | | | | | | | | | | |
| Tocopherol / Tocopherylacetat | 1 | | | | | | | | | | |
| Neo Heliopan® Hydro (Na-Salz) | | | | | | | | | | 3 | |
| Eusolex® OCR | 6 | | 9 | | 5 | 7 | 9 | | 4 | | 7 |
| Neo Heliopan® AP (Na-Salz) | | | | 0.5 | | 1 | | | | | |
| Neo Heliopan® BB | | | | | | | | 1 | 1 | | 1 |
| Neo Heliopan® MBC | | 2 | | 1 | | | | 3 | 1 | | 3 |
| Neo Heliopan® OS | 2 | | | | | | | | 7 | | |
| Neo Heliopan® E1000 | | 4 | | | | | | 5 | | | |
| Neo Heliopan® AV | | 4 | 7.5 | 5 | | | | 5 | 4 | 7.5 | |
| Uvinul® A PLUS | | | | | 1 | | 2 | | | | |
| Uvinul® T 150 | 1 | | | | | | | | 1.3 | 1 | 1 |
| Tinosorb® M | | 2 | | | 2 | | 2 | | | | |
| Tinosorb® S | | 1 | | | 2 | | 2 | | | | |
| Parsol® 1789 | 1 | | | | | | | | 2 | | 1 |
| Z-Cote® HP 1 | 7 | 2 | 5 | | | 7 | 5 | | 6 | 2 | |
| Eusolex® T 2000 | 5 | 2 | | | 10 | | | 10 | | 2 | |
| Veegum® Ultra | 1.5 | | 1.5 | | | 1.5 | 1.2 | | 1 | | |
| Keltrol® T | 0.5 | | 0.5 | | | 0.5 | 0.4 | | 0.5 | | |
| Cosmedia® SP | | | 0.2 | 0.3 | | | 0.1 | | | 0.2 | |
| Pemulen® TR 2 | | 0.3 | | 0.3 | | | | 0.2 | | | 0.3 |
| Ethanol | | 5 | | 8 | | | | | | | |
| Butylenglykol | 1 | | | 3 | 3 | | | | | 8 | 1 |
| Glycerin | 2 | 4 | 3 | 3 | | 3 | 3 | 3 | 5 | | 3 |
| Wasser/ Konservierungsmittel/ NaOH | ad 100/ q.s./ q.s | | | | | | | | | | |

**Tabelle 15: 0/W-PHeseemutsionen**

| Komponente | 34 | 35 | 36 | 37 | 38 | 39 | 40 | 41 | 42 | 43 | 44 |
|---|---|---|---|---|---|---|---|---|---|---|---|
| **L = Lotion, C = Creme** | C | C | L | C | L | C | L | L | L | L | C |
| Eumulgin® VL 75 | | | 5 | | 4 | | | | | | 2 |
| Generol® R | | | | | | 2 | | | | | |
| Eumulgin® B2 | | | | | | | | | | 1 | |
| Tween® 60 | | | | | | | | | | 1 | |
| Cutina® E 24 | | | | 0.5 | | | | | | | |
| Eumulgin® SG | | | 0,1 | 0,5 | | 0,4 | | 0,2 | 0,1 | | |
| Lanette® E | 0.5 | | | | | | | | | | |
| Amphisol® K | 0,5 | 0.5 | | | | | | | | | |
| Natriumstearat | | | | | 1 | | | | | | |
| Emulgade® L 68/50 | | 2 | | 2 | | | | 3 | 4 | | |
| Tego® Care 450 | | 1 | | | | | | | 1 | | |
| Cutina® MD | 2 | 1 | 1 | 1 | | 5 | | | | 2 | |
| Lanette® 14 | | | | | 1 | | | 2 | | 1 | |
| Lanette® O | 2 | | | 2 | 1 | 3 | 1 | | 1 | 1 | 3 |
| Cutina® PES | 1 | 2 | | 3 | 1 | | | | | | 3 |
| Novata® AB | | | | | | | | | 1 | 1 | |
| Lanolin, wasserfrei, USP | | | | | | 4 | | | | | |
| Cosmedia® DC | | | 2 | | | 1.5 | | | 1 | 1 | |
| Cetiol® SB 45 | | | | | | | 2 | | | | |
| Cegesoft® C 17 | 2 | | | | | | | | | | |
| Kohlenwasserstoff Gemisch nach Herstellbsp. 1 bis 5 | 5 | 5 | 4 | 4 | 3 | 4 | 5 | 4 | 5 | 10 | 2 |
| Myritol® PC | 6 | | | | | 5 | | | | | |
| Myritol® 331 | 2 | | 5 | | | | 2 | | | | 3 |
| Finsolv® TN | | | | 3 | 5 | | | 3 | 3 | | 1 |
| Cetiol® CC | | | | 3 | | | 4 | 3 | | | |
| Cetiol® OE | | | | | 2 | | 2 | | 5 | | |
| Dow Corning DC® 245 | | 2 | | | 1 | 4 | | | | 8 | 2 |
| Dow Corning DC® 2502 | | 1 | | | 1 | | | | | | 3 |
| Prisorine® 3758 | 3 | | | | | | | | | | 2 |
| Silikonöl Wacker AK® 350 | | | | | 1 | | | | | | 1 |
| Cetiol® 868 | | 2 | | | | | | | | | |
| Cetiol® J 600 | | 2 | | 2 | | | | | | | |
| Ceraphyl® 45 | | | | | | | 3 | | | | |
| Cetiol® SN | | | | 5 | | | | | | | |
| Cetiol® B | | | 5 | | | 5 | | 4 | | | 3 |
| Eutanol® G | | 3 | 5 | | 5 | | | | | | |

(fortgesetzt)

| Komponente | 34 | 35 | 36 | 37 | 38 | 39 | 40 | 41 | 42 | 43 | 44 |
|---|---|---|---|---|---|---|---|---|---|---|---|
| Cetiol® PGL | | | | | | | | 5 | 2 | | |
| Dry Flo® Plus | | 1 | | | | | | | | | 1 |
| SFE 839 | 1 | 1 | | | | | | | | | |
| Mandelöl | | | | | | 2 | | | | | |
| Photonyl® LS | | | | | | 2 | | | | | |
| Panthenol | | | | | | 1 | | | | | |
| Bisabolol | | | | | | 0.2 | | | | | |
| Tocopherol / Tocopherylacetat | | | | | | 1 | | | | | |
| Veegum® Ultra | | | | | | | | | 1 | | |
| Keltrol® T | | | | | | | | | 0.5 | | |
| Cosmedia® SP | 0.5 | | | | | 0.5 | 0.5 | 0.2 | | | 0.5 |
| Carbopol® ETD 2001 | | 0.3 | | 0.3 | | | | | | | |
| Pemulen® TR 2 | | | 0.3 | | | 0.3 | | | | | |
| Ethanol | | 5 | | 8 | | | | | | | 10 |
| Butylenglykol | 5 | | 2 | 3 | 3 | | | | | 8 | |
| Glycerin | 2 | 4 | 3 | 3 | | 7 | 5 | 3 | 5 | | |
| Wasser, Konservierungsmittel, NaOH | ad 100, q.s. (pH 6,5 - 7,5) | | | | | | | | | | |

**Formulierungen für den Sonnenschutz und die Hautpflege vom Typ Wasser in Öl**

In Tabelle 16 werden Sonnenschutzformulierungen vom W/O Emulsionstyp, in Tabelle 17 werden Pflegeemulsionen beschrieben. Durch den Einsatz der Kohlenwasserstoff Gemische wird das sensorische Verhalten bei Applikation positiv beeinflusst. Die Mengenangaben beziehen sich jeweils auf Gew.-% der handelsüblichen Substanzen in der Gesamtzusammensetzung.

**Tabelle 16: W/O - Sonnenschutzformulierungen**

| Ingredient | 1 | 2 | 3 | 4 | 5 | 6 | 7 | 8 | 9 | 10 | 11 |
|---|---|---|---|---|---|---|---|---|---|---|---|
| L = Lotion; C = Creme | C | L | C | L | C | L | L | L | L | C | C |
| Dehymuls® PGPH | 4 | 2 | 1 | 3 | 3 | 1 | 1 | 2 | 2 | 4 | 1 |
| Monomuls® 90-O18 | | | 2 | | | | | | | | |
| Lameform® TGI | 2 | | 4 | | 3 | | | | | 1 | 3 |
| Abil® EM 90 | | | | | | | 4 | | | | |
| Isolan® PDI | | | | | | 4 | | 2 | | | |
| Zinkstearat | 1 | | | 1 | 1 | | | 1 | | 1 | |
| Bienenwachs | 1 | | 5 | 1 | | | | 5 | | 7 | 5 |
| Tego® Care CG | | | | | 1 | | | | | | 0.5 |
| Prisorine® 3505 | | | 1 | | | 1 | 1 | | | | 1 |
| Cosmedia DC | 3 | 4 | 2 | 1 | 1 | 2 | 2 | 2 | 3 | 1 | 1 |
| Kohlenwasserstoff Gemisch nach Herstellbsp. 1 bis 5 | 5 | 4 | 4 | 3 | 2 | 4 | 3 | 4 | 2 | 3 | 5 |

(fortgesetzt)

| Ingredient | 1 | 2 | 3 | 4 | 5 | 6 | 7 | 8 | 9 | 10 | 11 |
|---|---|---|---|---|---|---|---|---|---|---|---|
| Myritol® 331 | 2 | | | | 3 | 6 | | | | | 3 |
| Finsolv® TN | | | | 5 | | | 2 | | | | |
| Cetiol® CC | 5 | | 2 | | 4 | 2 | | | 2 | 3 | 5 |
| Tegosoft DEC | | 4 | | 3 | | | 5 | 5 | | | |
| Cetiol® OE | | | | | 4 | | 5 | | 4 | 2 | |
| Dow Corning® DC 244 | | | 3 | | | | 2 | | 2 | 4 | |
| Dow Corning® DC 2502 | 1 | | 1 | | 2 | 1 | | | | | 1 |
| Silikonöl Wacker AK 350 | | 1 | | 4 | | | | 3 | | | |
| Cetiol® PGL | | 1 3 | | | | 2 | | | 4 | | |
| Cophero® F 1300 | | | | | | 1 | | | | | |
| Magnesium sulfat x 7 $H_2O$ | | | | | | 1 | | | | | |
| Neo Heliopan® Hydro (Na-Salz) | 2 | | 2.2 | | 3 | 3 | | | 1 | | 2 |
| Neo Heliopan® 303 | | 5 | | | | | | | 4 | | 4 |
| Uvasorb® HEB | 1 | | | 1 | 1 | | | | | | 2 |
| Neo Heliopan® MBC | 2 | | | | | 2 | 2 | 2 | | | |
| Uvinul® A plus | | | | | 2 | | | | 3 | 3 | |
| Neo Heliopan® AP (Na-Salz) | | 2 | 2 | | 1 | | | | 1 | | 6 |
| Neo Heliopan® AV | 3 | | 4 | 6 | 4 | 7.5 | 4 | 5 | | | 1 |
| Uvinul®T 150 | 1 | 1 | | | 2.5 | | | 1 | | | |
| Parsol® 1789 | 2 | 1 | | | | | 2 | | 2 | 2 | |
| Zinkoxid NDM | | | | | | 10 | | 3 | | | 4 |
| Tinosorb® M | | 3 | | 3 | | | | 2 | | 2 | |
| Tinosorb® S | | 3 | | 3 | | | | 2 | | 2 | |
| Eusolex® T Aqua | | | 8 | | | | | 5 | | | |
| Eusolex® T 2000 | | | | | 5 | | 3 | 3 | | | 4 |
| Ethanol | | | | | | | | | | 8 | |
| Glycerin | 5 | 3 | 3 | 3 | 5 | 3 | 2 | 3 | 10 | 4 | 3 |
| Wasser, Konservierungsmittel | | | | | | ad 100, q.s. | | | | | |

**Tabelle 17: W/O-Pflegeemulsionen**

| Komponente | 55 | 56 | 57 | 58 | 59 | 60 | 61 | 62 | 63 | 64 | 65 |
|---|---|---|---|---|---|---|---|---|---|---|---|
| **L = Lotion. C = Creme** | C | L | C | L | C | L | L | L | C | C | C |
| Dehymuls® PGPH | 1 | 3 | 1 | 2 | 3 | 1 | 1 | 2 | 1 | 1 | 1 |
| Monomuls® 90-018 | 2 | | | | | | | | 2 | | 2 |
| Lameform® TGI | 4 | 1 | | | 3 | | | 1 | 4 | 3 | 3 |
| Abil® EM 90 | | | | | | | 4 | | | | |
| Isolan® PDI | | | | | | 4 | | | | | |
| Glucate® DO | | | | 5 | | | | | | | |

(fortgesetzt)

| **Komponente** | **55** | **56** | **57** | **58** | **59** | **60** | **61** | **62** | **63** | **64** | **65** |
|---|---|---|---|---|---|---|---|---|---|---|---|
| Arlacel® 83 | | | 5 | | | | | | | | |
| Dehymuls® FCE | | | | | | | | | | | |
| Dehymuls® HRE 7 | | | | | | | | 4 | | 1 | |
| Zinkstearat | 2 | 1 | | 1 | 1 | | | 1 | 1 | 1 | |
| Bienenwachs | 4 | | | 1 | | | | 1 | 4 | 7 | |
| Tego Care® CG | | | | | 1 | | | | | | 0.5 |
| Prisorine® 3505 | | | 1 | 1 | | 1 | 1 | | | | 1 |
| Dry Flo® Plus | | | | | | | | | | | |
| SFE 839 | | | | | | | 3 | | | | |
| Lanolin; anhydrous USP | | | 5 | | | | | | | 4 | |
| Kohlenwasserstoff Gemisch nach Herstellbsp. 1 bis 5 | 3 | 4 | 2 | 12 | 10 | 2 | 2 | 6 | 3 | 12 | 1 |
| Cegesoft® C 17 | | | 3 | | | | | | | 1 | |
| Myritol® PC | | | | | | 2 | | 4 | | | |
| Myritol® 331 | 6 | | | | 2 | 6 | 2 | | | | 8 |
| Finsolv® TN | | | | 5 | | 2 | 5 | | | | |
| Cetiol® A | | 6 | | | | 4 | | | | | |
| Cetiol® CC | | 8 | | | 2 | 2 | 2 | | | | 5 |
| Cetiol® SN | | 5 | | | | | | 3 | | | |
| Cetiol® OE | 3 | | | | 4 | | 2 | | 4 | 2 | |
| Dow Corning DC® 244 | | | | | 1 | | 2 | | | | |
| Dow Corning DC® 2502 | | | 1 | | 2 | | | | | | |
| Prisorine® 3758 | | | | | 3 | | | | | | |
| Silikonöl Wacker AK® 350 | | | | 4 | | | | 3 | | | |
| Cetiol® 868 | | | | | | | | | | 2 | 7 |
| Cetiol® J 600 | | | 4 | | | 2 | | | | | |
| Ceraphyl® 45 | | | | 2 | | | | 2 | | 6 | |
| Cetiol® B | | | 2 | 4 | | | | | | 3 | |
| Eutanol® G 16 | | 1 | | | | | | | | 3 | |
| Eutanol® G | | | 3 | | | | | 8 | | | |
| Cetiol® PGL | | | | | | 4 | | | 9 | | |
| Mandelöl | | | | | 1 | | 5 | | | | |
| Insect Repellent® 3535 | 2 | | | | | | | | | | |
| N,N-Diethyl-m-toluamid | | | | 3 | | | | 5 | | | |
| Photonyl® LS | 2 | 2 | | | | | | | | | |
| Panthenol | 1.0 | | | | | | | | | | |
| Bisabolol | 0.2 | | | | | | | | | | |
| **Tocopherol / Tocopheryl Acetate** | 1.0 | | | | | | | | | | |

(fortgesetzt)

| Komponente | 55 | 56 | 57 | 58 | 59 | 60 | 61 | 62 | 63 | 64 | 65 |
|---|---|---|---|---|---|---|---|---|---|---|---|
| Magnesiumsulfat x 7 Wasser | 1 | | | | | | | | | | |
| Bentone® 38 | | | | | 1 | | | | | | |
| Propylencarbonat | | | | | 0.5 | | | | | | |
| Ethanol | | | | | | | | | | 8 | |
| Butylene Glycol | | | 2 | 6 | | | 2 | 5 | | | 2 |
| Glycerin | 5 | 3 | 3 | | 5 | 3 | 2 | | 10 | 4 | |
| Wasser, Konservierungsmittel | Ad 100, q.s. | | | | | | | | | | |

**Tabelle 18**

| Komponente | 1 | 2 | 3 | 4 | 5 | 6 | 7 |
|---|---|---|---|---|---|---|---|
| Ethanol | | 4,00 | | 3,00 | | 4,50 | |
| Wasser dem. (add to) | 100 | 100 | 100 | 100 | 100 | 100 | 100 |
| Phenonip | 1,00 | 1,00 | 1,00 | 1,00 | 1,00 | 1,00 | 1,00 |
| Glycerin 99% | 3,00 | 3,00 | 3,00 | 3,00 | 3,00 | 3,00 | 3,00 |
| Ultragel® 300 (Polyquaternium-37) | 0,8 | 1,00 | 1,50 | 0,90 | 2,00 | 1,50 | 0,75 |
| Eumulgin® VL 75 (Lauryl Glucoside (and) Polyglyceryl-2 Dipolyhydroxystearate (and) Glycerin) | 1,80 | - | 0,80 | - | - | - | - |
| Emulgade® PL 68/50 (Cetearyl Glucoside (and Cetearyl Alcohol) | - | - | - | - | - | 3,00 | - |
| Eumulgin® SG (Sodium Stearylglutamate) | 0,10 | 0,12 | 0,30 | 0,10 | - | - | 0,08 |
| Cutina® PES (Pentaerythrityl Distearate) | 2,50 | 3,5 0 | 1,70 | 4,00 | 2,00 | 4,00 | 5,00 |
| Cetiol® J600 (Oleyl Erucate) | 2,00 | - | 5,00 | - | - | 1,00 | 3,00 |
| Cetiol® CC (Dicaprylyl Carbonate) | 3,50 | - | 4,00 | 4,00 | 2,50 | 3,00 | 4,00 |
| Cetiol® PGL (Hexyldecanol (and) Hexyldecyl Laurate) | - | 2,00 | - | 4,00 | 5,00 | 5,00 | 1,00 |
| Tegosoft® DEC (Diethylhexyl Carbonate) | - | | - | 2,50 | - | - | 3,00 |
| DC® 345 (Cyclopentasiloxane) | 4,00 | - | - | - | 2,00 | - | - |
| Cetiol® B (Dibutyl Adipate) | 5,00 | 3,5 | 5,00 | - | - | 2,00 | - |
| Myritol® 331 (Cocoglycerides) | 3,00 | - | - | - | - | 4,00 | 4,50 |
| Kohlenwasserstoff Gemisch nach Herstellbeispiel 1 bis 5 | 3,00- | 3,00 | 5,00 | 2,00 | 7,00 | 5,00 | 3,00- |
| DHA (Dihydroxyaceton) | 2,00 | 1,00 | 2,50 | 1,00 | 2,00 | 2,00 | 3,50 |
| Uvinul® T 150 (Ethylhexyl Triazone) | | | 1,00 | - | - | - | - |
| Tinosorb® M (Methylene Bis-Benzotriazoyl Tetramethylbutylphenol) | - | 1,00 | 1,00 | - | - | - | - |
| Tinosorb® S (Bis-Ethylhexyloxyphenol Methoxyphenyl Triazine) | - | 2,00 | - | - | 0,50 | - | - |
| Uvasorb® HEB (Diethylhexyl Butamino Triazone) | - | - | - | - | 1,00 | - | - |
| Neo Heliopan® AV (Ethylhexyl Methoxycinnamate | - | 5.00 | 5,00 | - | 3,00 | - | - |
| Parsol® 1789 (Butyl Methoxydibenzoylmethane) | - | 3.00 | 3,00 | - | 2,00 | - | - |
| Neo Heliopan® 303 (Octocrylene) | - | 3,50 | 2,00 | - | - | - | - |

**Sprühbare W/O Emulsion (SPF 20)**

| Phase | Bestandteil | INCI | Gew.-% |
|---|---|---|---|
| I | Dehymuls® LE | PEG-30 Dipolyhydroxystearate | 3.00 |
| | DC 245 | Cyclopentasiloxane | 5.00 |
| | Cetiol® A | Hexyl Laurate | 5.00 |
| | Cetiol® CC | Dicaprylyl Carbonate | 1,50 |
| | | Kohlenwasserstoffgemisch gemäß Herstellbeispiel 1 bis 5 | 1,50 |
| | Cosmedia ® Gel CC | Dicaprylyl Carbonate (and) Stearalkonium Hectorite (and) Propylene Carbonate | 4.00 |
| | Cetiol® Sun | Titanium Dioxide Dispersion | 20.00 |
| II | Deionized Water | Aqua | qs 100 |
| | Butylene Glycol | Butylene Glycol | 3.00 |
| | Ajidew N50 | Sodium PCA | 2.00 |
| | Magnesium Sulphate | Magnesium Sulphate | 0.50 |
| | EDTA 4 Na | Tetrasodium EDTA | 0.05 |
| | Preservative | | q.s. |
| III | Parfum | | q.s. |
| Viscosity, Brook. RVT, 20˚C, Sp 4, 5 rpm 2700 mPas | | | |

**Sprühbare W/O Emulsion (SPF 20)**

| Phase | Bestandteil | INCI | Gew.-% |
|---|---|---|---|
| I | Dehymuls® LE | PEG-30 Dipolyhydroxystearate | 4.00 |
| | Dehymuls ® PGPH | Polyglyceryl-2 Dipolyhydroxystearate | 4.00 |
| | Cetiol® 868 | Ethylhexyl Stearate | 7.00 |
| | Cetiol® CC | Dicaprylyl Carbonate | 2,70 |
| | | **Kohlenwasserstoffgemisch gemäß Herstellbeispiel 1 bis 5** | 2,70 |
| | Cosmedia ® Gel CC | Dicaprylyl Carbonate (and) Stearalkonium Hectorite (and) Propylene Carbonate | 3.00 |
| | Cetiol ® OE | Dicaprylyl Ether | 5.00 |
| | Zincum N 29 | Zinc Oxide | 2.00 |
| II | Cetiol® Sun | Titanium Dioxide Dispersion | 20.00 |
| III | Deionized Water | Aqua | qs 100.00 |
| | Glycerine | Glycerine | 5.00 |
| | Sodium Chloride | Sodium Chloride | 0.50 |
| | Magnesium Sulphate | Magnesium Sulphate | 0.50 |
| | Preservative | | q.s. |
| IV | Parfum | | q.s. |

(fortgesetzt)

| Phase | Bestandteil | INCI | Gew.-% |
|---|---|---|---|
| Viscosität: Brookfield. RVT, 20˚C, Sp 5, 10 rpm 10400mPas | | | |

**Sonnenschutz Spray (SPF 6)**

| Phase | Bestandteil | INCI | Gew.-% |
|---|---|---|---|
| I | Deionized Water | Aqua | qs 100 |
| | Butylene Glycol | Butylene Glycol | 3.00 |
| II | Veegum Ultra | Magnesium Aluminum Silicate | 0.50 |
| | Keltrol T | Xanthan Gum | 0.30 |
| III | Eumulgin ® VL 75 | Lauryl Glucoside (and) Polyglyceryl-2-Dipolyhydroxystearate (and) Glycerin | 6.00 |
| | Cetiol® CC | Dicaprylyl Carbonate | 3,50 |
| | | Kohlenwasserstoffgemisch gemäß Herstellbeispiel 1 bis 5 | **3,50** |
| | Cetiol® AB | C12-15 Alkyl Benzoate | 7.00 |
| | Cetiol® Sun | Titanium Dioxide Dispersion | 10.00 |
| | Preservative | | q.s. |
| | Parfum | | q.s. |
| Viscosität: Brook. RVT, 20˚C, Sp 4, 5 rpm 3500 mPas pH= 6.5 | | | |

**Sonnenlotion (SPF 20)**

| | Bestandteil | INCI | Gew.-% |
|---|---|---|---|
| I | Myritol® 331 | Cocoglycerides | 2.00 |
| | Cetiol® SB 45 | Shea Butter | 1.00 |
| | Cosmedia Gel CC | Dicaprylyl Carbonate (and) Stearalkonium Hectorite | 2.00 |
| | Uvasorb® HEB | Dioctyl Butamido Triazone | 3.00 |
| | | Kohlenwasserstoffgemisch gemäß Herstellbeispiel 1 bis 5 | 2,00 |
| | Neo Heliopan® AV | Ethylhexyl Methoxycinnamate | 4.00 |
| | Neo Heliopan® E1000 | Isoamyl p-Methoxycinnamate | 4.00 |
| | Neo Heliopan® 303 | Octocrylene | 3.00 |
| | Copherol® 1250 C | Tocopheryl Acetate | 0.25 |
| | Cosmedia® DC | Hydrogenated Dimer Dilinoleyl/Dimethylcarbonate Copolymer | 2.00 |
| II | Cetiol® Sun | Titanium Dioxide Dispersion | 6.00 |
| | Satiaxane CX 91 | Xanthan gum | 0,30 |
| | Eumulgin® VL75 | Lauryl Glucoside (and) Polyglyceryl-2- | 3.13 |
| | | Dipolyhydroxystearate (and) Glycerin | |
| III | Deionized Water | Water | 58.22 |

(fortgesetzt)

| | Bestandteil | INCI | Gew.-% |
|---|---|---|---|
| | 2 NaEDTA | Disodium EDTA | 0.10 |
| | Liponic EG-1 | Glycereth-26 | 3.00 |
| IV | Tinosorb M | Methylene bis-benzotriazolyltetramethylbutylphenol | 3.00 |
| | Water | Water | 3.00 |
| | Preservative | | q.s. |
| | Parfum | | q.s |
| Viscosität Brook. RVT, 20˚C, spindle 5, 10 rpm 2560 mPaspH= 6.70 | | | |

**Sonnenlotion (SPF 50+)**

| | Bestandteil | INCI | Gew.-% |
|---|---|---|---|
| I | Cetiol® B | Bibutyl Adipate | 2.00 |
| | Cosmedia® DC | Hydrogenated Dimer Dilinoleyl/Dimethylcarbonate Copolymer | 1.00 |
| | | Kohlenwasserstoffgemisch gemäß Herstellbeispiel 1 bis 5 | 2.00 |
| II | Neo Heliopan® OS | Ethylhexyl Salicylate | 3.00 |
| | Tinosorb® S | Bis-ethylhexyloxyphenol Methoxyphenyl Triazine | 2.50 |
| | Neo Heliopan® AV | Ethylhexyl Methoxycinnamate | 6.00 |
| | Neo Heliopan® 303 | Octocrylene | 8.00 |
| | Neo Heliopan® 357 | Butyl Methoxydibenzoylmethane | 2.50 |
| III | Cosmedia® Gel CC | Dicaprylyl Carbonate (and) Steralkonium Hectorite (and) Propylene Carbonate | 2.00 |
| IV | Emulgade® PL 6850 | Cetearyl Glucoside (and) Cetearyl Alcohol | 2.50 |
| | Eumulgin ® VL 75 | Lauryl Glucoside (and) Polyglyceryl-2-Dipolyhydroxystearate (and) Glycerin | 3.00 |
| V | Cetiol® Sun | Titanium Dioxide Dispersion | 10.00 |
| VI | Deionized Water | Aqua | qs 100 |
| | 2 NaEDTA | Disodium EDTA | 0.10 |
| | Preservative | | q.s. |
| | Liponic EG-1 | Glycereth-26 | 3.00 |
| | Keltrol® T | Xanthan gum | 0.60 |
| | Veegum Ultra | Magnesium Aluminum Silicate | 2.00 |
| VII | Dry Flo Plus | Aluminum Starch Octenylsuccinate | 2.00 |
| VIII | Tinosorb® M | Methylene Bis-Benzotriazolyl Tetramethylbutylphenol | 5.00 |
| IX | Parfum | | q.s. |
| Viskosität Brookfield Helipath 20˚C, TB, 10 rpm 16200 mPas pH= 6.7 | | | |

**Anti-Ageing Sonnenschutz (SPF 30)**

| | Bestandteil | INCI | Gew.-% |
|---|---|---|---|
| I | Myritol® 331 | Cocoglycerides | 2.00 |
| | Cetiol® B | Bibutyl Adipate | 3.00 |
| | | Kohlenwasserstoffgemisch gemäß Herstellbeispiel 1 bis 5 | 2.00 |
| | Uvinul A plus | Diethylamino Hydroxybenzoyl Hexyl Benzoate | 5.00 |
| | Uvinul T 150 | Ethylhexyl Triazone | 2.50 |
| | Cosmedia® Gel CC | Dicaprylyl Carbonate (and) Stearalkonium Hectorite | 2.00 |
| | Cosmedia® DC | Hydrogenated Dimer Dilinoleyl/Dimethylcarbonate Copolymer | 2.00 |
| II | Cetiol® Sun | Titanium Dioxide Dispersion | 10.00 |
| | Satiaxane CX 91 | Xanthan gum | 0.40 |
| | Eumulgin® VL75 | Lauryl Glucoside (and) Polyglyceryl-2-Dipolyhydroxystearate (and) Glycerin | 3.50 |
| III | Deionized Water | Aqua | 49.60 |
| | 2NaEDTA | Disodium EDTA | 0.10 |
| | Glycerin | Glycerin | 3.00 |
| | Preservative | | q.s. |
| I V | Photonyl® LS | Arginine (and) Disodium Adenosine Triphosphate (and) Mannitol (and) Pyridoxine HCI (and) RNA (and) Histidine HCl (and) Phenylalanine (and) Tyrosine | 2.00 |
| | Water | | 12.00 |
| V | Copherol® 1250 C | Tocopheryl Acetate | 0.50 |
| | Parfum | | q.s. |
| Viskosität: Brook. RVT, 20˚C, Spindel 5, 10 rpm 3560 mPaspH= 6.50 | | | |

**Anti-Falten Creme**

| | Bestandteil | INCI | Gew.-% |
|---|---|---|---|
| I | Eumulgin ® BA 25 | Beheneth-25 | 3.30 |
| | Lameform® TGI | Polyglyceryl-3 Diisostearate | 0.70 |
| | | **Kohlenwasserstoffgemisch gemäß Herstellbeispiel 1 bis 5** | 2.00 |
| | Cutina ® PES | Pentaerythrityl Distearate | 2.00 |
| | Cetiol ®CC | Dicaprylyl Carbonate | 1.30 |
| | Cetiol ® AB | C12-C15 Alkyl Benzoate | 5.00 |
| | Cetiol ® LC | Coco-Caprylate/Caprate | 3.00 |
| | Cetiol® Sun | Titanium Dioxide Dispersion | 6.00 |
| | Phenonip | Phenoxyethanol (and) parabens | 0.80 |
| II | Deionized Water | Aqua | 61.85 |
| | 4NaEDTA | Tetrasodium EDTA | 0.05 |
| | Glycerin | Glycerin | 3.00 |

(fortgesetzt)

| | Bestandteil | INCI | Gew.-% |
|---|---|---|---|
| | Butylene Glycol | Butylene Glycol | 2.00 |
| III | Veegum Ultra | Magnessium Aluminum Silicate | 3.00 |
| | Keltrol T | Xanthan Gum | 0.30 |
| IV | | Perfume Waterfresh | 0.50 |
| | Coviox ® T70 C | Tocopherol | 0.10 |
| V | Deionized Water | Aqua | 1.00 |
| | Plantactiv ® Aesculus | Escin | 0.10 |
| Viskosität, Brookfield, RVT, 20˚C, Spindel 5, 5 rpm 11500 mPas pH= 6.2-6.7 | | | |

**Sonnenschutzlotion SPF 30**

| | Bestandteil | INCI | Gew.-% |
|---|---|---|---|
| I | Myritol® 331 | Cocoglycerides | 2.00 |
| | Cetiol® CC | Dicaprylyl Carbonate | 1.00 |
| | | **Kohlenwasserstoffgemisch gemäß Herstellbeispiel 1 bis 5** | 1.00 |
| | Cetiol® B | Bibutyl Adipate | 2.00 |
| | Neo Heliopan® MBC | 4-Methylbenzilidene Camphor | 3.50 |
| | Neo Heliopan® AV | Ethylhexyl Methoxycinnamate | 6.00 |
| | Neo Heliopan® E1000 | Isoamyl p-Methoxycinnamate | 6.00 |
| | Neo Heliopan® 357 | Butyl Methoxydibenzoylmethane | 1.00 |
| | Cosmedia ® DC | Hydrogenated Dimer Dilinoleyl/Dimethylcarbonate Copolymer | 1.00 |
| II | Cetiol® Sun | Titanium Dioxide Dispersion | 14.00 |
| | Satiaxane CX 91 | Xanthan gum | 0.10 |
| | Cosmedia ® SP | Sodium Polyacrylate | 0.40 |
| | Eumulgin® VL75 | Lauryl Glucoside (and) Polyglyceryl-2-Dipolyhydroxystearate (and) Glycerin | 3.75 |
| III | Deionized Water | Aqua | qs 100.00 |
| | Neo Heliopan® Hydro | Phenylbenzimidazole Sulfonic acid | 2.00 |
| | Trometamine | Trometamine | 1.60 |
| | Phenonip | | qs |
| Viscosität: Brookfield, RVT, 20˚C, Spindel 5, 10 rpm 2240 mPas pH= 7.35 | | | |

**Sonnencreme SPF 50+**

| | Bestandteil | INCI | Gew.-% |
|---|---|---|---|
| I | Myritol® 331 | Cocoglycerides | 2.00 |
| | Cetiol® CC | Dicaprylyl Carbonate | 1.00 |

(fortgesetzt)

| | Bestandteil | INCI | Gew.-% |
|---|---|---|---|
| | | **Kohlenwasserstoffgemisch gemäß Herstellbeispiel 1 bis 5** | 1.00 |
| | Cetiol® B | Bibutyl Adipate | 2.00 |
| | Neo Heliopan® MBC | 4-Methylbenzilidene Camphor | 3.50 |
| | Neo Heliopan® AV | Ethylhexyl Methoxycinnamate | 6.00 |
| | Neo Heliopan® E1000 | Isoamyl p-Methoxycinnamate | 6.00 |
| | Neo Heliopan® 357 | Butyl Methoxydibenzoylmethane | 1.00 |
| | Cosmedia® DC | Hydrogenated Dimer Dilinoleyl/Dimethylcarbonate Copolymer | 2.00 |
| | Emulgade® PL 6850 | Cetearyl Glucoside (and) Cetearyl Alcohol | 4.50 |
| | Lanette® E | Sodium Cetearyl Sulfate | 1.0 |
| II | Cetiol® Sun | Titanium Dioxide Dispersion | 14.00 |
| III | Deionized Water | Aqua | qs 100.00 |
| | Neo Heliopan® Hydro | Phenylbenzimidazole Sulfonic Acid | 2.00 |
| | NaOH 50% | Sodium Hydroxide | 0.6 |
| | | Preservative | q.s. |
| | | Parfum | q.s. |
| Viskosität: Brookfield, Helipath 20˚C, TE, 4 rpm , 195000 mPas pH= 7.5 | | | |

**AP/Deo in Aerosol Form (Gew.-%)**

| Bestandteil | INCI | A | B |
|---|---|---|---|
| IPM | Isopropyl Myristate | 10,8 | 10,8 |
| Cetiol® CC | Dicaprylyl Carbonate | 5.00 | 5.00 |
| Cosmedia® Gel CC | Dicaprylyl Carbonate (and) Stearalkonium Hectorite (and) Propylene Carbonate | - | 2.00 |
| | Caprylic capric triglyceride(and)Stearalkonium Hectorite (and) Propylene Carbonate | 2.00 | - |
| | **Kohlenwasserstoffgemisch gemäß Herstellbeispiel 1 bis 5** | 2.00 | 2.00 |
| Locron® P | Aluminium chlorhydrate | 3.00 | 3.00 |
| Hydagen® CAT | Triethyl Citrate | 1.00 | 1.00 |
| Perfume | Perfume | 1.20 | 1.20 |
| Propane | | 75.00 | 75.00 |

**Foundation oder Sonnenschutz enthaltend Titanium Dioxid**

| Ingredients | INCI Name | Gew.-% |
|---|---|---|
| Cetiol® CC | Dicaprylyl Carbonate | 95.00 |
| Cosmedia® Gel CC | Dicaprylyl Carbonate (and) Stearalkonium Hectorite (and) Propylene Carbonate | 1.00 |

(fortgesetzt)

| Ingredients | INCI Name | Gew.-% |
|---|---|---|
| | **Kohlenwasserstoffgemisch gemäß Herstellbeispiel 1 bis 5** | 1.00 |
| Titanium dioxide | | 5.00 |

**Sonnenschutz Spray SPF 40 (Gew.-%)**

| | Ingredient | INCI | A | B |
|---|---|---|---|---|
| I | Eumulgin ® VL 75 | Lauryl Glucoside (and) Polyglyceryl-2 Dipolyhydroxystearate (and) Glycerin | 6.0 | 6.0 |
| | Generol ® R | Brassica Campestris (Rapeseed) | 1.0 | 1.0 |
| | Cegesoft ® SH Cosmedia ® Gel CC | Sterols Shorea Stenoptera Dicaprylyl Carbonate (and) Stearalkonium Hectorite (and) Propylene Carbonate | 1.0 2.0 | 1.0 |
| | Eusolex 2292 | Ethylhexylmethoxycinnamate | 7.5 | 7.5 |
| | Tinosorb® S | Bis-ethylhexyloxyphenol methoxyphenyltriazine | 2.0 | 2.0 |
| | Cetiol ® CC | Dicaprylyl carbonate | 1.5 | |
| | | **Kohlenwasserstoffgemisch gemäß Herstellbeispiel 1 bis 5** | 1.5 | 1.5 |
| | Cetiol ® AB | C12-15 Alkyl Benzoate | 4.0 | 4.0 |
| | Cosmedia ® DC | Hydrogenated Dimer Dilinoleyl / Dimethylcarbonate Copolymer | 1.00 | 1.00 |
| II | Water | | 61.45 | 63.45 |
| | EDTA 4Na (Prolabo) | Tetrasodium EDTA | 0,05 | 0.05 |
| | Elestab 50J | Chlorphenesin & Methylparaben | 0.60 | |
| III | Cosmedia®SP | Sodium Polyacrylate | 0.40 | 0.40 |
| I V | Tinsorb® M | Methylenebis benzotriazolyl tetramethylbutylphenol | 10.0 | 10.0 |
| Viskosität, Brookfield. RVT, 23˚ C, spindle 2,5 rpm, ph:6 3500mPas 700mPas | | | | |

**Spray SPF 40**

| | Bestandteil | INCI | A | B |
|---|---|---|---|---|
| I | Eumulgin® VL75 | Lauryl Glucoside (and) Polyglyceryl-2 Dipolyhydroxystearate (and) Glycerin | 6.0 | 6.0 |
| | Generol® | Brassica Campestris (Rapeseed) Sterols | 1.0 | 1.0 |
| | Cegesoft® SH | Shorea Stenoptera | 1.0 | 1.0 |
| | Cosmedia® Gel CC | Dicaprylyl Carbonate(and) Stearalkonium Hectorite (and) Propylene Carbonate | 3.0 | |
| | | **Kohlenwasserstoffgemisch gemäß Herstellbeispiel 1 bis 5** | **2.0** | **2.0** |
| | Eusolex 2292 | Ethylhexylmethoxycinnamate | 7.5 | 7.5 |

(fortgesetzt)

| | Bestandteil | INCI | A | B |
|---|---|---|---|---|
| | Tinosorb S | Bis-ethylhexyloxyphenol methoxyphenyltriazine | 2.13 | 4.74 |
| | Cetiol® CC | Dicaprylyl Carbonate | 2.0 | 2.0 |
| | Cetiol® AB | C12-15 Alkyl Benzoate | 4.0 | 4.0 |
| II | Water | | 62.32 | 62.71 |
| | EDTA 4Na | Tetrasodium EDTA | 0.05 | 0.05 |
| | Elestab sol | Chlorphenesin & Methylparaben | 0.60 | 0.60 |
| III | Cosmedia® SP | Sodium Polyacrylate | 0.40 | 0.40 |
| IV | Tinosorb M | Methylenebis benzotriazolyl tetramethylbutylphenol | 10.00 | 10.00 |
| Viskosität Book. RVT, 23° C, spindle 2,5 rpm, ph:6 A: B: 730 mPas | | | 4020 mPas | |

**Feuchtigkeitsspendende Sonnenschutz Lotion W/O SPF 20**

| | Bestandteil | INCI | A | B |
|---|---|---|---|---|
| I | Dehymuls® | PEG - 30 Dipolyhydroxystearate | 3.00 | 3.00 |
| | DC 245 | Cyclopentasiloxane | 5.00 | 5.00 |
| | Cetiol® OE | Dicaprylyl Ether | 4.00 | 4.00 |
| | Cetiol® A | Hexyl Laurate | 5.00 | 5.00 |
| | Cetiol® CC | Dicaprylyl Carbonate | 1.50 | 1.50 |
| | | **Kohlenwasserstoffgemisch gemäß Herstellbeispiel 1 bis 5** | **1.50** | **1.50** |
| II | Cosmedia® Gel CC | Dicaprylyl Carbonate(and) Stearalkonium Hectorite (and) Propylene Carbonate | 4.00 | |
| III | Cetiol® SUN | Titanium dioxide (and) Alumina (and) Dimethicone and) Stearic acid ( and) Dicaprylyl Carbonate (and) Polyhydroxystearic acid | 20.00 | 20.00 |
| IV | Deionized water | Aqua | 49.55 | 53.55 |
| | Butylene Glycol | Butylene Glycol | 3.00 | 3.00 |
| | Ajidew N 50 | Sodium PCA | 2.00 | 2.00 |
| | Mg SO4 | Magnesium Sulfate | 0.50 | 0.50 |
| | EDTA, 4 Na | Tetrasodium EDTA | 0.05 | 0.05 |
| | Phenonip | Phenoxyethanol (and) Parabens | 0.50 | 0.50 |
| V | PerfumeSoleil/L27 8319/ C | Floressence | 0.40 | 0.40 |
| Viskosität, Brook. RVT, spindle 4, speed 5 [mPas] | | | 2000 | 5000 |

**Spray SPF 40** (Angaben in Gew.-%)

| | Bestandteil | INCI | A | B |
|---|---|---|---|---|
| I | Eumulgin® VL 75 | Lauryl Glucoside (and) Polyglyceryl-2 Dipolyhydroxystearate (and) Glycerin | 6.0 | 6.0 |
| | Generol® R | Brassica Campestris (Rapeseed) Sterols | 1.0 | 1.0 |
| | Cegesoft® SH | Shorea Stenoptera | 1.0 | 1.0 |
| | Cosmedia® Gel CC | Dicaprylyl Carbonate(and) Stearalkonium Hectorite (and) Propylene Carbonate | 2.0 | |
| | Eusolex 2292 | Ethylhexylmethoxycinnamate | 7.5 | 7.5 |
| | Tinosorb S | Bis-ethylhexyloxyphenol methoxyphenyltriazine | 2.0 | 2.0 |
| | Cetiol® CC | Dicaprylyl carbonate | 1.5 | 1.5 |
| | | **Kohlenwasserstoffgemisch gemäß Herstellbeispiel 1 bis 5** | 1.5 | 1.5 |
| | Cetiol® AB | C12-15 Alkyl Benzoate | 4.0 | 4.0 |
| | Cosmedia ® DC | Hydrogenated Dimer Dilinoleyl/Dimethylcarbonate Copolymer | 1.0 | 1.00 |
| II | Water | | 61.45 | 63.45 |
| | EDTA 4Na | Tetrasodium EDTA | 0.05 | 0.05 |
| | Elestab 50J | Chlorphenesin & Methylparaben | 0.60 | 0.60 |
| III | Cosmedia® SP | Sodium Polyacrylate | 0.40 | 0.40 |
| IV | Tinosorb M | Methylenebis benzotriazolyl tetramethylbutylphenol | 10.0 | 10.0 |
| Viskosität, Brook. RVT, 23˚ C, spindle 2, 5 rpm, pH: 6 [mPas] A: 3500, B: 700 | | | | |

**Mat finish fluid foundation**

| | Bestandteil | INCI | Gew.-% |
|---|---|---|---|
| I | Lameform® TGI | Polyglyceryl-3 Diisostearate | 1.50 |
| | Eumulgin® BA25 | Beheneth-25 | 3.50 |
| | Cetiol® CC | Dicaprylyl Carbonate | 4.00 |
| | Myritol® 331 | Cocoglycerides | 2.00 |
| | | **Kohlenwasserstoffgemisch gemäß Herstellbeispiel 1 bis 5** | 2.00 |
| | Fitoderm® | Squalane | 3.00 |
| | Cutina® PES | Pentaerythiryl Distearate | 1.00 |
| | Cegesoft® SH | Shorea Stenoptera | 1.00 |
| | Phenonip | Phenoxyethanol (and) Parabens | 0.80 |
| | KF 96, 100cs | Dimethicone | 3.00 |
| | Cetiol® OE | Dicaprylyl Ether | 3.00 |
| II | Sun Croma C47-051 | Titanium Dioxide | 6.00 |
| | SunPuro Yellow Iron Oxide C33-9001 | Iron Oxide CI 77492 | 1.30 |
| | SunPuro Red Iron Oxide C33-8001 | Iron Oxide CI 77491 | 0.60 |
| | SunPuro Black Iron Oxide C33-7001 | Iron Oxide CI 77499 | 0.20 |
| III | Deionized Water | Aqua | 52.50 |

(fortgesetzt)

| | Bestandteil | INCI | Gew.-% |
|---|---|---|---|
| | Glycerine | Glycerin | 3.00 |
| | Butylene Glycol | Butylene Glycol | 2.00 |
| IV | Veegum Ultra | Magnesium Aluminium Silicate | 1.00 |
| | Keltrol T | Xanthan Gum | 0.40 |
| V | Cosmedia® Gel CC | Dicaprylyl Carbonate (and) Stearalkonium Hectorite (and) Propylene Carbonate | 3.00 |
| VI | Cosmedia® PMMA V 12 | Polymethylmethacrylate | 2.00 |
| VII | Mirasil CDPDM | Cyclomethicone (and) Diphenyldimethicone | 3.00 |
| VIII | Perfume Caresse | | 0.20 |
| Viskosiät, Brook. RVT, spindle 3, speed 5, 8400mPas pH:6.7 | | | |

**Ultra protective spray SPF 40 0/W**

| | Bestandteil | INCI | Gew.-% |
|---|---|---|---|
| I | Eumulgin® VL 75 | Lauryl Glucoside (and) Polyglyceryl-2 Dipolyhydroxystearate (and) Glycerin | 7.00 |
| | Generol® R | Brassica Campestris (Rapeseed) Sterols | 1.00 |
| | Cosmedia® DC | Hydrogenated Dimer Dilinoleyl / Dimethylcarbonate Copolymer | 1.00 |
| | Eusolex 2292 | Ethylhexylmethoxycinnamate | 7.50 |
| | Tinosorb S | Bis-ethylhexyloxyphenol methoxyphenyltriazine | 2.00 |
| | Cetiol® CC | Dicaprylyl carbonate | 2.00 |
| | Cetiol® AB | C12-15 Alkyl Benzoate 4.00 | 4.00 |
| | | **Kohlenwasserstoffgemisch gemäß Herstellbeispiel 1 bis 5** | 2.00 |
| | Cegesoft® VP | Olus & Hydrogenated Vegetable Oil & Candelilla Cera | 2.50 |
| | Myritol® 331 | Cocoglycerides | 2.00 |
| | Cosmedia® Gel CC | Sodium polyacrylate Carbonate(and) Stearalkonium Hectorite (and) Propylene Carbonate | 4.00 |
| II | Water | qsp100 | 55.15 |
| | EDTA 4Na | Tetrasodium EDTA 0.05 | 0.05 |
| | Elestab 50J | Chlorphenesin & Methylparaben | 0.60 |
| III | Keltrol T | Xanthan Gum | 0.20 |
| IV | Tinosorb M | Methylenebis benzotriazolyl tetramethylbutylphenol | 10.00 |
| Viscosity, Brook. RVT, 23° C, spindle 2,5 rpm, 2500mPaspH:6.4 | | | |

**Soft moisture Gel Creme**

| | Bestandteil | INCI | Gew.-% |
|---|---|---|---|
| I | Deionized Water | Aqua | 65.35 |
| | Glycerine | Glycerin | 3.00 |
| | Phenonip | Phenoxyethanol (and) Parabens | 0.60 |
| II | Cosmedia® SP | Sodium Polyacrylate | 1.20 |
| III | Cegesoft® PFO | Passiflora Incarnata | 2.00 |
| | Eusolex 2292 | Ethylhexyl Methoxycinnamate | 2.00 |
| | KF 96,100cs | Dimethicone 2.00 | 2.00 |
| IV | Cetiol® CC | Dicaprylyl Carbonate | 2.50 |
| | Eutanol® G | Octyldodecanol | 4.0 |
| | | Kohlenwasserstoffgemisch gemäß Herstellbeispiel 1 bis 5 | 2.50 |
| | Eusolex 4360 | Benzophenone-3 | 0.10 |
| | Cegesoft® SBE | Butyrospermum Parkii | 1.50 |
| V | Cosmedia® Gel CC | Dicaprylyl Carbonate (and) Stearalkonium Hectorite (and) Propylene Carbonate | 5.00 |
| VI | Deionized Water | Aqua | 5.00 |
| | Osmhydran® LS 8453 | Mannitol (and) Arginine (and) Serine (and) Sucrose (and) PCA (and) Citrulline (and) Glycogen (and) Histidine HCI (and) Alanine (and) Threonine (and) Glutamic Acid (and) Lysine HCI | 2.00 |
| VII | Vegeseryl® LP LS 9058 | Hydrolyzed Soy Protein | 1.00 |
| VIII | Coviox® T70C | Tocopherol | 0.10 |
| | Perfume Raphaelle | | 0.15 |
| Viscosity, Brook. RV T, Helipath TE, speed 4, 101 000mPas pH:5. 5.5 | | | |

**Samtige Reinigungsmaske**

| | Bestandteil | INCI | Gew.-% |
|---|---|---|---|
| I | Emulgade® SE-PF | Glyceryl Stearate (and) Ceteareth-20 (and) Ceteareth-12 (and) Cetearyl Alcohol (and) Cetyl Palmitate | 8.00 |
| | Lanette® O | Cetearyl Alcohol | 2.00 |
| | Cegesoft® PS6 | Olus | 5.00 |
| | Cetiol® CC | Dicaprylyl Carbonate | 2.00 |
| | Cutina® PES | Pentaerythrityl Distearate | 3.00 |
| | | **Kohlenwasserstoffgemisch gemäß Herstellbeispiel 1 bis 5** | 2.00 |
| II | Cosmedia® Gel CC | Dicaprylyl Carbonate (and) Stearalkonium Hectorite (and) Propylene Carbonate | 5.00 |
| III | Deionized Water | Aqua | 45.00 |
| | Glycerine | Glycerin | 10.00 |
| | EDTA, 4Na | Tetrasodium EDTA | 0.10 |
| | Elestab® 50J | Chlorphenesin (and) Methylparaben | 0.40 |

(fortgesetzt)

| | Bestandteil | INCI | Gew.-% |
|---|---|---|---|
| IV | Dry Flo AF | Corn Starch Modified0 | 1.00 |
| | Propylene Glycol | Propylene Glycol | 3.00 |
| V | Kaolin | Kaolin | 10.00 |
| VI | Perfume Concombre | | 0.20 |
| | Coviox® T70C | Tocopherol | 0.10 |
| VII | Purisoft ® LS 9602 | Glycerin (and) Moringa Pterygosperma | 2.00 |
| VIII | Green Covasol W7035 (a.s.0.1 %) | CI 42090 (and) CI19140 | 3.20 |
| Viscosity, Brook. RVT, Helipath TE, Speed 4, 254 000mPas pH:6.3 | | | |

**Luxuriöse Körper Butter**

| | Bestandteil | INCI | Gew.-% |
|---|---|---|---|
| I | Deionized water | Aqua | 61.50 |
| | Glycerine | Glycerin | 5.00 |
| | Butylene Glycol | Butylene Glycol | 2.00 |
| | EDTA, 4 Na | Tetrasodium EDTA | 0.10 |
| | Phenonip | Phenoxyethanol (and) Parabens | 0.80 |
| II | Cosmedia® SP | Sodium Polyacrylate | 1.50 |
| III | Eumulgin® BA 25 | Beheneth-25 3.00 | 3.00 |
| | Lanette® O | Cetearyl Alcohol | 4.00 |
| | Cetiol® SN | Cetearyl Isononanoate | 3.00 |
| | Cetiol® SB45 | Butyrospermum Parkii | 5.00 |
| | Cetiol® CC | Dicaprylyl Carbonate | 0.50 |
| | | **Kohlenwasserstoffgemisch gemäß Herstellbeispiel 1 bis 5** | 0.50 |
| | KF 96, 100cs | Dimethicone | 1.00 |
| | Generol® R | Brassica Campestris (Rapeseed) Sterols | 0.50 |
| IV | Cosmedia® Gel CC | Dicaprylyl Carbonate (and) Stearalkonium Hectorite (and) Propylene Carbonate | 5.00 |
| V | Cosmedia® PMMA V12 | Polymethylmethacrylate | 2.00 |
| | Deionized water | Aqua | 4.00 |
| VI | Coviox® T70C | Tocopherol | 0.10 |
| | Perfume Pétale 139012E | | 0.50 |
| Viscosity, Brook. RVT Helipath TE, speed4, 800 000mPas pH:7 | | | |

**Happy Deo Mist**

| Bestandteil | INCI | Gew.-% |
|---|---|---|
| IPM | Isopropyl Palmitate | 6.50 |
| Cetiol® Sensoft | Propylheptyl Caprylate | 6.50 |
| Cosmedia® Gel | Caprylic/Capric Triglyceride (and) | 2.00 |
| CC | Stearalkonium Hectorite (and) Propylene Carbonate | |
| | **Kohlenwasserstoffgemisch gemäß Herstellbeispiel 1 bis 5** | 2.00 |
| Locron P | Aluminium Clorohydrate | 5.00 |
| Perfume Deo Talco | Perfume | 0.30 |
| Propellant | | 77.7 |
| Ratio propellant/other (3.34:1); Valve: RK 150P RA 634/5/8 (Vapor Phase); Button: V04.776 | | |

**Rich Hair conditioner / Haar Conditioner**

| Phase | Handelsname | INCI Bezeichnung | Gew.-% |
|---|---|---|---|
| I. | Dehyquart®L80 | Dicocoylethyl Hydroxyethylmonium Methosulfat (und) Propylene Glycol | 2 |
| | Lanette®O | Cetearyl Alcohol | 5 |
| | Cutina®GMS | Glyceryl Stearate | 1 |
| | **Kohlenwasserstoffgemisch gemäß Herstellbeispiel 1 bis 5** | | 0,3 |
| | Cetiol®J 600 | Oleyl Erucate | 0,5 |
| | Plantacare®1200 UP | Lauryl Glucoside | 2 |
| II. | Glycerol | | 2 |
| | Wasser, entionisiert | | 50 |
| III. | Hydroxyethylcellulose | Hydroxyethylcellulose | 0,4 |
| | Wasser, deionisiert | | Ad 100 |
| IV. | Aloe vera Gel | | 0,1 |
| | Konservierungsmittel | | q.s. |
| | Parfum | | q.s. |
| >> | pH Wert | | 4 - 4,5 |
| >> | Viskosität mPas | | Ca. 8000 |

Herstellung: Phase I und II wurden auf 85˚C erhitzt, Phase II wurde bei 85˚C zugefügt und die Temperatur wurde beim Rühren gehalten bis zur vollständigen Homogenität. Die Emulsion wurde unter Rühren abgekühlt und das vorher in Wasser aufgelöste Polymer (Phase III) wurde zugefügt. Danach wurden die übrigen Additive (Phase IV) zugefügt und der pH-Wert eingestellt (z.B. mit Zitronensäure).

**Hair-Rinse & Colouring /Haar Spülung und Färbung**

| Phase | Handelsname | INCI Bezeichnung | Gew.-% |
|---|---|---|---|
| I. | Dehyquart® F75 | Distearoylethyl Hydroxyethylmonium Methosulfate (and) Cetearyl Alcohol | 2 |
| | Eumulgin® B2 | Ceteareth-20 | 0,5 |
| | Lanette® O | Cetearyl Alcohol | 4 |
| | **Kohlenwasserstoffgemisch gemäß Herstellbeispiel 1 bis 5** | | **1,5** |
| II. | Wasser, deionisiert | | Ad 100 |
| III. | Sol. 5% Arianor Mahogany | Colour | 10 |
| IV. | Konservierungsmittel | | q.s. |
| >> | pH Wert | | 3,76 |

Herstellung: Schmelzen von Phase I bei 80-85˚C. Erhitzen von Phase II auf 80-85˚C and einrühren in Phase I. Rühren bei dieser Temperatur bis zur kompletten Homogenität. Kühlen unter Rühren auf 40˚C. Hinzufügen von Konservierungsmittel and wenn nötig, von hitzeempfindlichen Additiven. Kühlen beim Rühren auf 30˚C.Anpassen des pH Wertes, z.B. mit Zitronensäure. Hinzufügen der Arianor Lösung bei unter 40˚C.

**Styling Gel Typ "Out of bed"**

| Phase | Handelsname | INCI Bezeichnung | Gew.-% |
|---|---|---|---|
| I. | Alcool 96% (Prolabo) | Alcohol 96% | 12 |
| | Luviskol® VA 64 (BASF) | PVPNA | 4,5 |
| | Polyox® WSR-301 | PEG-90M | 0,25 |
| | Methocel®E4M Premium EP (Dow) | Hydroxypropyl Methylcellulose | 0,6 |
| | Dehyquart® L80 | Dicocoylethyl Hydroxyethylmonium Methosulfate (and) Propylene Glycol | 0,6 |
| II. | Eumulgin® HRE 40 | PEG-40 Hydrogenated Castor Oil | 1 |
| | **Kohlenwasserstoffgemisch gemäß Herstellbeispiel 1 bis 5** | | **1,5** |
| III. | Wasser, entionisiert | | a.d. |
| IV | Hispagel® 200 | Glycerin (und) Glyceryl Polyacrylate | 36,7 |
| V | Konservierungsmittel, Parfum | | q.s. |
| >> | pH Wert | | 6,8 |
| >> | Viskosität (mPas) Brookfield RVT 23˚C Spindel 5, 10rpm | | 220000 |

Herstellung: 1. Hinzufügen von Phase I bei Raumtemperatur nach und nach zum Alkohol. 2. Mischen von Phase II bis zur Homogenität und hinzufügen zu Phase I, 3. Hinzufügen von Phase III langsam zu Phase I und II, 4. Hinzufügen von Phase IV langsam zu Phase I-III, 5. wenn nötig hinzufügen von Phase V.

**Sprühbarer Hair-Care Conditioner mit Gluadin® WQTM**

| Phase | Handelsname | INCI | Gew.-% |
|---|---|---|---|
| I | Monomuls® 60-35C Powder | Hydrogenated Palm Glycerides | 1,24 |
| | Eumulgin® B 1 | Ceteareth-12 | 2,76 |

(fortgesetzt)

| Phase | Handelsname | INCI | Gew.-% |
|---|---|---|---|
| | Cetiol® OE | Dicaprylyl Ether | 5 |
| | **Kohlenwasserstoffgemisch gemäß Herstellbeispiel 1 bis 5** | | 4 |
| | Dow Corning® 345 | Cyclomethicone | 2 |
| II. | Glycerin (86%) | | 2 |
| | Wasser | | 16 |
| III. | Gluadin® WQTM | Hydroxypropyltrimonium hydrolyzed wheat protein | 2,85 |
| | Plantacare® 2000 UP | Decyl Glucoside | 1 |
| | Wasser | | Ad 100 |
| | Konservierungsmittel | | q.s. |
| | pH Wert | | 5,5 |
| | Viskosität mPas | | <100 |

Herstellung: 1. Erhitzen der Komponenten von Phase I auf 85˚C, 2. Erhitzen der Komponenten von Phase II ebenfalls auf 85˚C und hinzufügen von Phase II durch Einrühren in Phase 1, 3. Hinzufügen von Phase III kalt und rühren bis es abkühlt auf Raumtemperatur.

**Conditioner mit Dehyquart® C 4046**

| Phase | Handelsname | INCI | Gew.-% |
|---|---|---|---|
| I. | Dehyquart® C 4046 | Cetearyl Alcohol (und) Dipalmitoylethyl Hydroxyethylmonium Methosulfat (und) Ceteareth-20 | 4 |
| | Eutanol® G | Octyldodecanol | 1 |
| | **Kohlenwasserstoffgemisch gemäß Herstellbeispiel 1 bis 5** | | **0,5** |
| II. | Wasser, entionisiert | | Ad 100 |
| | Parfum | | q.s. |
| | Konservierungsmittel | | q.s. |
| >> | pH Wert | | 3,5 |
| >> | Viskosität (mPas) Brookfield RVF, 23˚C, Spindel 4, 10rpm | | 10300 |

Herstellung: Schmelzen von Phase I bei 80-85˚C. Erhitzen von Phase II auf 80-85˚C und Einrühren in Phase I. Fünf Minuten rühren bei dieser Temperatur. Abkühlen auf 40˚C während des Rührens. Hinzufügen des Konservierungsmittels und wenn nötig, hitzeempfindlicher Additive. Abkühlen auf 30˚C während des Rührens. Anpassen des pH Wertes, z.B. mit Zitronensäure.

**Conditioner mit Dehyquart® C 4046**

| Phase | Handelsname | INCI | Gew.-% |
|---|---|---|---|
| I. | Dehyquart® C 4046 | Cetearyl Alcohol (und) Dipalmitoylethyl Hydroxyethylmonium Methosulfat (und) Ceteareth-20 | 4 |
| | **Kohlenwasserstoffgemisch gemäß Herstellbeispiel 1 bis 5** | | **1** |

(fortgesetzt)

| Phase | Handelsname | INCI | Gew.-% |
|---|---|---|---|
| II. | Wasser, entionisiert | | Ad 100 |
| III. | Parfum | | q.s. |
| | Konservierungsmittel | | q.s. |
| >> | pH Wert | | 3,5 |
| >> | Viskosität (mPas) Brookfield RVF, 23˚C, Spindel 4, 10rpm | | 2060 |

Herstellung: Schmelzen von Phase I bei 80-85˚C. Erhitzen von Phase II auf 80-85˚C und Einrühren in Phase I. Fünf Minuten Rühren bei dieser Temperatur. Abkühlen auf 40˚C während des Rührens. Hinzufügen des Konservierungsmittels und wenn nötig, hitzeempfindlicher Additive. Abkühlen auf 30˚C während des Rührens. Anpassen des pH Wertes, z.B. mit Zitronensäure.

**Hair Polish Glossing Spray - Japan Hair Oil sprühbar**

| Handelsname | INCI | Gew.-% |
|---|---|---|
| Dow Corning® 345 Fluid (Dow Corning) | Cyclomethicone | 85 |
| Cetiol® CC | Dicaprylyl Carbonate | 4 |
| Cetiol® ISL | Isostearyl Lactate | 3 |
| **Kohlenwasserstoffgemisch gemäß Herstellbeispiel 1 bis 5** | | 5,2 |
| Neo Heliopan AV (Haarmann & Reimer) | Ethylhexyl Methoxycinnamate | 1,5 |
| Wacker PDM 1000 | Trimethylsiloxyphenyl Dimethicone | 0,5 |
| Konservierungsmittel | | q.s. |
| Parfum | | q.s. |
| Wasser, entionisiert | | Ad 100 |

Herstellung: Hinzufügen aller Zutaten in der Reihenfolge wie aufgeführt. Mischen bei Raumtemperatur.

**Hair gloss finishing serum**

| Handelsname | INCI | Gew.-% |
|---|---|---|
| DC®1501 (Dow Corning) | Cyclopentasiloxane (und) Dimethiconol | 70 |
| **Kohlenwasserstoffgemisch gemäß Herstellbeispiel 1 bis 5** | | 19,7 |
| Cetiol® CC | Dicaprylyl Carbonat | 8 |
| Myritol® 318 | Caprylic/Capric Triglycerid | 0,5 |
| Neo Heliopan E 1000 (Haarmann & Reimer) | Isoamyl p-Methoxycinnamat | 1 |
| Phenonip (SIPCA) | Phenoxyethanol (und) Parabens | 0,5 |
| Parfum | | 0,3 |
| Farbstoff | | q.s. |

Herstellung: Hinzufügen der Zutaten wie gezeigt. Mischen bei Raumtemperatur. Bemerkung: Der Farbstoff und das Parfum, die benutzt werden, müssen öllöslich sein (das Parfum außerdem verträglich mit Dimethiconol, um Trübheit zu vermeiden)

**Hair gloss Gelatine für sehr geschädigtes und stumpfes Haar**

| Handelsname | INCI | Gew.-% |
|---|---|---|
| Cetiol® CC | Dicaprylyl Carbonate | 33,6 |
| **Kohlenwasserstoffgemisch gemäß Herstellbeispiel 1 bis 5** | | 10 |
| Myritol® 318 | Caprylic/Capric Triglycerid | 43,6 |
| Wacker HDK H 20 (Wacker) | Silica Dimethyl Silylate | 12,5 |
| Parfum | | 0,3 |
| pH Wert | | N.A. |
| Viskosität bei 20˚C, Helipath, spindel E, 5rpm (mPas) | | 500000 |
| Herstellung: Vermischen der Zutaten in der angegebenen Reihenfolge. | | |

**Glossy light Hair Cream**

| Phase | HanaeJsname | INLI | Gew. % |
|---|---|---|---|
| I. | Dehyquart® F 75 | Distearoylethyl hydroxyethylmonium Methosulfat (und) Cetearyl Alkohol | 0,8 |
| | Dehyquart® L 80 | Dicocoylethyl Hydroxyethylmonium Methosulfat (und) Propylene Glycol | 1,8 |
| | **Kohlenwasserstoffgemisch gemäß Herstellbeispiel 1 bis 5** | | 1 |
| | Lanette® O | Cetearyl Alcohol | 5 |
| | Cetiol® CC | Dicaprylyl Carbonate | 4 |
| II. | Wasser, entionisiert | | 87,4 |
| III. | Konservierungsmitt el | | q.s. |
| | Parfum | | q.s. |
| >> | pH Wert | | 3,3 |
| | Viskosität bei 20˚C, Brookfield RVT, spindel 4, 10 rpm (mPas) | | 8600 |

Herstellung: 1. Erhitzen von Phase I auf 80-85˚C, 2. Erhitzen von Phase II auf 80-85˚C und hinzufügen über Phase I. Ca. 30 Minuten Rühren bei derselben Temperatur. 3. Abkühlen lassen bei leichtem Rühren. 4. Wenn die Temperatur unter 40˚C ist, hinzufügen von Phase III in angegebener Reihenfolge, 5. Anpassen des pH Wertes wenn nötig.

**Tiefen Haar Pflege Creme mit Wildmango Butter**

| Phase | Handelsname | INCI | Gew.-% |
|---|---|---|---|
| I. | Strukture XL (National Starch) | Hydroxypropyl Starch Phospat | 5 |
| | Wasser, entionisiert | | 86,3 |

(fortgesetzt)

| Phase | Handelsname | INCI | Gew.-% |
|---|---|---|---|
| II. | Dehyquart L 80 | Dicocoylethyl Hydroxyethylmonium Methosulfat (und) Propylene Glycol | 2,6 |
| | **Kohlenwasserstoffgemisch gemäß Herstellbeispiel 1 bis 5** | | 1 |
| | Irwinol® LS 9319 | Octyldodecanol (und) Irvingia gabonensis (und) Hydrogenated Coco Glycerides | 0,1 |
| | Lamesoft®TM Benz | Glycol distearate (and) Coco Glucoside (and) Glyceryl Oleate (and) Glyceryl Stearate | 4 |
| | Gluadin ®WLM | Hydrolyzed Wheat Protein | 0,5 |
| | Nutrilan®MILK | Hydrolyzed Milk Protein | 0,5 |
| III. | Konservierungsmittel | | q.s. |
| | Parfum | | q.s. |
| >> | pH Wert | | 3,8 |
| >> | Viskosität (mPas), Brookfield RVT, 20˚C, Spindel 4, 10 rpm | | 10000 |

Herstellung: 1.Auflösen von Struktur XL in Wasser und Rühren bis zur vollständigen Homogenität, 2. Anpassen des pH Wertes auf 3,5 - 4,0 mit Zitronensäure, verdünnt, 3. Hinzufügen der restlichen Zutaten in der aufgeführten Reihenfolge während des Rührens (Irwinol muss vor der Hinzugabe geschmolzen werden)

**Hair Cream gloss & Conditioning**

| Phase | Handelsname | INCI | Gew.-% |
|---|---|---|---|
| I. | Dehyquart® F 75 | Distearoylethyl Hydroxyethylmonium Methosulfat (und) Cetearyl Alcohol | 2 |
| | Lanette®O | Cetearyl Alcohol | 3 |
| | **Kohlenwasserstoffgemisch gemäß Herstellbeispiel 1 bis 5** | | 1 |
| | Dehyquart® L 80 | Dicocoylethyl Hydroxyethylmonium Methosulfat (und) Propylene Glycol | 2 |
| | Cacao Butter (Nederland) | Theobroma cacao Seed Butter | 0,5 |
| | Structure XL (National Starch) | Hydroxypropyl Starch Phosphat | 5 |
| II. | Wasser, entionisiert | | 86,02 |
| | Glycerin | | 0,48 |
| III. | Konservierungsmittel | | q.s. |
| | Parfum | | q.s. |
| >> | pH Wert | | 4,3 |
| >> | Viskosität (mPas) Brookfield RVT, 20˚C Spindel 5, 10 rpm | | 47600 |

Herstellung: 1. Schmelzen der Zutaten von Phase I bei 80-85˚C, 2. Hinzufügen des Wassers bei derselben Temperatur während des Rührens. Diese Temperatur halten und für 30 Minuten rühren, 3. Abkühlen unter langsamen Rühren, 4. Anpassen des pH Wertes, wenn nötig mit Zitronensäure, 5. Hinzufügen des Konservierungsmittels und des Parfums bei unter 40˚C.

Deep penetrating Hair Reconstructor

| Phase | Handelsname | INCI | Gew.-% |
|---|---|---|---|
| I. | Dehyquart® L 80 | Dicocoylethyl Hydroxyethylmonium Methosulfat (und) Propylene Glycol | 2 |
| | **Kohlenwasserstoffgemisch gemäß Herstellbeispiel 1 bis 5** | | 1 |
| | DC 949 (Dow Corning) | Amodimethicone (und) Cetrimonium Chloride (und) Trideceth-12 | 1 |
| | Gluadin® WLM | Hydrolyzed Wheat Protein | 2 |
| | Parfum | | q.s. |
| | Konservierungsmittel | | q.s. |
| II. | Lamesoft® PW 45 | Cetyl palmitate (and) Beheneth-10 (and) Hydrogenated Castor Oil (and) Glyceryl Stearate | 4 |
| | Wasser, entionisiert | | 37,75 |
| **III.** | Rheocare®CTH(E) (Cosmetic Rheologies) | Polyquaternium-37 (und) Propylene Glycol Dicaprylate/Dicaprate (and) PPG-1 Trideceth-6 | 2,25 |
| | Wasser, entionisiert | | 50 |
| >> | pH Wert | | 4 |
| | Viskosität (mPas) Brookfield | kfield RVT, 20˚C Spindel 4, 10 rpm | 10500 |

Herstellung: 1. Mischen der Komponenten von Phase I bis zur vollständigen Homogenität, 2. Auflösen von Lamesoft® PW 45 in Wasser und hinzufügen über Phase 1, 3. Auflösen von Rheocare Polymer in entionisiertem Wasser bis man eine homogene Creme erhält, 4. Einzufügen von Phase I + 11 über Phase III, 5. Anpassen des pH Wertes wenn nötig.

**Hair Conditioner Classic Care**

| Phase | Handelsname | INCI | Gew.-% |
|---|---|---|---|
| I. | Lanette® O | Cetearyl Alcohol | 4,5 |
| | Dehyquart® F 75 | Distearoylethyl Hydroxyethylmonium Methosulfat (und) Cetearyl Alcohol | 1,4 |
| | Cutina® GMS V | Glyceryl Stearate | 0,5 |
| | **Kohlenwasserstoffgemisch gemäß Herstellbeispiel 1 bis 5** | | 0,2 |
| | DC 200-1000 (Dow Corning) | Dimethicone | 0,2 |
| II. | Dehyquart® A-CA | Cetrimonium Chloride | 2 |
| | Wasser, entionisiert | | 91,2 |
| III. | Konservierungsmittel | | q.s. |
| | Parfum | | q.s. |
| | pH Wert | | 3,9 |
| | Viskosität (mPas) Brookfield RVT, 20˚C Spindel 4, 10 rpm | | 19700 |

Herstellung: 1. Mischen von Phase I bei 80-85˚C bis zur Homogenität, 2. Hinzufügen von Phase II über Phase

I bei derselben Temperatur (80-85°C) und Rühren. Weiterrühren während 30 Minuten, 3. Abkühlen bei langsamen Rühren und hinzufügen von Phase III bei einer Temperatur von weniger als 40°C, 4. Anpassen des pH Wertes wenn nötig (pH Wert ist = 3,5 - 4,5).

**Haar Glanz Nebel ("Mist")**

| Handelsname | INCI | Gew.-% |
|---|---|---|
| **Kohlenwasserstoffgemisch gemäß Herstellbeispiel 1 bis 5** | | **80** |
| Cetiol® CC | Dicaprylyl Carbonat | 15 |
| Dow Corning® 200 Fluid (Dow Corning) | Dimethicone | 2,7 |
| Neo® Heliopan AV (Haarmann & Reimer) | Ethylhexyl Methoxycinnamate | 1,5 |
| Wacker PDM 1000 | Trimethylsiloxyphenyl Dimethicone | 0,5 |
| Parfum | | 0,3 |
| pH Wert | | N.A. |
| Viskosität bei 20 °C (mPas) | | < 100 |
| Herstellung: Mischen der Zutaten in der angegebenen Reihenfolge | | |

**Sprühbarer Hair Conditioner, leave-on**

| Phase | Handelsname | INCI | Gew.-% |
|---|---|---|---|
| I. | Monomuls® 60 35 C | Hydrogenated Palm Glycerides | 1,24 |
| | Eumulgin® B 1 | Ceteareth-12 | 2,76 |
| | Cetiol® S | Dioctylcyclohexane | 9 |
| | Cetiol® OE | Dicaprylyl Ether | 9 |
| | **Kohlenwasserstoffgemisch gemäß Herstellbeispiel 1 bis 5** | | 2 |
| II. | Wasser | | 56,2 |
| | Gluadin® WQ | Laurdimonium Hydroxypropyl hydrolyzed wheat protein | 2,85 |
| | Plantacare® 1200 UP | Lauryl Glucoside | 1 |
| | Konservierungsmittel | | q.s. |
| | Parfum | | q.s. |
| | Viskosität mPas | | |

Herstellung: 1.Erhitzen von Phase I auf 85°C und Rühren bis es homogen ist, 2. Erhitzen von Phase II auf 85°C und langsam einzufügen zu Phase I während des Rührens, 3. Hinzufügen von Phase III (kalt) bei 78°C zu PhaseI/II während des Rührens, 4. Der Emulsion erlauben Abzukühlen während des Rührens, welches die Emulsion ständig in Bewegung hält. Vermeiden von Lufteinschluss. Hinzufügen von Phase IV bei 30°C.

**Cream to Powder Foundation**

| | Komponente INCI Bezeichung (Handelsname, Hersteller) | Gew.% |
|---|---|---|
| A | Diisostearyl malate (Corum 5015, Corum) | 7,7 |
| | Titanium dioxide (HD-0748, Lubrizol/Noveon) | 12,6 |
| | Iron oxides (HD-3170, Lubrizol/Noveon) | 1,25 |
| | Iron oxides (HD-3511, Lubrizol/Noveon) | 0,2 |
| B | Dimethicone (and) dimethicone crosspolymer (DC 9041 Sillcone Elastomer Blend, Dow Corning) | 8 |
| | **Kohlenwasserstoffgemisch gemäß Herstellbeispiel 1 bis 5** | 5 |
| | Ethylhexyl Isononanoate (Corum 5021, Corum) | 6 |
| | Pentaerythryl tetraisostearate (Corum 5041, Corum) | 7 |
| | Polyisobutane (and) $C_{15-19}$ alkane (and) $C_{12-14}$ isoparaffin (Permethyl 284C, Presperse) | 3 |
| | Cholesteryl hydroxystearate (Corum 5-89, Corum) | 0,5 |
| | Carnauba Wachs | 4 |
| | Cetyl Palmitate (Corum 5000, Corum) | 0,6 |
| | Myristyl myristate (Corum 5028, Corum) | 0,6 |
| | Tribehenine (Corum 5094, Corum) | 0,5 |
| | Mikrocristallines Wachs | 1,5 |
| | Methyl methacrylate crosspolymer (Glanzpearl GMX 0610, ISP) | 5,5 |
| | Boron nilinde (Soltouch CC6059, Momantive) | 1 |
| | Talk (und) methicone (Corum 5901, Corum) | 11,67 |
| | Mica (und) methicone (und) Parffinium liquidum (mineral) oil (Sericite SL012P, Nikko)10 | |
| | Lauroyl lysine (Corum 5106, Corum) | 6 |
| C | Pentaerythryl tetraisostearate (und) Paraffinium liquidum (mineral) oil (und) disteardimonium hectorite (und) propylene carbonate (und) palmitoyl tripeptide (Corum 9913, Corum) | 3 |
| | Pentaerythryl tetraisostearate (und) Paraffinium liquidum (mineral) oil (und) disteardimonium hectorite (und) propylene carbonate (und) palmitoyl oligopeptide (Corum 8616, Corum) | 3 |
| | Phenoxyethenol (und) methylparaben (und) butylparaben (und) ethylparaben (und) propylparaben (Paragon MEPB, Molmyre) Parfum | 1 0,06 |

Herstellung: Vormischung von A herstellen, B zu A geben und auf 90 ˚C erhitzen, gut mischen, Hitzezufuhr einstellen und C zugeben.

**Makeup foundation**

| Komponente INCI Bezeichung (Handelsname, Hersteller) | Gew.-% |
|---|---|
| Wasser | 78,2 |
| Glycerin | 1,89 |
| Triethanolamine 99% | 0,75 |
| Methylparaben (Nipagin M. Nipa) | 0,19 |
| Xanthan gum (Keltrol SP, CP Kelco) | 0,28 |
| Titanium dioxid (Softex Titanium dioxide, C47-7756, Sun Chemical) | 8,75 |
| Brown iron oxid (SunChroma Brown iron oxide, C33-31 Sun Chroma, Sun Chemical) | 1,08 |

(fortgesetzt)

| Komponente INCI Bezeichung (Handelsname, Hersteller) | Gew.-% |
|---|---|
| Red iron oxide (SunChroma red iron oxide, C33-124 Sun Chroma, Sun Chemical) | 0,17 |
| Phenyl trimethicone (DC 556 cosmetic Fluid, Dow Corning) | 3,41 |
| **Kohlenwasserstoffgemisch gemäß Herstellbeispiel 1 bis 5** | 1 |
| Stearic acid, 94% | 1,33 |
| Cetyl stearyl alcohol (Lanette O, Cognis) | 2,84 |
| Propylparaben (Nipasol, M. Nipa) | 0,1 |
| **"Skin Corrector"** | |
| Komponente INCI Bezeichung (Handelsname, Hersteller) | Gew.-% |
| Synthetisches Wachs (Cirebelle 108, Cirebella) | 2,9 |
| **Kohlenwasserstoffgemisch gemäß Herstellbeispiel 1 bis 5** | 19,51 |
| Synthetisches Wachs (Cirebelle 303, Cirebella) | 2,33 |
| Titanium dioxid Wachs dispersion (CWD-8906, Sun Chemical) | 65,12 |
| Yellow iron oxide wax dispersion (CWD-8942, Sun Chemical) | 4,18 |
| Red iron oxide wax dispersion (CWD-8801, Sun Chemical) | 1,91 |
| Black iron oxide wax dispersion (CWD-9921, Sun Chemical) | 0,05 |
| Alumina (und) titanium dioxid (SpectraFlex Focus White C88-1001, Sun Chemical) | 4 |

**Matte Antiaging Foundation**

| Phase | Komponente INCI Bezeichung (Handelsname, Hersteller) | Gew.-% |
|---|---|---|
| A | Cyclopentasiloxane (und) dimethicone copolyol (DC 5225C, Dow Corning) | 12 |
| | **Kohlenwasserstoffgemisch gemäß Herstellbeispiel 1 bis 5** | 10 |
| | Titanium dioxid (BTD-401, Kobo) | 9 |
| | Yellow iron oxide (BYO-12, Kobo) | 0,8 |
| | Red iron oxide (BRO-12, Kobo) | 0,07 |
| | Black iron oxide (BRO-12, Kobo) | 0,034 |
| | Methylmethacrylate crosspolymer (Sepimat SB, Seppic) | 1 |
| | Methylmethacrylate crosspolymer (Sepimat HB V, Seppic) | 1 |
| | Phenoxyethanol (und) methylparaben (und) ethylparaben (und) propylparaben (und) butylparaben (Sepicide HB, Seppic) | 17,296 |
| | Parfum | 0,3 |
| B | Dipalmitoyl hydroxyproline (Sepilift DPHP, Seppic) | 0,5 |
| | Palmitoyl praline (und) magnesium palmitoyl-glutamate (und) sodium palmitoyl sarcosinate (Sepifeel One, Seppic) | 05, |
| | Undecylenoyl phenylalanine (Sepiwhite MSH, Seppic) | 0,1 |
| C | Sodium hydroxide, 48% Lösung | 0,2 |
| | Glycerin | 5 |
| | Sodium chloride | 2 |

(fortgesetzt)

| Phase | Komponente INCI Bezeichung (Handelsname, Hersteller) | Gew.-% |
|---|---|---|
| | Imidazolidinyl urea (Sepicide Cl, Seppic) | 0,2 |
| D | Wasser | 40 |

Herstellung: Phase A wird hergestellt durch Dispersion der Puder in den Silikonen und den n-Undecan/n-Tridecan unter Rühren, in Kugelmühle zerkleinern. Phasen B, C und D werden separat hergestellt und jeweils auf 80 ˚C erhitzt. Phase D wird zu Phase C hinzugefügt, dieses Gemisch wird dann zu B gegeben. Homogenisierung für einige Minuten, Abkühlen unter mäßigem Rühren. Diese Mischung wird dann unter heftigem Rühren zu Phase A gegeben und die entstehende Emulsion wird homogenisiert.

**Super Matte Foundation**

| Phase | Komponente INCI Bezeichung (Handelsname, Hersteller) | Gew.-% |
|---|---|---|
| A | Wasser | 54,72 |
| | Disodium EDTA | 0,1 |
| | Wasser (und) montmorillonite (und) cetyl alcohol (und) glycerin (und) pentylene glycol (und) sucrose stearate (und) aderoglucan (Matte Lite Concentrate 269, MMP) | 20 |
| B | Sucrose distearate (Sistearna SP30-C, MMP/Sisterna) | 0,75 |
| C | Titanium dioxide | 6 |
| | Red iron oxide | 0,5 |
| | Yellow iron oxide | 1,25 |
| | Black iron oxide | 0,13 |
| | Nylon 12 | 0,25 |
| D | Dimethicone (und) dimethicone/vinyl dimethicone crosspolymer (Clearocast 200, MMP) | 5 |
| | **Kohlenwasserstoffgemisch gemäß Herstellbeispiel 1 bis 5** | 1 |
| | Isododecane (und) Isononyl Isononanoate (Clearocast 550, MMP) | 10 |
| E | Konservierungsmittel | 0,3 |

**"Chocolate Mousse"**

| Phase | Komponente INCI Bezeichung (Handelsname, Hersteller) | Gew.-% |
|---|---|---|
| A | Cl 77891 dimethicone (SAT-T-47051, US cosmetics) | 0,528 |
| | Cl 77491 dimethicone (SAT-R-33128, US cosmetics) | 0,456 |
| | Cl 77492 dimethicone (SAT-Y-33873, US cosmetics) | 0,732 |
| | Cl 77499 dimethicone (SAT-B-33134, US cosmetics) | 0,284 |
| B | Dimethicone/vinyl dimethicone crosspolymer (und) silica (Dow Corning 9701 Cosmetic Powder, Dow Corning) | 23 |
| | Zink oxid (und) dimethicone (Z-cote HP-1, BASF) | 6 |
| C | Wasser | 4 |

(fortgesetzt)

| Phase | Komponente INCI Bezeichung (Handelsname, Hersteller) | Gew.-% |
|---|---|---|
| | Wasser (und) propylene glycol (und) Helianthus annuus (Sonnnenblume) seed oil (und) Theobroma cacao extract (und) sclarolum gum (Cocoa Phytolat, Alban Muller) | 1 |
| | Dimethicone (Dow Corning 200 Fluid, 5cSt, Dow Corning) | 22,75 |
| | **Kohlenwasserstoffgemisch gemäß Herstellbeispiel 1 bis 5** | 22,75 |
| | Cyclopentasiloxane (und) dimethiconol (Dow Corning 1501 Fluid, Dow Corning) | 5 |
| | Cyclopentasiloxane (und) dimethicone crosspolymer (und) dimethiconol (Dow Corning 9546 Silicone Elastomer Blend, Dow Corning) | 5 |
| | Theobroma cacao seed butter (Cocoa Butter, Alban Muller) | 1 |
| | Cocos nucifera (Kokosnuss) Öl (und) Gardenia tahitensis Blüten Extrakt (Monoi de Tahip Butter frangaced, Pacific Sud Cosmetique) | 1 |
| | Tocopherol acetate (di-alpha-Tocopherol Acetate, DSM) | 0,5 |
| | Polysilicone-15 (Parsol-SLX, DSM) | 5 |
| D | Parfum (Chocolat Creme 0310585, Expressions Perfumées) | 1 |

Herstellung: A wird vermahlen, und B wird zugefügt und gerührt. Phase C wird vorgelegt und unter Rühren und auf 45 ˚C erhitzt. Dann wird AB in kleinen Portionen zugefügt und zum Schluss wird D zugegeben.

**Fresh Foundation Emulsion Mousse**

| Phase | Komponente INCI Bezeichung (Handelsname, Hersteller) | Gew.-% |
|---|---|---|
| A | Ceteareth-25 (und) PEG-2 stearate (und) Paraffinium liquidum (mineral) oil (und) hydrogenated coconut oil (und) cetyl alcohol (und) sodium stearate (Base O/W 097, LCW) | 5 |
| | Caprylic/capric triglyceride | 15 |
| | **Kohlenwasserstoffgemisch gemäß Herstellbeispiel 1 bis 5** | 1 |
| B | Wasser | 59 |
| | Konservierungsmittel | 0,3 |
| | Glycerin | 2 |
| | Algas extract (und) sorbitol (Fucosorb, LCW) | 2 |
| C | CI 77499 (und) silica (Unipure Black LC 989 EM, LCW) | 0,1 |
| | CI 77492 (und) silica (Unipure Yellow LC 162 EM, LCW) | 0,75 |
| | CI 77491 (und) silica (Unipure Red LC 361 EM, LCW) | 0,18 |
| | CI 77891 (und) silica (Unipure white LC 981 EM, LCW) | 2,95 |
| | Mica (Submica FL, LCW) | 1 |
| | Methylmethacrylate crosspolymer (und) silica (Covabeed PMMA 2MUSL, LCW) | 1 |
| | Mica (Mica 8 R2041, LCW) | 4 |
| | Talk (Talc LCW, LCW) | 4 |
| | Sodium plyacrylate (Covacryl MV50, LCW) | 1 |
| | Silica dimethyl silyata (Covasilic 15, LCW) | 0,5 |

Herstellung: A und B werden getrennt hergestellt und jeweils auf 70˚C erhitzt. B wird zu A unter Rühren zugefügt.

Phase C wurde separate hergestellt und mit einem Pulvermischer gut durchmischt und danach unter Rühren zur Mischung aus A und B gegeben.

**Mattifying Fluid Foundation**

| Phase | Komponente INCI Bezeichung (Handelsname, Hersteller) | Gew.-% |
|---|---|---|
| A | Wasser | 20 |
| | Butylene glycol | 4 |
| | PEG-400 | 4 |
| | Dimethicone copolyol PEG-7 phosphate (Pecostil PS 100, Pheonix) | 1 |
| B | Titanium dioxide | 10 |
| | Talk | 2 |
| | Iron oxide yellow | 0,9 |
| | Iron oxide red | 0,3 |
| | Iron oxide black | 0,05 |
| | Titanium dioxide (Cl 77891) (und) iron oxide (Cl 77491) (und) mica (und) triethoxy caprylylsilane | 3 |
| C | $C_{20-22}$ alkyl phosphate (und) $C_{20-22}$ alcohols (Sensanov WR, Seppic) | 1 |
| | $C_{14-22}$ alcohol (und) $C_{12-20}$ alkylglucoside (Montanov L, Seppic) | 2 |
| | Isostearyl isostearat | 8 |
| | **Kohlenwasserstoffgemisch gemäß Herstellbeispiel 1 bis 5** | 1 |
| | Ethylhexyl palmitate | 9 |
| | Vegetable oil (und) palm alcohol (und) lecithin (und) vegetable stearyl esters (Sensactive Veg. Chemyunion) | 2 |
| D | Wasser | q.s. to |
| | Xanthan gum | 100 0,15 |
| E | Tromethamine | q.s. |
| | Tetrasodium EDTA Polymethyl methacrylate (Micropearl M100, Seppic) | 0,05 3 |
| F | Sodium acrylate/acryloyldimethyllaurate copolymer (und) isohexadecanle (und) polysorbane (Simulgel EG, Seppic) | 0,5 |
| G | Phenoxyethanol (und) methylparaben (und) ethylparaben (und) propylparaben (und) butylparaben (Chemynoi, Chemyunion) | 0,1 |
| | Parfum | qs |

**Ultra gloss Lippenstifte**

| Komponente INCI Bezeichung (Handelsname, Hersteller) | Gew.-% |
|---|---|
| Polyglyceryl-10 hydroxystearate/stearata/eicosadioate (und) dextrin palmitate (Nikkol Nikkowax LM, Nikko) | 5 |
| Copernicia cerifera (carnauba) Wachs | 10 |
| Cera alba (Bienenwachs) | 15 |

(fortgesetzt)

| Komponente INCI Bezeichung (Handelsname, Hersteller) | Gew.-% |
|---|---|
| Cetyl alcohol | 5 |
| Ricinus communis (castor) seed oil | 63 |
| **Kohlenwasserstoffgemisch gemäß Herstellbeispiel 1 bis 5** | 2 |
| Herstellung: Mischung erhitzen und bei 80˚C schmelzen und in Gießform füllen. | |

**Maximum shine Lippenstifte**

| Phase | Komponente INCI Bezeichung (Handelsname, Hersteller) | Gew.-% |
|---|---|---|
| A | D&C Red No. 6 Barium Lake (Red 6 lake, Emerald Hilton Davis) | 0,7 |
| | Iron oxides (CC33-5136 Russet Iron Oxide, Sun Chemical) | 2,5 |
| | Iron oxides (CC33-135 Black iron oxide, Sun Chemical) | 0,25 |
| | Ricinus communis (castor) seed oil (Crystal O, CasChem) | 3 |
| B | Ricinus communis (castor) seed oil (Crystal O, CasChem) | 35,75 |
| | Copemica cerifera (carnauba) Wachs (Carnauba No. 1, Strahl & Pitsch) | 1,1 |
| | Euphorbia cerifera (candelilla) Wachs (Candelilla Pure, Strahl & Pitsch) | 5 |
| | Ozokerite (Ozokerite Wax 1700, Frank B, Ross Company) | 2,5 |
| | **Kohlenwasserstoffgemisch gemäß Herstellbeispiel 1 bis 5** | 1 |
| | Mikrocrystallines Wachs (Microwax SP 19, Strahl & Pitsch) | 2 |
| | Diisostearyl malata (Schercamol DISM Ester, Lubrizol/Noveon) | 24 |
| | Trisostearyl citrate (Schercomol TISC Ester, Lubrizol/Noveon) | 16,85 |
| | Methylparaben | 0,2 |
| | Propylparaben | 0,1 |
| C | Mica (und) iron oxides (Colorona Copper, EMD chemicals) | 5 |
| D | Ascorbyl palmitate | 0,05 |

**Ultra lasting lip color**

| Phase | Komponente INCI Bezeichung (Handelsname, Hersteller) | Gew.-% |
|---|---|---|
| A | D&C Red No. 7 Calcium Lake (31-DA-3707, Emerald Hilton Davis) | 0,6 |
| | D&C Red no. 6 Barium Lake (31-DA-3006, Emerald Hilton Davis) | 1,5 |
| | Iron oxides (CC33-5138 Russet iron oxide, Sun Chemical) | 4,5 |
| | Titanium dioxide | 0,5 |
| | Ricinus communis (castor) seed oil (Crystal O, CasChem) | 8,3 |
| B | Ricinus communis (castor) seed oil (Crystal O, CasChem) | 21,65 |
| | Copernica cerifera (carnauba) Wachs (Carnauba No. 1, Strahl & Pitsch) | 1,5 |
| | Euphorbia cerifera (candelilla) Wachs (Candelilla Pure, Strahl & Pitsch) | 6 |

(fortgesetzt)

| Phase | Komponente INCI Bezeichung (Handelsname, Hersteller) | Gew.-% |
|---|---|---|
| | Ozokerite (Ozokerite Wax 1700, Frank B. Ross Company) | 2,5 |
| | Mikrocrystallines Wachs (Microwax SP 19, Strahl & Pitsch) | 3,5 |
| | Triisostearyl polyglyceryl-3 dimer diinoleate (Schercemol PTTD Ester, Lubrizol/Noveon) | 10 |
| | Trisostearyl citrate (Schercomol TISC Ester, Lubrizol/Noveon) | 22 |
| | Isopropyl Isostearate (Schercamol 316 Ester, Lubrizol/Noveon) | 4 |
| | **Kohlenwasserstoffgemisch gemäß Herstellbeispiel 1 bis 5** | 1 |
| | Methylparaben | 0,2 |
| | Propylparaben | 0,1 |
| C | Mica (und) Titanium dioxide (Flamenco Red, Engelhard) | 12 |
| D | Ascorbyl palmitate (Ascorbyl palmitate, DSM) | 0,05 |

**Long lasting Shine Lip Gloss**

| Phase | Komponente INCI Bezeichung (Handelsname, Hersteller) | Gew.-% |
|---|---|---|
| A | Octyldodecanol | 47,93 |
| B | Ethylcellulose (Ethocel 100FP, Amerchol) | 5 |
| C | **Kohlenwasserstoffgemisch gemäß Herstellbeispiel 1 bis 5** | 18 |
| | bis-Biglyceryl polyacladipate-2 | 15 |
| | Octyl methoxycinnamate | 7,5 |
| | Polyethylene | 2 |
| D | Red No. 7 Aluminium lake (Sun Chemical) | 0,58 |
| | Iron oxide (Cl 77491, Sun Chemical) | 0,52 |
| | Iron oxide (Cl 77492, Sun Chemical) | 0,48 |
| | Iron oxide (Cl 77499, Sun Chemical) | 0,09 |
| E | Mica (und) titanium dioxide (Prepearis Shining Salin, Prespense) | 1,9 |
| | Dimethicone crosspolymer/silica (DC 7901 Powder, Dow Corning) | 1 |

**High-shine Lippenstift**

| | Komponente | Gew.-% |
|---|---|---|
| A | Hydrogenated Polydecene (und) ethylene propylene/styrene copolymer (und) butylene/ethylene/ styrene copolymer (Versagel MG 750, Penreco) | 25,89 |
| | Petrolatum (und) ethylene/propylene/styrene copolymer (und) | 10,38 |
| | butylene/ethylene/styrene copolymer (Versagel P100, Penreco) | |
| | Euphorbia cerifera (candelilla) wachs (Candelilla wax refined, Ross) | 8,8 |
| | Cera alba (Bienenwachs) (White Bleached Beeswax, Ross) | 5,18 |
| | Lanolin Wachs | 4,14 |

(fortgesetzt)

| | Komponente | Gew.-% |
|---|---|---|
| | Lanolin Öl | 4,14 |
| | Octyldodecanol (Eutanol® G, Cognis) | 4,14 |
| | **Kohlenwasserstoffgemisch gemäß Herstellbeispiel 1 bis 5** | 3,63 |
| | Copernicia cerifera (carnauba) Wachs (Carnauba wax, Ross) | 3,11 |
| | Octyldodecyl stearoyl stearate (Ceraphyl 647, ISP) | 3,11 |
| | Tridecyl trimellitate (Liponate TDTM, Lipo) | 3,11 |
| | Propylparaben | 0,1 |
| | Butylated hydroxytoluene | 0,1 |
| B | Butyrospermum parkii (shea butter) (Shea Butter, Ultra refined, Biochemica) | 5,18 |
| | Jojoba esters (Floraesters 30, Floratech) | 2,07 |
| | Theobroma grandiflorum seed butter (Cupuacu Butter, Beraca/Ross) | 2,07 |
| C | Iron oxides (und) synthetic fluorphlogopite (Sunshine Super Russet, Sun Chemical) | 8,29 |
| | Iron oxides (Soft-Tex Russet Iron Oxide, Sun Chemical) | 5,18 |
| | Red No. 7 Lake | 1,04 |
| | Yellow No. 6 Lake | 0,38 |

**Eleganter Lippenstift**

| Komponente INCI Bezeichnung (Hersteller) | Gew.-% |
|---|---|
| Dipentaerythrityl hexacaprylate/hexacaprate (und) ridecyl trimellitate (und) tri-decyl stearate (und) neopentyl glycol dicaprylate/dicaprate (Lipovol MOS-350, Lipo) | 72,2 |
| Synthetisches Bienenwachs (Lipobee 102, Lipo) | 8 |
| Copernicia cerifera (carnauba) Wachs | 4 |
| Euphorbia cerifera (candelilla) wachs | 4 |
| **Kohlenwasserstoffgemisch gemäß Herstellbeispiel 1 bis 5** | 1 |
| Propylparaben | 0,2 |
| Mica (und) boron nitride (Lipomic 501 BN, Lipo) | 1 |
| Tocopherol (Vitamin E Acetate, Ruger Chemical) | 0,5 |
| Titanium dioxide (Cl 77891) (und) dipentaerythrityl hexacaprylate/hexacaprate (und) tridecyl trimellitate (und) tridecyl stearate (und) neopentyl glycol dicaprylate/dicaprate (Titanium dioxide, 60% in Lipovol MOS-350, Lipo) | 2,8 |
| Iron oxide (Cl 77489) (und) dipentaerythrityl hexacaprylate/hexacaprate (und) tridecyl trimellitate (und) tridecyl stearate (und) neopentyl glycol dicaprylate/dicaprate (iron oxide Red, 60% in Lipovol MOS-350, Lipo) | 4,7 |
| Iron oxide (Cl 77489) (und) dipentaerythrityl hexacaprylate/hexacaprate (und) tridecyl trimellitate (und) tridecyl stearate (und) neopentyl glycol dicaprylate/dicaprate (iron oxide Yellow, 60% in Lipovol MOS-350, Lipo) | 0,8 |
| Iron oxide (Cl 77489) (und) dipentaerythrityl hexacaprylate/hexacaprate (und) tridecyl trimellitate (und) tridecyl stearate (und) neopentyl glycol dicaprylate/dicaprate (iron oxide Black, 60% in Lipovol MOS-350, Lipo) | 0,8 |

Herstellung: Alle Bestandteile werden unter Rühren auf 80-85 °C erhitzt. Der Ansatz wird auf 70-75°C abgekühlt und in eine Gießform gefüllt.

Lip Gloss

| | Komponente INCI Bezeichnung (Hersteller) | Gew.-% |
|---|---|---|
| A | **Kohlenwasserstoffgemisch gemäß Herstellbeispiel 1 bis 5** | 2 |
| | $C_{4-24}$ alkyl dimethicone/divinyldimethicone crosspolymer dimethicone (NuLastic Silk MA, DM, Alzo) | 15 |
| | Methylheptyldiisostearate (Beantree, Alzo) | 10 |
| | $C_{4-24}$ alkyl dimethicone/divinyldimethicone crosspolymer dimethicone (NuLastic Silk MA, DM-6, Alzo) | q.s to 100 |
| | Tocopherol (Vitamin E, BASF) | 0,2 |
| | Konservierungsmittel | qs |
| | Parfum (Peach Nectar 6104085, Bell Flavors & Fragances) | 0,2 |
| B | Calcium Sodium borosilicate (und) Iron oxides (Reflecks Dimensions Shiny Bronze G25000000, BASF) | 1 |
| | Mica (und) Titanium dioxide (Flamenco Winter Sparide 1300000 | 5 |
| | Hydrogenated polyisobutene (Luvitol Lite, BASF) | 10 |

Herstellung: Phasen A und B werden in getrennten Gefäßen jeweils homogenisiert, dann wird Phase B zu Phase A gefügt und gemischt, bis das Produkt homogen vorliegt.

**Creamy Sheer Lip Colour**

| | Komponente INCI | Gew.-% |
|---|---|---|
| A | Sesamum indicum (Sesam) Samen Öl | 21,28 |
| | Octyldodecanol (Jarcol 1-20, Jarchem) | 15,47 |
| | Euphorbia cerifera (candelilla) wachs (SP75, Strahl & Pitsch) | 12 |
| | Isohexyl Caprate | 10,07 |
| | Bis-Diglyceryl polyaryladipate-2 (Softisan 649, Sasol) | 9,95 |
| | Polyisobutene (Permethyl 104A, Presperse) | 9,67 |
| | **Kohlenwasserstoffgemisch gemäß Herstellbeispiel 1 bis 5** | 2,89 |
| | Polyethylene (Performalene PL Polyethylene, New Phrase) | 2,5 |
| | Mikrocrystallines Wachs (Be Square 195, New Phrase) | 2,5 |
| B | Synthetic wax and titanium dioxide (und) Isopropyl Titanium triisostearate (SW65U, Kobo) | 4,24 |
| | Synthetic wax and iron oxide (und) Isopropyl Titanium triisostearate (SW60ER, Kobo) | 1,41 |
| | Synthetic wax and D&C Red No. 6 Barium Lake (und) Isopropyl Titanium triisostearate (SW40R6E, Kobo) | 1,41 |
| | Synthetic wax and Blue No.1 Lake (und) Isopropyl Titanium triisostearate (SW30B1A, Kobo) | 0,71 |
| | Mica (und) titanium dioxide (KTZ Classic White, Kobo) | 0,96 |

(fortgesetzt)

| | Komponente INCI | Gew.-% |
|---|---|---|
| C | Vitis vinifera (grape) seed oil | 4,84 |
| | Vitamin E acetata | 0,1 |

**Lip volume booster**

| | INCI Bezeichnung (Handelsname, Hersteller) | Gew.-% |
|---|---|---|
| A | Ricinus communis (castor) oil | 39,7 |
| | Hydrogenated coco-glycerides (Softisan 100, Sasol) | 8 |
| | Candelilla cera (und) Paraffin (Cenilla G, Barlocher) | 9 |
| | Bis-Diglyceryl polyacyladipate-1 (Softisan 645, Sasol) | 8 |
| | Cera alba (Cerabell White, Barlocher) | 3 |
| | Cera carnauba Wachs (Cernauba T1, Barlocher) | 2 |
| | Tricaprylin (Trivant OC-G, Trivant Chemical) | 20 |
| | Tocopheryl acetate (D-alpha-Tocopheryl Acetate, DSM/Roche) | 0,5 |
| | **Kohlenwasserstoffgemisch gemäß Herstellbeispiel 1 bis 5** | 1 |
| | Propylparaben (Nipasol M. Nipa) | 0,1 |
| | BHT (Butylhydroxytoluol) | 0,05 |
| B | Ricinus communis (castor) oil (und) Cl 15850 (und) BHT (COD 8001, Sun Chemical) | 0,8 |
| | Calcium aluminum borosilicate (und) Cl 77891 (und) silica (und) tin oxide (Fionastar Noble Sparks, Merck) | 1 |
| | Mica (und) Cl 77891 (und) Cl 77491 (Timiron MP29 Sun Gold Sparkle, Merck) | 2 |
| | Mica (und) Cl 77891 (Timiron MP149 Diamond Cluster, Merck) | 2 |
| C | Glycerin (und) palmitoyl tripeptide-3 (Syn-Coll, Pentapharm) | 2,5 |
| | Alkyl methacrylate crosspolymer (Poly-Pore E200, Amcol HBS) | 0,35 |

Herstellung: alle Bestandteile der Phase A auf 80˚C erhitzen, Phase B zu A hinzufügen, Phase C mischen und zu AB zugeben kurz vor der Homogenisierung.

**Plumping effect Lippenstift**

| | Komponenten INCI Bezeichnung (Handelsname, Hersteller) | Gew.-% |
|---|---|---|
| A | D&C Red No. 7 Ca Lake (Cl 15850:1) (HD-3007, Noveon) | 0,4 |
| | Red iron oxide (Cl 77491) (HD-3511, Noveon) | 0,4 |
| | Titanium dioxide Cl 77891 (HD-0748, Noveon) | 1,2 |
| | Paraffinum liquidum (mineral) oil (und) disteardimonium hectorite (und) propylene carbonate (Bentone Gel MO V, Elementis Specialities) | 1,5 |
| | Diisostearyl maleate (Corum 5015, Corum) | 10,5 |
| | Mica (und) titanium dioxide (und) tin oxide (Prestige Sparkling blue, Eckart) | 1,5 |

(fortgesetzt)

| | Komponenten INCI Bezeichnung (Handelsname, Hersteller) | Gew.-% |
|---|---|---|
| B | Pentaerythrityl tetraisostearate (Corum 5041, Corum) | 15 |
| | Cholesteryl hydroxystearate (Corum 5069, corum) | 1 |
| | Hydrogenated polydecene (Nexbase 2006FG, Fortum) | 15 |
| | **Kohlenwasserstoffgemisch gemäß Herstellbeispiel 1 bis 5** | 10 |
| | Isostearyl isostearate (corum 5088, Corum) | 19,9 |
| | Phenyltrimethicone (DC 556, Dow Corning) | 2 |
| | PEG-8 Bienenwachs (Corum 2680, Corum) | 2 |
| | Ceresin | 9 |
| | Euphorbia cerifera (candelilla) wachs | 2 |
| | Cera alba (Bienenwachs) | 2 |
| | Copernicia cerifera (carnauba) Wachs | 3 |
| | Jojoba ester (Floraester-70, Floratech) | 1 |
| | Pentaerythrityl tetraisostearate (und) Paraffinum liquidum (mineral) oil (und) disteardimonium hectorite (und) propyl carbonate (und) palmitoyl oligopeptide (Corum 8813, Corum) | 2 |
| C | Vanilyl butyl ether (Corum 9235, Corum) | 0,4 |
| | Flavor | 0,2 |

Herstellung: Phase A wird zur Homogenität gemischt, die Phase B wird hinzugefügt und auf 90˚C erhitzt und gut gemischt. Die Hitzezufuhr wird gestoppt und Phase C hinzugefügt, danach gut gemischt.

**Lippenstift**

| | Komponenten INCI Bezeichnung (Handelsname, Hersteller) | Gew.-% |
|---|---|---|
| A | Propylene glycol dicaprylate/dicaprate (und) bis-diglyceryl polyacyladipate-1 (und) bis-diglyceryl polyadipate-2 (Softisan Gel, Sasol) | 9 |
| | Stearalkonium hectorite (und) propylene carbonate (Softisan 649, Sasol) | 6 |
| | Hydrogenated coco-glycerides (Softisan 100, Sasol) | 22,5 |
| | Hydrogenated palm oil (Softisan 154, Sasol) | 3 |
| | $C_{12-10}$ alkyl octanoate (Cosmacol EDI, Sasol) | 5 |
| | Petrolsturm (Merkur Vaseline773, Merkur) | 11 |
| | Cera alba (Bienenwachs) | 7 |
| | Cyclomethicone (Beisli CM 040, Wacker Chemie) | 3 |
| | Kohlenwasserstoffgemisch gemäß Herstellbeispiel 1 bis 5 | 4 |
| | Ricinus communis (castor) oil | 6 |
| | Paraffin | 14 |
| | Antioxidans | qs |
| B | Titanium dioxide (Cl 77891) (Timiron Splendid Red, Merck) | 3,5 |
| | Mica (und) Titanium dioxide (Cl 77891) (Timiron Starlusler MP-115, Merck) | 2,5 |
| | Mica (und) Titanium dioxide (Cl 77891) (Timiron Super Silver Fine, Merck) | 3 |

(fortgesetzt)

| | Komponenten INCI Bezeichnung (Handelsname, Hersteller) | Gew.-% |
|---|---|---|
| C | Parfum | qs |
| | Tocopheryl acetate | 0,5 |

**Volumizing Mascara**

| | Komponenten INCI Bezeichnung (Handelsname, Hersteller) | Gew.-% |
|---|---|---|
| A | Cera alba (Bienenwachs) (White Beeswax SP422P, Strahl & Pitsch) | 2,76 |
| | Glyceryl stearate | 2,3 |
| | Copernicia cerifera (carnauba) Wachs (Carnauba wax SP63, Strahl & Pitsch) | 1,98 |
| | Stearic acid | 3 |
| | Ethylcellulose (Ethocel Standard 100FP Premium, Amerchol) | 3 |
| | Kohlenwasserstoffgemisch gemäß Herstellbeispiel 1 bis 5 | 1 |
| | Octyldodecanol | 7 |
| | Premised Chitosan PCA, 2,5% (Kytamer PCA Polymer, Amerchol) | 15 |
| B | Iron oxide (Black Iron Oxide, Sun Chemical) | 6 |
| | Wasser | 55,45 |
| C | Triethanolamine 99% | 1,5 |
| D | Propylene glycol (und) diazolidinyl ures (und) methylparaben (und) propylparaben (NiPaguard, Clariant) | 1 |

**Lidschatten Stift (Eye shadow stick)**

| | Komponenten INCI Bezeichnung (Handelsname, Hersteller) | Gew.-% |
|---|---|---|
| A | Kohlenwasserstoffgemisch gemäß Herstellbeispiel 1 bis 5 | 1 |
| | Ocryldodacyl neopentanoate (Elefac 1-205, Alzo) | qs. to 100 |
| | Calcium sodium borosilicate (und) silica (und) titanium dioxide (Reflecks MultiDimensions Varying Violet G58000000, BASF) | 14 |
| | Mica (und) Titanium dioxide (und) iron oxides (Gemtone Moonstone G004, BASF) | 1 |
| B | Butylene glycol | 3,13 |
| | Acetylated glycol stearate (Unitwax, Guardian, ISP) | 22 |
| | Silica (Cab-o-Sil M5, Cabot) | 1,5 |
| C | Simmondsia chinensis (jojoba) seed oil (Lipovol J. Lipo) | 7,5 |
| | Stearic acid (Emersol 132, Cognis) | 3,6 |
| | Konservierungsmittel | qs |
| | Antioxidans | qs |

Phase B wird geschmolzen und gemischt, und dann zur vorgemischten Phase A gegeben. AB werden unter Rühren auf 80-05˚C erhitzt und dann wird langsam Phase C zugegeben.

**Metallic Silver shine Mascara**

| | Komponenten INCI Bezeichnung (Handelsname, Hersteller) | Gew.-% |
|---|---|---|
| A | Wasser | 56,25 |
| | Xanthan gum (Keltrol T, CP Kelco) | 0,15 |
| | Magnesium aluminium silicate (Veegum HV, RT Vandervilt) | 0,55 |
| | Carbon black (und) wasser (MBD 201 20% Dispersion, Geotech) | 5 |
| B | Disodium EDTA (Edeta BD, BASF) | 0,05 |
| | Methylparaben (Methyl-4-hydroxybenzoate H 5501, Sigma Aldrich) | 0,3 |
| | Triethanolamine | 0,5 |
| | Propylene glycol | 2 |
| C | **Aluminium powder (und) silica (Visionaire Bright Silver sea, Eckart)** | 3 |
| D | **Kohlenwasserstoffgemisch gemäß Herstellbeispiel 1 bis 5** | 5 |
| | Cyclopentasiloxane (und) PEG/PPG-18/18 dimethicone (Dow Corning 5225C Formulation Aid, Dow Corning) | 1 |
| | Cera alba (Bienenwachs) (Ewacers 12, Wagner) | 10 |
| | Stearic acid (Kortacid 1695, Akzo Nobel) | 2 |
| | Glyceryl stearate (Imwitor 960K, Sasol Wax) | 2 |
| | Stearyl alcohol (Lanette 18, Cognis) | 2 |
| | Polyisobutene (Permethyl-104A, Chesham Chemicals) | 1 |
| | Phenoxyethanol (und) methylparaben (und) butylparaben (und) ethylparaben (und) propylparaben (und) isobutylparaben (Phenonip, Clariant) | 1,2 |
| | PVP polyvinylpyrrolidone (Luviskol K30 Powder, BASF) | 4 |
| | Carnauba Wachs (Ewacera 34, Wagner) | 4 |

Herstellung: A wird gemischt, B wird gemischt und zu A zugefügt, C wird zu AB gegeben und auf 75˚C erhitzt. D wird separat vorgemischt und auf 75˚C erhitzt und zu ABC gegeben, während des Abkühlens wird gerührt.

**Creamy Shadow Stick**

| | Komponenten INCI Bezeichnung (Handelsname, Hersteller) | Gew.-% |
|---|---|---|
| A | Kohlenwasserstoffgemisch gemäß Herstellbeispiel 1 bis 5 | 38,5 |
| | PPG-3 myristyl ether (Varonic APM, Evonik, Degussa) | 7,0 |
| | Polyglyceryl-4- isostearate (and) cetyl PEG-PPG-10/1 dimethicone (and) hexyl laurate (Abil WE 09, Evonik, Degussa) | 1,0 |
| | Dimethicone, 10 mPas | 2,5 |
| | Cera alba (bees wax) | 4,5 |
| | Copernicia cerifera (carnauba) wax | 4,0 |
| | Ethylene/VA copolymer (A-C copolymer 400, Honeywell) | 2,5 |
| | Ozokerite | 5,8 |

(fortgesetzt)

| | Komponenten INCI Bezeichnung (Handelsname, Hersteller) | Gew.-% |
|---|---|---|
| | C 16-C36 acid triglyceride | 2,0 |
| | Isobutylparaben (and) isopropylparaben (and) butylparaben (Liquipar Oil, From Nature with Love) | 0,5 |
| B | Nylon-12 (egolon 12-10, Evonik, Degussa | 2,0 |
| | Titanium dioxide | 5,0 |
| | Chromium oxide green | 10,00 |
| | CI 77491 (and) aluminium powder (and) silica | 5,0 |
| | CI 77891 (and) CI 77288 (and)mica | 10,0 |

**Wasserfeste Mascara**

| | Komponenten INCI Bezeichnung (Handelsname, Hersteller) | Gew.-% |
|---|---|---|
| A | Wasser | 32,84 |
| | Magnesium aluminium silicate (Veegum HV, RT Vanderbilt) | 0,10 |
| | Xanthan gum (Keltrol 1000, Kelco Corp.) | 0,20 |
| | Methylparaben | 0,25 |
| | Trisodium EDTA (Protacide Na3 EDTA, Protameen) | 0,01 |
| | Premixed sodium acrylates/acrylnitrogens copolymer (Hydrilien 9, 2,5% gel. Lio - siehe unten) | 15,00 |
| B | Glycerin (and) polyester 5 (Lipo PE Base G-55, Lipo) | 12,00 |
| | Talc (Rose Talc, Prespersa) | 3,00 |
| | Iron oxide (CI 77499) (Iron Oxide Black, Ultra Chemical) | 9,00 |
| C | Triethanolamine, 99% | 1,00 |
| D | Synthetic bees wax (Lipobee 102, Lipo) | 10,00 |
| | Copernicia cerifera (carnauba) wax | 4,50 |
| | Kohlenwasserstoffgemisch gemäß Herstellbeispiel 1 bis 5 | 5,50 |
| | C12-C16 alkyl benzoate (Liponate NEB, Lipo) | 1,00 |
| | Stearic acid (Lipo Stearic Acid, Lipo) | 3,00 |
| | Sorbitan sesquioleate (Liposorb SQO, Lipo) | 1,30 |
| | Propylparaben | 0,20 |
| E | Water (aqua) | 1,00 |
| | Imidazolidinyl urea (Leposerve IU, Lipo) | 0,10 |

Herstellung: Die Bestandteile der Phase A werden gemischt und auf 70 ˚C erhitzt, Phase A wird in einer Kolloidmühle gemahlen, die Bestandteile der Phase B werden in der angegebenen Reihenfolge zugefügt unter beständigem Mahlen, bis das Pigment gleichmäßig dispergiert ist, danach wird Phase C zugefügt. Die Bestandteile von C werden gemischt und bei 80-84˚C gemischt (und dabei Entlüftet). Phase D wird zum Ansatz gegeben und emulgiert, der Ansatz wird auf 35 ˚C abgekühlt und die vorgemischte Phase E wird zugefügt. Bei 30˚C wird der Ansatz in geeignete Gefäße abgefüllt.

Sodium acrylates/acrylnitrogens copolymer Premix: 97,50 Gew.- % Wasser und 2,5 Gew.- % Sodium acrylates/acrylnitrogens copolymer werden gemischt und auf 78-80˚C erhitzt unter Rühren. Es wird für 15 bis 20 Minuten geführt bis die Lösung klar und homogen ist. Danach wird auf Raumtemperatur abgekühlt.

**Sebum resistenter Lidschatten**

| | Komponenten INCI Bezeichnung (Handelsname, Hersteller) | Gew.-% |
|---|---|---|
| A | Stearyl dimethicone (DC 2503 Cosmetic Wax, Dow Corning) | 10,00 |
| | C30-C45 alkyl methicone (and) C30-C45 olefin (DC AMS-C30 Cosmetic Wax, Dow Corning) | 5,00 |
| | Isododecane (and) arcylates/polytrimethylsiloxymethacrylate (DC FA 4002 ID Silicaone Acrylate, Dow Corning) | 10,00 |
| | Cyclopentasiloxane (and) triemthylsiloxymethacrylate (DC 749 Fluid, Dow Corning) | 51,00 |
| B | Cyclopentasiloxane (and) dimethicone crosspolymere (DC 9040 Silcone Elastomer Blend, Dow Corning) | 5,00 |
| | Kohlenwasserstoffgemisch gemäß Herstellbeispiel 1 bis 5 | 3 |
| C | Silica silylate (DC VM-2270 Aerogel Fine Particles, Dow Corning) | 1,00 |
| | Mica | 15,00 |

Herstellung: Die Bestandteile der Phase A werden gemischt und auf 80 ˚C erhitzt, unter Rühren wird abgekühlt. Die Phase B wird gemischt und unter Rühren zur Phase A gegeben, C wird unter langsamem Rühren zugegeben.

**Lippenstift**

| | Inhaltsstoff | INCI Bezeichnung | Gew.-% |
|---|---|---|---|
| I | Cerilla | Candellila Wax | 9.00 |
| | Cerewax | Microcristallina Wax | 3.50 |
| | Cerozo | Ozokerite Wax | 2.50 |
| | Cerauba | Carnauba Wax | 2.50 |
| | Cetiol MM | Myristyl Mysristate | 5.00 |
| | Eutanol® G | Octyldodecanol | 10.00 |
| | Myritol® 331 | Cocoglycerides | 5.00 |
| | Cetiol® CC | Dicaprylyl Carbonate | 4.00 |
| | | Kohlenwasserstoffgemisch gemäß Herstellbeispiel 1 bis 5 | 4.00 |
| | Cetiol® J600 | Oleyl Erucate | 1.00 |
| | Nipasol M | Propyl Paraben | 0.50 |
| | Lamesoft® TGI | Polyglyceryl-3 Diisostearate | 3.00 |
| | Cetiol® Sensoft | Propylheptyl Caprylate | 6.00 |
| II | COD 8008 | CI 77981 | 2.70 |
| | COD 8001 | CI 15850 | 12.90 |
| | COD 8010 | CI 15985 | 2.80 |
| | COD 8002 | CI 15850 | 1.40 |
| | COD 8007 | CI 42090 | 0.20 |
| | Ricinus Oil Codex | Ricinus Communis | 20.60 |
| III | | Fragance Astral 112 445 B | 0.40 |

(fortgesetzt)

| | Inhaltsstoff | INCI Bezeichnung | Gew.-% |
|---|---|---|---|
| | Irwinol® LS 9319 | Octyldodecanol & Irvingia Gabonesis & Hydrogenated Coco-Glycerides | 2.50 |
| | Coviox® T70C | Tocopherol | 0.50 |

**Lippenpflege**

| | Inhaltsstoff | INCI Bezeichnung | Gew.-% |
|---|---|---|---|
| I | Polybutene M100 | Polybutene | 53.50 |
| | Cetiol® Sensoft | Propylheptyl Caprylate | 10.00 |
| | | Kohlenwasserstoffgemisch gemäß Herstellbeispiel 1 bis 5 | **5.00** |
| | Isopropyl palmitate | Isopropyl Palmitate | 20.00 |
| | Cosmedia® DC | Hydrogenated Dimer Dilinoleyl Dimethylcarbonate Copolymer | 5.00 |
| | Red 28 Lake | Red 28 Lake | 0.05 |
| | | Perfume Fruits Rouges | 0.30 |
| | Coviox® T70C | Tocopherol | 0.10 |
| | Nipasol M | Propylparaben | 0.50 |
| II | Aerosil R972 | Silica Dimethyl Silylate | 5.30 |
| III | Ronastar Noble Spars | Calcium Aluminium Borosilicate (and) Silica (and) Titanium Dioxide | 0.25 |
| Viskosität (mPa.s): Brookfield RVT, Spindle 5, Speed 10 11 000 | | | |

**O/W Flüssig Foundation**

| | Inhaltsstoff | INCI Bezeichnung | Gew.-% |
|---|---|---|---|
| I | Deionized Water | Aqua | 50.50 |
| | Butylene Glycol | Butylene Glycol | 2.00 |
| | Glycerine | Glycerin | 3.00 |
| II | Veegum Ultra | Magnesium Aluminium Silicate | 1.00 |
| | Keltrol T | Xanthan Gum | 0.40 |
| III | Lameform® TGI | Polyglyceryl-3 Diisostearate | 1.50 |
| | Eumulgin® B2 | Ceteareth-20 | 3.50 |
| | Cutina® PES | Pentaerythrityl Distearate | 1.00 |
| | Cegesoft® SH | Shorea Stenoptera | 1.00 |
| | Cetiol® CC | Dicaprylyl Carbonate | 4.00 |
| | | Kohlenwasserstoffgemisch gemäß Herstellbeispiel 1 bis 5 | **2.00** |
| | Myritol® 331 | Cocoglycerides | 4.00 |
| | Fitoderm® | Squalane | 3.00 |
| | Cetiol® Sensoft | Propylheptyl Caprylate | 5.00 |

(fortgesetzt)

| | Inhaltsstoff | INCI Bezeichnung | Gew.-% |
|---|---|---|---|
| | Phenonip | Phenoxyethanol (and) Parabens | 0.80 |
| IV | Sun Croma C47-051 | Titanium Dioxide | 6.00 |
| | SunPuro Yellow Iron Oxide C33- | Iron Oxide CI 77492 | 1.30 |
| | 9001 | | |
| | SunPuro Red Iron Oxide C33-8001 | Iron Oxide CI 77491 | 0.60 |
| | SunPuro Black Iron Oxide C33-7001 | Iron Oxide CI 77499 | 0.20 |
| V | Cosmedia® Gel CC | Dicaprylyl Carbonate (and) Stearalkonium Hectorite (and) PropyleneCarbonate | 3.00 |
| VI | Micropearl M100 | Polymethylmethacrylate | 2.00 |
| VII | Mirasil CDPDM | Cyclomethicone (and) Diphenyldimethicone | 3.00 |
| VIII | Perfume Caresse | | 0.20 |
| Viskosität (mPa.s): Brk. RVT spindle 5, speed 10 5 500, pH 7.3 | | | |

**Anhang**

1) Abil® EM 90 INCI: Cetyl Dimethicone Copolyol Hersteller: Tego Cosmetics (Goldschmidt)
2) Allianz® OPT INCI: Acrylates/C12-22 Alkyl Methacrylate Copolymer
Hersteller: Rohm und Haas 3) Amphisol® K INCI: Potassium Cetyl Phosphate Hersteller: Hoffmann La Roche
4) Antaron® V 220 INCI: PVP/Eicosene Copolymer Hersteller: GAF General Aniline Firm
Corp. (IPS-Global) 5) Antaron® V 216 INCI: PVP/ Hexadecene Copolymer
Hersteller: GAF General Aniline Firm Corp. (IPS-Global) 6) Arlacel® 83
INCI: Sorbitan Sesquioleate Hersteller: Uniqema (ICI Surfacants) 7) Arlacel® P 135
INCI: PEG-30 Dipolyhydroxystearate Hersteller: Uniqema (ICI Surfacants) 8) Bentone® 38
INCI: Quaternium-18 Hectorite Hersteller: Rheox (Elementis Specialties)
9) Carbopol® 980 INCI: Carbomer Hersteller: Goodrich
10) Carbopol® 2984 INCI: Carbomer Hersteller: Noveon, Inc.
11) Carbopol® ETD 2001 INCI: Carbomer Hersteller: Noveon, Inc.
12) Carbopol® Ultrez 10 INCI: Carbomer Hersteller: Noveon, Inc.
13) Cegesoft® C 17 INCI: Myristyl Lactate Hersteller: Cognis Deutschland GmbH, Grünau
14) Cegesoft® PFO INCI: Passiflora Incarnata (EU) Hersteller: Cognis Deutschland GmbH
15) Cegesoft® PS 6 INCI: Olus Hersteller: Cognis Deutschland GmbH
16) Ceraphyl® 45 INCI: Diethylhexyl Malate Hersteller: International Specialty Products
17) Cetiol® 868 INCI: Ethylhexyl Stearate Hersteller: Cognis Deutschland GmbH
18) Cetiol® A INCI: Hexyl Laurate Hersteller: Cognis Deutschland GmbH
19) Cetiol® B INCI: Dibutyl Adipate Hersteller: Cognis Deutschland GmbH
20) Cetiol® CC INCI: Dicaprylyl Carbonate Hersteller: Cognis Deutschland GmbH
21) Cetiol® J 600 INCI: Oleyl Erucate Hersteller: Cognis Deutschland GmbH
22) Cetiol® LC INCI: Coco-Caprylate/Caprate Hersteller: Cognis Deutschland GmbH
23) Cetiol® OE INCI: Dicaprylyl Ether Hersteller: Cognis Deutschland GmbH
24) Cetiol® PGL INCI: Hexyldecanol, Hexyldecyl Laurate Hersteller: Cognis Deutschland GmbH
25) Cetiol® S INCI: Diethylhexylcyclohexane Hersteller: Cognis Deutschland GmbH
36) Dehymuls® FCE INCI: Dicocoyl Pentaerythrityl Distearyl Citrate Hersteller: Cognis Deutschland GmbH
37) Dehymuls® HRE 7 INCI: PEG-7 Hydrogenated Castor Oil Hersteller: Cognis Deutschland GmbH

(fortgesetzt)

26) Cetiol® SB 45 INCI: Shea Butter Butyrospermum Parkii (Linne) Hersteller: Cognis Deutschland GmbH

27) Cetiol® SN INCI: Cetearyl Isononanoate Hersteller: Cognis Deutschland GmbH

28) Copherol® F 1300 C INCI: Tocopherol Hersteller: Cognis Deutschland GmbH

29) Copherol 1250 C INCI: Tocopheryl Acetate Hersteller: Cognis Deutschland GmbH

30) Cosmedia® DC INCI: Hydrogenated Dimer Dilinoleyl / Dimethylcarbonate Copolymer Hersteller: Cognis Deutschland GmbH

31) Cosmedia® SP INCI: Sodium Polyacrylate Hersteller: Cognis Deutschland GmbH

32) Cutina® E 24 INCI: PEG-20 Glyceryl Stearate Hersteller: Cognis Deutschland GmbH

33) Cutina® HR INCI: Hydrogenated Castor Oil Hersteller: Cognis Deutschland GmbH

34) Cutina® MD INCI: Glyceryl Stearate Hersteller: Cognis Deutschland GmbH

35) Cuitina® PES INCI: Pentaerythrityl Distearate Hersteller: Cognis Deutschland GmbH

Glycerin and Ceteareth-12 and Cetyl Palmitate Hersteller: Cognis Deutschland GmbH 47) Emulgade®PL 68/50 INCI: Cetearyl Glucoside, Cetearyl Alcohol Hersteller: Cognis Deutschland GmbH

48) Emulgade® SE - PF INCI: Glyceryl Stearate (and) Ceteareth-20 (and) Ceteareth-12 (and) Cetearyl Alcohol (and) Cetyl Palmitate Hersteller: Cognis Deutschland GmbH

49) Emulgade® SUCRO INCI: Sucrose Polystearate (and) Hydrogenated Polyisobutene Hersteller: Cognis Deutschland GmbH

50) Eumulgin® B 1 INCI: Ceteareth-12 Hersteller: Cognis Deutschland G mbH

51) Eumulgin® B 2 INCI: Ceteareth- 20 Hersteller: Cognis Deutschland GmbH

52) Eumulgin® HRE 40 INCI: PEG-40 Hydrogenated Castor Oil Hersteller: Cognis Deutschland GmbH

53) Eumulgin® SG INCI: Sodium Stearoyl Glutamate Hersteller: Cognis Deutschland GmbH

54) Eumulgin® VL 75 INCI: Lauryl Glucoside (and) Polyglyceryl-2 Dipolyhydroxystearate (and) Glycerin Hersteller: Cognis Deutschland GmbH

55) Eusolex® OCR INCI: Octocrylene Hersteller: Merck

56) Eusolex® T 2000 INCI: Titanium Dioxide, Alumina, Simethicone

Hersteller: Cognis Deutschland GmbH

38) Dehymuls® PGPH INCI: Polyglyceryl-2 Dipolyhydroxystearate Hersteller: Cognis Deutschland GmbH

39) Dow Corning® 244 Fluid INCI: Cyclomethicone Hersteller: Dow Corning

40) Dow Corning® 246 Fluid INCI: Cyclopentasiloxane Hersteller: Dow Corning

41) Dow Corning® 2502 INCI: Cetyl Dimethicone Hersteller: Dow Corning

42) Dry®Flo Plus INCI: Aluminium Starch Octenylsuccinate Hersteller: National Starch

43) Elfacos®ST 37 INCI: PEG-22 Dodecyl Glycol Copolymer Hersteller: Akzo-Nobel

44) Elfacos®ST 9 INCI: PEG-45 Dodecyl Glycol Copolymer Hersteller: Akzo-Nobel

45) Emery® 1780 INCI: Lanolin Alcohol Hersteller: Cognis Corporation (Emery) INCI: Lanolin Alcohol Hersteller: Cognis Corporation

46) Emulgade® CM INCI: Cetearyl Isononanoate and Ceteareth-20 and Cetearyl

Hersteller: Merck

57) Eusolex® T AQUA INCI: Water and Titanium Dioxide and Alumina and Sodium Metaphosphate and Phenoxyethanol and Sodium Methylparaben Hersteller: Merck

58) Eutanol® G INCI: Octyldodecanol Hersteller: Cognis Deutschland GmbH

59) Eutanol®G 16 INCI: Hexyldecanol Hersteller: Cognis Deutschland GmbH Eutanol®G

60) 16 S INCI: Hexyldecyl Stearate Hersteller: Cognis Deutschland GmbH

61) Finsolv® TN INCI: C 12/15 Alkyl Benzoate Hersteller: Findex (Nordmann/Rassmann)

62) Generol® R INCI: Brassica Campestris (Rapseed) Sterols Hersteller: Cognis Deutschland GmbH

63) Glucate® DO INCI: Methyl Glucose Dioleate Hersteller: NRC Nordmann/Rassmann

64) Hispagel® 200 INCI: Glycerin, Glyceryl Polyacrylate Hersteller: Cognis Deutschland GmbH

65) Hostaphat® KL 340 N INCI: Trilaureth-4 Phosphate Hersteller: Clariant

66) Hydagen® C.A.T. INCI Triethyl Citrate Hersteller: Cognis Deutschland GmbH

67) Hydagen® DCMF INCI : Chitosan Hersteller: Cognis Deutschland GmbH

(fortgesetzt)

68) Insect Repellent® 3535 INCI : Ethyl Butylacetylaminopropionate Hersteller : EMD Chemicals Inc

69) Isolan® PDI INCI: Diisostearyl Polyglyceryl-3 Diisostearate

Hersteller: Goldschmidt AG 70) Keltrol® T INCI: Xanthan Gum

Hersteller: CP Kelco 71) Lameform® TGI INCI: Polyglyceryl-3 Diisostearate Hersteller: Cognis Deutschland GmbH

72) Lanette® 14 INCI: Myristyl Alcohol Hersteller: Deutschland GmbH

Cognis 73) Lanette 18 INCI: Stearyl Alcohol Hersteller: Cognis Deutschland GmbH

74) Lanette® 22 INCI: Behenyl Alcohol Hersteller: Cognis Deutschland GmbH

75) Lanette® E INCI: Sodium Cetearyl Sulfate Hersteller: Cognis Deutschland GmbH

76) Lanette® O INCI: Cetearyl Alcohol Hersteller: Cognis Deutschland GmbH

77) Locron® L INCI: Aluminium Chlorhydrate Hersteller: Clariant

78) Lucentite® SAN INCI: Quaternium-18 Hectoritr Hersteller: Co-Op Chemical Co., Ltd. Monomuls®

79) 90-O 18 INCI: Glyceryl Oleate

INCI: Ethylhexyl Salicylate Hersteller: Symrise

92) Novata® AB INCI: Cocoglycerides Hersteller: Cognis Deutschland GmbH

93) Parsol® 1789 INCI: Butyl Methoxydibenzoylmethane Hersteller: Hoffmann-La Roche (Givaudan)

94) Pemulen® TR-2 Polymer INCI: Acrylates / C10-30 Alkylacrylate Crosspolymer Hersteller: Noveon, Inc.

95) Photonyl® LS INCI: Arginine, Disodium Adenosine Triphosphate, Mannitol, Pyridoxine HCL, Phenylalanine, Tyrosine Hersteller: Laboratoires Serobiologiques (Cognis)

96) Prisorine® 3505 INCI: Isostearic Acid Hersteller: Uniqema

97) Prisorine® 3758 INCI: Hydrogenated Polyisobutene Hersteller: Uniqema

98) Rezal 36G INCI: Aluminum Zirconium Tetrachlorohydrex GLY Hersteller: Reheis, Inc

99) SFE® 839 INCI: Cyclopentasiloxane and Dimethicone/Vinyl Dimethicone Crosspolymer Hersteller: GE Silicones

100) Silikonöl Wacker AK® 350 INCI: Dimethicone Hersteller: Wacker

101) Tego® Care 450

Hersteller: R. T. Vanderbilt Company, Inc

80) Myrj® 51 INCI: PEG-30-Sterate Hersteller: Uniqema

81) Myritol® 312 INCI: Caprylic/Capric Triglyceride Hersteller: Cognis Deutschland GmbH

82) Myritol® 331 INCI: Cocoglycerides Hersteller: Cognis Deutschland GmbH

83) Myritol® PC INCI: Propylene Glycol Dicaprylate/ Dicaprate Hersteller: Cognis Deutschland GmbH

84) Neo Heliopan® 303 INCI: Octocrylene Hersteller: Symrise

85) Neo Heliopan® AP INCI: Disodium Phenyl Dibenzimidazole Tetrasulfonate Hersteller: Symrise

86) Neo Heliopa® AV INCI: Ethylhexyl Methoxycinnamate Hersteller: Symrise

87) Neo Heliopan® BB INCI: Benzophenone-3 Hersteller: Symrise

88) Neo Heliopan® E 1000 INCI: Isoamyl-p-Methoxycinnamate Hersteller: Symrise

89) Neo Heliopan® Hydro INCI: Phenylbenzimidazole Sulfonic Acid Hersteller: Symrise

90) Neo Heliopan® MBC INCI: 4-Methylbenzylidene Camphor Hersteller: Symrise

91) Neo Heliopan® OS

INCI: Polyglyceryl-3 Methylglucose Distearate Hersteller: Tego Cosmetics

(Goldschmidt) 102) Tego® Care CG 90 INCI: Cetearyl Glucoside

Hersteller: Goldschmidt 103) Tegosoft® DEC INCI: Diethylhexyl Carbonate Hersteller: Goldschmidt

104) Tinosorb® S INCI: Bis-Ethylhexyloxyphenol Methoxyphenyl Triazine Hersteller: Ciba Specialty Chemicals Corporation

105) Tinosorb® M INCI: Methylene Bis-Benzotriazolyl Tetramethylbutylphenol Herstelller: Ciba Specialty Chemicals Corporation

106) Tween® 60 INCI: Polysorbate 60 Hersteller: Uniqema (ICI Surfactants)

107) Uvasorb® HEB INCI: Diethylhexyl Butamido Triazone Hersteller: 3V Inc.

108) Unirep® U-18 INCI: Dimethyl Phthalate and Diethyl Toluamide and Ethyl Hexanediol Hersteller: Induchem AG

109) Uvinul® T 150 INCI: Ethylhexyl Triazone Hersteller: BASF

110) Uvinul® A plus INCI: Diethylamino Hydroxybenzoyl Hexyl Benzoate Hersteller: BASF

111) Veegum® Ultra INCI: Magnesium Aluminium Silicate

(fortgesetzt)

112) Veegum® Plus INCI: Magnesium Aluminum Silicate and Cellulose Gum Hersteller: R. T. Vanderbilt Company, Inc

113) Z-Cote® HP 1 INCI: Zinc Oxide and Triethoxycaprylylsilane Hersteller: BASF

114) Zinc Oxide NDM INCI: Zinc Oxide Hersteler: Symrise

## Patentansprüche

1. Kohlenwasserstoff Gemisch, das $^{14}C$ Isotope enthält und wobei das Kohlenwasserstoff Gemisch mindestens 2 voneinander verschiedene Kohlenwasserstoffe enthält, deren Kohlenstoff Zahl sich um mehr als 1 unterscheidet.

2. Kohlenwasserstoff Gemisch nach Anspruch 1, wobei das Kohlenwasserstoff Gemisch mindestens 2 voneinander verschiedene Kohlenwasserstoffe enthält, deren Kohlenstoff Zahl sich um 2 unterscheidet.

3. Kohlenwasserstoff Gemisch nach einem der vorgenannten Ansprüche, **dadurch gekennzeichnet, dass** der Anteil an $^{14}C$ Isotopen zu $^{12}C$ Isotopen im Bereich von $6 \times 10^{-13}$ bis $1{,}2 \times 10^{-12}$ liegt.

4. Kohlenwasserstoff Gemisch nach einem der vorgenannten Ansprüche, **dadurch gekennzeichnet, dass** das Kohlenwasserstoff Gemisch Kohlenwasserstoffe enthält, die ausgewählt sind aus der Gruppe der Kohlenwasserstoffen mit 7 bis 23 Kohlenstoffatomen, vorzugsweise 11 bis 21 Kohlenstoffatome.

5. Kohlenwasserstoff Gemisch nach einem der vorgenannten Ansprüche, worin 2 voneinander verschiedene Kohlenwasserstoffe mindestens 50 Gew.-%, vorzugsweise mindestens 60 Gew.-% - bezogen auf die Summe der Kohlenwasserstoffe ausmachen.

6. Kohlenwasserstoff Gemisch nach einem der vorgenannten Ansprüche, enthaltend lineare C11 und lineare C13 Kohlenwasserstoffe.

7. Kohlenwasserstoff Gemisch nach einem der vorgenannten Ansprüche, enthaltend lineare C 17 und lineare C 19 Kohlenwasserstoffe, vorzugsweise zusätzlich mindestens einen linearen C21 Kohlenwasserstoff.

8. Verwendung eines Kohlenwasserstoff Gemischs nach einem der vorgenannten Ansprüche in kosmetischen und/oder pharmazeutischen Zubereitungen, insbesondere als Ölkörper und/oder als Dispergiermittel.

9. Kosmetische und/oder pharmazeutische Zubereitungen, enthaltend 0,1 bis 80 Gew.% eines Kohlenwasserstoff Gemischs, das $^{14}C$ Isotope enthält.

10. Kosmetische und/oder pharmazeutische Zubereitung nach Anspruch 9, **dadurch gekennzeichnet, dass** der Anteil an $^{14}C$ Isotopen zu $^{12}C$ Isotopen im Kohlenwasserstoff Gemisch im Bereich von $6 \times 10^{-13}$ bis $1{,}2 \times 10^{-12}$ liegt.

11. Kosmetische und/oder pharmazeutische Zubereitung nach Anspruch 9 und/oder 10, enthaltend mindestens einen Antiperspirant /Desodorant Wirkstoff.

12. Kosmetische und/oder pharmazeutische Zubereitung nach einem der Ansprüche 9 bis 11, enthaltend mindestens einen UV-Lichtschutzfilter.

13. Kosmetische und/oder pharmazeutische Zubereitungen nach einem der Ansprüche 9 bis 12, enthaltend mindestens einen Selbstbräuner.

14. Kosmetische und/oder pharmazeutische Zubereitungen nach einem der Ansprüche 9 bis 13, enthaltend mindestens ein Pigment und/oder Farbstoff.

15. Kosmetische und/oder pharmazeutische Zubereitungen nach einem der Ansprüche 9 bis 14, enthaltend mindestens

einen Emulgator und/oder ein Tensid und/oder eine Wachskomponente und/oder ein Polymer und/oder einen weiteren Ölkörper.

Europäisches Patentamt
European Patent Office
Office européen des brevets

# EUROPÄISCHER RECHERCHENBERICHT

Nummer der Anmeldung
EP 09 00 4222

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X,P | EP 2 014 274 A1 (COGNIS IP MAN GMBH [DE]) 14. Januar 2009 (2009-01-14)<br>* Absätze [0079] - [0107]; Ansprüche 1-11 * | 1-6,8-15 | INV.<br>A61K8/31<br>A61K8/92<br>A61K8/02<br>A61Q1/02<br>A61Q1/04<br>A61Q1/06<br>A61Q1/10<br>A61Q5/00<br>A61Q5/06<br>A61Q5/12<br>A61Q7/00<br>A61Q15/00<br>A61Q17/04<br>A61Q19/00<br>A61Q19/08<br>A61Q19/10<br>C07C1/22 |
| E | WO 2009/080966 A2 (OREAL [FR]; DOP FLORENCE [FR]) 2. Juli 2009 (2009-07-02)<br>* Beispiele 2,3,5 * | 1-6, 8-10, 14-15 | |
| X,P | WO 2008/128232 A1 (SYNTROLEUM CORP [US]; ABHARI RAMIN [US])<br>23. Oktober 2008 (2008-10-23)<br>* Absatz [0108]; Anspruch 1; Beispiele 1-5 * | 1-4,8 | |
| X,D | WO 2007/068371 A1 (COGNIS IP MAN GMBH [DE]; FALKOWSKI JUERGEN [DE]; DIERKER MARKUS [DE];) 21. Juni 2007 (2007-06-21)<br>* das ganze Dokument * | 1-15 | **RECHERCHIERTE SACHGEBIETE (IPC)** |
| X | WO 2004/011581 A1 (CRODA INT PLC [GB]; STEEL IAN [GB])<br>5. Februar 2004 (2004-02-05)<br>* Anspruch 30; Beispiele 1-5 * | 1-4, 8-10,15 | A61K<br>A61Q<br>C07C |
| X | US 2007/135316 A1 (KOIVUSALMI EIJA [FI] ET AL) 14. Juni 2007 (2007-06-14)<br>* Absatz [0103]; Anspruch 1 * | 1-4 | |
| X | WO 2007/068800 A2 (NESTE OIL OYJ [FI]; KOIVUSALMI EIJA [FI]; MYLLYOJA JUKKA [FI]; MATIKAI) 21. Juni 2007 (2007-06-21)<br>* Beispiele 1-8; Tabelle 2 * | 1-3 | |

Der vorliegende Recherchenbericht wurde für alle Patentansprüche erstellt

| Recherchenort | Abschlußdatum der Recherche | Prüfer |
|---|---|---|
| München | 26. August 2009 | Werner, Stefan |

2

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument
& : Mitglied der gleichen Patentfamilie, übereinstimmendes Dokument

EPO FORM 1503 03.82 (P04C03)

Europäisches Patentamt
European Patent Office
Office européen des brevets

# EUROPÄISCHER RECHERCHENBERICHT

Nummer der Anmeldung
EP 09 00 4222

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| | | | C07C9/15 |
| | | | RECHERCHIERTE SACHGEBIETE (IPC) |
| | | | |

Der vorliegende Recherchenbericht wurde für alle Patentansprüche erstellt

2

| Recherchenort | Abschlußdatum der Recherche | Prüfer |
|---|---|---|
| München | 26. August 2009 | Werner, Stefan |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument
..........................................................................................................
& : Mitglied der gleichen Patentfamilie, übereinstimmendes Dokument

EPO FORM 1503 03.82 (P04C03)

**ANHANG ZUM EUROPÄISCHEN RECHERCHENBERICHT ÜBER DIE EUROPÄISCHE PATENTANMELDUNG NR.** EP 09 00 4222

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

26-08-2009

| Im Recherchenbericht angeführtes Patentdokument | Datum der Veröffentlichung | Mitglied(er) der Patentfamilie | Datum der Veröffentlichung |
|---|---|---|---|
| EP 2014274 A1 | 14-01-2009 | DE 102008017031 A1 | 24-12-2008 |
| | | DE 102008022434 A1 | 24-12-2008 |
| WO 2009080966 A2 | 02-07-2009 | WO 2009071662 A2 | 11-06-2009 |
| | | WO 2009080951 A2 | 02-07-2009 |
| | | WO 2009080952 A2 | 02-07-2009 |
| | | WO 2009080953 A2 | 02-07-2009 |
| | | WO 2009080954 A2 | 02-07-2009 |
| | | WO 2009080955 A2 | 02-07-2009 |
| | | WO 2009077709 A2 | 25-06-2009 |
| | | WO 2009080956 A2 | 02-07-2009 |
| | | WO 2009080957 A2 | 02-07-2009 |
| | | WO 2009080958 A2 | 02-07-2009 |
| | | WO 2009080960 A2 | 02-07-2009 |
| | | WO 2009080961 A2 | 02-07-2009 |
| | | WO 2009080962 A2 | 02-07-2009 |
| | | WO 2009080963 A2 | 02-07-2009 |
| | | WO 2009080964 A2 | 02-07-2009 |
| | | WO 2009080965 A2 | 02-07-2009 |
| | | WO 2009080967 A2 | 02-07-2009 |
| WO 2008128232 A1 | 23-10-2008 | US 2008253936 A1 | 16-10-2008 |
| WO 2007068371 A1 | 21-06-2007 | CN 101360699 A | 04-02-2009 |
| | | EP 1798213 A1 | 20-06-2007 |
| | | EP 1960326 A1 | 27-08-2008 |
| | | JP 2009519264 T | 14-05-2009 |
| | | US 2008269352 A1 | 30-10-2008 |
| | | ZA 200803235 A | 28-01-2009 |
| WO 2004011581 A1 | 05-02-2004 | AU 2003251362 A1 | 16-02-2004 |
| | | GB 2391808 A | 18-02-2004 |
| US 2007135316 A1 | 14-06-2007 | KEINE | |
| WO 2007068800 A2 | 21-06-2007 | CA 2631848 A1 | 21-06-2007 |
| | | EP 1960497 A2 | 27-08-2008 |
| | | KR 20080079674 A | 01-09-2008 |

EPO FORM P0461

Für nähere Einzelheiten zu diesem Anhang : siehe Amtsblatt des Europäischen Patentamts, Nr.12/82

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- EP 2006011647 W, Cognis
- EP 0693471 B1
- EP 0818450 A1
- EP 0694521 B1
- DE 19712033 A1
- EP 1371359 A2
- EP 766661 B1
- WO 07048757 A


### In der Beschreibung aufgeführte Nicht-Patentliteratur


- **A. Barel ; M. Paye ; H. Maibach.** Handbook of Cosmetic Science and Technology. Marcel Dekker Inc, 2001
- PIT Emulsionen beispielsweise. *Parfümerie und Kosmetik,* vol. 77 (4/96), 250-254
- **P.Finkel.** *SÖFW-Journal,* 1996, vol. 122 (8), 543-548
- *Parf.Kosm.,* 1999, vol. 80 (3), 10-16
- **Kirk-Othmer.** Encyclopedia of Chemical Technology. 1979, vol. 8, 913